(19) Europäisches Patentamt
European Patent Office
Office européen des brevets

(11) Numéro de publication: **0 127 543 B1**

(12) # FASCICULE DE BREVET EUROPÉEN

(45) Date de publication du fascicule du brevet: **13.09.89**

(51) Int. Cl.⁴: **C 07 D 501/34**, C 07 D 501/36, A 61 K 31/545

(21) Numéro de dépôt: **84401080.1**

(22) Date de dépôt: **25.05.84**

(54) **Nouveaux composés antibiotiques dérivés des céphalosporines, procédé d'obtention et compositions pharmaceutiques les contenant.**

(30) Priorité: **27.05.83 FR 8308862**

(43) Date de publication de la demande: **05.12.84 Bulletin 84/49**

(45) Mention de la délivrance du brevet: **13.09.89 Bulletin 89/37**

(84) Etats contractants désignés: **AT BE CH DE FR GB IT LI LU NL SE**

(56) Documents cité:
**EP-A-0 060 745**
**FR-A-2 501 209**

**Le dossier contient des informations techniques présentées postérieurement au dépôt de la demande et ne figurant pas dans le présent fascicule.**

(73) Titulaire: **SANOFI, 40, Avenue George V, F-75008 Paris (FR)**

(72) Inventeur: **Olliero, Dominique Bat. Lorraine, Res. des Facultés Avenue de la Justice, F-34100 Montpellier (FR)**
Inventeur: **Salhi, Ali, 639, rue de Valène, F-34980 Saint- Gely- du- Fesc (FR)**

(74) Mandataire: **Gillard, Marie- Louise, Cabinet Beau de Loménie 55, Rue d'Amsterdam, F-75008 Paris (FR)**

EP 0 127 543 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

## Description

La présente invention concerne des dérivés de la famille des céphalosporines, leur procédé de préparation et leur application en thérapeutique.

La Demanderesse a déjà décrit des dérivés de la famille des céphalosporines dans les demandes FR-2 501 209 et EP-60 745.

Les nouveaux dérivés, selon la présente invention, ont des spectres d'activité antibactérienne différents.

Les composés selon l'invention répondent à la formule:

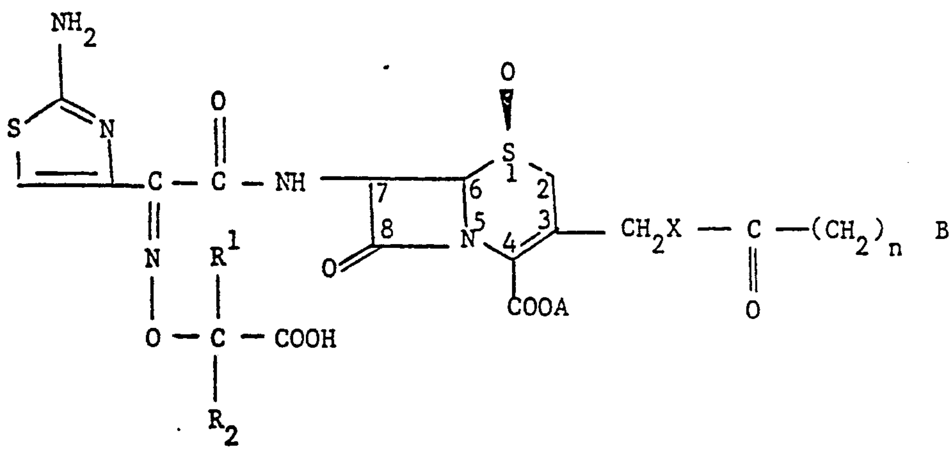

(I)

dans laquelle:

. le groupe COOA en position 4 est un radical acide, ou un sel alcalin ou alcalino-terreux, ou un sel d'aminoacide ou d'amine, par exemple la triéthylamine ou les éthanolamines, ou un radical ester facilement hydrolysable ou métaboliquement labile et pharmaceutiquement acceptable.

. X désigne un atome d'oxygène ou un atome de soufre.

. n est zéro ou 1.

. $R_1$ et $R_2$ désignent chacun indépendamment l'hydrogène ou un groupe méthyle, ou

. $R_1$ et $R_2$ pris ensemble avec l'atome de carbone auquel ils sont liés forment un noyau cyclobutyle ou cyclopentyle.

. B est le reste d'une amine primaire choisi parmi les groupements suivants:

- Z-NH-R où Z est un groupe alkylène à chaîne droite ou ramifiée ayant de 2 à 7 atomes de carbone, éventuellement interrompue par un atome de soufre et éventuellement substituée par un groupe hydroxyle, thiol, méthylthio, amino, acétamido, carbamoyle, phényle, hydroxyphényle ou imidazolyle.

Z peut aussi être un groupe cyclopentylidène ou cyclohexylidène.

R représente un atome d'hydrogène ou un groupe alkyle ayant de 1 à 3 atomes de carbone;

- Z'-Alk-NH-R où Z' représente un groupe 1,2-phénylène ou 1,3-phénylène ou 1,4-phénylène éventuellement substitué par un atome d'halogène ou par 1 ou 2 groupes méthoxy ou par 1, 2 ou 3 groupes méthyle ou bien Z' représente un groupe 1,2-cyclohexylène, 1,3-cyclohexylène ou 1,4-cyclohexylène.

Alk représente un groupe alkylène droit ou ramifié ayant de 1 à 3 atomes de carbone.

R est tel que défini ci-dessus;

- Z'-N-CO-Y-NH-R''
     |
     R'

où Z' est tel que défini ci-dessus.

- Y désigne un groupe alkylène $(CH_2)_m$ dans lequel m = 0, 1, 2, 3 ou 4, un groupe alkylène ramifié ayant 2 ou 3 atomes de carbone

ou encore Y avec NH-R'' constitue un cycle 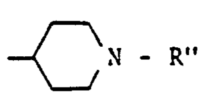

R' et R'', identiques ou différents, ont la même signification que celle donnée pour R ci-dessus.

- Z''-NH-R où Z'' est un groupe 1,3-cyclohexylène ou 1,4-cyclohexylène et R est tel que défini précédemment;

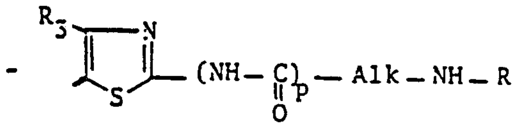

où $R_3$ représente un atome d'hydrogène ou un groupe méthyle.
p = 0 ou 1 et Alk et R sont tels que définis précédemment;
- un groupe 2-pipéridyle, 3-pipéridyle ou 4-pipéridyle éventuellement substitué sur l'atome d'azote par un groupe -CO-Alk-$NH_2$ ou

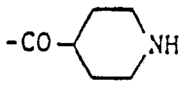

où Alk est tel que défini précédemment.

Les sels que les composés de formule (I) sont susceptibles de donner avec les acides pharmaceutiquement acceptables font partie intégrante de l'invention.

Par suite de la présence dans la formule d'un groupement oxime, les composés (I) existent sous 2 formes isomères syn et anti. Les isomères syn dont l'activité thérapeutique est supérieure sont les composés préférés.

Il est entendu que les composés (I) indiqués ci-dessus peuvent exister:
- soit sous la forme indiquée dans la formule (I),
- soit sous la forme tautomère (I'):

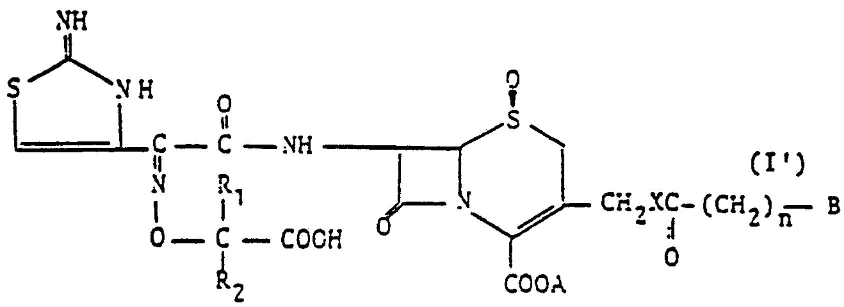

(I')

dans laquelle A, X, $R_1$, $R_2$, B et n ont les significations indiquées précédemment.

L'invention concerne également un procédé de préparation des composés de formule (I).

L'invention a également pour objet des trifluoroacétates de l'acide de formule:

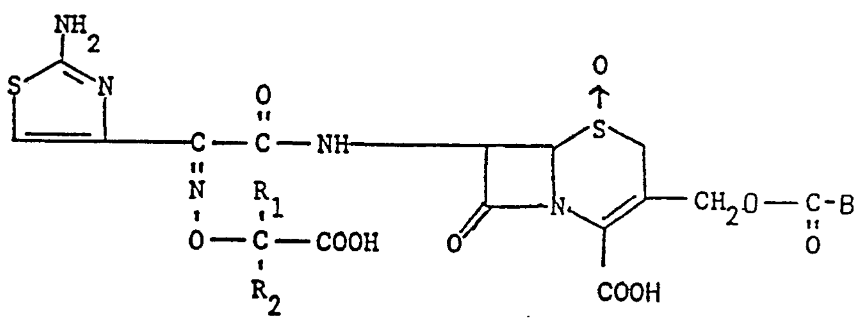

dans laquelle:
- lorsque $R_1$ et $R_2$ représentent chacun un groupe méthyle, B est le reste d'une amine de formule:

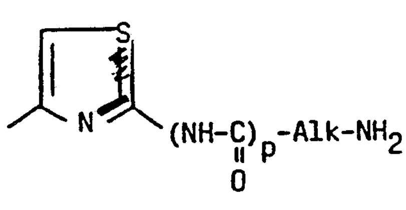

dans laquelle le groupe

$(NH-C(=O))_p-Alk-NH_2$

est choisi parmi les groupes:

$NH-C(=O)-CH_2-NH_2$ ; $NH-C(=O)-CH_2-CH_2-NH_2$ ; $NH-C(=O)-CH(CH_3)-NH_2$ ; $-CH_2-CH_2-NH_2$ ; $-CH_2-NH_2$

ou B est le reste d'une amine de formule:

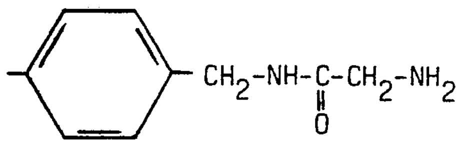

- lorsque $R_1$ et $R_2$ représentent avec l'atome de carbone auquel ils sont liés un groupe cyclobutyle, B est le reste d'une amine de formule:

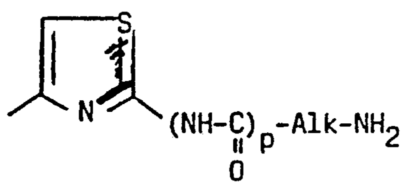

dans laquelle le groupe

$(NH-C(=O))_p-Alk-NH_2$

est choisi parmi les groupes

$-NH-C(=O)-CH_2-NH_2$ et $CH_2-NH_2$ ;

isomère syn;
et de l'acide de formule:

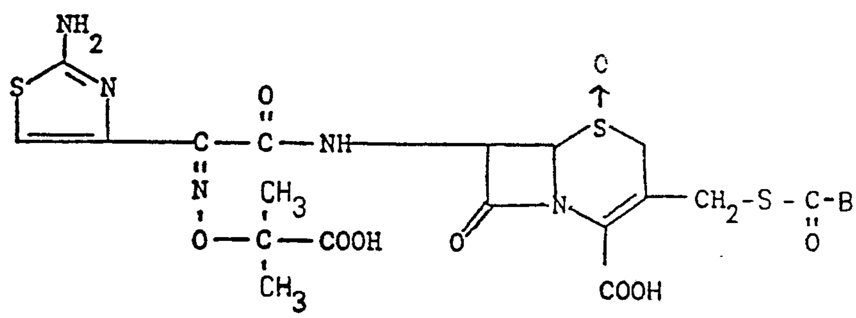

dans laquelle B le reste d'une amine de formule:

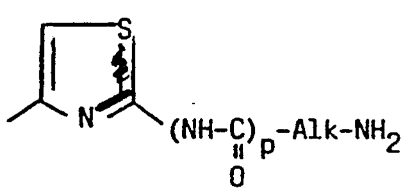

dans laquelle le groupe

est choisi parmi les groupes: 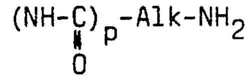

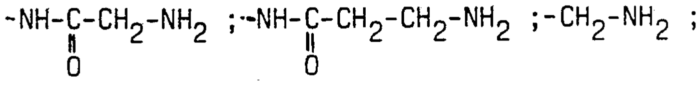

ou

B est le reste d'une amine de formule:

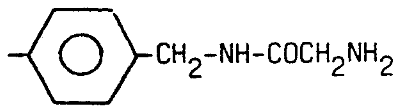

isomère syn.

Le procédé de préparation desdits dérivés est le même que celui, explicité ci-après pour les composés de formule (I). Il est plus précisément décrit dans les exemples 1 à 3.

Selon le schéma réactionnel ci-après, ce procédé consiste à acyler tout d'abord l'amino-7 bromométhyl-3 céphème-3 carboxylate de tertiobutyle-4 S-oxyde (II) par l'acide (III) pour obtenir le composé (IV) décrit dans la demande de brevet européen numéro 60 745. Au composé IV, on ajoute ensuite l'acide B - $(CH_2)_n$ - COOH ou le thioacide $B(CH_2)_nCOSH$ dont la fonction amine doit être préalablement protégée, selon une méthode connue, par un groupement protecteur comme le tertiobutoxycarbonyle ou le trichloréthoxycarbonyle par exemple; B' représente alors le groupe B dans lequel la fonction amine est protégée.

L'addition du sel de sodium ou de potassium de l'acide B' - $(CH_2)_nCOOH$ au composé (IV) se fait de préférence dans un solvant polaire aprotique, par exemple le diméthylformamide, tandis que l'addition du sel de sodium ou de potassium du thioacide B' - $(CH_2)_nCOSH$ peut se faire dans un solvant apolaire comme le tétrahydrofurane. On obtient le composé (V).

Dans le cas des thioacides, pour préparer les composés (V), on peut utiliser le thioacide lui-même au lieu d'un sel alcalin. On opère alors dans l'acétone anhydre en présence d'hydrogéno carbonate de potassium et d'iodure de sodium.

Enfin, pour aboutir au composé (I), les groupes protecteurs sur les amines et les esters tertiobutyliques sont éliminés par un procédé connu, en particulier par hydrolyse en milieu acide en utilisant, par exemple, l'acide trifluoracétique ou un mélange d'acide formique et d'acide chlorhydrique.

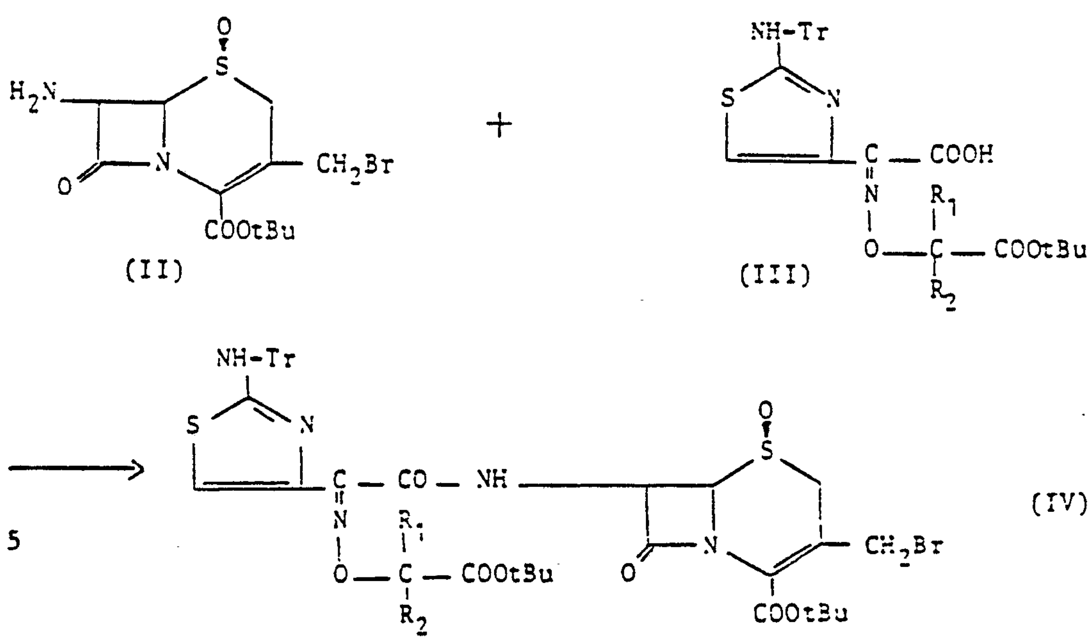

(II) + (III) → (IV)

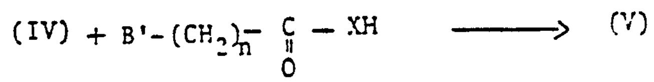

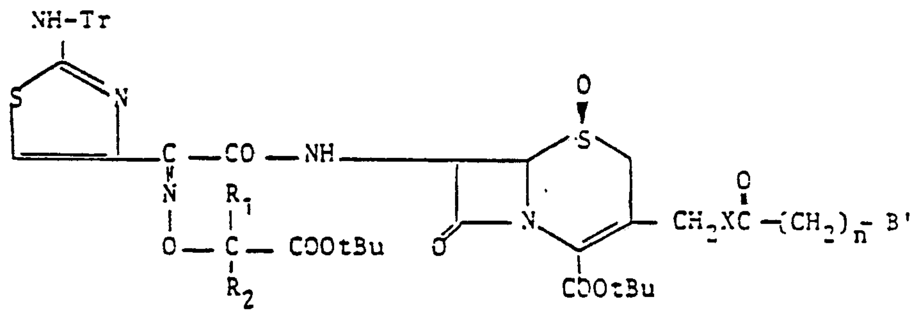

(V )

$$(V) + H^+ \longrightarrow (I)$$

Tr représente le trityle, tBu le tertiobutyle, X, $R_1$, $R_2$, n, B' et B ont les significations précédemment indiquées.

Les composés (I) de l'invention dans lesquels A est autre que H s'obtiennent à partir des composés (I) dans lesquels A est H par des réactions connues en elles-mêmes.

Ainsi les sels minéraux sont obtenus par action sur les composés (I), dans lesquels A est H, d'une base minérale, telle que la soude ou la potasse ou le bicarbonate de sodium en quantité équimoléculaire; la réaction de salification est réalisée dans un solvant, tel que l'eau ou l'éthanol et le sel obtenu est isolé par évaporation de la solution.

Les sels de bases organiques sont obtenus par action, sur une solution de l'acide I (A = H), dans un solvant ou un mélange de solvants convenables, d'une quantité équimoléculaire de la base organique. Le sel est isolé par précipitation avec l'éther.

Les esters sont obtenus par les procédés connus d'estérification; par exemple on utilisera avantageusement l'action d'un dérivé halogéné sur un sel, tel que le sel de sodium de l'acide; on réalisera de préférence la réaction dans un solvant capable de dissoudre le dérivé acide de départ, par exemple dans le diméthylformamide.

Les isomères de forme syn et anti s'obtiennent par un choix convenable des réactifs.

Les exemples suivants permettent de mieux comprendre la portée de l'invention, sans toutefois la limiter.

Ainsi qu'il est habituel dans cette famille de composés, les produits selon l'invention ne présentent pas de point de fusion net mais seulement des points de décomposition ne permettant pas de les caractériser.

Les produits seront donc caractérisés par leur spectre de résonance magnétique nucléaire enregistré à 60 MHz ou à 250 MHz, l'étalon interne étant l'hexaméthyldisiloxane. Les spectres sont enregistrés dans le diméthylsulfoxyde deutérié sauf exceptions signalées dans la description du spectre: 50 mg de produit dans 0,5 ml de solvant à 60 MHz et 10 mg dans 0,5 ml à 250 MHz. Les déplacements chimiques sont mesurés à ± 0,01 ppm et les constantes de couplage à ± 0,5 Hz.

Les abréviations suivantes seront utilisées:
- S: singulet
- D: doublet
- D de D: doublet de doublet
- S. e.: singulet élargi
- M: multiplet
- Q: quadruplet
- AB: système AB
- J: représente la constante de couplage.

De plus les microanalyses élémentaires ont été effectuées dans chaque cas et sont en accord avec les formules indiquées.

Les spectres infra-rouge servent également à caractériser les produits obtenus. Ils sont enregistrés entre 4000 $cm^{-1}$ et 600 $cm^{-1}$ à partir d'une préparation constituée d'une pastille de bromure de potassium contenant le produit à une concentration d'environ 2 %; lorsque le spectre est enregistré en solution à 1 % dans un solvant chloré, la nature de celui-ci est mentionnée. On repère la fréquence de vibration d'élongation des groupements carbonyles de la molécule (ν CO).

**Exemple 1**

**Acide [(Amino-2 thiazolyl-4)-2 (carboxy-2 propyl-2 oxyimino)-2 acetamido]-7 (piperidinyl-4) carbonyl oxyméthyl-3 céphème-3 carboxylique-4 S-oxyde-1β isomère syn trifluoroacétate. SR 41 862**

a) [(Tritylamino-2 thiazolyl-4)-2 (tertiobutoxycarbonyl-2 propyl-2 oxyimino)-2 acétamido]-7 (tertiobutoxycarbonyl-1 piperidinyl-4) carbonyl oxyméthyl-3 céphème-3 carboxylate de tertiobutyle-4 S-oxyde-1β isomère syn

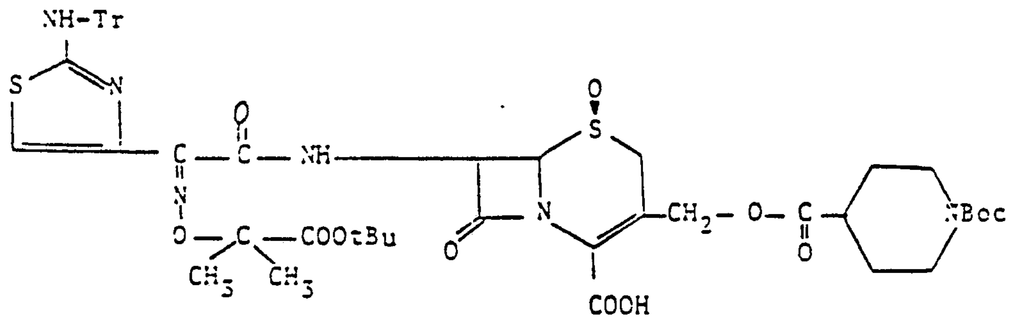

tBu et Tr ont les significations précédemment indiquées, Boc désigne le groupement tertiobutoxycarbonyle.

A une solution de 1,38 g de [(tritylamino-2 thiazolyl-4)-2 (tertiobutoxycarbonyl-2 propyl-2 oxyimino)-2 acétamido]-7 bromométhyl-3 céphème-3 carboxylate de tertiobutyle-4 S-oxyde-1β isomère syn, dans 20 ml de diméthylformamide anhydre, on ajoute 1 g d'acide tertiobutyloxycarbonyl-1 piperidinyl-4 carboxylique et 1,5 g d'hydrogénocarbonate de potassium.

Après 17 heures d'agitation à température ambiante (20 - 25°C) le mélange réactionnel est versé sur 200 ml d'eau glacée. Après une agitation vigoureuse, les cristaux sont filtrés et lavés à l'eau. Ils sont ensuite repris par 70 ml de dichlorométhane. La phase organique est alors lavée par une solution saturée de chlorure de sodium, séchée sur sulfate de magnésium et évaporée. La laque obtenue est chromatographiée sur une colonne de 50 g de silice. On élue par un mélange dichlorométhane-acétate d'éthyle 90 - 10 (vol/vol). Après évaporation des fractions contenant le composé et trituration dans l'hexane, on obtient 1,3 g du composé attendu.

**Spectre IR:** ν **CO**

. 1805 $cm^{-1}$: C = 0 en 8 du β lactame
. 1735 $cm^{-1}$: C = 0 des esters tertiobutyliques et de l'ester en 3
. 1695 $cm^{-1}$: C = 0 de l'amide en 7, et C = 0 du tertiobutoxycarbonyle protecteur de la pipéridine.

**Spectre de RMN à 250 MHz**

1H à 8,75 ppm (S, N<u>H</u> Tr) - 1H à 8,10 ppm (D, J = 9 Hz, CON<u>H</u>) - 15H à 7,25 ppm (M, H aromatiques) - 1H à 6,73 ppm (S, <u>H</u> thiazole) - 1H à 5,81 ppm (M, $\underline{H_7}$) - 1H à 5,25 ppm (D, J = 13 Hz, $C\underline{H_2}OCO$) - 1H à 4,94 ppm (D, J = 4 Hz, $\underline{H_6}$) - 1H à 4,55 ppm (D, J = 13 Hz, $C\underline{H_2}OCO$) - 1H à 3,90 ppm (D, J = 17 Hz, $C\underline{H_2}SO$) - 2H à 3,79 ppm (D, J = 12 Hz, <u>H</u>CN Boc équatoriaux) - 1H à 3,53 ppm (D, J = 17 Hz, $C\underline{H_2}SO$) - 2H à 2,75 ppm (M, <u>H</u>CN Boc axiaux) - 1H à 2,50 ppm (M, $\underline{H}CCO_2$) - 2H à 1,75 ppm (D, J = 12 Hz, $\underline{H}CHCO_2$ équatoriaux) - 9H à 1,46 ppm (S, $CO_2$ <u>tBu</u>) - 2H à 1,40 ppm (M, $\underline{H}CCHCO_2$ axiaux) - 6H à 1,39 ppm (S, $(\underline{CH_3})_2C$) - 9H à 1,36 ppm (S, $CO_2$<u>tBu</u>) - 9H à 1,29 ppm (S, <u>Boc</u> N).

b) **SR 41 862**

On dissout 0,8 g du composé obtenu ci-dessus dans 10 ml d'acide trifluoroacétique. Après 45 minutes à 23°C l'acide est évaporé sous vide sans chauffer et le résidu huileux est cristallisé par addition de 50 ml d'éther isopropylique. Les cristaux sont filtrés et lavés à l'éther isopropylique puis à l'hexane. Ils sont ensuite séchés sous vide sur anhydride phosphorique. On obtient 0,66 g du produit attendu.

**Spectre IR:** ν **CO**

. 1795 $cm^{-1}$: C = 0 en 8 du β lactame
. 1735 $cm^{-1}$: C = 0 de l'ester en 3
. 1680 $cm^{-1}$ bande large: C = 0 des acides de la molécule, de l'amide en 7, des ions $CF_3CO_2$-

**Spectre de RMN à 250 MHz**

2H à 8,60 ppm (S.e., $NH_2$+) - 1H à 8,50 ppm (D, J = 9 Hz, CONH) - 3H à 8,40 ppm (S.e., $NH_3^{\oplus}$) - 1H à 6,68 ppm (S, H thiazole) - 1H à 6,0 ppm (D de D, $J_1$ = 9 Hz, $J_2$ = 4 Hz, $H_7$) - 1H à 5,16 ppm (D, J = 13 Hz, $CH_2OCO$) - 1H à 4,97 ppm (D, J = 4 Hz, $H_6$) - 1H à 4,60 ppm (D, J = 13 Hz, $CH_2OCO$) - 1H à 3,90 ppm (D, J = 17 Hz, $CH_2SO$) - 1H à 3,55 ppm (D, J = 17 Hz, $CH_2SO$) - 2H à 3,25 ppm (M, $CH_2NH$) - 2H à 2,90 ppm (M, $CH_2NH$) - 1H à 2,66 ppm (M, $CHCO_2$) - 2H à 1,90 ppm (M, $CH_2CH$) - 2H à 1,70 ppm (M, $CH_2CH$) - 6H à 1,44 ppm (2S, $(CH_3)_2C$).

**Exemple 2**

**Acide [(amino-2 thiazolyl-4)-2 (carboxy-2 propyl-2 oxyimino)-2 acétamido]-7 (amino-3 propionyl) thiométhyl-3 céphème-3 carboxylique-4 S-oxyde-1β isomère syn trifluoracétate. SR 41 884.**

a) Acide tertiobutoxycarbonyl amino-3 thiopropionique

On solubilise 1,8 g d'acide tertiobutoxycarbonylamino-3 propionique dans 50 ml de dichlorométhane anhydre. Le tricol est muni d'une garde à chlorure de calcium et refroidi dans un bain d'eau et de glace. On ajoute, dans l'ordre, 1,4 ml de triéthylamine et 1,3 ml de chloroformiate d'isobutyle. Après 20 minutes d'agitation à froid, on ajoute 1,5 ml de triétylamine et on fait barboter un léger courant d'hydrogène sulfuré pendant 15 minutes. Puis le mélange est agité à froid pendant 15 minutes avant d'être évaporé à sec. 150 ml de tampon sulfate (pH2) sont ajoutés et le thioacide est extrait deux fois par 70 ml de dichlorométhane. Les phases organique réunies sont séchées sur sulfate de magnésium et évaporées.

Le produit huileux obtenu est utilisé tel quel.

b) [(Tritylamino-2 thiazolyl-4)-2 (tertiobutoxycarbonyl-2 propyl-2 oxyimino)-2 acétamido]-7 (tertiobutoxycarbonylamino-3 propionyl) thiométhyl-3 céphème-3 carboxylate de tertiobutyle-4 S-oxyde-1β isomère syn

A une solution de 0,46 g de [(tritylamino-2 thiazolyl-4)-2 (tertiobutoxycarbonyl-2 propyl-2 oxyimino)-2 acétamido]-7 bromo méthyl-3 céphème-3 carboxylate de tertiobutyle-4 S-oxyde-1β isomère syn, dans 10 ml de tétrahydrofurane, on ajoute 0,4 g du thioacide décrit ci-dessus ainsi que 0,6 g d'hydrogénocarbonate de potassium. Après 4 heures d'agitation à température ambiante, le solvant est évaporé et le résidu repris par 100 ml d'eau et 50 ml de dichlorométhane. Après décantation la phase aqueuse est extraite par 50 ml de dichlorométhane. Les phases organiques sont réunies, séchées sur sulfate de magnésium et évaporées.

La laque ainsi obtenue est chromatographiée sur une colonne de silice (40 g), on élue par un mélange dichlorométhane-acétate d'éthyle 90 - 10 (vol/vol).

**Spectre IR: ν CO**

. 1805 $cm^{-1}$: C = 0 en 8 du β lactame
. 1720 $cm^{-1}$: C = 0 des esters tertiobutyliques
. 1690 $cm^{-1}$: C = 0 de l'amide en 7, du thioester en 3 et du tertiobutoxy carbonyle protecteur de l'amine.

**Spectre de RMN à 250 MHz (CD $CL_3$)**

1H à 7,75 ppm (D, J = 9 H; CONH) - 15H à 7,27 ppm (M, H ar Trit) - 1H à 6,95 ppm (S.e., NH- Trit) - 1H à 6,63 ppm (S, H thiazole) - 1H à 6,21 ppm (D de D, $J_1$ = 9 Hz, $J_2$ = 4 Hz, $H_7$) - 1H à 4,85 ppm (S.e., NH-Boc) - 1H à 4,50 ppm (D, J = 4 Hz, $H_6$) - 1H à 4,29 ppm (D, J = 13 Hz, $CH_2SCO$) - 1H à 3,75 ppm (D, J = 13 Hz, $CH_2SCO$) - 1H à 3,58 ppm (A de AB, J = 17 Hz, $CH_2SO$) - 2H à 3,36 ppm (M, $CH_2$ NH Boc) - 1H à 3,22 ppm (B de AB, J = 17 Hz, $CH_2SO$) - 2H à 2,75 ppm (T, J = 6 Hz, $CH_2$ -
CS) - 15H à 1,52 ppm (S, Boc NH et $(CH_3)_2C$) - 18H à 1,39 ppm (2S, $CO_2$ t Bu).
||

c) **SR 41 884**

La totalité du composé obtenu ci-dessus est solubilisée dans 10 ml d'acide trifluoroacétique. Après 45 minutes à 23° C l'acide est évaporé sous vide sans chauffer, et le résidu huileux est cristallisé par addition de 50 ml d'éther isopropylique. Les cristaux sont filtrés et lavés à l'éther isopropylique puis à l'hexane. Ils sont ensuite séchés sous vide sur anhydride phosphorique.

On obtient 0,37 g du produit attendu.

**Spectre IR:** ν **CO**

. 1785 $cm^{-1}$ C = 0 en 8 du β lactame
. 1680 $cm^{-1}$ bande large: C = 0 des acides de la molécule, de l'amide en 7, du thioester, des ions $CF_3CO_2^-$.

**Spectre de RMN à 250 MHz**

1H à 8,40 ppm (D, J = 9 Hz, CONH) - 3H à 7,80 ppm (S.e., $NH_3^+$) - 3H à 7,30 ppm (S.e., $NH_3^+$) - 1H à 6,78 ppm (S, H, thiazole) - 1H à 5,95 ppm (D de D, $J_1$ = 9 Hz, $J_2$ = 4 Hz, $H_7$) - 1H à 4,92 ppm (D, J = 4 Hz, $H_6$) - 1H à 4,18 ppm (D, J = 13 Hz, $CH_2SCO$) - 1H à 3,79 ppm (D, J = 13 Hz, $CH_2SCO$) - 2H à 3,66 ppm (S, $CH_2SO$) - 2H à 3,0 ppm (M, $CH_2NH_2$) - 2H à 2,92 ppm (M, $CH_2COS$) - 6H à 1,44 ppm (S, $(CH_3)_2C$).

**Exemple 3**

**Acide [(amino-2 thiazolyl-4)-2 (carboxy-2 propyl-2 oxyimino)-2 acétamido]-7 (amino-3 propionyl)-thiométhyl-3 céphème-3 carboxylique-4 S-oxyde-1 β isomère syn, trifluoracétate. SR 41 884.**

a) [(Tritylamino-2 thiazolyl-4)-2 tertiobutoxycarbonyl-2 propyl-2 oximino)-2 acétamido]-7 (tertiobutoxycarbonylamino-3 propionyl)-thiométhyl-3 céphème-3 carboxylate de tertiobutyle-4 S-oxyde-1 β isomère syn

A 0,46 g de [(tritylamino-2 thiazolyl-4)-2 (tertiobutoxy carboxylamino-2 propyl-2 oxyimino)-2 acétamido]-7 bromométhyl-3 céphème-3 carboxylate de tertiobutyl-4 S-oxyde 1 isomère syn dans 10 ml d'acétone anhydre, on ajoute 0,4 g d'acide tertiobutoxycarbonylamino-3 thiopropionique, 0,6 g d'hydrogénocarbonate de potassium et 0,25 g d'iodure de sodium.

Après 2 heures d'agitation à température ambiante, on évapore le solvant à siccité. On reprend le résidu dans 100 ml d'eau et extrait par 50 ml de dichlorométhane. On sépare la phase organique et réextrait la phase aqueuse par 50 ml de dichlorométhane. On réunit les extraits organiques, sèche sur sulfate de magnésium et évapore à siccité.

On chromatographie le produit obtenu sur colonne de silice en éluant avec un mélange dichlorométhane-acétate d'éthyle 90 - 10 (vol/vol).

On obtient un produit identique (spectre IR et spectre de RMN) au produit de l'exemple 2 b).

b) **SR 41 884**

On effectue la déprotection comme indiqué dans l'exemple 2 c).

En opérant comme dans l'exemple 1, on prépare les composés selon l'invention, sous forme de trifluoroacétate, décrits dans le tableau I ci-après.

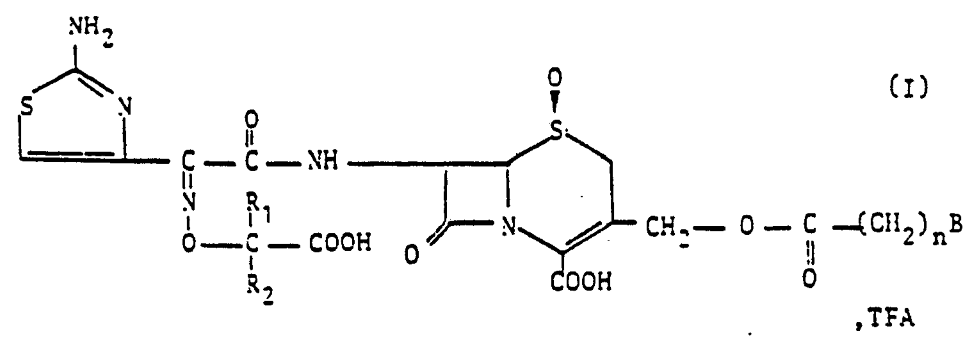

(I)

,TFA

Ces composés sont identifiés par un numéro de référence. On donne pour chacun d'entre eux les valeurs de $R_1$, $R_2$, B et n et le spectre de RMN.

L'acide B' - $(CH_2)_n$ - COOH qui réagit sur (IV) pour donner (V) est un aminoacide de la série L ou de la série D ou racémique; l'indication correspondante apparaît dans le tableau I, à la colonne B.

On donne également l'éluant de chromatographie qui sert pour isoler (V): dernier produit intermédiaire avant le déblocage des fonctions acides et amines de la molécule. Cet intermédiaire V est caractérisé par son

spectre infra-rouge, les longueurs d'ondes indiquées en $cm^{-1}$ correspondent dans l'ordre aux fréquences de vibrations d'élongation du carbonyl en 8 du bêta lactame, des esters tertiobutyliques et de l'ester en 3, de l'amide en 7 et du carbamate protecteur de l'amine. Lorsque longueurs d'onde seulement sont indiquées, la seconde correspond à une bande large qui recouvre les fréquences de vibration d'élongation à la fois des esters, de l'amide et du carbamate protecteur de l'amine.

La liste des spectres de RMN des composés cités dans le tableau I est donnée à la suite de ce tableau.

**Tableau I**

| n°SR | n | 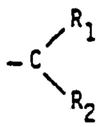 | B | Eluant de chromatographie de l'intermédiaire V vol/vol | IR $\vartheta$ CO $cm^{-1}$ intermédiaire V | n° RMN |
|---|---|---|---|---|---|---|
| 41730 | 0 | 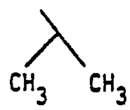 | $(CH_2)_2 NH_2$ | $CH_2Cl_2$ 85<br>Ac O Et 15 | 1805<br>1725<br>1690 | 1 |
| 41731 | " | " | 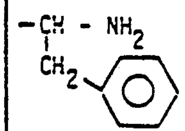 Ⓛ | $CH_2Cl_2$ 92,5<br>Ac O Et 7,5 | 1805<br>1720 | 2 |
| 41732 | " | " | 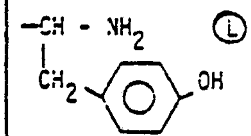 Ⓛ | $CH_2Cl_2$ 90<br>Ac O Et 10 | 1805<br>1725 | 3 |
| 41733 | " | " | 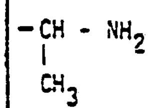 Ⓛ | $CH_2Cl_2$ 90<br>Ac O Et 10 | 1805<br>1725 | 4 |
| 41806 | " | " | 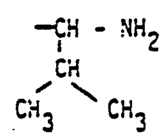 Ⓛ | $CH_2Cl_2$ 95<br>Ac O Et 5 | 1805<br>1730 | 5 |

| n°SR | n | 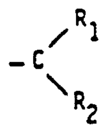 | B | Eluant de chromatographie de l'intermédiaire V. vol/vol | IR $\nu$ CO $cm^{-1}$ intermédiaire V | n° RMN |
|---|---|---|---|---|---|---|
| 41807 | 0 | 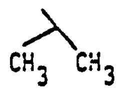 | $-CH - NH_2$ (L)<br>$CH_3 - CH_2-CO-NH_2$ | $CH_2Cl_2$ 50<br>Ac O Et 50 | 1805<br>1735<br>1680 | 6 |
| 41810 | " | " | $-CH - NH_2$ (L)<br>$CH_2OH$ | $CH_2Cl_2$ 80<br>Ac O Et 20 | 1805<br>1725 | 7 |
| 41854 | " | " | $-(CH_2)_3NH_2$ | $CH_2Cl_2$ 90<br>Ac O Et 10 | 1805<br>1725 | 8 |
| 41855 | " | " | $-(CH_2)_5NH_2$ | $CH_2Cl_2$ 90<br>Ac O Et 10 | 1805<br>1725<br>1690 | 9 |
| 41856 | " | " | $-(CH_2)_7NH_2$ | $CH_2Cl_2$ 90<br>Ac O Et 10 | 1805<br>1725<br>1695 | 10 |
| 41857 | " | " | $-CH - NH_2$ (L)<br>$CH_2S - CH_2NHCOCH_3$ | $CH_2Cl_2$ 100<br>Me OH 1,5 | 1805<br>1725<br>1680 | 11 |
| 41858 | " | " | $CH_3$<br>$- C - NH_2$<br>$CH_3$ | $CH_2Cl_2$ 90<br>Ac O Et 10 | 1805<br>1725 | 12 |
| 41859 | " | " | $-CH - NH_2$ (L)<br>$CH_2 - CH - CH_3$<br>$CH_3$ | $CH_2Cl_2$ 92,5<br>Ac O Et 7,5 | 1810<br>1725 | 13 |
| 41860 | " | " | $-CH - NH_2$ (L)<br>$CH - CH_2CH_3$<br>$CH_3$ | $CH_2Cl_2$ 92,5<br>Ac O Et 7,5 | 1805<br>1725 | 14 |

| n°SR | n | 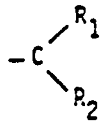 | B | Eluant de chromatographie de l'intermédiaire V vol/vol | IR $\partial$ CO $cm^{-1}$ intermédiaire V | n° RMN |
|---|---|---|---|---|---|---|
| 41885 | 0 | 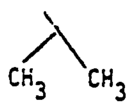 | $-CH - NH_2$. (L)<br>\|<br>$CH - OH$<br>\|<br>$CH_3$ | $CH_2 Cl_2$ 85<br>Ac O Et 15 | 1805<br>1720 | 15 |
| 41886 | " | " | $-CH - NH_2$ (L)<br>\|<br>$CH_2$ CO $NH_2$ | $CH_2 Cl_2$ 100<br>Me OH 1,5 | 1805<br>1720<br>1620 | 16 |
| 41887 | " | " | $-CH - NH_2$ (L)<br>\|<br>$CH_2 - CH_2$ S $CH_3$ | $CH_2 Cl_2$ 95<br>Ac O Et 5 | 1805<br>1725 | 17 |
| 41888 | " | " | $- CH - NH_2$ (L)<br>\|<br>$(CH_2)_4 - NH_2$ | $CH_2 Cl_2$ 100<br>Me OH 1 | 1505<br>1720 | 18 |
| 41889 | " | " | $-CH - NH_2$ (L)<br>\|<br>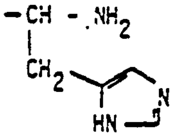 | $CH_2 Cl_2$ 80<br>Ac O Et 20 | 1805<br>1755<br>1720 | 19 |
| 41891 | " | " | $- CH - NH_2$ (D)<br>\|<br>$CH_3$ | $CH_2 Cl_2$ 90<br>Ac O Et 10 | 1805<br>1725 | 20 |
| 41967 | " | " | $(CH_2)_4$ $NH_2$ | $CH_2 Cl_2$ 90<br>Ac O Et 10 | 1805<br>1725<br>1690 | 21 |
| 41975 | " | " | 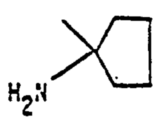 | $CH_2 Cl_2$ 92,5<br>Ac O Et 7,5 | 1805<br>1725 | 22 |
| 41976 | " | " | 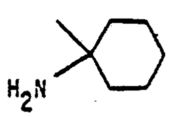 | $CH_2 Cl_2$ 92,5<br>Ac O Et 7,5 | 1805<br>1730 | 23 |

| n° SR | n | 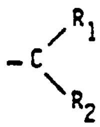 | B | Eluant de chromatographie de l'intermédiaire V vol/vol | IR $\vartheta$ CO $cm^{-1}$ intermédiaire V | n° RMN |
|---|---|---|---|---|---|---|
| 41977 | " | " | 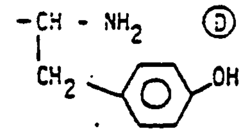 | $CH_2Cl_2$ 90<br>Ac O Et 10 | 1805<br>1720 | 24 |
| 41987 | " | " | $-CH - CH_2CH_2NH_2$<br>\|<br>$NH_2$ (L) | $CH_2Cl_2$ 85<br>Ac O Et 15 | 1805<br>1720 | 25 |
| 42022 | " | " | 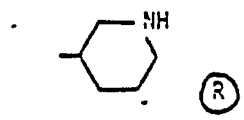 | $CH_2Cl_2$ 90<br>Ac O Et 10 | 1805<br>1730<br>1690 | 26 |
| 42023 | " | " | $-CH - (CH_2)_3NH_2$<br>\|<br>$NH_2$ (L) | $CH_2Cl_2$ 90<br>Ac O Et 10 | 1805<br>1720 | 27 |
| 42024 | " | " | $-CH_2 - CH - NH_2$<br>\|<br>$CH_3$ (R) | $CH_2Cl_2$ 85<br>Ac O Et 15 | 1805<br>1730<br>1690 | 28 |
| 42025 | " | " | $-CH - CH_2NH_2$<br>\|<br>$CH_3$ (R) | $CH_2Cl_2$ 90<br>Ac O Et 10 | 1805<br>1725<br>1690 | 29 |
| 42026 | " | " | 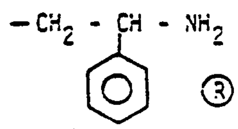 | $CH_2Cl_2$ 90<br>Ac O Et 10 | 1805<br>1725 | 30 |
| 42027 | 0 | " | $-CH_2-CH_2-CH-NH_2$<br>\|<br>$CH_3$ (R) | $CH_2Cl_2$ 92<br>Ac O Et 8 | 1805<br>1730<br>1690 | 31 |
| 42028 | " | " | $-(CH_2)_3$ NH $CH_3$ | $CH_2Cl_2$ 90<br>Ac O Et 10 | 1805<br>1730<br>1690 | 32 |
| 42029 | " | " | $-(CH_2)_4$ NH $CH_3$ | $CH_2Cl_2$ 90<br>Ac O Et 10 | 1805<br>1730<br>1690 | 33 |

| n° SR | n | 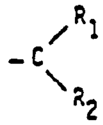 | B | Eluant de chromatographie de l'intermédiaire V. vol/vol | IR ϑ CO cm$^{-1}$ intermédiaire V | n° RMN |
|---|---|---|---|---|---|---|
| 42031 | 0 | " | 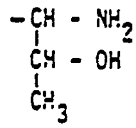 (D) | $CH_2Cl_2$ 85<br>Ac O Et 15 | 1805<br>1725<br>1675 | 34 |
| 42042 | " | " | 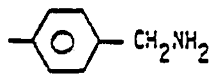 | $CH_2Cl_2$ 90<br>Ac O Et 10 | 1810<br>1730<br>1690 $CCl_4$ | 35 |
| 42073 | " | " | 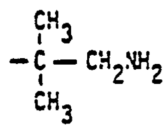 | $CH_2Cl_2$ 90<br>Ac O Et 10 | 1805<br>1725<br>1690 | 36 |
| 42117 | " | " | 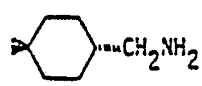 | $CH_2Cl_2$ 100<br>Me OH 1 | 1810<br>1725<br>1685 $CCl_4$ | 37 |
| 42120 | " | " | 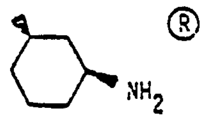 (R) | $CH_2Cl_2$ 90<br>Ac O Et 10 | 1805<br>1725 | 38 |
| 42121 | " | " | (R) 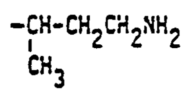 | $CH_2Cl_2$ 90<br>Ac O Et 10 | 1805<br>1730<br>1690 | 39 |
| 42139 | " | " | 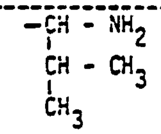 (D) | $CH_2Cl_2$ 95<br>Ac O Et 5 | 1805<br>1725<br>1690 | 40 |
| 42140 | " | " | $-CH_2CH_2NHCH_3$ | $CH_2Cl_2$ 90<br>Ac O Et 10 | 1805<br>1730<br>1690 | 41 |
| 42181 | " | " | $-(CH_2)_3NHCH_2CH_3$ | $CH_2Cl_2$ 100<br>Me OH 1 | 1805<br>1730<br>1685 $CH_2Cl_2$ | 42 |
| 42182 | " | " | 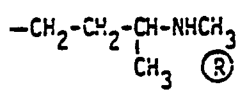 (R) | $CH_2Cl_2$ 90<br>Ac O Et 10 | 1805<br>1735<br>1685 | 43 |

| n° SR | n | 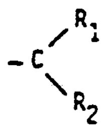 | B | Eluant de chromatographie de l'intermédiaire V vol/vol | IR $\nu$ CO $cm^{-1}$ intermédiaire V | n° RMN |
|---|---|---|---|---|---|---|
| 42183 | 0 | 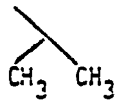 | $-(CH_2)_5NH\ CH_3$ | $CH_2Cl_2$ 100<br>Me OH 1 | 1810<br>1735<br>1690 $CCl_4$ | 44 |
| 42191 | " | $-\ CH_2$ | 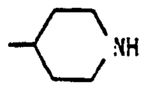 | $CH_2\ Cl_2$ 85<br>Ac O Et 15 | 1805<br>1730<br>1690 | 45 |
| 42192 | " | 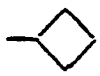 | 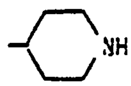 | $CH_2\ Cl_2$ 90<br>Ac O Et 10 | 1805<br>1730<br>1690 | 46 |
| 42193 | " | $-\ CH_2$ | 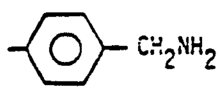 | $CH_2\ Cl_2$ 90<br>Ac O Et 10 | 1805<br>1720 | 47 |
| 42194 | " | 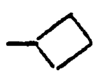 | 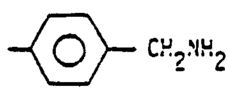 | $CH_2\ Cl_2$ 90<br>Ac O Et 10 | 1805<br>1725 | 48 |
| 42195 | " | $-\ CH_2$ | $-(CH_2)_3\ NH_2$ | $CH_2\ Cl_2$ 85<br>Ac O Et 15 | 1805<br>1730<br>1690 | 49 |
| 42196 | " | 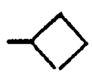 | " | $CH_2\ Cl_2$ 90<br>Ac O Et 10 | 1805<br>1725<br>1695 | 50 |
| 42197 | " | $-\ CH_2$ | $-(CH_2)_4\ NH_2$ | $CH_2\ Cl_2$ 85<br>Ac O Et 15 | 1805<br>1725<br>1690 | 51 |
| 42198 | " | 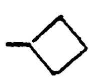 | " | $CH_2\ Cl_2$ 90<br>Ac O Et 10 | 1805<br>1725<br>1695 | 52 |

| n°SR | n | 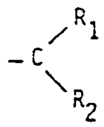 | B | Eluant de chromatographie de l'intermédiaire V vol/vol | IR ∂ CO $cm^{-1}$ intermédiaire V | n° RMN |
|---|---|---|---|---|---|---|
| 42200 | 0 | 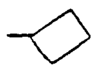 | 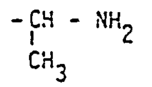 (D) | $CH_2Cl_2$ 90<br>Ac O Et 10 | 1805<br>1725 | 53 |
| 42201 | " | " | 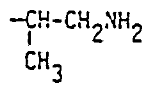 (R) | $CH_2Cl_2$ 90<br>Ac O Et | 1805<br>1725<br>1690 | 54 |
| 42208 | " | " | 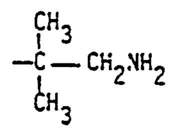 | $CH_2Cl_2$ 92,5<br>Ac O Et 7,5 | 1805<br>1725 | 55 |
| 42209 | " | " | 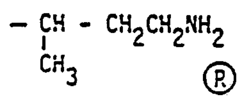 (R) | $CH_2Cl_2$ 90<br>Ac O Et 10 | 1805<br>1725<br>1690 | 56 |
| 42210 | " | " | 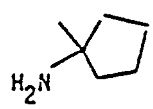 | $CH_2Cl_2$ 92,5<br>Ac O Et 7,5 | 1805<br>1725 | 57 |
| 42211 | " | $-CH_2$ | 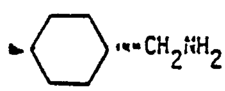 | $CH_2Cl_2$ 85<br>Ac O Et | 1805<br>1725<br>1690 | 58 |
| 42212 | " | 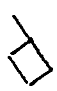 | " | $CH_2Cl_2$ 90<br>Ac O Et 10 | 1805<br>1725<br>1690 | 59 |
| 42213 | " | 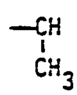 | $-(CH_2)_3NH_2$ | $CH_2Cl_2$ 85<br>Ac O Et 15 | 1805<br>1730<br>1690 | 60 |
| 42214 | " | " | $-(CH_2)_4NH_2$ | $CH_2Cl_2$ 85<br>Ac O Et 15 | 1805<br>1730<br>1690 | 61 |
| 42215 | " | " | 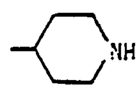 | $CH_2Cl_2$ 90<br>Ac O Et 10 | 1805<br>1735<br>1690 | 62 |

| n° SR | n | 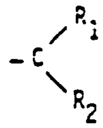 | B | Eluant de chromatographie de l'intermédiaire V vol/vol | IR $\nu$ CO $cm^{-1}$ intermédiaire V | n° RMN |
|---|---|---|---|---|---|---|
| 42 216 | 0 | 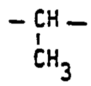 | 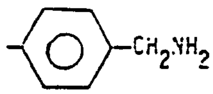 | $CH_2Cl_2$ 90<br>Ac O Et 10 | 1810<br>1725 $CCl_4$ | 63 |
| 42 217 | " | " | 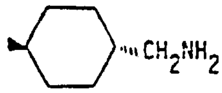 | $CH_2Cl_2$ 90<br>Ac O Et 10 | 1810<br>1725<br>1690 | 64 |
| 42 320 | " | 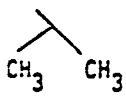 | 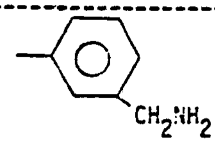 | $CH_2Cl_2$ 95<br>Ac O Et 5 | 1805<br>1725 | 65 |
| 42 321 | " | " | 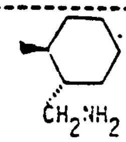 | $CH_2Cl_2$ 100<br>Me OH 1 | 1805<br>1730<br>1690 | 66 |
| 42 371 | 1 | " | 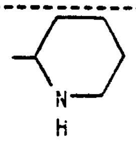 | $CH_2Cl_2$ 100<br>Me OH 0,7 | | 67 |
| 42 372 | " | " | 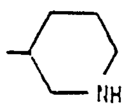 | $CH_2Cl_2$ 100<br>Me OH 0,7 | | 68 |
| 42 374 | 0 | " | 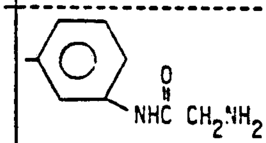 | $CH_2Cl_2$ 100<br>Me OH 1 | 1805<br>1725<br>1690 | 69 |
| 42 379 | " | " | 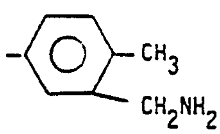 | $CH_2Cl_2$ 90<br>Ac O Et 10 | 1805<br>1725 | 70 |
| 42 380 | " | " | 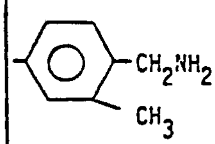 | $CH_2Cl_2$ 95<br>Ac O Et 5 | 1805<br>1725 | 71 |
| 42 395 | " | " | 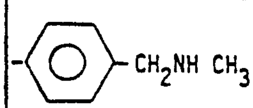 | $CH_2Cl_2$ 90<br>Ac O Et 10 | 1805<br>1725<br>1685 | 72 |

| n° SR | n | 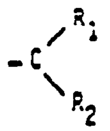 | B | Eluant de chromatographie de l'intermédiaire V vol/vol | IR ν CO $cm^{-1}$ intermédiaire V | n° RMN |
|---|---|---|---|---|---|---|
| 42 396 | 0 | 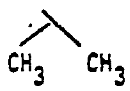 | 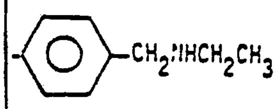 | $CH_2Cl_2$ 100<br>Me OH 0,6 | 1805<br>1725<br>1685 | 73 |
| 42 397 | " | " | 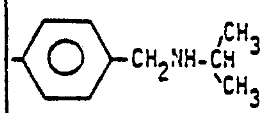 | $CH_2Cl_2$ 90<br>Ac O Et 10 | 1805<br>1725<br>1685 | 74 |
| 42 456 | " | " | 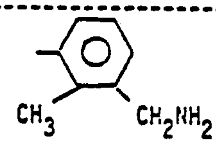 | $CH_2Cl_2$ 95<br>Ac O Et 5 | | 75 |
| 42 457 | " | " | 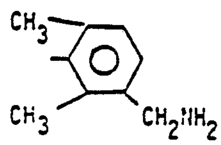 | $CH_2Cl_2$ 100<br>Me OH 1 | 1805<br>1725 | 76 |
| 42 458 | " | " | 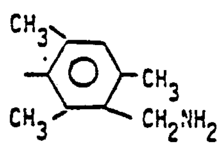 | $CH_2Cl_2$ 95<br>Ac O Et 5 | 1805<br>1725 | 77 |
| 42 459 | " | " | 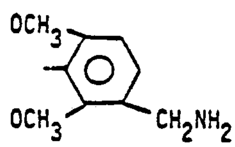 | $CH_2Cl_2$ 85<br>Ac O Et 15 | | 78 |
| 42 460 | " | " | 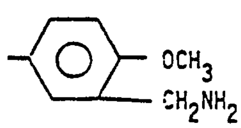 | $CH_2Cl_2$ 100<br>Me OH 1 | 1805<br>1720 | 79 |
| 42 461 | " | " | 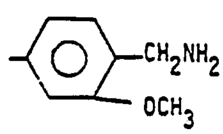 | $CH_2Cl_2$ 100<br>Me OH 1 | 1802<br>1720 | 80 |
| 42 462 | " | 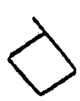 | 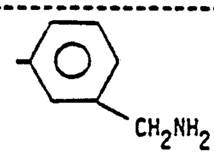 | $CH_2Cl_2$ 100<br>Me OH 1 | 1805<br>1720 | 81 |

| n° SR | n | 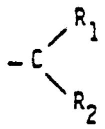 | B | Eluant de chromatographie de l'intermédiaire V vol/vol | IR $\nu$ CO $cm^{-1}$ intermédiaire V | n° RMN |
|---|---|---|---|---|---|---|
| 42 463 | 0 | 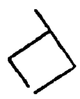 | 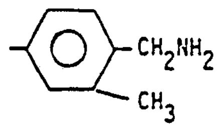 | $CH_2$ $Cl_2$ 100<br>Me OH 1 | 1805<br>1720 | 82 |
| 42 464 | " | " | 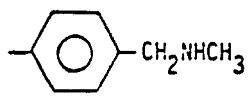 | $CH_2$ $Cl_2$ 100<br>Me OH 1 | 1805<br>1720<br>1690 | 83 |
| 42 465 | " | 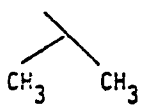 | 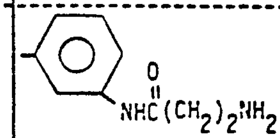 | $CH_2$ $Cl_2$ 100<br>Me OH 1,5 | 1805<br>1725<br>1690 | 84 |
| 42 466 | " | " | 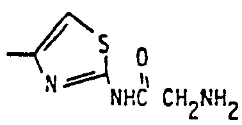 | $CH_2$ $Cl_2$ 100<br>Me OH 1,5 | 1805<br>1720 | 85 |
| 42 467 | " | " | 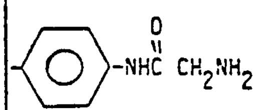 | $CH_2$ $Cl_2$ 100<br>Me OH 2 | 1805<br>1720 | 86 |
| 42 471 | 1 | 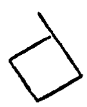 | 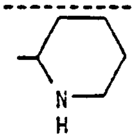 | $CH_2Cl_2$ 100<br>Me OH 0,5 | 1805 C $Cl_4$<br>1720<br>1675 | 87 |
| 42 472 | " | " | 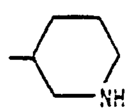 | $CH_2$ $Cl_2$ 95<br>Ac O Et 5 | 1805 C $Cl_4$<br>1725<br>1675 | 88 |
| 42 473 | " | " | 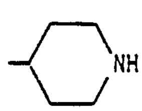 | $CH_2$ $Cl_2$ 100<br>Me OH 0,5 | 1805 C $Cl_4$<br>1725<br>1665 | 89 |
| 42 474 | " | 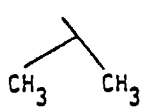 | 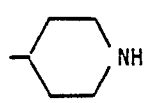 | $CH_2$ $Cl_2$ 95<br>Ac O Et 5 | 1805 C $Cl_4$<br>1725<br>1685 | 90 |
| 42 537 | 0 | " | 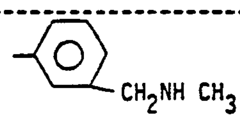 | $CH_2$ $Cl_2$ 100<br>Me OH 1 | 1805<br>1725<br>1690 | 91 |

| n° SR | n | 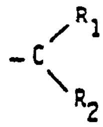 | B | Eluant de chromatographie de l'intermé-diaire V vol/vol | IR ϑ CO cm⁻¹ intermédiaire V | n° RMN |
|---|---|---|---|---|---|---|
| 42 538 | 0 | 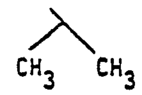 | 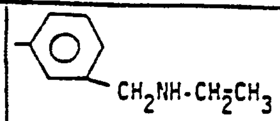 | $CH_2Cl_2$ 100<br>Me OH 1 | 1805<br>1725<br>1690 | 92 |
| 42 539 | " | " | 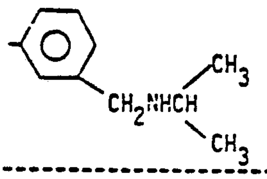 | $CH_2Cl_2$ 100<br>Me OH 0,8 | 1805<br>1725<br>1685 | 93 |
| 42 540 | " | 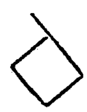 | 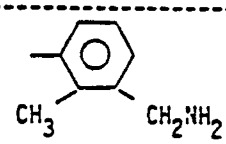 | $CH_2Cl_2$ 100<br>Me OH 0,7 | 1805<br>1720 | 94 |
| 42 541 | " | " | 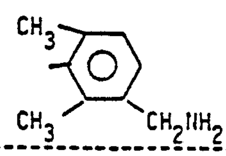 | $CH_2Cl_2$ 100<br>Me OH 0,7 | 1805<br>1720 | 95 |
| 42 542 | " | " | 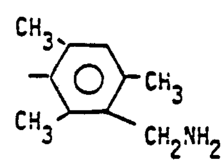 | $CH_2Cl_2$ 100<br>Me OH 0,5 | 1805<br>1720 | 96 |
| 42 544 | " | 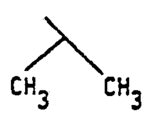 | 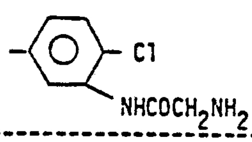 | $CH_2Cl_2$ 100<br>Me OH 1 | 1802<br>1720 | 97 |
| 42 545 | " | " | 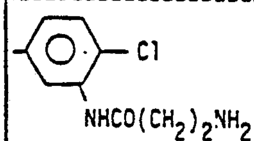 | $CH_2Cl_2$ 100<br>Me OH 1 | 1803<br>1720 | 98 |
| 42 546 | " | " | 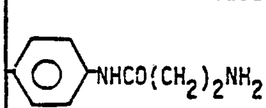 | $CH_2Cl_2$ 100<br>Me OH 1 | 1805<br>1720 | 99 |
| 42 547 | | 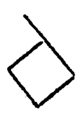 | 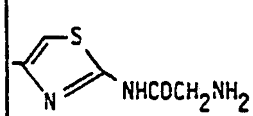 | $CH_2Cl_2$ 100<br>Me OH 1,5 | 1805<br>1720 | 100 |

| n° SR | n | 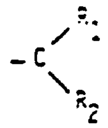 | B | Eluant de chromatographie de l'intermédiaire V vol/vol | IR $\nu$ CO $cm^{-1}$ intermédiaire V | n° RMN |
|---|---|---|---|---|---|---|
| 42 548 | 0 | 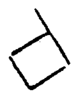 | 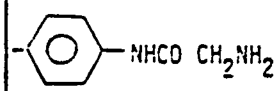 | $CH_2Cl_2$ 100<br>Me OH 2 | 1802<br>1720 | 101 |
| 42 549 | " | 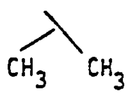 | 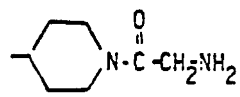 | $CH_2Cl_2$ 100<br>Me OH 1 | 1805<br>1720 | 102 |
| 42 581 | " | " | 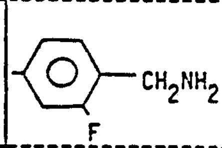 | $CH_2Cl_2$ 100<br>Me OH 0,8 | 1805<br>1725 | 103 |
| 42 582 | " | " | 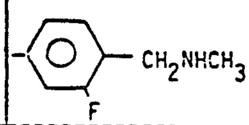 | $CH_2Cl_2$ 100<br>Me OH 0,8 | 1805<br>1725<br>1690 | 104 |
| 42 583 | " | " | 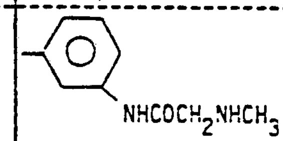 | $CH_2Cl_2$ 100<br>Me OH 0,5 | 1805<br>1720<br>1685 | 105 |
| 42 584 | " | " | 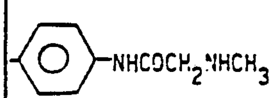 | $CH_2Cl_2$ 100<br>Me OH 1 | 1802<br>1720 | 106 |
| 42 585 | " | " | 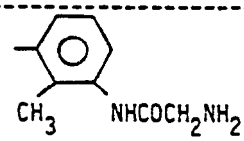 | $CH_2Cl_2$ 100<br>Me OH 0,8 | 1805<br>1720<br>1690 | 107 |
| 42 586 | " | " | 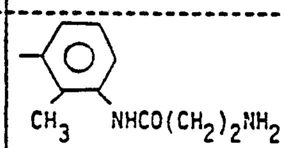 | $CH_2Cl_2$ 100<br>Me OH 1 | 1805<br>1725<br>1690 | 108 |
| 42 587 | " | " | 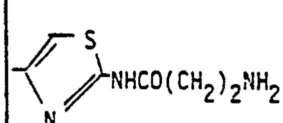 | $CH_2Cl_2$ 100<br>Me OH 1,5 | 1805<br>1720<br>1690 | 109 |
| 42 657 | " | " | 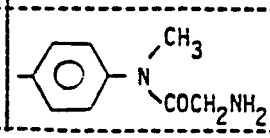 | $CH_2Cl_2$ 100<br>Me OH 0,7 | 1805<br>1725<br>1675 | 110 |

| n° SR | n | 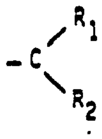 | B | Eluant de chromatographie de l'intermédiaire V vol/vol | IR $\nu$ CO $cm^{-1}$ intermédiaire V | n° RMN |
|---|---|---|---|---|---|---|
| 42 658 | 0 | 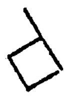 | 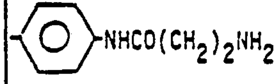 | $CH_2Cl_2$ 100<br>Me OH 1 | 1805<br>1720 | 111 |
| 42 675 | " | " | 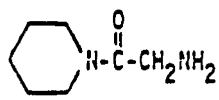 | $CH_2Cl_2$ 90<br>Ac O Et 10 | 1805<br>1725 | 112 |
| 42 676 | " | 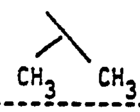 | 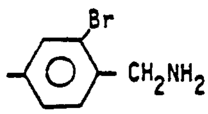 | $CH_2Cl_2$ 100<br>Me OH 0,5 | 1805<br>1720 | 113 |
| 42 677 | " | " | 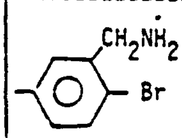 | $CH_2Cl_2$ 100<br>Me OH 0,5 | 1805<br>1720 | 114 |
| 42 687 | " | " | 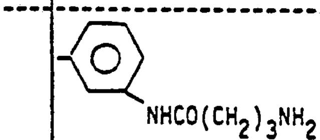 | $CH_2Cl_2$ 100<br>Me OH 0,9 | 1805<br>1725<br>1690 | 115 |
| 42 688 | " | " | 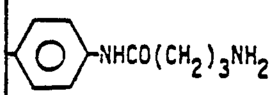 | $CH_2Cl_2$ 100<br>Me OH 0,8 | 1805<br>1720 | 116 |
| 42 689 | " | " | 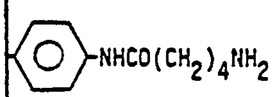 | $CH_2Cl_2$ 100<br>Me OH 0,8 | 1805<br>1720 | 117 |
| 42 690 | " | " | 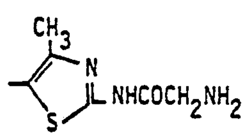 | $CH_2Cl_2$ 100<br>Me OH 1 | 1805<br>1715 | 118 |
| 42 811 | " | " | Cis Trans<br>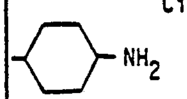 | $CH_2Cl_2$ 100<br>Me OH 0,5 | 1805 $CH_2Cl_2$<br>1725 | 119 |
| 42 812 | " | " | 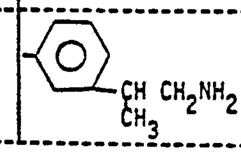 | $CH_2Cl_2$ 92,5<br>Ac O ET 7,5 | 1805<br>1725 | 120 |

| n° SR | n | 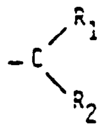 | B | Eluant de chromatographie de l'intermédiaire V vol/vol | IR $\vartheta$ CO $cm^{-1}$ intermédiaire V | n° RMN |
|---|---|---|---|---|---|---|
| 42 814 | 0 | 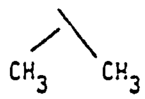 | 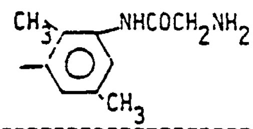 | $CH_2Cl_2$ 100<br>Me OH 1 | 1805<br>1725<br>1690 | 121 |
| 42 815 | " | " | 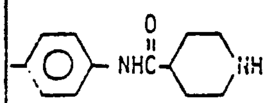 | $CH_2Cl_2$ 100<br>Me OH 0,7 | 1805<br>1725<br>1690 | 122 |
| 42 816 | " | " | 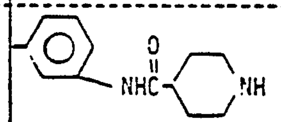 | $CH_2Cl_2$ 100<br>Me OH 0,7 | 1805<br>1725<br>1690 | 123 |
| 42 817 | " | " | 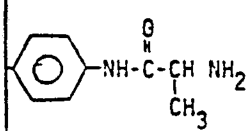 | $CH_2Cl_2$ 100<br>Me OH 0,9 | 1805<br>1720 | 124 |
| 42 818 | " | 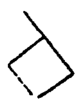 | " " | $CH_2Cl_2$ 100<br>Me OH 0,9 | 1805<br>1720 | 125 |
| 42 781 | " | 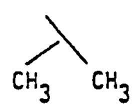 | 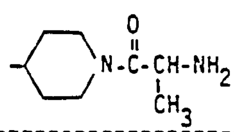 | $CH_2Cl_2$ 100<br>Me OH 1 | 1805<br>1725<br>1675 $CH_2Cl_2$ | 126 |
| 42 782 | " | " | 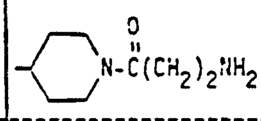 | $CH_2Cl_2$ 100<br>Me OH 1,5 | 1805<br>1725 | 127 |
| 42 783 | " | " | 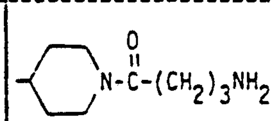 | $CH_2Cl_2$ 100<br>Me OH 1 | | 128 |
| 42 846 | " | " | 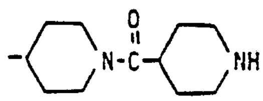 | $CH_2Cl_2$ 100<br>Me OH 1 | 1805<br>1725<br>1680 $CH_2Cl_2$ | 129 |
| 42 848 | " | " | 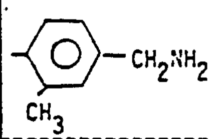 | $CH_2Cl_2$ 100<br>Me OH 0,7 | 1805<br>1720 | 130 |

| n° SR | n | 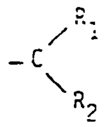 | B | Eluant de chromatographie de l'intermédiaire V vol/vol | IR $\nu$ CO $cm^{-1}$ intermédiaire V | n° RMN |
|---|---|---|---|---|---|---|
| 42849 | 0 | 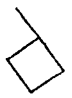 | 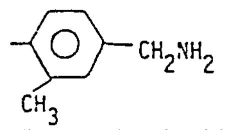 | $CH_2Cl_2$ 100<br>Me OH 0,7 | 1805<br>1720 | 131 |
| 42852 | " | 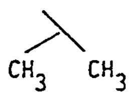 | 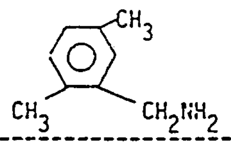 | $CH_2Cl_2$ 100<br>Me OH 0,5 | 1805<br>1725 | 132 |
| 42857 | " | " | 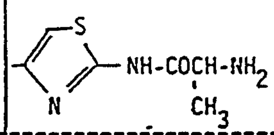 | $CH_2Cl_2$ 100<br>Me OH 1,5 | 1805<br>1725 | 133 |
| 42862 | " | " | 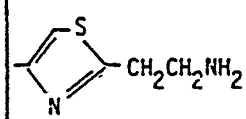 | $CH_2Cl_2$ 100<br>Me OH 1 | 1805<br>1725 | 134 |
| 42869 | " | " | 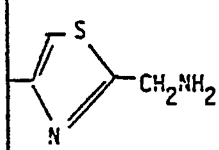 | $CH_2Cl_2$ 85<br>Ac O Et 15 | 1805<br>1725 | 135 |
| 42870 | " | 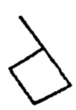 | 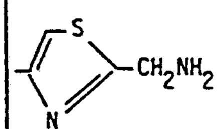 | $CH_2Cl_2$ 80<br>Ac O Et 20 | 1805<br>1720 | 136 |
| 42901 | " | 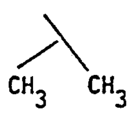 | 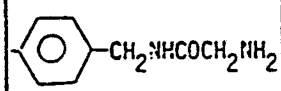 | $CH_2Cl_2$ 100<br>Me OH 1 | 1805<br>1720<br>1680 | 137 |

**Spectres de RMN**:

Les spectres sont enregistrés à 60 MHz, signalés par (a) ou à 250 MHz, signalés par (b); lorsqu'il existe deux diastéréoisomères dans la molécule, les signaux dédoublés sont indiqués par x.

**RMN n° 1 - (b)**:

1H à 8,44 ppm (D, J = 9 Hz, CON<u>H</u>) - 3H à 7,90 ppm (S.e., $N^{\oplus}\underline{H_3}$) - 3H à 7,50 ppm $N^{\oplus}\underline{H_3}$) - 1H à 6,78 ppm (S, <u>H</u> thiazole) - 1H à 5,97 ppm (D de D, $J_1$ = 9 Hz, $J_2$ = 4 Hz, $\underline{H_7}$) - 1H à 5,18 ppm (D, J = 13 Hz, $C\underline{H_2}OCO$) - 1H à 4,94 ppm (D, J = 4 Hz, $\underline{H_6}$) - 1H à 4,64 ppm (D, J = 13 Hz, $C\underline{H_2}OCO$) - 1H à 3,90 ppm (D, J = 17 Hz, $C\underline{H_2}SO$) - 1H à 3,58 ppm (D, J = 17 Hz, $C\underline{H_2}SO$) - 2H à 3,00 ppm (M, $C\underline{H_2}NH_2$) - 2H à 2,61 ppm (M, $C\underline{H_2}CO_2$) - 6H à 1,44 ppm (S, $(C\underline{H_3})_2C$).

**RMN n° 2 - (b)**:

4H à 8,45 ppm (M, $N^{\oplus}H_3$, CONH) - 3H à 7,35 ppm (S.e., $N^{\oplus}H_3$) - 5H à 7,25 ppm (M, H aromatiques) - 1H à 6,82 ppm (S, H thiazole) - 1H à 6,0 ppm (D de D, $J_1$ = 9 Hz, $J_2$ = 4 Hz, $H_7$) - 1H à 5,20 ppm (D, J = 13 Hz, $CH_2OCO$) - 1H à 4,92 ppm (D, J = 4 Hz, $H_6$) - 1H à 4,81 ppm (D, J = 13 Hz, $CH_2OCO$) - 1H à 4,31 ppm (S.e., $CHNH_2$) - 1H à 3,50 ppm (D, J = 17 Hz, $CH_2SO$) - 1H à 3,30 ppm (D, J = 17 Hz, $CH_2$ SO) - 2H à 3,10 ppm (M, $CH_2C_6H_5$) - 6H à 1,44 ppm (2S, $(CH_3)_2C$).

**RMN n° 3 - (b)**:

5H à 8,45 ppm (S.e., $N^{\oplus}H_2$, OH, CONH) - 3H à 7,50 ppm (S.e., $N^{\oplus}H_3$) - 2H à 6,95 ppm (D, J = 8 Hz, H méta OH) - 1H à 6,78 ppm (S, H thiazole) - 2H à 6,68 ppm (D, J = 8 Hz, H ortho OH) - 1H à 6,0 ppm (D de D, $J_1$ = 9 Hz, $J_2$ = 4 Hz, $H_7$) - 1H à 5,16 ppm (D, J = 13 Hz, $CH_2OCO$) - 1H à 4,92 ppm (D, J = 4 Hz, $H_6$) - 1H à 4,74 ppm (D, J = 13 Hz, $CH_2OCO$) - 1H à 4,19 ppm (M, $CHNH_2$) - 1H à 3,66 ppm (D, J = 17 Hz, $CH_2SO$) - 1H à 3,34 ppm (D, J = 17 Hz, $CH_2SO$) - 2H à 2,93 ppm (M, $CH_2$ - CH - $NH_2$) - 6H à 1,44 ppm (2S, $(CH_3)_2C$).

**RMN n° 4 - (b)**:

1H à 8,50 ppm (D, J = 9 Hz, CONH) - 3H à 8,40 ppm (S.e., $N^{\oplus}H_3$) - 3H à 7,60 ppm (S.e., $N^{\oplus}H_3$) - 1H à 6,79 ppm (S, H thiazole) - 1H à 6,00 ppm (D de D, $J_1$ = 9 Hz, $J_2$ = 4 Hz, $H_7$) - 1H à 5,45 ppm (D, J = 13 Hz, $CH_2OCO$) - 1H à 4,97 ppm (D, J = 4 Hz, $H_6$) - 1H à 4,80 ppm (D, J = 13 Hz, $CH_2OCO$) - 1H à 4,08 ppm (M, $CHNH_2$) - 1H à 3,92 ppm (D, J = 17 Hz, $CH_2SO$) - 1H à 3,58 ppm (D, J = 17 Hz, $CH_2SO$) - 6H à 1,44 ppm (S, $(CH_3)_2C$) - 3H à 1,34 ppm (D, J = 7 Hz, $CH_3CH$).

**RMN n° 5 - (b)**:

1H à 8,45 ppm (D, J = 9 Hz, CONH) - 3H à 8,40 ppm (S.e., $N^{\oplus}H_3$) - 3H à 7,60 ppm (S.e., $N^{\oplus}H_3$) - 1H à 6,78 ppm (S, H thiazole) - 1H à 6,0 ppm (D de D, $J_1$ = 9 Hz, $J_2$ = 4 Hz, $H_7$) - 1H à 5,25 ppm (D, J = 13 Hz, $CH_2OCO$) - 1H à 4,95 ppm (D, J = 4 Hz, $H_6$) - 1H à 4,82 ppm (D, J = 13 Hz, $CH_2OCO$) - 2H à 3,92 ppm (M, $CHNH_2$ et $CH_2SO$) - 1H à 3,56 ppm (D, J = 17 Hz, $CH_2SO$) - 1H à 2,11 ppm (M, CH $(CH_3)_2$) - 6H à 1,44 ppm (2S, $(CH_3)_2C$) - 6H à 0,9 ppm (D, J = 7 Hz, $(CH_3)_2CH$).

**RMN n° 6 - (b)**:

1H à 8,45 ppm (D, J = 9 Hz, CONH) - 3H à 8,35 ppm (S.e., $N^{\oplus}H_3$) - 4H à 7,40 ppm (S.e., $CONH_2$, $N^{\oplus}H_3$) - 1H à 6,92 ppm (S.e., $CONH_2$) - 1H à 6,76 ppm (S, H thiazole) - 1H à 6,0 ppm (D de D, $J_1$ = 9 Hz, $J_2$ = 4 Hz, $H_7$) - 1H à 5,25 ppm (D, J = 13 Hz, $CH_2OCO$) - 1H à 5,00 ppm (D, J = 4 Hz, $H_6$) - 1H à 4,82 ppm (D, J = 13 Hz, $CH_2OCO$) - 1H à 4,00 ppm (M, $CHNH_2$) - 1H à 3,95 ppm (D, J = 17 Hz, $CH_2SO$) - 1H à 3,56 ppm (D, J = 17 Hz, $CH_2SO$) - 2H à 2,20 ppm (M, $CH_2$-$CONH_2$) - 2H à 1,95 ppm (M, $CH_2$-CH) - 6H à 1,44 ppm (2S, $(CH_3)_2C$).

**RMN n° 7 - (b)**:

1H à 8,50 ppm (D, J = 9 Hz, CONH) - 3H à 8,40 ppm (S.e., $N^{\oplus}H_3$) - 3H à 7,40 ppm (S.e., $N^{\oplus}H_3$) - 1H à 6,76 ppm (S, H thiazole) - 1H à 6,0 ppm (D de D, $J_1$ = 9 Hz, $J_2$ = 4 Hz, $H_7$) - 1H à 5,25 ppm (D, J = 13 Hz, $CH_2OCO$) - 1H à 4,92 ppm (D, J = 4 Hz, $H_6$) - 1H à 4,82 ppm (D, J = 13 Hz, $CH_2OCO$) - 1H à 4,16 ppm (M, $CHNH_2$) - 1H à 3,95 ppm (D, J = 17 Hz, $CH_2SO$) - 1H à 3,81 ppm (A de AB, $J_{AB}$ = 13 Hz, $CH_2OH$) - 1H à 3,69 ppm (B de AB, $J_{AB}$ = 13 Hz, $CH_2OH$) - 1H à 3,55 ppm (D, J = 17 Hz, $CH_2$ SO) - 6H à 1,44 ppm (2S, $(CH_3)_2C$).

**RMN n° 9 - (a)**:

8H entre 5,5 et 8,5 ppm (signal élargi, $NH_2$, $CO_2H$, TFA) - 1H à 8,40 ppm (D, J = 9 Hz, CONH) - 1H à 6,82 ppm (S, H thiazole) - 1H à 6,0 ppm (D de D, $J_1$ = 9 Hz, $J_2$ = 4 Hz, $H_7$) - 1H à 5,15 ppm (A de AB, $J_{AB}$ = 13 Hz, $CH_2OCO$) - 1H à 5,00 ppm (D, J = 4 Hz, $H_6$) - 1H à 4,67 ppm (B de AB, J = 13 Hz, $CH_2OCO$) - 1H à 3,85 ppm (A

de AB, J = 17 Hz, $C\underline{H}_2SO$) - 1H à 3,62 ppm (B de AB, J = 17 Hz, $C\underline{H}_2SO$) - 2H à 2,80 ppm (M, $C\underline{H}_2NH_2$) - 2H à 2,40 ppm (M, $C\underline{H}_2CO_2$) - 2H à 1,80 (M, $CH_2C\underline{H}_2CH_2$) - 6H à 1,43 ppm (S, $(C\underline{H}_3)_2C$).

**RMN n° 9 - (a):**

8H entre 5,5 et 8,7 ppm (signal large, $CO_2\underline{H}$, $N\underline{H}_2$, TFA) - 1H à 8,40 ppm (D, J = 9 Hz, $CON\underline{H}$) - 1H à 6,87 ppm (S, $\underline{H}$ thiazole) - 1H à 6,0 ppm (D de D, $J_1$ = 9 Hz, $J_2$ = 4 Hz, $\underline{H}_7$) - 1H à 5,15 ppm (A de AB, J = 13 Hz, $C\underline{H}_2OCO$) - 1H à 5,0 ppm (D, J = 4 Hz, $\underline{H}_6$) - 1H à 4,60 ppm (B de AB, J = 13 Hz, $C\underline{H}_2OCO$) - 1H à 3,85 ppm (A de AB, J = 17 Hz, $C\underline{H}_2SO$) - 1H à 3,60 ppm (B de AB, J = 17 Hz, $C\underline{H}_2SO$) - 2H à 2,80 ppm (M, $C\underline{H}_2NH_2$) - 2H à 2,30 ppm (M, $C\underline{H}_2CO$) - 12H à 1,45 ppm (S.e., $(C\underline{H}_3)_2C$ et $CH_2(C\underline{H}_2)_3CH_2$).

**RMN n° 10 - (a):**

8H entre 5,5 et 8,0 ppm (signal large, $N\underline{H}_2$, $CO_2\underline{H}$, TFA) - 1H à 8,45 ppm (D, J = 9 Hz, $CON\underline{H}$) - 1H à 6,87 ppm (S, $\underline{H}$ thiazole) - 1H à 6,0 ppm (D de D, $J_1$ = 9 Hz, $J_2$ = 4 Hz, $\underline{H}_7$) - 1H à 5,15 ppm (A de AB, J = 13 Hz, $C\underline{H}_2OCO$) - 1H à 5,0 ppm (D, J = 4 Hz, $\underline{H}_6$) - 1H à 4,65 ppm (B de AB, J = 13 Hz, $C\underline{H}_2OCO$) - 1H à 3,85 ppm (A de AB, J = 17 Hz, $C\underline{H}_2SO$) - 1H à 3,62 ppm (B de AB, J = 17 Hz, $C\underline{H}_2SO$) - 2H à 2,80 ppm (M, $C\underline{H}_2NH_2$) - 2H à 2,30 ppm (M, $C\underline{H}_2CO_2$) - 6H à 1,45 ppm (S, $(C\underline{H}_3)_2C$) - 10H à 1,35 ppm (S.e., $CH_2(C\underline{H}_2)_5CH_2$).

**RMN n° 11 - (a):**

2H à 8,40 ppm (M, $CON\underline{H}$, $CH_3CON\underline{H}$) - 8H à 7,50 ppm (S.e., $N^{\oplus}\underline{H}_3$, $CO_2\underline{H}$) - 1H à 6,90 ppm (S, $\underline{H}$ thiazole) - 1H à 6,05 ppm (D de D, $J_1$ = 9 Hz, $J_2$ = 4 Hz, $\underline{H}_7$) - 1H à 5,40 ppm (A de AB, J = 13 Hz, $C\underline{H}_2OCO$) - 1H à 4,98 ppm (D, J = 4 Hz, $\underline{H}_6$) - 1H à 4,90 ppm (B de AB, J = 13 Hz, $C\underline{H}_2OCO$) - 3H à 4,30 ppm (M, $C\underline{H}_2NHCOCH_3$, $C\underline{H}NH_2$) - 1H à 4,00 ppm (A de AB, $J_{AB}$ = 17 Hz, $C\underline{H}_2SO$) - 1H à 3,65 ppm (B de AB, $J_{AB}$ = 17 Hz, $C\underline{H}_2SO$) - 2H à 3,00 ppm (M, $C\underline{H}_2S$) - 3H à 1,80 ppm (S, $C\underline{H}_3CONH$) - 6H à 1,45 ppm (S, $(C\underline{H}_3)_2C$).

**RMN n° 12 - (a):**

8H entre 6,5 et 9 ppm (signal large, $CO_2\underline{H}$, TFA, $N\underline{H}_2$) - 1H à 8,5 ppm (D, J = 9 Hz, $CON\underline{H}$) - 1H à 6,90 ppm (S, $\underline{H}$ thiazole) - 1H à 6,0 ppm (D de D, $J_1$ = 9 Hz, $J_2$ = 4 Hz, $\underline{H}_7$) - 1H à 5,30 ppm (A de AB, J = 13 Hz, $C\underline{H}_2OCO$) - 1H à 5,0 ppm (D, J = 4 Hz, $\underline{H}_6$) - 1H à 4,80 ppm (B de AB, J = 13 Hz, $C\underline{H}_2OCO$) - 1H à 3,92 ppm (A de AB, J = 17 Hz, $C\underline{H}_2SO$) - 1H à 3,67 ppm (B de AB, J = 17 Hz, $C\underline{H}_2SO$) - 12H à 1,45 ppm (S, $(C\underline{H}_3)_2C\text{-}CO_2H$, $(C\underline{H}_3)_2C\ NH_2$).

**RMN n° 13 - (a):**

8H entre 6,5 et 9,5 ppm (signal large, $CO_2\underline{H}$, $N\underline{H}_2$, TFA) - 1H à 8,5 ppm (D, J = 9 Hz, $CON\underline{H}$) - 1H à 6,90 ppm (S, $\underline{H}$ thiazole) - 1H à 6,05 ppm (D de D, $J_1$ = 9 Hz, $J_2$ = 4 Hz, $\underline{H}_7$) - 1H à 5,30 ppm (A de AB, J = 13 Hz, $C\underline{H}_2OCO$) - 1H à 5,05 ppm (D, J = 4 Hz, $\underline{H}_6$) - 1H à 4,90 ppm (B de AB, J = 13 Hz, $C\underline{H}_2OCO$) - 3H à 3,80 ppm (M, $C\underline{H}NH_2$ et $C\underline{H}_2SO$) - 3H à 1,50 ppm (M, $C\underline{H}_2\text{-}C\underline{H}$) - 6H à 1,45 ppm (S, $(C\underline{H}_3)_2C$) - 6H à 0,85 ppm (D, J = 7 Hz, $(C\underline{H}_3)_2C\underline{H}$).

**RMN n° 14 - (a):**

8H entre 7 et 9 ppm (signal large, $N\underline{H}_2$, $CO_2\underline{H}$, TFA) - 1H à 8,50 ppm (D, J = 9 Hz, $CON\underline{H}$) - 1H à 6,90 ppm (S, $\underline{H}$ thiazole) - 1H à 6,08 ppm (D de D, $J_1$ = 9 Hz, $J_2$ = 4 Hz, $\underline{H}_7$) - 1H à 5,30 ppm (A de AB, $J_{AB}$ = 13 Hz, $C\underline{H}_2OCO$) - 1H à 5,05 ppm (D, J = 4 Hz, $\underline{H}_6$) - 1H à 4,49 ppm (B de AB, $J_{AB}$ = 13 Hz, $C\underline{H}_2OCO$) - 3H à 3,90 ppm (M, $C\underline{H}_2SO$ et $C\underline{H}CH_2$) - 1H à 1,80 ppm (M, $C\underline{H}CH_3$) - 6H à 1,45 ppm (S, $(C\underline{H}_3)_2C$) - 2H à 1,30 ppm (M, $C\underline{H}_2CH_3$) - 6H à 0,88 ppm (M, $C\underline{H}_3CH_2$ et $C\underline{H}_3CH$).

**RMN n° 15 - (b):**

1H à 8,5 ppm (D, J = 9 Hz, $CON\underline{H}$) - 3H à 8,30 ppm (S.e., $N^{\oplus}\underline{H}_3$) - 3H à 7,40 ppm (S.e., $N^{\oplus}\underline{H}_3$) - 1H à 6,76 ppm (S, $\underline{H}$ thiazole) - 1H à 6,0 ppm (D de D, $J_1$ = 9 Hz, $J_2$ = 4 Hz, $\underline{H}_7$) - 1H à 5,24 ppm (D, J = 13 Hz, $C\underline{H}_2OCO$) - 1H à 4,92 ppm (D, J = 4 Hz, $\underline{H}_6$) - 1H à 4,84 ppm (D, J = 13 Hz, $C\underline{H}_2OCO$) - 1H à 4,10 ppm (M, $C\underline{H}$-$NH_2$) - 1H à 3,97 ppm (M, $C\underline{H}$-OH) - 1H à 3,94 ppm (D, J = 17 Hz, $C\underline{H}_2SO$) - 1H à 3,55 ppm (D, J = 17 Hz, $C\underline{H}_2SO$) - 6H à 1,44 ppm (S, $(C\underline{H}_3)_2C$) - 3H à 1,14 ppm (D, J = 7 Hz, $C\underline{H}_3CH$).

**RMN n° 16 - (b):**

1H à 8,5 ppm (D, J = 9 Hz, $CON\underline{H}$) - 3H à 8,40 ppm (S.e., $N^{\oplus}\underline{H}_3$) - 1H à 7,66 ppm (S, $CON\underline{H}_2$) - 3H à 7,50 ppm (S.e., $N^{\oplus}\underline{H}_3$) - 1H à 7,22 ppm (S, $CON\underline{H}_2$) - 1H à 6,78 ppm (S, $\underline{H}$ thiazole) - 1H à 6,0 ppm (D de D, $J_1$ = 9 Hz, $J_2$ = 4 Hz, $\underline{H}_7$) - 1H à 5,29 ppm (D, J = 13 Hz, $C\underline{H}_2OCO$) - 1H à 4,92 ppm (D, J = 4 Hz, $\underline{H}_6$) - 1H à 4,79 ppm (D, J = 13 Hz, $C\underline{H}_2OCO$) - 1H à 4,26 ppm (M, $C\underline{H}NH_2$) - 1H à 3,92 ppm (D, J = 17 Hz, $C\underline{H}_2SO$) - 1H à 3,52 ppm (D, J = 17 Hz, $C\underline{H}_2SO$) - 2H à 2,71 ppm (M, $C\underline{H}_2CONH_2$) - 6H à 1,44 ppm (S, $(C\underline{H}_3)_2C$).

**RMN n° 17 - (b):**

1H à 8,50 ppm (D, J = 9 Hz, $CON\underline{H}$) - 3H à 8,45 ppm (S.e., $N^{\oplus}\underline{H}_3$) - 3H à 7,45 ppm (S.e., $N^{\oplus}\underline{H}_3$) - 1H à 6,78 ppm (S, $\underline{H}$ thiazole) - 1H à 6,0 ppm (D de D, $J_1$ = 9 Hz, $J_2$ = 4 Hz, $\underline{H}_7$) - 1H à 5,24 ppm (D, J = 13 Hz, $C\underline{H}_2OCO$) - 1H à 4,98 ppm (D, J = 4 Hz, $\underline{H}_6$) - 1H à 4,82 ppm (D, J = 13 Hz, $C\underline{H}_2OCO$) - 1H à 4,13 ppm (M, $C\underline{H}NH_2$) - 1H à 3,92 ppm (D, J = 17 Hz, $C\underline{H}_2SO$) - 1H à 3,10 ppm (D, J = 17 Hz, $C\underline{H}_2SO$) - 1H à 2,50 ppm (M, $C\underline{H}_2S$) - 5H à 2,0 ppm (M, $C\underline{H}_3S$ et $C\underline{H}_2$-$CH_2$-S) - 6H à 1,44 ppm (S, $(C\underline{H}_3)_2C$).

**RMN n° 18 - (b):**

4H à 8,50 ppm (M, $CON\underline{H}$, $N^{\oplus}H_3$) - 3H à 7,80 ppm (S.e., $N^{\oplus}\underline{H}_3$) - 3H à 7,50 ppm (S.e., $N^{\oplus}\underline{H}_3$) - 1H à 6,79 ppm (S, $\underline{H}$ thiazole) - 1H à 6,0 ppm (D de D, $J_1$ = 9 Hz, $J_2$ = 4 Hz, $\underline{H}_7$) - 1H à 5,24 ppm (D, J = 13 Hz, $C\underline{H}_2OCO$) - 1H à 4,97 ppm (D, J = 4 Hz, $\underline{H}_6$) - 1H à 4,82 ppm (D, J = 13 Hz, $C\underline{H}_2OCO$) - 1H à 3,97 ppm (M, $C\underline{H}NH_2$) - 1H à 3,92 ppm (D, J = 17 Hz, $C\underline{H}_2SO$) - 1H à 3,60 ppm (D, J = 17 Hz, $C\underline{H}_2SO$) - 2H à 2,75 ppm (M, $C\underline{H}_2NH_2$ - 1H à 1,75 ppm (M, $C\underline{H}_2CH$) - 11H à 1,40 ppm (S.e., $(C\underline{H}_3)_2C$, $(C\underline{H}_2)_3CH_2NH_2$).

**RMN n° 19 - (b):**

1H à 9 ppm (S, $\underline{H}_2$ imidazole) - 3H à 8,6 ppm (S.e., $N^{\oplus}\underline{H}_3$) - 1H à 8,5 ppm (D, J = 9 Hz, $CON\underline{H}$) - 1H à 7,40 ppm (S, $\underline{H}_4$ imidazole) - 3H à 7,30 ppm (S.e., $N^{\oplus}\underline{H}_3$) - 1H à 6,79 ppm (S, $\underline{H}$ thiazole) - 1H à 6,0 ppm (D de D, $J_1$ = 9 Hz, $J_2$ = 4 Hz, $\underline{H}_7$) - 1H à 5,22 ppm (D, J = 13 Hz, $C\underline{H}_2OCO$) - 1H à 4,97 ppm (D, J = 4 Hz, $\underline{H}_6$) - 1H à 4,90 ppm (D, J = 13 Hz, $C\underline{H}_2OCO$) - 1H à 4,40 ppm (M, $C\underline{H}NH_2$) - 1H à 3,89 ppm (D, J = 17 Hz, $C\underline{H}_2SO$) - 1H à 3,52 ppm (D, J = 17 Hz, $C\underline{H}_2SO$) - 2H à 3,20 ppm (M, $C\underline{H}_2CH$) - 6H à 1,44 ppm (S, $(C\underline{H}_3)_2C$).

**RMN n° 20 - (b):**

1H à 8,50 ppm (D, J = 9 Hz, $CON\underline{H}$) - 3H à 8,40 ppm (S.e., $N^{\oplus}\underline{H}_3$) - 3H à 7,40 ppm (S.e., $N^{\oplus}\underline{H}_3$) - 1H à 6,77 ppm (S, $\underline{H}$ thiazole) - 1H à 6,0 ppm (D de D, $J_1$ = 9 Hz, $J_2$ = 4 Hz, $\underline{H}_7$)- 1H à 5,76 ppm (D, J = 13 Hz, $C\underline{H}_2OCO$) - 1H à 4,95 ppm (D, J = 4 Hz, $\underline{H}_6$) - 1H à 4,81 ppm (D, J = 13 Hz, $C\underline{H}_2OCO$) - 1H à 4,06 ppm (M, $C\underline{H}NH_2$) - 1H à 3,95 ppm (D, J = 17 Hz, $C\underline{H}_2SO$) - 1H à 3,63 ppm (D, J = 17 Hz, $C\underline{H}_2SO$) - 6H à 1,44 ppm (S, $(C\underline{H}_3)_2C$) - 3H à 1,36 ppm (D, J = 7 Hz, $C\underline{H}_3CH$).

**RMN n° 21 - (a):**

8H entre 6,5 et 9 ppm (signal élargi, $N\underline{H}_2$, $CO_2\underline{H}$, TFA) - 1H à 8,35 ppm (D, J = 9 Hz, $CON\underline{H}$) - 1H à 6,80 ppm (S, $\underline{H}$ thiazole) - 1H à 6,0 ppm (D de D, $J_1$ = 9 Hz, $J_2$ = 4 Hz, $\underline{H}_7$) - 1H à 5,15 ppm (A de AB, $J_{AB}$ = 13 Hz, $C\underline{H}_2OCO$) - 1H à 5,0 ppm (D, J = 4 Hz, $\underline{H}_6$) - 1H à 4,65 ppm (B de AB, $J_{AB}$ = 13 Hz, $C\underline{H}_2OCO$) - 1H à 3,95 ppm (A de AB, $J_{AB}$

= 17 Hz, $CH_2SO$) - 1H à 3,65 ppm (B de AB, $J_{AB}$ = 17 Hz, $CH_2SO$) - 2H à 2,80 ppm (M, $CH_2NH_2$) 2H à 2,35 ppm (M, $CH_2CO$) - 10H à 1,45 ppm (S.e., $(CH_3)_2C + CH_2(CH_2)_2CH_2$).

**RMN n° 22 - (a):**

8H entre 6 et 9 ppm (signal large, TFA, $NH_2$, $CO_2H$) - 1H à 8,42 ppm (D, J = 9 Hz, CONH) - 1H à 6,85 ppm (S, H thiazole) - 1H à 6,05 ppm (D de D, $J_1$ = 9 Hz, $J_2$ = 4 Hz, $H_7$) - 1H à 5,30 ppm (A de AB, J = 13 Hz, $CH_2OCO$) - 1H à 5,05 ppm (D, J = 4 Hz, $H_6$) - 1H à 4,68 ppm (B de AB, $J_{AB}$ = 13 Hz, $CH_2OCO$) - 1H à 4,0 ppm (A de AB, $J_{AB}$ = 17 Hz, $CH_2SO$) - 1H à 3,65 ppm (B de AB, $J_{AB}$ = 17 Hz, $CH_2SO$) - 8H à 1,75 ppm (M, H, cyclopentane) - 6H à 1,45 ppm (S, $(CH_3)_2C$).

**RMN n° 23 - (a):**

8H entre 7 et 10 ppm (signal large, TFA, $NH_2$, $CO_2H$) - 1H à 8,50 ppm (D, J = 9 Hz, CONH) - 1H à 6,92 ppm (S, H thiazole) - 1H à 6,10 ppm (D de D, $J_1$ = 9 Hz, $J_2$ = 4 Hz, $H_7$) - 1H à 5,35 ppm (A de AB, J = 13 Hz, $CH_2OCO$) - 1H à 5,0 ppm (D, J = 4 Hz, $H_6$) - 1H à 4,75 ppm (B de AB, J = 13 Hz, $CH_2OCO$) - 1H à 4,0 ppm (A de AB, $J_{AB}$ = 17 Hz, $CH_2SO$) - 1H à 3,70 ppm (B de AB, $J_{AB}$ = 17 Hz, $CH_2SO$) - 16H entre 1 et 2,3 ppm (M, $(CH_3)_2C$ et cyclohexane).

**RMN n° 24 - (a):**

9H entre 8 et 10 ppm (signal large, $NH_2$, OH, $CO_2H$, TFA) - 1H à 8,55 ppm (D, J = 9 Hz, CONH) - 2H à 7,10 ppm (D, J = 8 Hz, H méta OH) - 1H à 6,90 ppm (S, H thiazole) - 2H à 6,80 ppm (D, J = 8 Hz, H ortho, OH) - 1H à 6,10 ppm (D de D, $J_1$ = 9 Hz, $J_2$ = 4 Hz, $H_7$) - 1H à 5,80 ppm (A de AB, J = 13 Hz, $CH_2OCO$) - 1H à 5,05 ppm (D, J = 4 Hz, $H_6$) - 1H à 4,80 ppm (B de AB, J = 13 Hz, $CH_2OCO$) - 1H à 4,30 ppm (M, $CHNH_2$) - 2H à 3,70 ppm (M, $CH_2SO$) - 2H à 3,0 ppm (M, $CH_2$-$C_6H_4OH$ - 6H à 1,46 ppm (S, $(CH_3)_2C$).

**RMN n° 25 - (a):**

10H entre 6,5 et 9,5 ppm (signal large, $NH_2$, $CO_2H$, TFA) - 1H à 8,40 ppm (D, J = 9 Hz, CONH) - 1H à 6,85 ppm (S, H thiazole) - 1H à 6,05 ppm (D de D, $J_1$ = 9 Hz, $J_2$ = 4 Hz, $H_7$) - 1H à 5,30 ppm (A de AB, $J_{AB}$ = 13 Hz, $CH_2OCO$) - 1H à 5,0 ppm (D, J = 4 Hz, $H_6$) - 1H à 4,85 ppm (B de AB, $J_{AB}$ = 13 Hz, $CH_2OCO$) - 1H à 4,20 ppm (M, $CHNH_2$) - 2H à 3,80 ppm (M, $CH_2SO$) - 2H à 2,95 ppm (M, $CH_2NH_2$) - 2H à 2,20 ppm (M, $CH_2CH_2NH_2$) - 6H à 1,45 ppm (S, $(CH_3)_2C$).

**RMN n° 26 - (b):**

3H à 8,60 ppm (S.e., $N^{\oplus}H_3$) - 1H à 8,44 ppm (D, J = 9 Hz, CONH) - 1H à 6,78 ppm (S, H thiazole) - 1H à 6,0 ppm (D de D, $J_1$ = 9 Hz, $J_2$ = 4 Hz, $H_7$) - 1H à 5,16 ppm (2D, J = 13 Hz, $CH_2OCO$)* - 1H à 4,97 ppm (D, J = 4 Hz, $H_6$) - 1H à 4,66 ppm (2D, J = 13 Hz, $CH_2OCO$)* - 1H à 3,92 ppm (D, J = 17 Hz, $CH_2SO$) - 1H à 3,56 ppm (D, J = 17 Hz, $CH_2SO$) - 5H entre 2,5 et 3,5 ppm (M, $CH_2N$ et $CHCO_2$) - 4H entre 1,5 et 2,0 ppm (M, $(CH_2)_2$ $CH_2N$) - 6H à 1,44 ppm (S, $(CH_3)_2C$).

**RMN n° 27 - (b):**

3H à 8,50 ppm (S.e., $N^{\oplus}H_3$) - 1H à 8,44 ppm (D, J = 9 Hz, CONH) - 3H à 7,80 ppm (S.e., $N^{\oplus}H_3$) - 3H à 7,30 ppm (S.e., $N^{\oplus}H_3$) - 1H à 6,78 ppm (S, H thiazole) - 1H à 6,0 ppm (D de D, $J_1$ = 9 Hz, $J_2$ = 4 Hz, $H_7$) - 1H à 5,26 ppm (D, J = 13 Hz, $CH_2OCO$) - 1H à 4,97 ppm (D, J = 4 Hz, $H_6$) - 1H à 4,84 ppm (D, J = 13 Hz, $CH_2OCO$) - 1H à 4,08 ppm (M, $CHCO_2$) - 1H à 3,94 ppm (D, J = 17 Hz, $CH_2SO$) - 1H à 3,56 ppm (D, J = 17 Hz, $CH_2SO$) - 2H à 2,8 ppm (M, $CH_2NH_2$) - 4H à 1,60 ppm (M, $(CH_2)_2CH_2NH_2$) - 6H à 1,44 ppm (S, $(CH_3)_2C$).

**RMN n° 28 - (b):**

1H à 8,37 ppm (D, J = 9 Hz, CONH) - 3H à 7,90 (S.e., $N^{\oplus}H_3$) - 3H à 7,20 ppm (S.e., $N^{\oplus}H_3$) - 1H à 6,76 ppm (S, H thiazole) - 1H à 6,0 ppm (D de D, $J_1$ = 9 Hz, $J_2$ = 4 Hz, $H_7$) - 1H à 5,16 ppm (D, J = 13 Hz, $CH_2OCO$) - 1H à 4,97 ppm (D, J = 4 Hz, $H_6$) - 1H à 4,66 ppm (D, J = 13 Hz, $CH_2OCO$) - 1H à 3,92 ppm (D, J = 17 Hz, $CH_2SO$) - 1H à 3,58 ppm (D, J = 17 Hz, $CH_2SO$) - 1H à 3,50 ppm (M, $CHNH_2$) - 2H à 2,56 ppm (M, $CH_2CHNH_2$) - 6H à 1,44 ppm (S, $(CH_3)_2C$) - 3H à 1,16 ppm (D, J = 6 Hz, $CH_3$-CH).

**RMN n° 29 - (b):**

1H à 8,44 ppm (D, J = 9 Hz, CONH) - 3H à 7,95 ppm (S.e., $N^{\oplus}H_3$) - 3H à 7,50 ppm (S.e., $N^{\oplus}H_3$) - 1H à 6,78 ppm (S, H thiazole) - 1H à 6,0 ppm (D de D, $J_1$ = 9 Hz, $J_2$ = 4 Hz, $H_7$) - 1H à 5,20 ppm (2D, J = 13 Hz, $CH_2OCO$)* - 1H à 4,95 ppm (M, $H_6$) - 1H à 4,62 ppm (2D, J = 13 Hz, $CH_2OCO$)* - 1H à 3,94 ppm (D, J = 17 Hz, $CH_2SO$) - 1H à 3,58 ppm (D, J = 17 Hz, $CH_2SO$) - 1H à 3,0 ppm (M, $CHCO_2$) - 2H à 2,75 ppm (M, $CH_2NH_2$) - 6H à 1,44 ppm (M, $(CH_3)_2C$) - 3H à 1,10 ppm (D, J = 7 Hz, $CH_3CH$).

**RMN n° 30 - (b):**

4H à 8,45 ppm (M, $N^{\oplus}H_3$, CONH) - 8H à 7,35 ppm (M, $N^{\oplus}H_3$, H aromatique) - 1H à 6,78 ppm (S, H thiazole) - 1H à 6,0 ppm (M, $H_7$) - 1H à 5,05 ppm (2D, J = 13 Hz, $CH_2OCO$)* 1H à 4,94 ppm (2D, J = 4 Hz, $H_6$)* - 1H à 4,60 ppm (M, $CHNH_2$) - 1H à 3,66 ppm (2D, J = 17 Hz, $CH_2SO$)* - 1H à 3,40 ppm (2D, J = 17 Hz, $CH_2SO$)* - 2H à 3,0 ppm (M, $CH_2CO_2$) - 6H à 1,44 ppm (S, $(CH_3)_2C$).

**RMN n° 31 - (b):**

1H à 8,40 ppm (D, J = 9 Hz, CONH) - 3H à 7,74 ppm (S.e., $N^{\oplus}H_3$) - 3H à 7,40 ppm (S.e., $N^{\oplus}H_3$) - 1H à 6,81 ppm (S, H thiazole) - 1H à 6,0 ppm (D de D, $J_1$ = 9 Hz, $J_2$ = 4 Hz, $H_7$) - 1H à 5,13 ppm (D, J = 13 Hz, $CH_2OCO$) - 1H à 4,97 ppm (D, J = 4 Hz, $H_6$) - 1H à 4,63 ppm (D, J = 13 Hz, $CH_2OCO$) - 1H à 3,90 ppm (D, J = 17 Hz, $CH_2SO$) - 1H à 3,55 ppm (D, J = 17 Hz, $CH_2SO$) - 1H à 3,16 ppm (M, $CHNH_2$) - 2H à 2,40 ppm (M, $CH_2CO$) - 1H à 1,77 ppm (M, $CH_2CHNH_2$) - 1H à 1,61 ppm (M, $CH_2CHNH_2$) - 6H à 1,44 ppm (2S, $(CH_3)_2C$) - 1H à 1,1 ppm (D, J = 7 Hz, $CH_3$-CH).

**RMN n° 32 - (a):**

7H entre 7 et 9 ppm (NH, $NH_2$, $CO_2H$, TFA) - 1H à 8,50 ppm (D, J = 9 Hz, CONH) - 1H à 6,90 ppm (S, H thiazole) - 1H à 6,05 ppm (D de D, $J_1$ = 9 Hz, $J_2$ = 4 Hz, $H_7$) - 1H à 5,15 ppm (A de AB, J = 13 Hz, $CH_2OCO$) - 1H à 5,0 ppm (D, J = 4 Hz, $H_6$) - 1H à 4,70 ppm (B de AB, J = 13 Hz, $CH_2OCO$) - 1H à 3,95 ppm (A de AB, $J_{AB}$ = 17 Hz, $CH_2SO$) - 1H à 3,65 ppm (B de AB, $J_{AB}$ = 17 Hz, $CH_2SO$) - 2H à 2,90 ppm (M, $CH_2NH$) - 3H à 2,45 ppm (D, J = 6 Hz, $CH_3NH$) - 2H à 2,4 ppm (M, $CH_2CO$) - 2H à 1,70 ppm (M, $CH_2CH_2CH_2$) - 6H à 1,42 ppm (S, $(CH_3)_2C$).

**RMN n° 33 - (a):**

7H entre 7 et 9,5 ppm ($NH_2$, NH, $CO_2H$, TFA) - 1H à 8,46 ppm (D, J = 9 Hz, CONH) - 1H à 6,90 ppm (S, H thiazole) - 1H à 6,05 ppm (D de D, $J_1$ = 9 Hz, $J_2$ = 4 Hz, $H_7$) - 1H à 5,15 ppm (A de AB, $J_{AB}$ = 13 Hz, $CH_2OCO$) - 1H à 5,0 ppm (D, J = 4 H; $H_6$) - 1H à 4,70 ppm (B de AB, $J_{AB}$ = 13 Hz, $CH_2OCO$) - 1H à 3,90 ppm (A de AB, $J_{AB}$ = 17 Hz, $CH_2SO$) - 1H à 3,65 ppm (B de AB, $J_{AB}$ = 17 Hz, $CH_2SO$) - 2H à 2,75 ppm (M, $CH_2NHCH_3$) - 5H à 2,45 ppm (M, $CH_3NH$ et $CH_2CO$) - 10H à 1,45 ppm (S.e., $(CH_3)_2C$ et $CH_2$ $(CH_2)_2CH_2$).

**RMN n° 34 - (a):**

8H entre 6 et 9 ppm (signal élargi, $NH_2$, TFA, OH, $CO_2H$) - 1H à 8,47 ppm (D, J = 9 Hz, CONH) - 1H à 6,90 ppm (S, H thiazole) - 1H à 6,15 ppm (D de D, $J_1$ = 9 Hz, $J_2$ = 4 Hz, $H_7$) - 1H à 5,35 ppm (AB, $J_{AB}$ = 13 Hz, $CH_2OCO$) - 1H à 5,0 ppm (D, J = 4 Hz, $H_6$) - 1H à 4,85 ppm (B de AB, $J_{AB}$ = 13 Hz, $CH_2OCO$) - 4H à 3,95 ppm (M, $CH_2SO$ et CHOH et $CHNH_2$) - 6H à 1,45 ppm (S, $(CH_3)_2C$) - 3H à 1,20 ppm (D, J = 7 Hz, $CH_3CHOH$).

**RMN n° 35 - (b):**

1H à 8,50 ppm (D, J = 9 Hz, CONH) - 3H à 8,35 ppm (S.e., $N^{\oplus}H_3$) - 2H à 7,94 ppm (D, J = 8 Hz, H ortho CO) - 2H à 7,55 ppm (D, J = 8 Hz, H méta CO) - 1H à 6,84 ppm (S, H thiazole) - 1H à 6,0 ppm (D de D, $J_1$ = 9 Hz, $J_2$ = 4 Hz, $H_7$) - 1H à 5,44 ppm (D, J = 13 Hz, $CH_2OCO$) - 1H à 4,99 ppm (D, J = 4 Hz, $H_6$) - 1H à 4,86 ppm (D, J = 13 Hz, $CH_2OCO$) - 3H à 4,1 ppm (M, $CH_2NH_2$, $CH_2SO$) - 1H à 3,72 ppm (D, J = 17 Hz, $CH_2SO$) - 6H à 1,44 ppm (2S, $(CH_3)_2C$).

**RMN n° 36 - (a):**

3H à 9,30 ppm (S.e., $N^{\oplus}H_3$) - 1H à 8,55 ppm (D, J = 9 Hz, CONH) - 3H à 8,05 ppm (S.e., $N^{\oplus}H_3$) - 1H à 6,92 ppm (S, H thiazole) - 1H à 6,05 ppm (D de D, $J_1$ = 9 Hz, $J_2$ = 4 Hz, $H_7$) - 1H à 5,30 ppm (A de AB, $J_{AB}$ = 13 Hz, $CH_2OCO$) - 1H à 5,05 ppm (D, J = 4 Hz, $H_6$) - 1H à 4,70 ppm (B de AB, $J_{AB}$ = 13 Hz, $CH_2OCO$) - 1H à 3,95 ppm (A de AB, $J_{AB}$ = 17 Hz, $CH_2SO$) - 1H à 3,65 ppm (B de AB, $J_{AB}$ = 17 Hz, $CH_2SO$) - 2H à 3,0 ppm (S.e., $CH_2NH_2$) - 6H à 1,45 ppm (S, $(CH_3)_2C$-ON) - 6H à 1,17 ppm (S, $(CH_3)_2CO_2CH_2$).

**RMN n° 37 - (b):**

8H entre 6 et 9 ppm (signal large, $CO_2H$, TFA, $NH_2$) - 1H à 8,50 ppm (D, J = 9 Hz, CONH) - 1H à 6,90 ppm (S, H thiazole) - 1H à 6,0 ppm (D de D, $J_1$ = 9 Hz, $J_2$ = 4 Hz, $H_7$) - 1H à 5,15 ppm (D, J = 13 Hz, $CH_2OCO$) - 1H à 5,0 ppm (D, J = 4 Hz, $H_6$) - 1H à 4,58 ppm (D, J = 13 Hz, $CH_2OCO$) - 1H à 3,9 ppm (D, J = 17 Hz, $CH_2SO$) - 1H à 3,54 ppm (D, J = 17 Hz, $CH_2SO$) - 2H à 2,6 ppm (M, $CH_2NH_2$) - 1H à 2,25 ppm (T, J = 12 Hz, $CHCO_2$) - 4H à 1,84 ppm (M, $CH_2CHCO$) - 6H à 1,45 ppm (S, $(CH_3)_2C$) - 3H à 1,25 ppm (M, $CHCH_2NH_2$ et $CH_2CHCH_2NH_2$) - 2H à 0,95 ppm (M, $CH_2CHCH_2NH_2$).

**RMN n° 38 - (b):**

1H à 8,37 ppm (D, J = 9 Hz, CONH - 3H à 7,90 ppm (S.e., $N^{\oplus}H_3$) - 3H à 7,40 ppm (S.e., $N^{\oplus}H_3$) - 1H à 6,79 ppm (S, H thiazole) - 1H à 6,0 ppm (D de D, $J_1$ = 9 Hz, $J_2$ = 4 Hz, $H_7$) - 1H à 5,11 ppm (2D, J = 13 Hz, $CH_2OCO$)* - 1H à 4,98 ppm (D, J = 4 Hz, $H_6$) - 1H à 4,61 ppm (2D, J = 13 Hz, $CH_2OCO$)* - 1H à 3,90 ppm (D, J = 17 Hz, $CH_2SO$) - 1H à 3,59 ppm (D, J = 17 Hz, $CH_2SO$) - 1H à 3,0 ppm (S.e., $CHNH_2$) - 1H à 2,40 ppm (M, $CHCO_2$) - 1H à 2,10 ppm (M, $CH_2CHNH_2$) - 3H à 1,80 ppm (M, $CH_2CHNH_2$) - 6H à 1,44 ppm (2S, $(CH_3)_2C$) - 4H entre 1 et 1,5 ppm (M, $CH_2$-$CH_2$-CH-$CO_2$).

**RMN n° 39 - (b):**

1H à 8,5 ppm (D, J = 9 He, CONH) - 6H entre 7 et 8 ppm (signal large, $NH_2$, TFA) - 1H à 6,81 ppm (S, H thiazole) - 1H à 6,0 ppm (D de D, $J_1$ = 9 Hz, $J_2$ = 4 Hz, $H_7$) - 1H à 5,15 ppm (2D, J = 13 Hz, $CH_2OCO$)* - 1H à 5,0 ppm (D, J = 4 Hz, $H_6$) - 1H à 4,63 ppm (2D, J = 13 Hz, $CH_2OCO$)* - 1H à 3,89 ppm (D, J = 17 Hz, $CH_2SO$) - 1H à 3,56 ppm D, J = 17 Hz, $CH_2SO$) - 2H à 2,77 ppm (M, $CH_2NH_2$) - 1H à 2,52 ppm (M, $CHCO_2$) - 1H à 1,84 ppm (M, $CH_2CH$) - 1H à 1,56 ppm (M, $CH_2CH$) - 6H à 1,44 ppm (S, $(CH_3)_2C$) - 3H à 1,06 ppm (D, J = 7 Hz, $CH_3CH$).

**RMN n° 40 - (a):**

1H à 8,45 ppm (D, J = 9 Hz, CONH) - 8H à 7,30 ppm (S.e., $N^{\oplus}H_3$, $CO_2H$) - 1H à 6,80 ppm (S, H thiazole) - 1H à 6,0 ppm (M, $H_7$) - 1H à 5,20 ppm (A de AB, $J_{AB}$ = 13 Hz, $CH_2OCO$) - 1H à 4,90 ppm (B de AB, $J_{AB}$ = 13 Hz, $CH_2OCO$) - 1H à 4,90 ppm (D, J = 4 Hz, $H_6$) - 3H à 3,80 ppm (M, $CH_2SO$, $CHNH_2$) - 1H à 2,00 ppm (M, $CH(CH_3)_2$) - 6H à 1,45 ppm (S, $(CH_3)_2C$) - 6H à 0,95 ppm (2D, J = 7 Hz, $(CH_3)_2CH$).

**RMN n° 41 - (a):**

2H à 8,50 ppm (S.e., $N^{\oplus}H_2$) - 1H à 8,45 ppm (D, J = 9 Hz, CONH) - 3H à 7,30 ppm (S.e., $N^{\oplus}H_3$) - 1H à 6,80 ppm (S, H thiazole) - 1H à 6,0 ppm (M, $H_7$) - 1H à 5,15 ppm (A de AB, $J_{AB}$ = 13 Hz, $CH_2OCO$) - 1H à 4,96 ppm (D, J =

4 Hz, $\underline{H}_6$) - 1H à 4,65 ppm (B de AB, 13 Hz, $C\underline{H}_2OCO$) - 1H à 3,90 ppm (A de AB, J = 17 Hz, $C\underline{H}_2SO$) - 1H à 3,65 ppm (B de AB, J = 17 Hz, $C\underline{H}_2SO$) - 2H à 3,00 ppm (M, $C\underline{H}_2NH$) - 5H à 2,50 ppm (M, $C\underline{H}_3NH$, $C\underline{H}_2CO_2$) - 6H à 1,42 ppm (S, $(C\underline{H}_3)_2C$).

**RMN n° 42 - (a):**

7H à 9,4 ppm (S.e., $N^{\oplus}\underline{H}_3$, $N^{\oplus}\underline{H}_2$, $CO_2\underline{H}$) - 1H à 8,45 ppm (D, J = 9 Hz, $CON\underline{H}$) - 1H à 6,85 ppm (S, $\underline{H}$ thiazole) - 1H à 6,00 ppm (M, $\underline{H}_7$) - 1H à 5,00 ppm (D, J = 4 Hz, $\underline{H}_6$) - 1H à (A de AB, $J_{AB}$ = 13 Hz, $C\underline{H}_2OCO$) - 1H à 4,65 ppm (B de AB, $J_{AB}$ = 13 Hz, $C\underline{H}_2OCO$) - 1H à 3,85 ppm (A de AB, $J_{AB}$ = 17 Hz, $C\underline{H}_2SO$) - 1H à 3,60 ppm (B de AB, $J_{AB}$ = 17 Hz, $C\underline{H}_2SO$) - 4H à 2,85 ppm (M, $C\underline{H}_2NHCH_2$) - 2H à 2,40 ppm (M,
$C\underline{H}_2CO$)

||
O

- 2H à 1,80 ppm (M, $CH_2C\underline{H}_2CH_2NH$) - 6H à 1,45 ppm (S, $(C\underline{H}_3)_2C$) - 3H à 1,10 ppm (T, J = 7 Hz, $C\underline{H}_3CH_2NH$).

||
O

**RMN n° 43 - (a):**

2H à 8,50 ppm (S.e., $N^{\oplus}\underline{H}_2$) - 1H à 8,40 ppm (D, J = 9 Hz, $CON\underline{H}$) - 3H à 7,00 ppm (S.e., $N^{\oplus}\underline{H}_3$) - 1H à 6,76 ppm (S, $\underline{H}$ thiazole) - 1H à 5,95 ppm (M, $\underline{H}_7$) - 1H à 5,10 ppm (A de AB, $J_{AB}$ = 13 Hz, $C\underline{H}_2OCO$) - 1H à 5,0 ppm (D, J = 4 Hz, $\underline{H}_6$) - 1H à 4,65 ppm (B de AB, $J_{AB}$ = 13 Hz, $C\underline{H}_2OCO$) - 1H à 3,85 ppm (A de AB, $J_{AB}$ = 17 Hz, $C\underline{H}_2SO$) - 1H à 3,50 ppm B de AB, $J_{AB}$ = 17 Hz, $C\underline{H}_2SO$) - 1H à 3,30 ppm (M, $C\underline{H}NH$) - 5H à 2,45 ppm (M, $C\underline{H}_3NH$ et
$C\underline{H}_2CO$)

||
O

- 2H à 1,80 ppm (M, $C\underline{H}_2CH_2$
CO)
||

- 6H à 1,45 ppm (S, $(C\underline{H}_3)_2C$) - 3H à 1,10 ppm (D, J = 7 Hz, $C\underline{H}_3CH$).
||

**RMN n° 44 - (a):**

1H à 8,40 ppm (D, J = 9 Hz, $CON\underline{H}$) - 7H à 7,80 ppm (S.e., $N^{\oplus}H_3$, $CO_2\underline{H}$) - 1H à 6,80 ppm (S, $\underline{H}$ thiazole) - 1H à 6,00 ppm (M, $\underline{H}_7$) - 1H à 5,00 ppm (M, $\underline{H}_6$) - 1H à 5,00 ppm (A de AB, $J_{AB}$ = 13 Hz, $C\underline{H}_2OCO$) - 1H à 4,65 ppm (B de AB, $J_{AB}$ = 13 Hz, $C\underline{H}_2OCO$) - 1H à 3,85 ppm (A de AB, $J_{AB}$ = 17 Hz, $C\underline{H}_2SO$) - 1H à 3,55 ppm (B de AB, $J_{AB}$ = 17 Hz, $C\underline{H}_2SO$) - 2H à 2,80 ppm (M, $C\underline{H}_2NH$) - 6H à 2,40 ppm (M, $C\underline{H}_3NH$, $C\underline{H}_2CO_2$) - 12H à 1,45 ppm (S.e., $(C\underline{H}_3)_2C$ et $CH_2(C\underline{H}_2)_3CH_2NH$).

**RMN n°45 - (b):**

1H à 8,75 ppm (D, J = 9 Hz, $CON\underline{H}$) - 1H à 8,60 ppm (S.e., $N^{\oplus}\underline{H}_2$) - 1H à 8,40 ppm (S.e., $N^{\oplus}\underline{H}_2$) - 3H à 7,30 ppm (S.e., $N^{\oplus}\underline{H}_3$) - 1H à 6,78 ppm (S, $\underline{H}$ thiazole) - 1H à 5,91 ppm (D de D, $J_1$ = 9 Hz, $J_2$ = 4 Hz, $\underline{H}_7$) - 1H à 5,13 ppm (D, J = 13 Hz, $C\underline{H}_2OCO$) - 1H à 4,95 ppm (D, J = 4 Hz, $\underline{H}_6$) - 1H à 4,61 ppm (D, J = 13 Hz, $C\underline{H}_2OCO$) - 2H à 4,55 ppm (S, $C\underline{H}_2$ ON) - 1H à 3,85 ppm (A de AB, $J_{AB}$ = 17 Hz, $C\underline{H}_2SO$) - 1H à 3,55 ppm (B de AB, $J_{AB}$ = 17 Hz, $C\underline{H}_2SO$) - 2H à 3,20 ppm (M, $C\underline{H}_2NH$) - 2H à 2,90 ppm (M, $C\underline{H}_2NH$) - 1H à 2,64 ppm (M, $C\underline{H}CO_2$) - 2H à 1,95 ppm (M, $C\underline{H}_2CH_2NH$) - 2H à 1,66 ppm (M, $C\underline{H}_2CH_2NH$).

**RMN n°46 - (b):**

1H à 8,75 ppm (D, J = 9 Hz, CON$\underline{H}$) - 1H à 8,60 ppm (S.e., $N^{\oplus}\underline{H_2}$) - 1H à 8,40 ppm (S.e., $N^{\oplus}\underline{H_2}$) - 3H à 7,30 ppm (S.e., $N^{\oplus}\underline{H_3}$) - 1H à 6,78 ppm (S, $\underline{H}$ thiazole) - 1H à 5,94 ppm (D de D, $J_1$ = 9 Hz, $J_2$ = 4 Hz, $\underline{H_7}$) - 1H à 5,13 ppm (D, J = 13 Hz, $C\underline{H_2}OCO$) - 1H à 4,95 ppm (D, J = 4 Hz, $\underline{H_6}$) - 1H à 4,61 ppm (D, J = 13 Hz, $CH_2OCO$) - 1H à 3,90 ppm (A de AB, J = 17 Hz, $C\underline{H_2}SO$) - 1H à 3,55 ppm (B de AB, J = 17 Hz, $C\underline{H_2}SO$) - 2H à 3,20 ppm (M, $C\underline{H_2}$ NH) - 2H à 2,90 ppm (M, $CH_2NH$) - 1H à 2,64 ppm (M, $C\underline{H}CO_2$) - 4H à 2,40 ppm (M,

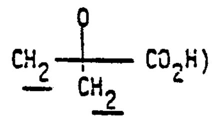

- 6H entre 1,5 et 2 ppm

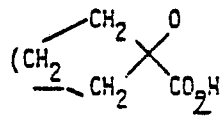

et $C\underline{H_2}$-$CH_2NH$).

**RMN n° 47 - (b):**

1H à 8,79 ppm (D, J = 9 Hz, CON$\underline{H}$) - 3H à 8,30 ppm (S.e., $N^{\oplus}\underline{H_3}$) - 2H à 7,97 ppm (D, J = 8 Hz, $\underline{H}$ ortho CO) - 2H à 7,55 ppm (D, J = 8 Hz, $\underline{H}$ méta CO) - 3H à 7,30 ppm (S.e., $N^{\oplus}\underline{H_3}$) - 1H à 6,84 ppm (S, $\underline{H}$ thiazole) - 1H à 5,92 ppm (D de D, $J_1$ = 9 Hz, $J_2$ = 4 Hz, $\underline{H_7}$) - 1H à 5,40 ppm (D, J = 13 Hz, $C\underline{H_2}OCO$) - 1H à 4,95 ppm (D, J = 4 Hz, $\underline{H_6}$) - 1H à 4,84 ppm (D, J = 13 Hz, $CH_2OCO$) - 2H à 4,56 ppm (S, $C\underline{H_2}ON$) - 1H à 4,08 ppm (M, $C\underline{H_2}$ $NH_2$) - 1H à 4,00 ppm (A de AB, $J_{AB}$ = 17 Hz, $C\underline{H_2}SO$) - 1H à 3,71 ppm (B de AB, $J_{AB}$ = 17 Hz, $C\underline{H_2}SO$).

**RMN n° 48 - (b):**

1H à 8,75 ppm (D, J = 9 Hz, CON$\underline{H}$) - 3H à 8,30 ppm (S.e., $N^{\oplus}\underline{H_3}$) - 2H à 7,95 ppm (D, J = 8 Hz, $\underline{H}$ ortho CO) - 2H à 7,55 ppm (D, J = 8 Hz, $\underline{H}$ méta CO) - 3H à 7,30 ppm (S.e., $N^{\oplus}\underline{H_3}$) - 1H à 6,78 ppm (S, $\underline{H}$ thiazole) - 1H à 5,95 ppm (D de D, $J_1$ = 9 Hz, $J_2$ = 4 Hz, $\underline{H_7}$) - 1H à 4,44 ppm (D, J = 13 Hz, $C\underline{H_2}OCO$) - 1H à 4,98 ppm (D, J = 4 Hz, $\underline{H_6}$) -1H à 4,81 ppm (D, J = 13 Hz, $C\underline{H_2}OCO$) - 1H à 4,10 ppm (S.e., $C\underline{H_2}NH_2$) - 1H à 4,05 ppm (A de AB, $J_{AB}$ = 17 Hz, $C\underline{H_2}SO$ - 1H à 3,71 ppm (B de AB, $J_{AB}$ = 17 Hz, $C\underline{H_2}SO$) - 4H à 2,40 ppm

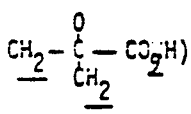

- 2H à 1,85 ppm

(M, 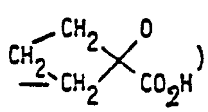).

**RMN n° 49 - (b):**

1H à 8,75 ppm (D, J = 9 Hz, CON$\underline{H}$) - 3H à 7,70 ppm (S.e., $N^{\oplus}\underline{H_3}$) - 3H à 7,30 ppm (S.e., $N^{\oplus}\underline{H_3}$ - 1H à 6,82 ppm (S, $\underline{H}$ thiazole) - 1H à 5,90 ppm (D de D, $J_1$ = 9 Hz, $J_2$ = 4 Hz, $\underline{H_7}$) - 1H à 5,10 ppm (D, J = 13 Hz, $CH_2OCO$) - 1H à 4,95 ppm (D, J = 4 Hz, $\underline{H_6}$) - 1H à 4,56 ppm (D, J = 13 Hz, $C\underline{H_2}CCO$) - 2H à 4,53 ppm (S, $CH_2ON$) - 1H à 3,84 ppm (A de AB, $J_{AB}$ = 17 Hz, $C\underline{H_2SO}$) - 1H à 3,55 ppm (B de AB, $J_{AB}$ = 17 Hz, $C\underline{H_2}SO$) - 2H à 2,76 ppm (M, $C\underline{H_2}NH_2$) - 2H à 2,40 ppm (M, $C\underline{H_2}CO_2$) - 2H à 1,72 ppm (M, $CH_2C\underline{H_2}CH_2NH_2$).

**RMN n° 50 - (b):**

1H à 8,75 ppm (D, J = 9 Hz, CONH) - 3H à 7,75 ppm (S.e., $N^{\oplus}H_3$) - 3H à 7,25 ppm (S.e., $N^{\oplus}H_3$) - 1H à 6,77 ppm (S, H thiazole) - 1H à 5,94 ppm (D de D, $J_1$ = 9 Hz, $J_2$ = 4 Hz, $H_7$) - 1H à 5,11 ppm (D, J = 13 Hz, $CH_2OCO$) - 1H à 4,95 ppm (D, J = 4 Hz, $H_6$) - 1H à 4,63 ppm (D, J = 13 Hz, $CH_2OCO$) - 1H à 3,89 ppm (A de AB, J = 17 Hz, $CH_2SO$) - 1H à 3,55 ppm (B de AB, J = 17 Hz, $CH_2SO$) - 2H à 2,78 ppm (M, $CH_2NH_2$) - 6H à 2,40 ppm

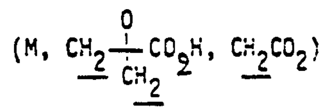

- 4H à 1,85 ppm

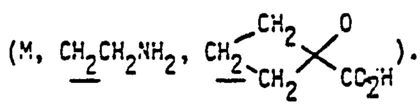

**RMN n° 51 - (b):**

1H à 8,75 ppm (D, J = 9 Hz, CONH) - 3H à 7,60 ppm (S.e., $N^{\oplus}H_3$) - 3H à 7,30 ppm (S.e., $^{\oplus}NH_3$) - 1H à 6,82 ppm (S, H thiazole) - 1H à 5,87 ppm (D de D, $J_1$ = 9 Hz, $J_2$ = 4 Hz, $H_7$) - 1H à 5,61 ppm (D, J = 13 Hz, $CH_2OCO$) - 1H à 4,92 ppm (D, J = 4 Hz, $H_6$) - 1H à 4,58 ppm (D, J = 13 Hz, $CH_2OCO$) - 2H à 4,57 ppm (S, $CH_2ON$) - 1H à 3,81 ppm (A de AB, J = 13 Hz, $CH_2SO$) - 1H à 3,55 ppm (B de AB, J = 13 Hz, $CH_2SO$) - 2H à 2,75 ppm (M, $CH_2$ $NH_2$) - 2H à 2,31 ppm (M, $CH_2CO_2$) - 4H à 1,50 ppm (M, $CH_2(CH_2)_2CH_2N$).

**RMN n° 52 - (b):**

1H à 8,75 ppm (D, J = 9 Hz, CONH) - 3H à 7,70 ppm (S.e., $N^{\oplus}H_3$) - 3H à 7,30 ppm (S.e. $N^{\oplus}H_3$) - 1H à 6,78 ppm (S, H thiazole) - 1H à 5,92 ppm (D de D, $J_1$ = 9 Hz, $J_2$ = 4 Hz, $H_7$) - 1H à 5,13 ppm (D, J = 13 Hz, $CH_2OCO$) - 1H à 4,95 ppm (D, J = 4 Hz, $H_6$) - 1H à 4,58 ppm (D, J = 13 Hz, $CH_2OCO$) - 1H à 3,86 ppm (A de AB, J = 17 Hz, $CH_2SO$) - 1H à 3,55 ppm (B de AB, J = 17 Hz, $CH_2SO$) - 2H à 2,74 ppm (M, $CH_2NH_2$) - 6H à 2,40 ppm

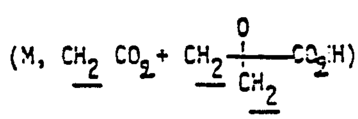

- 2H à 1,85 ppm

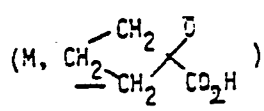

- 4H à 1,50 ppm (M, $CH_2CH_2CH_2CO_2$).

**RMN n° 53 - (b):**

1H à 8,80 ppm (D, J = 9 Hz, CONH) - 3H à 8,40 ppm (S.e., $N^{\oplus}H_3$) - 3H à 7,40 ppm (S.e., $N^{\oplus}H_3$) - 1H à 6,78 ppm (S, H thiazole) - 1H à 5,95 ppm (D de D, $J_1$ = 9 Hz. $J_2$ = 4 Hz, $H_7$ - 1H à 5,25 ppm (D, J = 13 Hz, $CH_2OCO$) - 1H à 4,95 ppm (D, J = 4 Hz, $H_6$) - 1H à 4,78 ppm (D, J = 13 Hz, $CH_2OCO$) - 1H à 4,08 ppm (M, $CHNH_2$) - 1H à 3,95 ppm (A de AB, J = 17 Hz, $CH_2SO$) - 1H à 3,60 ppm (B de AB, J = 17 Hz, $CH_2SO$) - 4H à 2,40 ppm

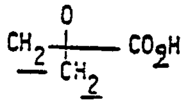 )

- 2H à 1,80 ppm

(M, 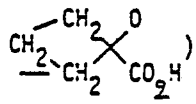 )

- 3H à 1,36 ppm (D, J = 7 Hz, $C\underline{H}_3CH$).

**RMN n° 54 - (b):**

1H à 8,75 ppm (D, J = 9 Hz, CON$\underline{H}$) - 3H à 7,87 ppm (S.e., $N^{\oplus}\underline{H}_3$) - 3H à 7,30 ppm (S.e., $N^{\oplus}\underline{H}_3$) - 1H à 6,78 ppm (S, $\underline{H}$ thiazole) - 1H à 5,95 ppm (D de D, $J_1$ = 9 Hz, $J_2$ = 4 Hz, $\underline{H}_7$) - 1H à 5,19 ppm (2D, J = 13 Hz, $C\underline{H}_2OCO$)* - 1H à 4,95 ppm (D, J = 4 Hz, $\underline{H}_6$) - 1H à 4,64 ppm (2D, J = 13 Hz, $C\underline{H}_2OCO$)* - 1H à 3,92 ppm (A de AB, J = 17 Hz, $C\underline{H}_2SO$) - 1H à 3,55 ppm (B de AB, J = 17 Hz, $C\underline{H}_2SO$) - 1H à 3,0 ppm (M, $C\underline{H}CO_2$) - 2H à 2,80 ppm (M, $C\underline{H}_2NH_2$) - 4H à 2,40 ppm

(M, 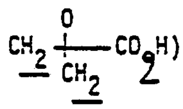 )

- 2H à 1,80 ppm

(M, 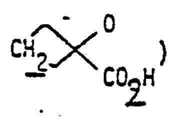 )

- 3H à 1,08 ppm (D, J = 7 Hz, $C\underline{H}_3CH$).

**RMN n° 55 - (b):**

1H à 8,75 ppm (D, J = 9 Hz, CON$\underline{H}$) - 3H à 7,80 ppm (S.e., $N^{\oplus}\underline{H}_3$) - 3H à 7,30 ppm (S.e., $N^{\oplus}\underline{H}_3$) - 1H à 6,80 ppm (S, $\underline{H}$ thiazole) - 1H à 5,95 ppm (M, $\underline{H}_7$) - 1H à 5,20 ppm (D, J = 13 Hz, $C\underline{H}_2OCO$) - 1H à 4,95 ppm (S.e., $\underline{H}_6$) - 1H à 4,63 ppm (D, J = 13 Hz, $C\underline{H}_2OCO$) - 1H à 3,90 ppm (A de AB, J = 17 Hz, $C\underline{H}_2SO$) - 1H à 3,58 ppm (B de AB, J = 17 Hz, $C\underline{H}_2SO$) 2H à 2,93 ppm (M, $C\underline{H}_2NH_2$) - 4H à 2,40 ppm

(M, 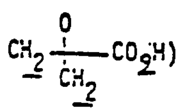 )

- 2H à 1,90 ppm (M,

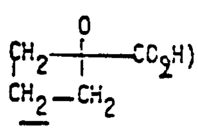 )

- 6H à 1,14 ppm (S, $(C\underline{H}_3)_2C$).

**RMN n° 56 - (b):**

1H à 8,75 ppm (D, J = 9 Hz, CON$\underline{H}$) - 3H à 7,75 ppm (S.e., N$^{\oplus}\underline{H}_3$) - 3H à 7,35 ppm (S.e., N$^{\oplus}\underline{H}_3$) - 1H à 6,80 ppm (S, $\underline{H}$ thiazole) - 1H à 5,95 ppm (D de D, $J_1$ = 9 Hz, $J_2$ = 4 Hz, $\underline{H}_7$) - 1H à 5,15 ppm (2D, J = 13 Hz, $C\underline{H}_2OCO$)* - 1H à 4,95 ppm (D, J = 4 Hz, $\underline{H}_6$) - 1H à 4,60 ppm (2D, J = 13 Hz, $C\underline{H}_2OCO$)* - 1H à 3,90 ppm (A de AB, J = 17 Hz, $C\underline{H}_2SO$) - 1H à 3,56 ppm (B de AB, J = 17 Hz, $C\underline{H}_2SO$) - 2H à 2,74 ppm (M, $C\underline{H}_2NH_2$) - 1H à 2,50 (M, $C\underline{H}CO_2$) - 4H à 2,40 ppm

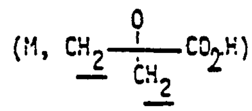

- 3H à 1,90 ppm

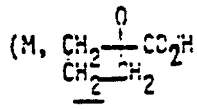

et $C\underline{H}_2CH_2NH_2$) - 1H à 1,55 ppm (M, $C\underline{H}_2CH_2NH_2$) - 3H à 1,08 ppm (D, J = 7 Hz, $C\underline{H}_3CH$).

**RMN n° 57 - (b):**

1H à 8,80 ppm (D, H = 9 Hz, CON$\underline{H}$) - 3H à 8,40 ppm (S.e., N$^{\oplus}\underline{H}_3$) - 3H à 7,40 ppm (S.e., N$^{\oplus}\underline{H}_3$) - 1H à 6,79 ppm (S, $\underline{H}$ thiazole) - 1H à 5,95 ppm (D de D, $J_1$ = 9 Hz, $J_2$ = 4 Hz, $\underline{H}_7$) - 1H à 5,28 ppm (D, J = 13 H; $C\underline{H}_2OCO$) - 1H à 4,95 ppm (D, J = 4 Hz, $\underline{H}_6$) - 1H à 4,75 ppm (D, J = 13 Hz, $C\underline{H}_2OCO$) - 1H à 3,95 ppm (A de AB, J = 17 Hz, $C\underline{H}_2SO$) - 1H à 3,61 ppm (B de AB, J = 17 Hz, $C\underline{H}_2SO$) - 4H à 2,40 ppm

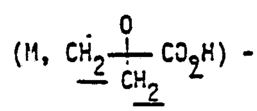

2H à 2,10 ppm (M, $C\underline{H}_2$ cyclopentane) - 2H à 1,90

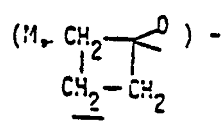

- 6H à 1,80 ppm (M, $C\underline{H}_2$ cyclopentane).

**RMN n° 58 - (b):**

1H à 8,75 ppm (D, J = 9 Hz, CON$\underline{H}$) - 3H à 2,70 ppm (S.e., N$^{\oplus}\underline{H}_3$) - 3H à 7,30 ppm (S.e., N$^{\oplus}\underline{H}_3$) - 1H à 6,84 ppm (S, $\underline{H}$ thiazole) - 1H à 5,89 ppm (D de D, $J_1$ = 9 Hz, $J_2$ = 4 Hz, $\underline{H}_7$ - 1H à 5,13 ppm (D, J = 13 Hz, $C\underline{H}_2OCO$) - 1H à 4,95 ppm (D, J = 4 Hz, $\underline{H}_6$) - 3H à 4,55 ppm (M, $C\underline{H}_2ON$ et $C\underline{H}_2OCO$) - 1H à 3,82 ppm (A de AB, J = 17 Hz, $C\underline{H}_2SO$ - 1H à 3,55 ppm (B de AB, J = 17 Hz, $C\underline{H}_2SO$) - 2H à 2,60 ppm (M, $CH_2NH_2$) - 1H à 2,21 ppm (M, $C\underline{H}CO_2$) - 4H à 1,80 ppm, (M, $C\underline{H}_2$ cyclohexane) - 1H à 1,45 ppm (M, $\underline{C}HCH_2NH_2$) - 2H à 1,25 ppm (M, $C\underline{H}_2$ cyclohexane) - 2H à 0,90 ppm (M, $C\underline{H}_2$ cyclohexane).

**RMN n° 59 - (b):**

1H à 8,67 ppm (D, J = 9 Hz, CON$\underline{H}$) - 3H à 7,70 ppm (S.e., N$^{\oplus}\underline{H}_3$) - 3H à 7,30 ppm (S.e., N$^{\oplus}\underline{H}_3$) - 1H à 6,82 ppm (S, $\underline{H}$ thiazole) - 1H à 5,94 ppm (D de D, $J_1$ = 9 Hz, $J_2$ = 4 Hz, $\underline{H}_7$) - 1H à 5,13 ppm (D, J = 13 Hz, $C\underline{H}_2OCO$) - 1H à 4,95 ppm (D, J = 4 Hz, $\underline{H}_6$) - 1H à 4,56 ppm (D, J = 13 Hz, $C\underline{H}_2OCO$) - 1H à 3,87 ppm (A de AB, J = 17 Hz, $C\underline{H}_2SO$) - 2H à 3,55 ppm (B de AB, J = 17 Hz, $C\underline{H}_2SO$) - 2H à 2,60 ppm (M, $C\underline{H}_2NH_2$) - 5H entre 2,0 et 2,5 ppm, 6H entre 1,6 et 2,0 ppm, 3H entre 1,1 et 1,6 ppm, 2H à 0,90 ppm (M,

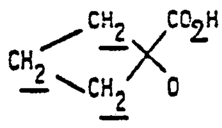 et 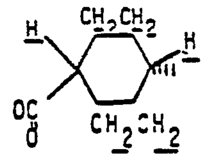

**RMN n° 60 - (b):**

1H à 8,60 ppm (2D, J = 9 Hz, CONH)* - 3H à 7,70 ppm (S.e., $N^{\oplus}H_3$) - 3H à 7,30 ppm S.e., $N^{\oplus}H_3$) - 1H à 6,82 ppm (2S, H thiazole)* - 1H à 5,95 ppm (D de D, $J_1$ = 9 Hz, $J_2$ = 4 Hz, $H_7$) - 1H à 5,13 ppm (D, J = 13 Hz, $CH_2OCO$) - 1H à 4,95 ppm (2D, $H_6$)* - 2H à 4,60 ppm (M, $CH_2OCO$ + CHON) - 1H à 3,86 ppm (2D, J = 17 Hz, $CH_2SO$)* - 1H à 3,56 ppm (2D, J = 17 Hz, $CH_2SO$)* - 2H à 2,75 ppm (M, $CH_2NH_2$) - 2H à 2,40 ppm (T, J = 7 Hz, $CH_2CO_2$) - 2H à 1,75 ppm (M, $CH_2CH_2CH_2NH_2$) - 3H à 1,39 ppm (D, J = 7 Hz, $CH_3CH$).

**RMN n° 61 - (b):**

1H à 8,70 ppm (2D, J = 9 Hz, CONH)* - 3H à 7,70 ppm (S.e., $N^{\oplus}H_3$) - 3H à 7,30 ppm S.e., $N^{\oplus}H_3$) - 1H à 6,82 ppm (2S, H thiazole)* - 1H à 5,95 ppm (D de D, $J_1$ = 9 Hz, $J_2$ = 4 Hz, $H_7$) - 1H à 5,12 ppm (D, J = 13 Hz,$CH_2OCO$) - 1H à 4,94 ppm (2D, $H_6$)* - 2H à 4,60 ppm (M, HCON, et $CH_2OCO$) - 1H à 3,86 ppm (A de AB, J = 17 Hz, $CH_2SO$)-1H à 3,55 ppm (B de AB, J = 17 Hz, $CH_2SO$) - 2H à 2,75 ppm (M, $CH_2NH_2$) - 2H à 2,31 ppm (M, $CH_2CO_2$) - 4H à 1,50 ppm (M, $CH_2CH_2CH_2CH_2NH_2$) - 3H à 1,45 ppm (D, J = 7 Hz, $CH_3CH$).

**RMN n° 62 - (b):**

2H à 8,60 ppm (M, CONH, $N^{\oplus}H_3$) - 1H à 8,40 ppm (S.e., $N^{\oplus}H_2$) - 3H à 7,30 ppm (S.e., $N^{\oplus}H_3$) - 1H à 6,82 ppm (2S, H thiazole)* - 1H à 5,95 ppm (D de D, $J_1$ = 9 Hz, $J_2$ = 4 Hz, $H_7$) - 1H à 5,14 ppm (D, J = 13 Hz, $CH_2OCO$) - 1H à 4,95 ppm (2D, $H_6$)* - 2H à 4,60 ppm (M, CH-ON, et $CH_2OCO$) - 1H à 3,88 ppm (2D, J = 17 Hz, $CH_2SO$) - 1H à 3,55 ppm (D, J = 17 Hz, $CH_2SO$) - 2H à 3,20 ppm et 2H à 2,95 ppm

(M, 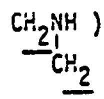 )

- 1H à 2,66 ppm (M, $CHCO_2$) - 2H à 1,95 ppm et 2H à 1,70 ppm

(M, 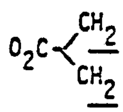 )

- 3H à 1,45 ppm (D, J = 7 Hz, $CH_3CH$).

**RMN n° 63 - (b):**

1H à 8,70 ppm (2D, J = 9 Hz, CONH)* - 3H à 8,20 ppm (S.e., $N^{\oplus}H_3$) - 2H à 7,95 ppm (D, J = Hz, H ortho $CO_2$) - 2H à 7,55 ppm (D, J = 8 Hz, H méta $CO_2$) - 3H à 7,30 ppm (S.e., $N^{\oplus}H_3$) - 1H à 6,82 ppm (2S, H thiazole)* - 1H à 5,95 ppm (D de D, $J_1$ = 9 Hz, $J_2$ = 4 Hz, $H_7$,) - 1H à 5,44 ppm (D, J = 13 Hz, $CH_2OCO$) - 1H à 4,96 ppm (2D, $H_6$)* - 1H à 4,84 ppm (D, J = 13 Hz, $CH_2OCO$) - 1H à 4,60 ppm (Q, J = 7 Hz, CHON) - 2H à 4,10 ppm (M, $CH_2NH_2$) 1H à 3,90 ppm (D, J = 17 Hz, $CH_2SO$) - 1H à 3,70 ppm (D, J = 17 Hz, $CH_2SO$) - 3H à 1,45 ppm (D, J = 7 Hz, $CH_3CH$).

**RMN n° 64 - (b):**

1H à 8,60 ppm (2D, J = 9 Hz, CONH)* - 3H à 7,70 ppm (S.e., $N^{\oplus}H_3$) - 1H à 6,80 ppm (2S, H thiazole)* - 1H à 5,95 ppm (D de D, $J_1$ = 9 Hz, $J_2$ = 4 Hz, $H_7$) - 5H entre 4 et 6 ppm (S.e., $N^{\oplus}H_3CO_2H$) - 1H à 5,13 ppm (D, J = 13 Hz, $CH_2OCO$) - 1H à 4,95 ppm (2D, $H_6$)* - 2H à 4,60 ppm (M, $CH_2OCO$ et CHON) - 1H à 3,86 ppm (A de AB, J = 17 Hz, $CH_2$ SO) - 1H à 3,55 ppm (B de AB, J = 17 Hz, $CH_2SO$) - 2H à 2,61 ppm (M, $CH_2NH_2$) 1H à 2,21 ppm (M, $CHCO_2$) - 4H à 1,80 ppm

(M, 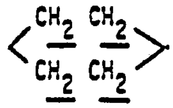 )

- 3H à 1,45 ppm (D, J = 7 Hz, $CH_3CH$) - 2H à 1,25 ppm et 2H à 0,90 ppm

(M, 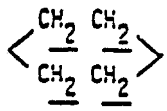 ).

**RMN n° 65 - (b):**

1H à 8,66 ppm (D, J = 9 Hz, CONH) - 3H à 8,15 ppm (S.e., $CH_2N^{\oplus}H_3$) - 1H à 8,05 ppm (S, HAr 2') - 1H à 7,94 ppm (D, J = 7 Hz, HAr 6') - 1H à 7,71 ppm (D, J = 7 Hz, HAr 4') - 1H à 7,55 ppm (T, J = 7 Hz, HAr 5') - 1H 6,95 ppm (S, H thiazole) - 1H à 6,00 ppm (D de D, $J_1$ = 9 Hz, $J_2$ = 4 Hz, $H_7$) 1H à 5,45 ppm (D, J = 13 Hz, $CH_2OCO$) - 1H à 5,00 ppm (D, J = 4 Hz, $H_6$) - 1H à 4,87 ppm (D, J = 13 Hz, $CH_2OCO$) - 3H à 4,05 ppm (M, $CH_2N^{\oplus}H_3$ et $CH_2SO$) - 1H à 3,75 ppm (D, J = 17 Hz, $CH_2SO$) - 3H à 1,48 ppm (S, $CH_3$) - 3H à 1,47 ppm (S, $CH_3$).

**RMN n° 66 - (b):**

1H à 8,80 ppm (D, J = 9 Hz, CONH) - 3H à 8,05 ppm (S.e., $CH_2N^{\oplus}H_3$) - 1H à 6,95 ppm (S, H thiazole) - 1H à 6,00 ppm (D de D, $J_1$ = 9 Hz, $J_2$ = 4 Hz, $H_7$) - 1H à 5,20 ppm (2D superposés, J = 13 Hz, $CH_2OCO$) - 1H à 5,10 ppm (2D, J = 4 Hz, $H_6$) - 1H à 4,56 ppm (2D superposés, J = 13 Hz, $CH_2OCO$) - 4H à 3,90 ppm (M, $CH_2SO$ et $CH_2N^{\oplus}H_3$) - 3H à 2,80 ppm (M, $CHCO_2$, $CHCH_2N^{\oplus}H_3$, et H Eq en α du $CO_2$) = 1H à 2,00 ppm (M, HAx en α du $CO_2$) - 6H à 1,50 ppm (2S $(CH_3)_2C$) - 6H entre 1,20 et 1,80 ppm (M, $3CH_2$ restant du cyclohexane).

**RMN n° 67 - (b):**

3H à 8,50 ppm (M, $N^{\oplus}H_2$ et CONH) - 2H à 7,50 ppm (S.e., $NH_2$ thiazole) - 1H à 6,79 ppm (S, H thiazole) - 1H à 5,99 ppm (D de D, $J_1$ = 9 Hz, $J_2$ = 4 Hz, $H_7$) - 1H à 5,16 ppm (2D, J = 13 Hz, $CH_2OCO$) - 1H à 4,95 ppm (D, J = 4 Hz, $H_6$) - 1H à 4,66 ppm (D, J = 13 Hz, OCO) - 1H à 3,92 ppm (D, J = 17 Hz, $CH_2SO$) - 1H à 3,58 ppm (D, J = 17 Hz, $CH_2SO$) - 1H à 3,36 ppm (M, $CHN^{\oplus}H_2$) - 1H à 3,25 ppm (M, $CH_2$ α $N^{\oplus}H_2$) - 1H à 2,95 ppm (M, $CH_2$ α $N^{\oplus}H_2$) - 2H à 2,60 ppm (2D superposés $CH_2$) $CO_2$) - 3H à 1,70 ppm (M, H pipéridine) - 6H à 1,48 ppm (2S $(CH_3)_2C$) - 3H à 1,45 ppm (M, H pipéridine).

**RMN n° 68 - (b):**

1H à 8,60 ppm (S.e., $N^{\oplus}H_2$ pipéridine) - 1H à 8,45 ppm (S.e., $NH_2^{\oplus}$ pipéridine) - 1H à 8,45 ppm (D, J = 9 Hz, CONH) - 1H à 7,50 ppm (S.e., $NH_2$ thiazole)-1H à 6,70 ppm (S, H thiazole) - 1H à 5,97 ppm (D de D, $J_1$ = 9 Hz, $J_2$ = 4 Hz, $H_7$) - 1H à 5,10 ppm (D, J = 13 Hz, $CH_2OCO$) - 1H à 4,97 ppm (D, J = 4 Hz, $H_6$) 1H à 4,63 ppm (D, J = 13 Hz) - $CH_2OCO$) - 1H à 3,90 ppm (D, J = 17 Hz, $CH_2SO$) - 1H à 3,57 ppm, (D, J = 17 Hz, $CH_2SO$) - 2H à 3,20 ppm (M, $CH_2$ α $N^{\oplus}H_2$) - 2H à 2,70 ppm (M, $CH_2$ α $N^{\oplus}H_2$) - 2H à 2,30 ppm (D, J = 7 Hz, $CH_2CO_2$) - 1H à 2,05 ppm (M, $CHCH_2CO_2$) - 3H à 1,55 ppm (M, $CH_2$ pipéridine) - 6H à 1,48 ppm (2S, $(CH_3)_2C$) - 1H à 1,20 ppm (M, $CH_2$ pipéridine).

**RMN n° 69 - (b):**

1H à 10,9 ppm (S.e., Ar NHCO) - 1H à 8,45 ppm (D, J = 9 Hz, CONH) - 4H à 8,15 ppm (2S, $CH_2N^{\oplus}H_3$ et HAr 2') - 1H à 7,81 ppm (D, J = 7 Hz, HAr 6') - 1H à 7,67 ppm (D, J = 7 Hz, HAr 4') - 1H à 7,50 ppm (T, J = 7 Hz, HAr 5') - 2H à 7,20 ppm (S.e., $NH_2$ thiazole) - 1H à 6,78 ppm (S, H thiazole) - 1H à 6,00 ppm (D de D $J_1$ = 9 Hz, $J_2$ = 4 Hz, $H_7$) - 1H à 5,45 ppm (D, J = 13 Hz, $CH_2OCO$) - 1H à 5,00 ppm (D, J = 4 Hz, $H_6$) - 1H à 4,84 ppm (D, J = 13 Hz, $CH_2OCO$) - 1H à 4,00 ppm (D, J = 17 Hz, $CH_2SO$) - 1H à 3,75 ppm
(M, - C - $CH_2N^{\oplus}H_3$ et $CH_2SO$) - 6H à 1,47 ppm (2S, $(CH_3)_2C$)

||

O

**RMN n° 70 - (b):**

1H à 8,70 ppm (D, J = 9 Hz, CONH) - 3H à 8,50 ppm (S.e., $CH_2$ $N^{\oplus}H_3$) - 1H à 8,05 ppm (S, HAr 2') - 1H à 7,80 ppm (D, J = 7 Hz, HAr 6') - 1H à 7,34 ppm (D, J = 7 Hz, HAr 5') - 1H à 6,95 ppm (S, H thiazole) - 1H à 6,00 ppm (D de D, $J_1$ = 9 Hz, $J_2$ = 4 Hz, $H_7$) - 1H à 5,45 ppm (D, J = 13 Hz, $CH_2OCO$) - 1H à 5,05 ppm (D, J = 4 Hz, $H_6$) - 1H à 4,82 ppm (D, J = 13 Hz, $CH_2OCO$) - 3H à 4,05 ppm (M, Ar $CH_2N^{\oplus}H_3$ et $CH_2SO$) - 1H à 3,80 ppm (D, J = 17 Hz, $CH_2SO$) - 3H à 2,37 ppm (S, $CH_3$ Ar) - 6H à 1,48 ppm (2S, $(CH_3)_2C$).

**RMN n° 71 - (b):**

1H à 8,70 ppm (D, J = 9 Hz, CONH) - 9H à 8,50 ppm (S.e., $CH_2N^{\oplus}H_3$) - 2H à 7,78 ppm (M, HAr 2', 6') - 1H à 7,50 ppm (D, J = 7 Hz, HAr 5') - 1H à 6,92 ppm (S, H thiazole) - 1H à 6,00 ppm (D de D, $J_1$ = 9 Hz, $J_2$ = 4 Hz, $H_7$) - 1H à 5,45 ppm (D, J = 13 Hz, $CH_2OCO$) - 1H à 5,00 ppm (D, J = 4 Hz, $H_6$) - 1H à 4,81 ppm (D, J = 13 Hz, $CH_2OCO$) - 3H à 4,05 ppm (M Ar $CH_2N^{\oplus}H_3$ et $CH_2SO$) - 1H à 3,75 ppm (D, J = 17 Hz, $CH_2SO$) - 3H à 2,36 ppm (S, $CH_3Ar$) - 6H à 1,47 ppm (2S, $(CH_3)_2C$).

**RMN n° 72 - (b):**

1H à 8,70 ppm (D, J = 9 Hz, CONH) - 2H à 7,95 ppm (D, J = 8 Hz, HAr 2', 6') - 2H à 7,67 ppm (D, J = 8 Hz, HAr 3', 5') - 1H à 6,95 ppm (5, H thiazole) - 1H à 6,00 ppm (D de D, $J_1$ = 9 Hz, $J_2$ = 4 Hz, $H_7$) - 1H à 5,45 ppm (D, J = 13 Hz, $CH_2OCO$) - 1H à 5,00 ppm (D, J = 4 Hz, $H_6$) - 1H à 4,82 ppm (D, J = 13 Hz, $CH_2OCO$) - 2H à 4,16 ppm (M, Ar $CH_2NHCH_3$) - 1H à 4,08 ppm (D, J = 17 Hz, $CH_2SO$) - 1H à 3,78 ppm (D, J = 17 Hz, $CH_2SO$) - 3H à 2,43 ppm (S, $CH_3N$) - 6H à 1,48 ppm (2S, $(CH_3)_2C$).

**RMN n° 73 - (b):**

1H à 8,70 ppm (D, J = 9 Hz, CONH) - 2H à 7,95 ppm (D, J = 8 Hz, HAr 2',6') - 2H à 7,67 ppm (D, J = 8 Hz, HAr 3', 5') - 1H à 6,92 ppm (S, H thiazole) - 1H à 6,00 ppm (D de D, $J_1$= 9 Hz, $J_2$ = 4 Hz, $H_7$) - 1H à 5,43 ppm (D, J = 13 Hz, $CH_2OCO$) - 1H à 5,00 ppm (D, J = 4 Hz, $H_6$) - 1H à 4,82 ppm (D, J = 13 Hz, $CH_2OCO$) - 2H à 4,18 ppm (M, $CH_2Ar$) - 1H à 4,10 ppm (D, J = 17 Hz, $CH_2SO$) - 1H à 3,76 ppm (D, J = 17 Hz, $CH_2SO$) - 2H à 2,90 ppm (M, $CH_3CH_2NH$) - 6H à 1,47 ppm (2S, $(CH_3)_2C$) - 3H à 1,16 ppm (T, J = 7 Hz, $CH_3CH_2$).

**RMN n° 74 - (b):**

1H à 8,70 ppm (D, J = 9 Hz, CONH) - 2H à 7,95 ppm (D, J = 8 Hz, H Ar 2', 6') - 2H à 7,67 ppm (D, J = 8 Hz, HAr 3', 5') - 1H à 6,95 ppm (S, H thiazole) - 1H à 6,00 ppm (D de D, $J_1$ = 9 Hz, $J_2$ = 4 Hz, $H_7$) - 1H à 5,45 ppm (D, J = 13 Hz, $CH_2OCO$) - 1H à 5,00 ppm (D, J = 4 Hz, $H_6$) - 1H à 4,82 ppm (D, J = 13 Hz, $CH_2OCO$) - 2H à 4,16 ppm (M, Ar $CH_2N$ Pr) - 1H à 4,08 ppm (D, J = 17 Hz, $CH_2SO$) - 1H à 3,78 ppm (D, J = 17 Hz, $CH_2SO$) - 1H à 3,20 ppm (M, NH-CH $(CH_3)_2$) - 6H à 1,48 ppm (2S, $(CH_3)_2C$) - 6H à 1,26 ppm (D, J = 7 Hz, $(CH_3)_2CH$).

**RMN n° 75 - (b):**

1H à 8,62 ppm (D, J = 9 Hz, CONH) - 3H à 8,10 ppm (S.e., $CH_2N^{\oplus}H_3$) - 1H à 7,68 ppm (D, J = 7 Hz, HAr 6') - 1H à 7,55 ppm (D, J = 7 Hz, H Ar 4') - 1H à 7,34 ppm (T, J = 7 Hz, HAr 5') - 1H à 6,92 ppm (S, H thiazole) - 1H à 6,00 ppm (D de D, $J_1$ = 9 Hz, $J_2$ = 4 Hz, $H_7$) - 1H à 5,37 ppm (D, J = 13 Hz, $CH_2OCO$) - 1H à 5,00 ppm (D, J = 4 Hz, $H_6$) - 1H à 4,84 ppm (D, J = 13 Hz, $CH_2OCO$) - 3H à 4,10 ppm (M, $CH_2N^{\oplus}H_3$ et $CH_2SO$) - 1H à 3,75 ppm (D, J = 17 Hz, $CH_2SO$) - 3H à 2,40 ppm (S, $CH_3Ar$) - 6H à 1,47 ppm (2S, $(CH_3)_2C$).

**RMN n° 76 - (b)**

1H à 8,62 ppm (D, J = 9 Hz, CONH) - 3H à 8,00 ppm (S.e., $CH_2N^{\oplus}H_3$)- 1H à 7,31 ppm (D, J = 7 Hz, HAr 4') - 1H à 7,14 ppm (D, J = 7 Hz, HAr 5') - 1H à 6,90 ppm (S, H thiazole) - 1H à 6,00 ppm (D de D, $J_1$ = 9 Hz, $J_2$ = 4 Hz, $H_7$) - 1H à 5,27 ppm (D, J = 13 Hz, $CH_2OCO$) - 1H à 5,00 ppm (D, J = 4 Hz, $H_6$) - 1H à 4,92 ppm (D, J = 13 Hz, $CH_2OCO$) - 3H à 4,00 ppm (M, $CH_2N^{\oplus}H_3$ et $CH_2SO$) - 1H à 3,64 ppm (D, J = 17 Hz, $CH_2SO$) - 6H à 2,18 ppm (S, $CH_3$ - Ar) - 6H à 1,48 ppm (2S, $(CH_3)_2C$).

**RMN n° 77 (b):**

1H à 8,65 ppm (D, J = 9 Hz, CONH) - 3H à 7,90 ppm (S.e., $CH_2N^{\oplus}H_3$) - 1H à 7,00 ppm (S, HAr) - 1H à 6,90 ppm (S, H thiazole) - 1H à 6,00 ppm (D de D, $J_1$ = 9 Hz, $J_2$ = 4 Hz, $H_7$) - 1H à 5,25 ppm (D, J = 13 Hz, $CH_2OCO$) - 1H à 5,00 ppm (D, J = 4 Hz, $H_6$) - 1H à 4,92 ppm (D, J = 13 Hz, $CH_2OCO$) - 3H à 4,00 ppm (M, Ar $CH_2N^{\oplus}H_3$ et $CH_2SO$) - 1H à 3,65 ppm (D, J = 17 Hz, $CH_2SO$) - 3H à 2,31 ppm (S, $CH_3Ar$) - 3H à 2,25 ppm (S, $CH_3Ar$) - 3H à 2,14 ppm (S, $CH_3Ar$) - 6H à 1,45 ppm (2S, $(CH_3)_2C$).

**RMN n° 78 - (b):**

1H à 8,70 ppm (D, J = 9 Hz, CONH) - 3H à 8,00 ppm (S.e., $CH_2N^{\oplus}H_3$) - 1H à 7,45 ppm (D, J = 7 Hz, HAr 4') - 2H à 6,92 ppm (M, H thiazole et HAr 5') - 1H à 6,00 ppm (D de D, $J_1$ = 9 Hz, $J_2$ = 4 Hz, $H_7$) - 1H à 5,42 ppm (D, J = 13 Hz, $CH_2OCO$) - 1H à 5,00 ppm (D, J = 4 Hz, $H_6$) - 1H à 4,84 ppm (D, J = 13 H; $CH_2OCO$) - 3H à 3,95 ppm (M, $CH_2N^{\oplus}H_3$ et $CH_2SO$) - 3H à 3,78 ppm (S, $OCH_3$) - 3H à 3,73 ppm (S, $OCH_3$) - 1H à 3,58 ppm (D, J = 17 Hz, $CH_2SO$) - 6H à 1,47 ppm (2S, $(CH_3)_2C$).

**RMN n° 79 - (b):**

1H à 8,62 ppm (D, J = 9 Hz, CONH) - 5H à 8,00 ppm (M, $CH_2N^{\oplus}H_3$ et HAr 2', 6') - 1H à 7,20 ppm (D, J = 7 Hz, HAr 5') - 1H à 6,95 ppm (S, H thiazole) - 1H à 6,00 ppm (D de D, $J_1$ = 9 Hz, $J_2$ = 4 Hz, $H_7$) - 1H à 5,45 ppm (D, J = 13 Hz, $CH_2OCO$) - 1H à 5,00 ppm (D, J = 4 Hz, $H_6$) - 1H à 4,82 ppm (D, J = 13 Hz, $CH_2OCO$) - 3H à 4,00 ppm (M, $CH_2N^{\oplus}H_3$ et $CH_2SO$) - 3H à 3,88 ppm (S, $OCH_3$) - 1H à 3,72 ppm (D, J = 17 Hz, $CH_2SO$) - 3H à 1,48 ppm (S, $(CH_3)_2C$) - 3H à 1,47 ppm (S, $(CH_3)_2C$).

**RMN n° 80 - (b):**

1H g 8,66 ppm (D, J = 9 Hz, CONH) - 3H à 8,05 ppm (S.e., $CH_2N^{\oplus}H_3$) - 3H à 7,50 ppm (M, HAr) - 1H à 6,95 ppm (S, H thiazole) - 1H à 6,00 ppm (D de D, $J_1$ = 9 Hz, $J_2$ = 4 Hz, $H_7$) - 1H à 5,45 ppm (D, J = 13 Hz, $CH_2OCO$) - 1H à 5,00 ppm (D, J = 4 Hz, $H_6$) - 1H à 4,84 ppm (D, J = 13 Hz, $CH_2OCO$) - 3H à 4,00 ppm (M, $CH_2N^{\oplus}H_3$ et $CH_2SO$) - 3H à 3,86 ppm (S, $CH_3O$ - Ar) - 1H à 3,75 ppm (D, J = 17 Hz, $CH_2SO$) - 3H à 1,48 ppm (S, $(CH_3)_2C$) - 3H à 1,47 ppm (S, $(CH_3)_2C$).

**RMN n° 81 - (b):**

1H à 8,82 ppm (D, J = 9 Hz, CONH) - 3H à 8,20 ppm (S.e., $CH_2N^{\oplus}H_3$) - 1H à 8,13 ppm (S.e., HAr 2') - 1H à 7,95 ppm (D, J = 7 Hz, HAr 6') - 1H 7,69 ppm (D, J = 7 Hz, HAr 4') - 1H à 7,55 ppm (T, J = 7 Hz, HAr 5') - 1H à 6,95 ppm (S, H thiazole) - 1H à 6,00 ppm (D de D, $J_1$ = 9 Hz, $J_2$ = 4 Hz, $H_7$) - 1H à 5,48 ppm (D, J = 13 Hz, $CH_2OCO$) -

1H à 5,00 ppm (D, J = 4 Hz, $\underline{H}_6$) 1H à 4,86 ppm (D, J = 13 Hz, $C\underline{H}_2OCO$) - 3H à 4,05 ppm (M, $C\underline{H}_2N^{\oplus}H_3$ et $C\underline{H}_2SO$) - 1H à 3,75 ppm (D, J = 17 Hz, $C\underline{H}_2SO$) - 4H à 2,40 ppm

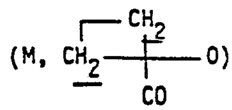

- 2H à 1,90 ppm

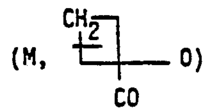

**RMN n° 82 - (b):**

1H à 8,82 ppm (D, J = 9 Hz, $CON\underline{H}$) - 3H à 8,20 ppm (S.e., $CH_2N^{\oplus}\underline{H}_3$) - 2H à 7,80 ppm (M, $\underline{H}$Ar 2', 6') - 1H à 7,45 ppm (D, J = 7 Hz, $\underline{H}$Ar 5') - 1H à 6,92 ppm (S, $\underline{H}$ thiazole) - 1H à 5,97 ppm (D de D, $J_1$ = 9 Hz, $J_2$ = 4 Hz, $\underline{H}_7$) - 1H à 5,45 ppm (D, J = 13 Hz, $C\underline{H}_2OCO$) - 1H à 5,00 ppm (D, J = 4 Hz, $\underline{H}_6$) - 1H à 4,82 ppm (D, J = 13 Hz, $C\underline{H}_2OCO$) - 3H à 4,05 ppm (M, $C\underline{H}_2N^{\oplus}H_3$ et $C\underline{H}_2SO$) - 1H à 3,75 ppm (D, J = 17 Hz, $C\underline{H}_2SO$) - 4H à 2,40 ppm

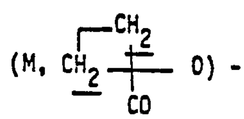

- 3H à 2,34 ppm (S, $C\underline{H}_3Ar$) - 2H à 1,90 ppm

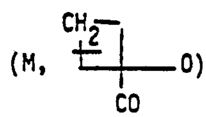

**RMN n° 83 - (b):**

1H à 8,97 ppm (D, J = 9 Hz, $CON\underline{H}$) - 2H à 8,79 ppm (S.e., $CH_2N^{\oplus}H_2$) - 2H à 8,00 ppm (D, J = 8 Hz, $\underline{H}$Ar 2', 6') - 2H à 7,58 ppm (D, J = 8 Hz, $\underline{H}$Ar 3', 5') - 1H à 6,95 ppm (S, $\underline{H}$ thiazole) - 1H à 5,97 ppm (D de D, $J_1$ = 9 Hz, $J_2$ = 4 Hz, $\underline{H}_7$) - 1H à 5,45 ppm (D, J = 13 Hz, $C\underline{H}_2OCO$) - 1H à 5,00 ppm (D, J = 4 Hz, $\underline{H}_6$) - 1H à 4,84 ppm (D, J = 13 Hz, $C\underline{H}_2OCO$) - 2H à 4,19 ppm (M, $C\underline{H}_2N^{\oplus}H_2 - CH_3$) - 1H à 4,08 ppm (D, J = 17 Hz, $C\underline{H}_2SO$) - 1H à 3,75 ppm (D, J = 17 Hz, $C\underline{H}_2SO$) - 3H à 2,50 ppm (M, $CH_2N^{\oplus}H_2 - CH_3$) - 4H à 2,40 ppm

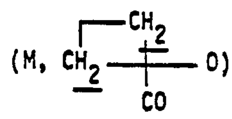

- 2H à 1,90 ppm

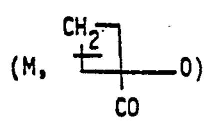

**RMN n° 84 (b):**

1H à 10,7 ppm (S, CONHAr) - 1H à 8,60 ppm (D, J = 9 Hz, CONH) - 1H à 8,20 ppm (S, HAr 2') - 1H à 7,80 ppm (D, J = 8 Hz, HAr 6') - 3H à 7,65 ppm (S.e., $CH_2N^{\oplus}H_3$) - 1H à 7,60 ppm (D, J = 8 Hz, HAr 4') - 1H à 7,45 ppm (T, J = 8 Hz, HAr 5') - 1H à 6,92 ppm (S, H thiazole) - 1H à 6,00 ppm (D de D, $J_1$ = 9 Hz, $J_2$ = 4 Hz, $H_7$) -1H à 5,45 ppm (D, J = 13 Hz, $CH_2OCO$) - 1H à 5,00 ppm (D, J = 4 Hz, $H_6$) - 1H à 4,84 ppm (D, J = 13 Hz, $CH_2OCO$) - 1H à 4,00 ppm (D, J = 17 Hz, $CH_2SO$) - 1H à 3,75 ppm (D, J = 17 Hz, $CH_2SO$) - 2H à 3,05 ppm (Q, J = 7 Hz, $CH_2CH_2N^{\oplus}H_3$) - 2H à 2,70 ppm (T, J = 7 Hz, $CH_2CH_2N^{\oplus}H_3$) - 6H à 1,47 ppm (2S, $(CH_3)_2C$).

**RMN n° 85 - (b):**

1H à 8,80 ppm (D, J = 9 Hz, CONH) - 4H à 8,20 ppm (M, $CH_2N^{\oplus}H_3$ et H thiazole en 3) - 1H a 6,95 ppm (S, H thiazole) - 1H à 6,00 ppm (D de D, $J_1$ = 9 Hz, J = 4 Hz, $H_7$) - 1H à 5,38 ppm (D, J = 13 Hz, $CH_2OCO$) - 1H à 3,98 ppm (D, J = 4 Hz, $H_6$) - 1H à 4,78 ppm (D, J = 13 Hz, $CH_2OCO$) - 1H à 3,98 ppm (D, J = 17 Hz, $CH_2SO$) - 2H à 3,82 ppm (Q, J = 7 Hz, $CH_2N^{\oplus}H_3$) - 1H à 3,66 ppm (D, J = 17 Hz, $CH_2SO$) - 6H à 1,49 ppm (2S, $(CH_3)_2C$).

**RMN n° 86 (b):**

1H à 10,75 ppm (S, Ar NHCO) - 1H à 8,70 ppm (D, J = 9 Hz, CONH) - 3H à 8,07 ppm (S.e., $CH_2N^{\oplus}H_3$) - 2H à 7,94 ppm (D, J = 8 Hz, HAr 2' 6') - 2H à 7,70 ppm (D, J = 8 Hz, HAr 3' 5') - 1H à 6,95 ppm (S, H thiazole) - 1H à 6,00 ppm (D de D, $J_1$ = 9 Hz, $J_2$ = 4 Hz, $H_7$) - 1H à 5,40 ppm (D, J = 13 Hz, $CH_2OCO$) - 1H à 4,98 ppm (D, J = 4 Hz, $H_6$) - 1H à 4,80 ppm (D, J = 13 Hz, $CH_2OCO$) - 1H à 4,05 ppm (D, J = 17 Hz, $CH_2SO$) - 3H à 3,80 ppm (M, $CH_2SO$ et $CH_2N^{\oplus}H_3$) - 6H à 1,48 ppm (2S, $(CH_3)_2C$).

**RMN n° 87 - (b):**

1H à 8,75 ppm (D, J = 9 Hz, CONH) - 1H à 8,50 ppm (S.e., $NH^{\oplus}_2$ pipéridine) - 1H à 8,40 ppm (S.e., $NH^{\oplus}_2$ pipéridine) - 2H à 7,40 ppm (S.e., $NH_2$ thiazole) - 1H à 6,78 ppm (S, H thiazole) - 1H à 5,95 ppm (D de D, $J_1$ = 9 Hz, $J_2$ = 4 Hz, $H_7$) - 1H à 5,16 ppm (2D, J = 13 Hz, $CH_2OCO$) - 1H à 4,95 ppm (D, J = 4 Hz, $H_6$) - 1H à 4,68 ppm (D, J = 13 Hz, $CH_2OCO$) - 1H à 3,92 ppm (D, J = 17 Hz, $CH_2SO$) - 1H à 3,60 ppm (D, J = 17 Hz, $CH_2SO$) - 1H à 3,40 ppm (M, $CHN^{\oplus}H_2$) - 1H à 3,25 ppm (M, $CH_2N^{\oplus}H_2$) - 1H à 2,89 ppm (M, $CH_2N^{\oplus}H_2$) - 2H à 2,60 ppm (2D superposés, J = 7 Hz, $CH_2COO$) - 4H à 2,40 ppm

(M, 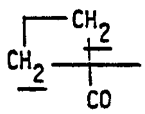 0) -

- 5H à 1,85 ppm (M, 3H de la pipéridine et

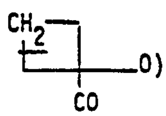 0) -

- 3H à 1,45 ppm (M, 3H pipéridine).

**RMN n° 88 - (b):**

1H à 8,80 ppm (D, J = 9 Hz, CONH) - 1H à 8,60 ppm (S.e., $N^{\oplus}H_2$ pipéridine) - 1H à 8,45 ppm (S.e., $N^{\oplus}H_2$ pipéridine) - 2H à 7,25 ppm (S.e., $NH_2$ thiazole) - 1H à 6,79 ppm (S, H thiazole) - 1H à 6,00 ppm (D de D, $J_1$ = 9 Hz, $J_2$ = 4 Hz, $H_7$) - 1H à 5,16 ppm (D, J = 13 Hz, $CH_2OCO$) - 1H à 5,00 ppm (D, J = 4 Hz, $H_6$) - 1H à 4,69 ppm (D, J = 13 Hz, $CH_2OCO$) - 1H à 3,90 ppm (D, J = 17 Hz, $CH_2SO$) - 1H à 3,60 ppm (D, J = 17 Hz, $CH_2SO$) - 2H à 3,24 ppm (M, $CH_2$ en α de $N^{\oplus}H_2$ pipéridine) - 2H à 2,70 ppm (M, $CH_2$ en α de $N^{\oplus}H_2$ pipéridine) 6H à 2,40 ppm

(M, 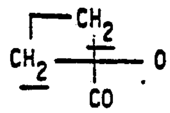

et $CH_2COO$) - 6H entre 1,5 et 2,2 ppm

(M, 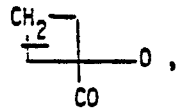

$CHCH_2CO_2$, 3H pipéridine) - 1H à 1,20 ppm (M, H pipéridine).

**RMN n° 89 - (b):**

1H à 8,80 ppm (D, J = 9 Hz, CONH) - 2H à 8,30 ppm (2S.e., $N^{\oplus}H_2$ pipéridine) - 2H à 7,40 ppm (S.e., $NH_2$ thiazole) - 1H à 6,78 ppm (S, H thiazole) - 1H à 5,92 ppm (D de D, $J_1$ = 9 Hz, $J_2$ = 4 Hz, $H_7$) - 1H à 5,14 ppm (D, J = 13 Hz, $CH_2OCO$) - 1H à 4,96 ppm (D, J = 4 Hz, $H_6$) - 1H à 4,60 ppm (D, J = 13 Hz, $CH_2OCO$) - 1H à 3,89 ppm (D, J = 17 Hz, $CH_2SO$) - 1H à 3,58 ppm (D, J = 17 Hz, $CH_2SO$) - 2H à 3,18 ppm (M H α $N^{\oplus}H_2$ pipéridine) - 2H à 2,81 ppm (M, H α $N^{\oplus}H_2$ pipéridine) - 4H à 2,40 ppm

(M, 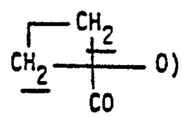

- 2H à 2,28 ppm (D, J = 7 Hz, $CH_2CO$ - O) - 5H à 1,90 ppm

(M, 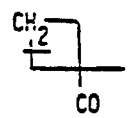

O, $CHCH_2COO$, 2H β $N^{\oplus}H_2$ pipéridine) - 2H à 1,30 ppm (M, 2H β $N^{\oplus}H_2$ pipéridine).

**RMN n° 90 - (b):**

1H à 8,45 ppm (D, J = 9 Hz, CONH) - 1H à 8,50 ppm (S.e., $NH^{\oplus}_2$ pipéridine) - 1H à 8,20 ppm (S.e., $NH_2$ pipéridine) - 2H à 7,30 ppm (S.e., $NH_2$ thiazole) - 1H à 6,77 ppm (S, H thiazole) - 1H à 5,95 ppm (D de D, $J_1$ = 9 Hz, $J_2$ = 4 Hz, $H_7$) - 1H à 5,11 ppm (D, J = 13 Hz, $CH_2OCO$) - 1H à 4,95 ppm (D, J = 4 Hz, $H_6$) - 1H à 4,61 ppm (D, J = 13 Hz, $CH_2OCO$) - 1H à 3,89 ppm (D, J = 17 Hz, $CH_2SO$) - 1H à 3,58 ppm (D, J = 17 Hz, $CH_2SO$) - 2H à 3,21 ppm (M, $CH_2$ en α $N^{\oplus}H_2$ pipéridine) - 2H à 2,81 ppm (M, $CH_2$ en α $N^{\oplus}H_2$ pipéridine) - 2H à 2,27 ppm (D, J = 7 Hz, $CH_2COO$) - 1H à 1,95 ppm (M, $CHCH_2COO$) - 2H à 1,75 ppm (M, $CH_2$ β $N^{\oplus}H_2$ pipéridine) - 6H à 1,47 ppm (2S, $(CH_3)_2C$) - 2H à 1,40 ppm (M, $CH_2$ β $N^{\oplus}H_2$ pipéridine).

**RMN n° 91 - (b):**

2H à 8,70 ppm (S.e., $CH_2N^{\oplus}H_2CH_3$) - 1H à 8,60 ppm (D, J = 9 Hz, CONH) - 1H à 8,10 ppm (S.e., HAr 2') - 1H à 7,96 ppm (D, J = 8 Hz, H Ar 6') - 1H à 7,75 ppm (D, J = 8 Hz, HAr 4') - 1H à 7,56 ppm (T, J = 8 Hz, H Ar 5')-1H à 6,92 ppm (S, H thiazole) - 1H à 6,00 ppm (D de D, $J_1$ = 9 Hz, $J_2$ = 4 Hz, $H_7$) - 1H à 5,45 ppm (D, J = 13 Hz, $CH_2OCO$) - 1H à 5,00 ppm (D, J = 4 Hz, $H_6$) - 1H à 4,87 ppm (D, J = 13 Hz, $CH_2OCO$) - 2H à 4,16 ppm (M, Ar $CH_2N^{\oplus}H_2CH_3$) - 1H à 4,05 ppm (D, J = 17 Hz, $CH_2SO$) - 1H à 3,75 ppm (D, J = 17 Hz, $CH_2SO$) - 3H à 2,50 ppm (M, $CH_3N^{\oplus}H_2CH_2$) - 6H à 1,45 ppm (2S, $(CH_3)_2C$).

**RMN n° 92 - (b):**

2H à 8,70 ppm (S.e., $CH_2N^{\oplus}\underline{H_2}CH_2CH_3$) - 1H à 8,60 ppm (D, J = 9 Hz, CON$\underline{H}$) - 1H à 8,10 ppm (S.e., $\underline{H}$Ar 2') - 1H à 7,96 ppm (D, J = 8 Hz, $\underline{H}$Ar 6') - 1H à 7,75 ppm (D, J = 8 Hz, $\underline{H}$Ar 4') - 1H à 7,56 ppm (T, J = 8 Hz, $\underline{H}$Ar 5') - 1H à 6,92 ppm (S, $\underline{H}$ thiazole) - 1H à 6,02 ppm (D de D, $J_1$ = 9 Hz, $J_2$ = 4 Hz, $\underline{H_7}$) 1H à 5,45 ppm (D, J = 13 Hz, $C\underline{H_2}OCO$) - 1H à 5,00 ppm (D, J = 4 Hz, $\underline{H_6}$) - 1H à 4,87 ppm (D, J = 13 Hz, $C\underline{H_2}OCO$) - 2H à 4,17 ppm (S.e., Ar, $C\underline{H_2}N^{\oplus}H_2$ Et) - 1H à 4,05 ppm (D, J = 17 Hz, $C\underline{H_2}SO$) - 1H à 3,75 ppm (D, J = 17 Hz, $C\underline{H_2}SO$) - 2H à 2,95 ppm (M, $CH_3C\underline{H_2}N^{\oplus}H_2$) - 6H à 1,45 ppm (2S, $(C\underline{H_3})_2C$) - 3H à 1,15 ppm (T, J = 7 Hz, $C\underline{H_3}CH_2N^{\oplus}H_2$).

**RMN n° 93 - (b):**

1H à 8,75 ppm (D, J = 9 Hz, CON$\underline{H}$) - 2H à 8,70 ppm (S.e., $CH_2$, $N^{\oplus}\underline{H_2}$-i Pr) - 1H à 8,10 ppm (S.e., $\underline{H}$Ar 2') - 1H à 7,95 ppm (D, J = 8 Hz, $\underline{H}$Ar 6') - 1H à 7,75 ppm (D, J = 8 Hz, $\underline{H}$Ar 4') - 1H à 7,58 ppm (T, J = 8 Hz, $\underline{H}$Ar 5') - 1H à 7,00 ppm (S, $\underline{H}$ thiazole) - 1H à 6,00 ppm (D de D, $J_1$ = 9 Hz, $J_2$ = 4 Hz, $\underline{H_7}$) - 1H à 5,46 ppm (D, J = 13 Hz, $C\underline{H_2}OCO$) - 1H à 5,00 ppm (D, J = 4 Hz, $\underline{H_6}$) - 1H à 4,87 ppm (D, J = 13 Hz, $C\underline{H_2}OCO$) - 2H à 4,19 ppm (M, Ar $C\underline{H_2}N^{\oplus}H_2$ i Pr) - 1H à 4,05 ppm (D, J = 17 Hz, $C\underline{H_2}SO$) - 1H à 3,79 ppm (D, J = 17 Hz, $C\underline{H_2}SO$) - 1H à 3,31 ppm (M, $(CH_3)_2$-C$\underline{H}$) - 6H à 1,45 ppm (2S, $(C\underline{H_3})_2C$) - 6H à 1,22 ppm (D, J = 7 Hz, $(C\underline{H_3})_2CH\ N^{\oplus}H_2$).

**RMN n° 94 - (b):**

1H à 8,90 ppm (D, J = 9 Hz, CON$\underline{H}$) - 3H à 8,10 ppm (S.e., $CH_2N^{\oplus}\underline{H_3}$) - 1H à 7,66 ppm (D, J = 8 Hz, $\underline{H}$Ar 6') - 1H à 7,55 ppm (D, J = 8 Hz, $\underline{H}$Ar 4') - 1H à 7,45 ppm (T, J = 8 Hz, $\underline{H}$Ar 5') - 1H à 6,95 ppm (S, $\underline{H}$ thiazole) - 1H à 6,00 ppm (D de D, $J_1$ = 9 Hz, $J_2$ = 4 Hz, $\underline{H_7}$) - 1H à 5,40 ppm (D, J = 13 Hz, $C\underline{H_2}OCO$) - 1H à 5,00 ppm (D, J = 4 Hz, $\underline{H_6}$) - 1H à 4,86 ppm (D, J = 13 Hz, $C\underline{H_2}OCO$) - 3H à 4,07 ppm (M, Ar $C\underline{H_2}N^{\oplus}H_3$ et $C\underline{H_2}SO$) - 1H à 3,67 ppm (D, J = 17 Hz, $C\underline{H_2}SO$) - 3H à 2,40 ppm (S, $C\underline{H_3}Ar$) - 4H à 2,50 ppm

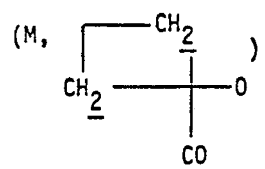

- 2H à 1,90 ppm

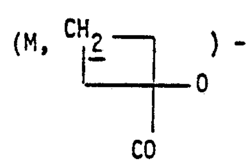

**RMN n° 95 - (b):**

1H à 8,90 ppm (D, J = 9 Hz, CON$\underline{H}$) - 3H à 8,05 ppm (S.e., $CH_2N^{\oplus}\underline{H_3}$) - 1H à 7,40 ppm (D, J = 8 Hz, $\underline{H}$Ar 4') - 1H à 7,20 ppm (D, J = 8 Hz, $\underline{H}$ Ar 5') - 1H à 6,95 ppm (S, $\underline{H}$ thiazole) - 1H à 6,00 ppm (D de D, $J_1$ = 9 Hz, $J_2$ = 4 Hz, $\underline{H_7}$) - 1H à 5,30 ppm (D, J = 13 Hz, $C\underline{H_2}OCO$) - 1H à 5,00 ppm (D, J = 4 Hz, $\underline{H_6}$) - 1H à 4,95 ppm (D, J = 13 Hz, $C\underline{H_2}OCO$) - 3H à 4,00 ppm (Ar $C\underline{H_2}N^{\oplus}H_3$ et $C\underline{H_2}SO$) - 1H à 3,63 ppm (D, J = 17 Hz, $C\underline{H_2}SO$) - 4H à 2,40 ppm

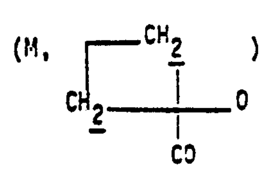

- 6H à 2,20 ppm (S, $C\underline{H_3}Ar$) - 2H à 1,90 ppm

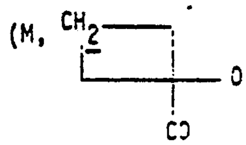

**RMN n° 96 - (b):**

1H à 8,90 ppm (D, J = 9 Hz, CON<u>H</u>) - 3H à 7,90 ppm (S.e., $CH_2N^{\oplus}\underline{H_3}$) - 1H à 7,00 ppm (S, <u>H</u>Ar) - 1H à 6,9 ppm (S, <u>H</u> thiazole) - 1H à 6,00 ppm (D de D, $J_1$ = 9 Hz, $J_2$ = 4 Hz, $\underline{H_7}$) - 1H à 5,30 ppm (D, J = 13 Hz, $C\underline{H_2}OCO$) - 1H à 5,00 ppm (D, J = 4 Hz, $\underline{H_6}$) - 1H à 4,94 ppm (D, J = 13 Hz, $C\underline{H_2}OCO$) - 3H à 4,00 ppm (M, Ar $C\underline{H_2}N^{\oplus}H_3$ et $C\underline{H_2}SO$) - 1H à 3,62 ppm (D, J = 17 Hz, $C\underline{H_2}SO$) - 4H à 2,50 ppm

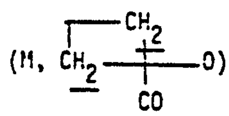

- 3H à 2,32 ppm (S, $C\underline{H_3}Ar$) - 3H à 2,2 ppm (S, $C\underline{H_3}Ar$) - 3H à 2,16 ppm (S, $C\underline{H_3}Ar$) - 2H à 1,90 ppm

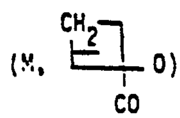

**RMN n° 97 - (b):**

1H à 10,30 ppm (S, Ar N<u>H</u>CO) - 1H à 8,70 ppm (D, J = 9 Hz, CON<u>H</u>) - 1H à 8,30 ppm (S.e., <u>H</u>Ar 2') - 3H à 8,05 ppm (M, $CH_2N^{\oplus}\underline{H_3}$) - 2H à 7,70 ppm (M <u>H</u>Ar 5', 6') - 1H à 6,95 ppm (S, <u>H</u> thiazole) - 1H à 6,00 ppm (D de D, $J_1$ = 9 Hz, $J_2$ = 4 Hz, $\underline{H_7}$) - 1H à 5,45 ppm (D, J = 13 Hz, $C\underline{H_2}OCO$) - 1H à 4,97 ppm (D, J = 4 Hz, $\underline{H_6}$) - 1H à 4,80 ppm (D, J = 13 Hz, $C\underline{H_2}OCO$) - 1H à 4,03 ppm (D, J = 17 Hz, $C\underline{H_2}SO$) - 2H à 3,85 ppm (M, $C\underline{H_2}N^{\oplus}H_3$) - 1H à 3,75 ppm (D, J = 17 Hz, $C\underline{H_2}SO$) - 6H à 1,47 ppm (2S, $(C\underline{H_3})_2C$).

**RMN n° 98 - (b):**

1H à 9,90 ppm (S, Ar N<u>H</u>CO) - 1H à 8,66 ppm (D, J = 9 Hz, CON<u>H</u>) - 1H à 8,28 ppm (S, <u>H</u>Ar 2') - 5H à 7,70 ppm (M, <u>H</u>Ar 5', 6' et $CH_2N^{\oplus}\underline{H_3}$) - 1H à 6,95 ppm (S, <u>H</u> thiazole) - 1H à 6,00 ppm (D de D, $J_1$ = 9 Hz, $J_2$ = 4 Hz, $H_7$) - 1H à 5,45 ppm (D, J = 13 Hz, $C\underline{H_2}OCO$) - 1H à 5,00 ppm (D, J = 4 Hz, $\underline{H_6}$) - 1H à 4,84 ppm (D, J = 13 Hz, $C\underline{H_2}OCO$) - 1H à 4,03 ppm (D, J = 17 Hz, $C\underline{H_2}SO$) - 1H à 3,68 ppm (D, J = 17 Hz, $C\underline{H_2}SO$) - 2H à 3,05 ppm

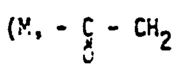

$C\underline{H_2}N^{\oplus}H_3$) - 2H à 2,75 ppm (T, J = 7 Hz,

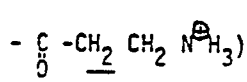

- 6H à 1,46 ppm (2S, $(C\underline{H_3})_2C$).

**RMN n° 99 - (b):**

1H à 10,5 ppm (S, Ar NHCO) - 1H à 8,56 ppm (D, J = 9 Hz, CONH) - 2H à 7,89 ppm (D, J = 8 Hz, HAr 2', 6') - 2H à 7,70 ppm (D, J = 8 Hz, HAr 3', 5') - 3H à 7,70 ppm (S.e., $(CH_2)_2N^{\oplus}H_3$) - 1H à 6,95 ppm (S, H thiazole) - 1H à 5,98 ppm (D de D, $J_1$ = 9 Hz, $J_2$ = 4 Hz, $H_7$) - 1H à 5,40 ppm (D, J = 13 Hz, $CH_2OCO$) - 1H à 4,98 ppm (D, J = 4 Hz, $H_6$) - 1H à 4,79 ppm (D, J = 13 Hz, $CH_2OCO$) - 1H à 4,05 ppm (D, J = 17 Hz, $CH_2SO$) - 1H à 3,75 ppm (D, J = 17 Hz, $CH_2SO$) - 2H à 3,06 ppm

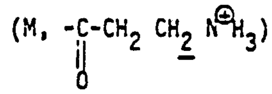

2H à 2,70 ppm

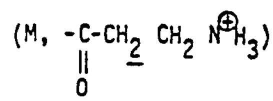

- 6H à 1,45 ppm (2S, $(CH_3)_2C$).

**RMN n° 100 - (b):**

1H à 12,75 ppm (S.e., thiazole NHCO) - 1H à 8,90 ppm (D, J = 9 Hz, CONH) - 4H à 8,15 ppm (M, $CH_2NH_3$ et H thiazole en 3) - 1H à 6,96 ppm (S, H thiazole) - 1H à 6,00 ppm (D de D, $J_1$ = 9 Hz, $J_2$ = 4 Hz, $H_7$) - 1H à 5,40 ppm (D, J = 13 Hz, $CH_2OCO$) - 1H à 4,98 ppm (D, J = 4 Hz, $H_6$) - 1H à 4,81 ppm (D, J = 13 Hz, $CH_2OCO$) - 1H à 4,0 ppm (D, J = 17 Hz, $CH_2SO$) - 2H à 3,86 ppm (M, $CH_2N^{\oplus}H_3$) - 1H à 3,66 ppm (D, J = 17 Hz, $CH_2SO$) - 4H à 2,5 ppm

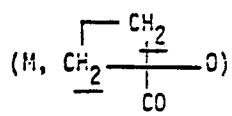

- 2H à 1,90 ppm

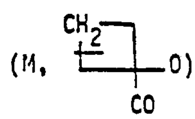

**RMN n° 101 - (b):**

1H à 10,8 ppm (S, Ar NHCO) - 1H à 8,95 ppm (D, J = 9 Hz, CONH) - 3H à 8,15 ppm (S.e., $CH_2N^{\oplus}H_3$) - 2H à 7,90 ppm (D, J = 8 Hz, H Ar 2', 6') - 2H à 7,70 ppm (D, J = 8 Hz, HAr 3', 5') - 1H à 6,97 ppm (S, H thiazole) - 1H à 6,00 ppm (D de D, $J_1$ = 9 Hz, $J_2$ = 4 Hz, $H_7$) - 1H à 5,36 ppm (D, J = 13 Hz, $CH_2OCO$) - 1H à 5,00 ppm (D, J = 4 Hz, $H_6$) - 1H à 4,82 ppm (D, J = 13 Hz, $CH_2OCO$) - 1H à 4,05 ppm (D, J = 17 Hz, $CH_2SO$) - 3H à 3,80 ppm (M, $CH_2N^{\oplus}H_3$ et $CH_2SO$) - 4H à 2,40 ppm

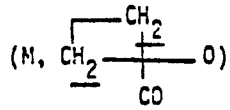

- 2H à 1,90 ppm

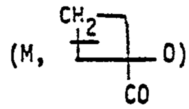

**RMN n° 102 - (b):**

1H à 8,45 ppm (D, J = 9 Hz, CONH) - 3H à 8,0 ppm (S.e., $CH_2N^{\oplus}H_3$) - 1H à 6,79 ppm (S, H thiazole) - 1H à 5,98 ppm (D de D, $J_1$ = 9 Hz, $J_2$ = 4 Hz, $H_7$) - 1H à 5,15 ppm (2D, J = 13 Hz, $CH_2OCO$) - 1H à 4,97 ppm (D, J = 4 Hz, $H_6$) - 1H 4,62 ppm (D, J = 1; Hz, $CH_2OCO$) - 1H à 4,15 ppm (De., J = 12 Hz, $H_2$ Eq pipéridine) - 3H à 3,84 ppm (M, $CH_2N^{\oplus}H_3$ et $CH_2SO$) - 2H à 3,58 ppm (M, $CH_2SO$ et $H_6$ Eq pipéridine) - 1H à 3,05 ppm (Te., J = 12 Hz, $H_2$Ax pipéridine) - 1H à 2,81 ppm (Te., J = 12 Hz, $H_6$Ax pipéridine) - 1H à 2,60 ppm (M, $CHCO_2$) - 2H à 1,84 ppm (M, $H_3$ et $H_5$ piperidine) - 2H à 1,50 ppm (M, $H_3$ et $H_5$ pipéridine) - 6H à 1,45 ppm (2 D, $(CH_3)_2C$).

**RMN n° 103 - (b):**

1H à 8,72 ppm (D, J = 9 Hz, CONH) - 3H à 8,25 ppm (S.e., $CH_2N^{\oplus}H_3$) - 3H à 7,75 ppm (M, HAr) - 1H à 6,97 ppm (S, H thiazole) - 1H à 6,00 ppm (D de D, $J_1$ = 9 Hz, $J_2$ = 4 Hz, $H_7$) - 1H à 5,45 ppm (D, J = 13 Hz, $CH_2OCO$) - 1H à 5,00 ppm (D, J = 4 Hz, $H_6$) - 1H à 4,84 ppm (D, J = 13 Hz, $CH_2OCO$) - 3H à 4,05 ppm (M, $CH_2N^{\oplus}H_3$ et $CH_2SO$) - 1H à 3,75 ppm (D, J = 17 Hz, $CH_2SO$) - 6H à 1,45 ppm (2S, $(CH_3)_2C$).

**RMN n° 104 - (b):**

2H à 8,80 ppm (S.e., $CH_2N^{\oplus}H_2CH_3$) - 1H à 8,77 ppm (D, J = 9 Hz, CONH) - 3H à 7,75 ppm (M, HAr) - 1H à 6,95 ppm (S, H thiazole) - 1H à 6,00 ppm (D de D, $J_1$ = 9 Hz, $J_2$ = 4 Hz, $H_7$) - 1H à 5,45 ppm (D, J = 13 Hz, $CH_2OCO$) - 1H à 5,00 ppm (D, J = 4 Hz, $H_6$) - 1H à 4,86 ppm (D, J = 13 Hz, $CH_2OCO$) - 2H à 4,25 ppm (M, $CH_2N^{\oplus}H_2CH_3$) - 1H à 4,10 ppm (D, J = 17 Hz, $CH_2SO$) - 1H à 3,74 ppm (D, J = 17 Hz, $CH_2SO$) - 3H à 2,58 ppm (M, $CH_2N^{\oplus}H_2$ - $CH_3$) - 6H à 1,45 ppm (2S, $(CH_3)_2C$).

**RMN n° 105 - (b):**

1H à 10,70 ppm (S, Ar NHCO) - 2H à 8,70 ppm (S.e., $CH_2N^{\oplus}H_2CH_3$) - 1H à 8,68 ppm (D, J = 9 Hz, CONH) - 1H à 8,16 ppm (S, HAr 2') - 1H à 7,92 ppm (D, J = 8 Hz, HAr 6') - 1H à 7,66 ppm (D, J = 8 Hz, HAr 4') - 1H à 7,50 ppm (T, J = 8 Hz, HAr 5') - 1H à 6,95 ppm (S, H thiazole) - 1H à 6,00 ppm (D de D, $J_1$ = 9 Hz, $J_2$ = 4 Hz, $H_7$) - 1H à 5,45 ppm (D, J = 13 Hz, $CH_2OCO$) - 1H à 5,00 ppm (D, J = 4 Hz, $H_6$) - 1H à 4,82 ppm (D, J = 13 Hz, $CH_2OCO$) - 1H à 4,02 ppm (D, J = 17 Hz, $CH_2SO$) - 2H à 3,90 ppm (M, $CH_2N^{\oplus}H$ $CH_3$) - 1H à 3,74 ppm (D, J = 17 Hz, $CH_2SO$) - 3H à 2,60 ppm (M, $CH_2N^{\oplus}H_2CH_3$) - 6H à 1,45 ppm (2S, $(CH_3)_2C$).

**RMN n° 106 - (b):**

1H à 10,80 ppm (S, Ar NHCO) - 3H à 8,70 ppm (M, $CH_2N^{\oplus}H_2CH_3$ et CONH) - 2H à 7,92 ppm (D, J = 8 Hz, HAr 2', 6') - 2H à 7,69 ppm (D, J = 8 Hz, HAr 3', 5') - 1H à 6,95 ppm (S, H thiazole) - 1H à 6,00 ppm (D de D, $J_1$ = 9 Hz, $J_2$ = 4 Hz, $H_7$) - 1H à 5,42 ppm (D, J = 13 Hz, $CH_2OCO$) - 1H à 5,00 ppm (D, J = 4 Hz, $H_6$) - 1H à 4,81 ppm (D, J = 13 Hz, $CH_2OCO$) - 1H à 4,05 ppm (D, J = 17 Hz, $CH_2SO$) - 2H à 3,95 ppm (M, $CH_2N^{\oplus}H_2CH_3$) - 1H à 3,75 ppm (D, J = 17 Hz, $CH_2SO$) - 3H à 2,62 ppm (M, $CH_2N^{\oplus}H_2CH_3$) - 6H à 1,45 ppm (2S, $(CH_3)_2C$).

**RMN n° 107 - (b):**

1H à 9,95 ppm (S, Ar NHCO) - 1H à 8,75 ppm (D, J = 9 Hz, CONH) - 3H à 8,05 ppm (S.e., $CH_2N^{\oplus}H_3$) - 1H à 7,61 ppm (D, J = 8 Hz, HAr 6') - 1H à 7,52 ppm (D, J = 8 Hz, HAr 4') - 1H à 7,31 ppm (T, J = 8 Hz, HAr 6') - 1H à 6,96 ppm (S, H thiazole) - 1H à 6,00 ppm (D de D, $J_1$ = 9 Hz, $J_2$ = 4 Hz, $H_7$) - 1H à 5,40 ppm (D, J = 13 Hz, $CH_2OCO$) -

1H à 5,00 ppm (D, J = 4 Hz, $H_6$) - 1H à 4,81 ppm (D, J = 13 Hz, $CH_2OCO$) - 1H à 4,05 ppm (D, J = 17 Hz, $CH_2SO$) - 2H à 3,81 ppm (M, $CH_2N^{\oplus}H_3$) - 1H à 3,72 ppm (D, J = 17 Hz, $CH_2SO$) - 3H à 2,28 ppm (S, Ar $CH_3$) - 6H à 1,45 ppm (2S, $(CH_3)_2C$).

**RMN n° 108 - (b):**

1H à 9,75 ppm (S, Ar NHCO) - 1H à 8,70 ppm (D, J =9 Hz, CONH) - 3H à 7,70 ppm (S.e., $CH_2CH_2N^{\ominus}H_3$) - 1H à 7,60 ppm (D, J = 8 H; HAr 6') - 1H à 7,50 ppm (D, J = 8 Hz, HAr 4') - 1H à 7,25 ppm (T, J = 8 Hz, HAr 5') - 1H à 6,95 ppm (S, H thiazole) - 1H à 6,00 ppm (D de D, $J_1$ = 9 Hz, $J_2$ = 4 Hz, $H_7$) - 1H à 5,39 ppm (D, J = 13 Hz, $CH_2OCO$) - 1H à 5,00 ppm (D, J = 4 Hz, $H_6$) - 1H à 4,82 ppm (D, J = 13 Hz, $CH_2OCO$) - 1H à 4,05 ppm (D, J = 17 Hz, $CH_2SO$) - 1H à 3,72 ppm (D, J = 17 Hz, $CH_2SO$) - 2H à 3,02 ppm (M, $CH_2$ $CH_2N^{\oplus}H_3$) - 2H à 2,69 ppm (T, J = 7, $CH_2CH_2N^{\oplus}H_3$) - 3H à 2,29 ppm (S, Ar $CH_3$) - 6H à 1,45 ppm (2S, $(CH_3)_2C$) -

**RMN n° 109 - (b):**

1H à 12,7 ppm (S.e., NHCO thiazole) - 1H à 8,79 ppm (D, J = 9 Hz, CONH) - 1H à 8,08 ppm (S, H thiazole en 3) - 3H à 7,75 ppm (S.e., $CH_2N^{\oplus}H_3$) - 1H à 6,98 ppm (S, H thiazole) - 1H à 6,00 ppm (D de D, $J_1$ = 9 Hz, $J_2$ = 4 Hz, $H_7$) - 1H à 5,37 ppm (D, J = 13 Hz, $CH_2OCO$) - 1H à 5,00 ppm (D, J = 4 Hz, $H_6$) - 1H à 4,81 ppm (D, J = 13 Hz, $CH_2OCO$) - 1H à 4,03 ppm (D, J = 17 Hz, $CH_2SO$) - 1H à 3,68 ppm (D, J = 17 Hz, $CH_2SO$) - 2H à 3,06 ppm (M, $CH_2CH_2$ $N^{\oplus}H_3$) - 2H à 2,77 ppm (T, J = 7 Hz, $CH_2CH_2N^{\oplus}H_3$) - 6H à 1,45 ppm (2S, $(CH_3)_2C$).

**RMN n° 110 - (b):**

1H à 8,70 ppm (D, J = 9 Hz, CONH) - 5H à 7,96 ppm (M, HAr 2', 6' et $CH_2N^{\oplus}H_3$) - 2H à 7,55 ppm (D, J = 8 Hz, HAr 3', 5') - 1H à 6,95 ppm (S, H thiazole) - 1H à 6,00 ppm (D de D, $J_1$ = 9 Hz, $J_2$ = 4 Hz, $H_7$) - 1H à 5,46 ppm (D, J = 13 Hz, $CH_2OCO$) - 1H à 4,99 ppm (D, J = 4 Hz, $H_6$) - 1H à 4,84 ppm (D, J = 13 Hz, $CH_2OCO$) - 1H à 4,06 ppm (D, J = 17 Hz, $CH_2SO$) - 1H à 3,74 ppm (D, J = 17 Hz, $CH_2SO$) - 2H à 3,55 ppm (S.e., $CH_2$ $N^{\oplus}H_3$) - 3H à 3,25 ppm (S.e., N $CF_3$) - 6H à 1,45 ppm (2S, $(CH_3)_2C$).

**RMN n° 111 - (b):**

1H à 10,5 ppm (S.e., Ar NHCO) - 1H à 8,95 ppm (D, J = 9 Hz, CONH) - 2H à 7,89 ppm (D, J = 8 Hz, HAr 2', 6') - 2H à 7,71 ppm (D, J = 8 Hz, HAr 3', 5') - 1H à 6,96 ppm (S, H thiazole) - 1H à 5,98 ppm (D de D, $J_1$ = 9 Hz, $J_2$ = 4 Hz, $H_7$) - 1H à 5,42 ppm (D, J = 13 Hz, $CH_2O$ CO) - 1H à 5,00 ppm (D, J = 4 Hz, $H_6$) - 1H à 4,80 ppm (D, J = 13 Hz, $CH_2O$ CO) - 1H à 4,05 ppm (D, J = 17 Hz, $CH_2SO$) - 1H à 3,75 ppm (D, J = 17 Hz, $CH_2SO$) - 2H à 3,05 ppm (M, $CH_2CH_2N^{\oplus}H_3$) - 2H à 2,70 ppm (T, J = 7 Hz, $CH_2CH_2N^{\oplus}H_3$) - 4H à 2,40 ppm

(M, 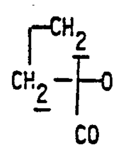 )

2H à 1,90 ppm

(M, 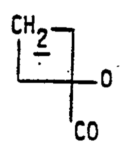 )

**RMN n° 112 - (b):**

1H à 8,80 ppm (D, J = 9 Hz, CONH) - 3H à 7,90 ppm (S.e., $CH_2N^{\oplus}H_3$) - 1H à 6,80 ppm (S, H thiazole) - 1H à 5,95 ppm (D de D, $J_1$ = 9 Hz, $J_2$ = 4 Hz, $H_7$) - 1H à 5,17 ppm (2 D, J = 13 Hz, $CH_2O$ CO) - 1H à 4,96 ppm (D, J = 4 Hz, $H_6$) - 1H à 4,60 ppm (D, J = 13 Hz, $CH_2O$ CO) - 1H à 4,18 ppm (M, $H_2$e pipéridine) 3H à 3,90 ppm (M, $CH_2N^{\oplus}H_3$ et $CH_2SO$) - 2H à 3,58 ppm (M, $CH_2SO$ et $H_6$e pipéridine) - 1H à 3,06 ppm (M, $H_2$a pipéridine) - 1H à 2,75 ppm (M, $H_6$a pipéridine) - 1H à 2,60 ppm (M, $H_4$ pipéridine) - 4H à 2,40 ppm

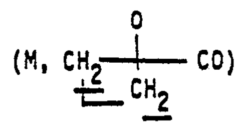

- 4H à 1,80 ppm et 2H à 1,50 ppm

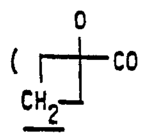

et $H_3$ et $H_5$ pipéridine).

**RMN n° 113 - (b):**

1H à 8,75 ppm (D, J = 9 Hz, CONH) - 3H à 8,40 ppm (S.e., CH $N^{\oplus}H_3$) - 1H à 8,13 ppm (S, HAr 6') - 1H à 8,00 ppm (D, J = 8 Hz, HAr 2') - 1H à 7,64 ppm (D, J = 8 Hz, HAr 3') - 1H à 6,97 ppm (S, H thiazole) - 1H à 6,00 ppm (D de D, $J_1$ = 9 Hz, $J_2$ = 4 Hz, $H_7$) - 1H à 5,45 ppm (D, J = 13 Hz, $CH_2O$ CO) - 1H à 5,00 ppm (D, J = 4 Hz, $H_6$) - 1H à 4,86 ppm (D, J = 13 Hz, $CH_2O$ CO) - 2H à 4,19 ppm (M, $CH_2N^{\oplus}H_3$) - 1H à 4,10 ppm (D, J = 17 Hz, $CH_2SO$) - 1H à 3,76 ppm (D, J = 17 Hz, $CH_2SO$) - 6H à 1,45 ppm (2S, $(CH_3)_2C$).

**RMN n° 114 - (b):**

1H à 8,72 ppm (D, J = 9 Hz, CONH) - 3H à 8,40 ppm (S.e., $CH_2N^{\oplus}H_3$) - 2H à 8,11 ppm (M, HAr 2', 6') - 1H à 7,86 ppm (M, HAr 3') - 1H à 6,99 ppm (S, H thiazole) - 1H à 6,01 ppm (D de D, $J_1$ = 9 Hz, $J_2$ = 4 Hz, $H_7$) - 1H à 5,46 ppm (D, J = 13 Hz, $CH_2OCO$) - 1H à 5,00 ppm (D, J = 4 Hz, $H_6$) - 1H à 4,89 ppm (D, J = 13 Hz, $CH_2OCO$) - 2H à 4,18 ppm (M, $CH_2N^{\oplus}H_3$) - 1H à 4,06 ppm (D, J = 17 Hz, $CH_2SO$) - 1H à 3,78 ppm (D, J = 17 Hz, $CH_2SO$) - 6H à 1,45 ppm (2S, $(CH_3)_2C$).

**RMN n° 115 - (b):**

1H à 10,20 ppm (S.e., Ar NHCO) - 1H à 8,70 ppm (D, J = 9 Hz, CONH) - 1H à 8,16 ppm (S.e., HAr 2') - 1H à 7,84 ppm (D, J = 8 Hz, HAr 6') - 3H à 7,66 ppm (S.e., $CH_2N^{\oplus}H_3$) - 1H à 7,60 ppm (D, J = 8 Hz, HAr 4') - 1H à 7,45 ppm (T, J = 8 Hz, HAr 5') - 1H à 6,97 ppm (S, H thiazole) - 1H à 6,00 ppm (D de D, $J_1$ = 9 Hz, $J_2$ = 4 Hz, $H_7$) - 1H à 5,45 ppm (D, J = 13 Hz, $CH_2OCO$) - 1H à 5,00 ppm (D, J = 4 Hz, $H_6$) - 1H à 4,84 ppm (D, J = 13 Hz, $CH_2OCO$) - 1H à 4,05 ppm (D, J = 17 Hz, $CH_2SO$) - 1H à 3,75 ppm (D, J = 17 Hz, $CH_2SO$) - 2H à 2,81 ppm (M, $CH_2$ - $N^{\oplus}H_3$) - 2H à 2,40 ppm (T, J = 7 Hz, $CH_2CO_2$) - 2H à 1,82 ppm (M, $CH_2CH_2CO_2$) - 6H à 1,45 ppm (2S, $(CH_3)_2C$).

**RMN n° 116 - (b):**

1H à 10,3 ppm (S.e., Ar NHCO) - 1H à 8,70 ppm (D, J = 9 Hz, CONH) - 2H à 7,90 ppm (D, J = 8 Hz, HAr 2', 6') - 1H à 7,67 ppm (D, J = 8 Hz, HAr 3', 5') - 3H à 7,60 ppm (S.e., $CH_2N^{\oplus}H_3$) - 1H à 6,95 ppm (5, H thiazole) - 1H à 6,00 ppm (D de D, $J_1$ = 9 Hz, $J_2$ = 4 Hz, $H_7$) - 1H à 5,42 ppm (D, J = 13 Hz, $CH_2OCO$) - 1H à 4,98 ppm (D, J = 4 Hz, $H_6$) - 1H à 4,79 ppm (D, J = 13 Hz, $CH_2OCO$) - 1H à 4,06 ppm (D, J = 17 Hz, $CH_2SO$) - 1H à 3,75 ppm (D, J = 17 Hz, $CH_2SO$) - 2H à 2,79 ppm (M, $CH_2N^{\oplus}H_3$) - 2H à 2,42 ppm (M, $CH_2$ - CONH Ar) - 2H à 1,84 ppm (M, $CH_2CH_2N^{\oplus}H_3$) - 6H à 1,48 ppm (2S, $(CH_3)_2C$).

**RMN n° 117 - (b):**

1H à 10,30 ppm (S.e., Ar $N\underline{H}CO$) - 1H à 8,75 ppm (D, J = 9 Hz, $N\underline{H}CO$) - 2H à 7,04 ppm (D, J = 8 Hz, $\underline{H}$Ar 2', 6') - 2H à 7,72 ppm (D, J = 8 Hz, $\underline{H}$Ar 3', 5') - 3H à 7,60 ppm (S.e., $CH_2N^{\oplus}H_3$) - 1H à 6,97 ppm (S, $\underline{H}$ thiazole) - 1H à 6,00 ppm (D de D, $J_1$ = 9 Hz, $J_2$ = 4 Hz, $\underline{H}_7$) - 1H à 5,40 ppm (D, J = 13 Hz, $C\underline{H}_2OCO$) - 1H à 4,90 ppm (D, J = 4 Hz, $\underline{H}_6$) - 1H à 4,78 ppm (D, J = 13 Hz, $C\underline{H}_2OCO$) - 1H à 4,05 ppm (D, J = 17 Hz, $C\underline{H}_2SO$) - 1H à 3,75 ppm (D, J = 17 Hz, $C\underline{H}_2SO$) - 2H à 2,75 ppm (M, $C\underline{H}_2N^{\oplus}H_3$) - 2H à 2,36 ppm (M, $C\underline{H}_2CONH$ Ar) - 4H à 1,58 ppm (M, $COCH_2$ $(C\underline{H}_2)$, $CH_2N^{\oplus}H_3$) - 6H à 1,45 ppm (2S, $(C\underline{H}_3)_2C$).

**RMN n° 118 - (b):**

1H à 12,7 ppm (S.e., $N\underline{H}CO$ thiazole) - 1H à 8,65 ppm (D, J = 9 Hz, $CON\underline{H}$) - 3H à 8,20 ppm (S.e., $CH_2N^{\oplus}\underline{H}_3$) - 1H à 6,93 ppm (S, $\underline{H}$ thiazole) - 1H à 6,00 ppm (D de D, $J_1$ = 9 Hz, $J_2$ = 4 Hz, $\underline{H}_7$) - 1H à 5,45 ppm (D, J = 13 Hz, $C\underline{H}_2OCO$) - 1H à 5,02 ppm (D, J = C Hz, $\underline{H}_6$) - 1H à 4,75 ppm (D, J = 13 Hz, $C\underline{H}_2OCO$) - 1H à 4,02 ppm (D, J = 17 Hz, $C\underline{H}_2SO$) - 2H à 3,90 ppm (M, $C\underline{H}_2$ Gly) - 1H à 3,70 ppm (D, J = 17 Hz, $CH_2SO$) - 3H à 2,50 ppm (S, $C\underline{H}_3$ thiazole) - 6H à 1,45 ppm (2S, $(C\underline{H}_3)_2C$).

**RMN n° 119 - (b):**

1H à 8,45 ppm (D, J = 9 Hz, $CON\underline{H}$) - 3H à 7,80 ppm (S.e., $CHN^{\oplus}\underline{H}_3$) - 1H à 6,80 ppm (S, $\underline{H}$ thiazole) - 1H à 6,00 ppm (D de D, $J_1$ = 9 Hz, $J_2$ = 4 Hz, $\underline{H}_7$) - 1H à 5,10 ppm (D, J = 13 Hz, $C\underline{H}_2OCO$) - 1H à 4,95 ppm (D, J = 4 Hz, $\underline{H}_6$) - 1H à 4,58 ppm (D, J = 13 Hz, $C\underline{H}_2OCO$) - 1H à 3,90 ppm (D, J = 17 Hz, $C\underline{H}_2SO$) - 1H à 3,56 ppm (D, J = 17 Hz, $C\underline{H}_2SO$) - 1H à 3,00 ppm (2M, $C\underline{H}N^{\oplus}H_3$) - 1H à 2,25 ppm (M, $C\underline{H}CO_2$) - 4H à 1,80 ppm et 4H à 1,40 ppm (M, $C\underline{H}_2$ cyclohexane) - 6H à 1,44 ppm (2S, $(C\underline{H}_3)_2C$).

**RMN n° 120 - (b):**

1H à 8,75 ppm (D, J = 9 Hz, $CON\underline{H}$) - 2H à 7,80 ppm (S.e., $\underline{H}$Ar 2', 6') - 3H à 7,65 ppm (S.e., $C\underline{H}_2N^{\oplus}\underline{H}_3$) - 1H à 7,57 ppm (D, J = 8 Hz, $\underline{H}$Ar 4') - 1H à 7,47 ppm (T, J = 8 Hz, $\underline{H}$Ar 5') - 1H à 7,00 ppm (S, $\underline{H}$ thiazole) - 1H à 6,00 ppm (D de D, $J_1$ = 9 Hz, $J_2$ = 4 Hz, $\underline{H}_7$) - 1H à 5,45 ppm (D, J = 13 Hz, $C\underline{H}_2OCO$) - 1H à 4,98 ppm (D, J = 4 Hz, $\underline{H}_6$) - 1H à 4,82 ppm (D, J = 13 Hz, $C\underline{H}_2OCO$) - 1H à 4,05 ppm (D, J = 17 Hz, $C\underline{H}_2SO$) - 1H à 3,75 ppm (D, J = 17 Hz, $C\underline{H}_2SO$) - 3H à 3,00 ppm (M,
$C\underline{H}$
|
$CH_3C\underline{H}_2N^{\oplus}H_3$) - 6H à 1,50 ppm (2S, $(C\underline{H}_3)_2C$) - 3H à 1,20 ppm (D, J = 7 Hz,
CH
|
$C\underline{H}_3CH_2N^{\oplus}H_3$).

**RMN n° 121 - (b):**

1H à 10,0 ppm (S.e., Ar $N\underline{H}CO$) - 1H à 8,85 ppm (D, J = 9 Hz, $CON\underline{H}$) - 3H à 8,05 ppm (S.e., $CH_2N^{\oplus}H_3$) - 1H à 7,45 ppm (S, $\underline{H}$Ar 6') - 1H à 7,30 ppm (S, $\underline{H}$Ar 4') - 1H à 7,00 ppm (S, $\underline{H}$ thiazole) - 1H à 6,00 ppm (D de D, $J_1$ = 9 Hz, $J_2$ = 4 Hz, $\underline{H}_7$) - 1H à 5,43 ppm (D, J = 13 Hz, $C\underline{H}_2OCO$) - 1H à 5,00 ppm (D, J = 4 Hz, $\underline{H}_6$) - 1H à 4,78 ppm (D, J = 13 Hz, $C\underline{H}_2OCO$) - 1H à 4,05 ppm (D, J = 17 Hz, $C\underline{H}_2SO$) - 2H à 3,80 ppm (M, $C\underline{H}_2$ Gly) - 1H à 3,75 ppm (D, J = 17 Hz, $C\underline{H}_2SO$) - 6H à 2,25 ppm (2S, $C\underline{H}_3Ar$) - 6H à 1,50 ppm (2S, $(C\underline{H}_3)_2C$).

**RMN n° 122 - (b):**

1H à 10,45 ppm (S.e., Ar $N\underline{H}CO$) - 1H à 8,70 ppm (D, J = 9 Hz, $CON\underline{H}$) - 1H à 8,50 ppm (S.e., $N^{\oplus}\underline{H}_2$, pipéridine) - 1H à 8,20 ppm (S.e., $N^{\oplus}\underline{H}_2$, pipéridine) - 2H à 7,84 ppm (D, J = 8 Hz, $\underline{H}$Ar 2', 6') - 2H à 7,70 ppm (D, J = 8 Hz, $\underline{H}$Ar 3', 5') - 1H à 6,98 ppm (S, $\underline{H}$ thiazole) - 1H à 6,00 ppm (D de D, $J_1$ = 9 Hz, $J_2$ = 4 Hz, $\underline{H}_7$) - 1H à 5,42 ppm (D, J = 13 Hz, $C\underline{H}_2OCO$) - 1H à 4,98 ppm (D, J = 4 Hz, $\underline{H}_6$) - 1H à 4,78 ppm (D, J = 13 Hz, $C\underline{H}_2OCO$) - 1H à 4,05 ppm (D, J = 17 Hz, $C\underline{H}_2SO$) - 1H à 3,75 ppm (D, J = 17 Hz, $C\underline{H}_2SO$) - 2H à 3,30 ppm (M, $C\underline{H}_2$ en α $N^{\oplus}H_2$ pipéridine) - 2H à 2,90 ppm (M, $C\underline{H}_2$ en α $N^{\oplus}H_2$ pipéridine) - 1H à 2,56 ppm (M, $C\underline{H}CONH$) - 4H à 1,80 ppm (M, $C\underline{H}_2$ en β $N^{\oplus}H_2$ pipéridine) - 6H à 1,45 ppm (2S, $(C\underline{H}_3)_2C$).

**RMN n° 123 - (b):**

1H à 10,25 ppm (S.e., Ar NHCO) - 1H à 8,70 ppm (D, J = 9 Hz, CONH) - 1H à 8,50 ppm (S.e., $N^{\ominus}H_2$, pipéridine) - 1H à 8,25 ppm (S.e., $N^{\oplus}H_2$, pipéridine) - 1H à 8,19 ppm (S.e., HAr 2') - 1H à 7,86 ppm (D, J = 8 Hz, HAr 6') - 1H à 7,60 ppm (D, J = 8 Hz, HAr 4') - 1H à 7,42 ppm (T, J = 8 Hz, HAr 5') - 1H à 6,95 ppm (S, H thiazole) - 1H à 6,00 ppm (D de D, $J_1$ = 9 Hz, $J_2$ = 4 Hz, $H_7$) - 1H à 5,45 ppm (D, J = 13 Hz, $CH_2OCO$) - 1H à 5,00 ppm (D, J = 4 Hz, $H_6$) - 1H à 4,81 ppm (D, J = 13 Hz, $CH_2OCO$) - 1H à 4,05 ppm (D, J = 17 Hz, $CH_2SO$) - 1H à 3,71 ppm (D, J = 17 Hz, $CH_2SO$) - 2H à 3,30 ppm (M, $CH_2$ en α $N^{\ominus}H_2$ pipéridine) - 2H à 2,90 ppm (M, $CH_2$ en α $N^{\ominus}H_2$ pipéridine) - 1H à 2,66 ppm (M, CHCONH) - 4H à 1,80 ppm (N, $CH_2$ en β $N^{\ominus}H_2$ pipéridine) - 6H à 1,45 ppm (2S, $(CH_3)_2C$).

**RMN n° 124 - (b):**

1H à 10,75 ppm (S.e., Ar NHCO) - 1H à 8,80 ppm (D, J = 9 Hz, CONH) - 3H à 8,20 ppm (S.e., CH $N^{\ominus}H_3$) - 2H à 7,92 ppm (D, J = 8 Hz, HAr 2', 6') - 2H à 7,70 ppm (D, J = 8 Hz, HAr 3', 5') - 1H à 7,00 ppm (S, H thiazole) - 1H à 6,00 ppm (D de D, $J_1$ = 9 Hz, $J_2$ = 4 Hz, $H_7$) - 1H à 5,45 ppm (D, J = 13 Hz, $CH_2OCO$) - 1H à 5,00 ppm (D, J = 4 Hz, $H_6$) - 1H à 4,80 ppm (D, J = 13 Hz, $CH_2OCO$) - 2H à 4,00 ppm (M, H α Ala et $CH_2SO$) - 1H à 3,75 ppm (D, J = 17 Hz, $CH_2SO$) - 6H à 1,50 ppm (2S, $(CH_3)_2C$) - 3H à 1,42 ppm (D, J = 7 Hz, $CH_3$Ala).

**RMN n° 125 - (b):**

1H à 10,80 ppm (S.e., Ar NHCO) - 1H à 9,00 ppm (D, J = 9 Hz, CONH) - 3H à 8,20 ppm (S.e., CH - $N^{\ominus}H_3$) - 2H à 7,85 ppm (D, J = 8 Hz, HAr 2', 6') - 2H à 7,75 ppm (D, J = 8 Hz, HAr 3', 5') - 1H à 7,00 ppm (S, H thiazole) - 1H à 6,00 ppm (D de D, $J_1$ = 9 Hz, $J_2$ = 4 Hz, $H_7$) - 1H à 5,45 ppm (D, J = 13 Hz, $CH_2OCO$) - 1H à 5,00 ppm (D, J = 4 Hz, $H_6$) - 1H à 4,80 ppm (D, J = 13 Hz, $CH_2OCO$) - 2H à 4,00 ppm (M, H α Ala et $CH_2SO$) - 1H à 3,75 ppm (D, J = 17 Hz, $CH_2SO$) - 4H à 2,40 ppm

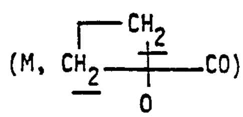

- 2H à 1,80 ppm

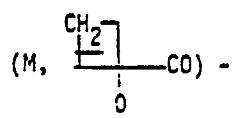

- 3H à 1,43 ppm (D, J = 7 Hz, $CH_3$ Ala).

**RMN n° 126 - (b):**

1H à 8,45 ppm (D, J = 9 Hz, CONH) - 3H à 8,00 ppm (S.e., $N^{\ominus}H_3$ Ala) - 3H à 7,90 ppm (S.e., $N^{\ominus}H_3$ thiazole) - 1H à 6,80 ppm (S, H thiazole) - 1H à 6,00 ppm (D de D, $J_1$ = 9 Hz, $J_2$ = 4 Hz, $H_7$) - 1H à 5,20 ppm (M, $CH_2OCO$) - 1H à 4,95 ppm (D, J = 4 Hz, $H_6$) - 1H à 4,50 ppm (D, $CH_2OCO$) - 1H à 3,15 ppm (M, $H_2$a pipéridine) - 1H à 2,80 ppm (M, $H_6$a pipéridine) - 1H à 2,60 ppm (M, - $CHCO_2$ pipéridine) - 2H à 1,80 ppm et 2H à 1,50 ppm (2 M, $H_3$ et $H_5$ pipéridine) - 6H à 1,45 ppm (2S, $(CH_3)$, C) - 3H à 1,20 ppm (2D, $CH_3CH$).

**RMN n° 127 - (b):**

1H à 8,43 ppm (D, J = 9 Hz, CONH) - 6H à 7,50 ppm (S.e., $N^{\ominus}H_3$) - 1H à 6,80 ppm (S, H thiazole) - 1H à 6,00 ppm (D de D, $J_1$ = 9 Hz, $J_2$ = 4 Hz, $H_7$) - 1H à 5,16 ppm (2D, J = 13 Hz, $CH_2OCO$) - 1H à 4,95 ppm (D, J = 4 Hz, $H_6$) - 1H à 4,58 ppm (D, J = 13 Hz, $CH_2OCO$) - 1H à 4,20 ppm (M, $H_2$e pipéridine) - 1H à 3,90 ppm (D, J = 17 Hz,

$CH_2SO$) - 1H à 3,64 ppm (M, $H_6$e pipéridine) - 1H à 3,57 ppm (D, J = 17 Hz, $CH_2SO$) - 1H à 3,07 ppm (M, $H_2$a pipéridine) - 2H à 2,96 ppm (M, $CH_2N^{\oplus}H_3$) - 1H à 2,75 ppm (M, $H_6$a pipéridine) - 3H à 2,65 ppm (M, $CHCO_2$ et $CH_2CON$) - 2H à 1,80 ppm et 2H à 1,50 ppm ($H_3$ et $H_5$ pipéridine) - 6H à 1,45 ppm (2S, $(CH_3)_2C$).

**RMN n° 128 - (b):**

1H à 8,45 ppm (D, J = 9 Hz, CONH) - 6H à 7,70 ppm (S.e., $N^{\oplus}H_3$) - 1H à 6,80 ppm (S, H thiazole) - 1H à 5,98 ppm (D de D, $J_1$ = 9 Hz, $J_2$ = 4 Hz, $H_7$) - 1H à 5,20 ppm (2D, J = 13 Hz, $CH_2OCO$) - 1H à 4,96 ppm (D, J = 4 Hz, $H_6$) - 1H à 4,58 ppm (D, J = 13 Hz, $CH_2OCO$) - 1H à 4,18 ppm ($H_2$e pipéridine) - 1H à 3,90 ppm (D, J = 17 Hz, $CH_2SO$) - 1H à 3,75 ppm (M, $H_6$e pipéridine) - 1H à 3,55 ppm (D, J = 17 Hz, $CH_2SO$) - 1H à 3,00 ppm (N, $H_2$a pipéridine) - 4H à 2,65 ppm (M, $CH_2N^{\oplus}H_3$, $H_6$a et $H_4$ pipéridine) - 2H à 2,40 ppm (T, $CH_2CON$) - 4H à 1,70 ppm (M, $CHCH_2$ $CH_2N^{\oplus}H_3$, $H_3$ et $H_6$ pipéridine) - 2H à 1,50 ppm (M, $H_3$ et $H_5$ pipéridine) - 6H à 1,44 ppm (2S, $(CH_3)_2C$).

**RMN n° 129 - (b):**

1H à 8,50 ppm (S.e., $N^{\oplus}H_2$ pipéridinium) - 1H à 8,45 ppm (D, J = 9 Hz, CONH) - 1H à 8,25 ppm (S.e., $N^{\oplus}H_2$ pipéridinium) - 3H à 7,40 ppm (S.e., $N^{\oplus}H_3$ thiazole) - 1H à 6,80 ppm (S, H thiazole) - 1H à 6,00 ppm (D de D, $J_1$ = 9 Hz, $J_2$ = 4 Hz, $H_7$) - 1H à 5,16 ppm (D, J = 13 Hz, $CH_2OCO$) - 1H à 4,15 ppm (M, $H_2$e pipéridine) - 2H à 3,90 ppm (M, $H_6$e pipéridine et $CH_2SO$) - 1H à 3,55 ppm (D, J = 17 Hz, $CH_2SO$) - 2H à 3,25 ppm (M, $H_2$e et $H_6$e pipéridinium) - 1H à 3,15 ppm (M, $H_2$a pipéridine) - 3H à 2,85 ppm (M, $H_6$a pipéridine et $H_2$a et $H_6$a pipéridinium) - 2H à 2,70 ppm (M, $H_4$ pipéridine et $H_4$ pipéridinium) - 6H à 1,75 ppm et 2H à 1,50 ppm (2M, $H_3$ et $H_5$ pipéridinium $H_3$ et $H_5$ pipéridine) - 6H à 1,44 ppm (2S, $(CH_3)_2C$).

**RMN n° 130 - (b):**

1H à 8,75 ppm (D, J = 9 Hz, CONH) - 3H à 8,20 ppm (S.e., $CH_2N^{\oplus}H_3$) - 1H à 7,80 ppm (D, J = 8 Hz, HAr 6') - 2H à 7,45 ppm (M, HAr 3', 5') 1H à 7,00 ppm (S, H thiazole) - 1H à 6,00 ppm (D de D, $J_1$ = 9 Hz, $J_2$ = 4 Hz, $H_7$) - 1H à 5,42 ppm (D, J = 13 Hz, $CH_2OCO$) - 1H à 5,00 ppm (D, J = 4 Hz, $H_6$) - 1H à 4,80 ppm (D, J = 13 Hz, $CH_2OCO$) - 3H à 4,00 ppm (M, $CH_2N^{\oplus}H_3$ et $CH_2SO$) - 1H à 3,75 ppm (D, J = 17 Hz, $CH_2SO$) - 3H à 2,45 ppm (S, $CH_3Ar$) - 6H à 1,45 ppm (2S, $(CH_3)_2C$).

**RMN n° 131 - (b):**

1H à 8,85 ppm (D, J = 9 Hz, CONH) - 3H à 8,20 ppm (S.e., $CH_2N^{\oplus}H_3$) - 1H à 7,80 ppm (D, J = 8 Hz, HAr 6') - 2H à 7,45 ppm (M, HAr 3', 5') - 1H à 6,95 ppm (S, H thiazole) - 1H à 6,00 ppm (D de D, $J_1$ = 9 Hz, $J_2$ = 4 Hz, $H_7$) - 1H à 5,45 ppm (D, J = 13 Hz, $CH_2OCO$) - 1H à 5,00 ppm (D, J = 4 Hz, $H_6$) - 1H à 4,80 ppm (D, J = 13 Hz, $CH_2OCO$) - 3H à 4,00 ppm (M, $CH_2N^{\oplus}H_3$ et $CH_2SO$) - 1H à 3,73 ppm (D, J = 17 Hz, $CH_2SO$) - 3H à 2,50 ppm (S, $CH_3$ Ar) - 4H à 2,40 ppm

(M, 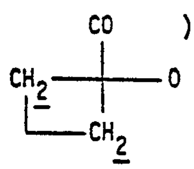 ) -

2H à 1,80 ppm

(M, 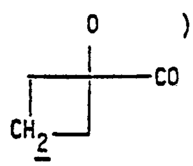 ) -

**RMN n° 132 - (b):**

1H à 8,80 ppm (D, J = 9 Hz, CON$\underline{H}$) - 3H à 8,10 ppm (S.e., $CH_2N^{\oplus}\underline{H_3}$) - 1H à 7,55 ppm (S, $\underline{H}$Ar) - 1H à 7,45 ppm (S, $\underline{H}$Ar) - 1H à 7,00 ppm (S, H thiazole) - 1H à 6,00 ppm (D de D, $J_1$ = 9 Hz, $J_2$ = 4 Hz, $\underline{H_7}$) - 1H à 5,40 ppm (D, J = 13 Hz, $C\underline{H_2}OCO$) - 1H à 5,00 ppm (D, J = 4 Hz, $\underline{H_6}$) - 1H à 4,83 ppm (D, J = 13 Hz, $C\underline{H_2}OCO$) - 3H à 3,70 ppm (M, $C\underline{H_2}N^{\oplus}H_3$ et $C\underline{H_2}SO$) - 1H à 3,70 ppm (D, J = 17 Hz, $C\underline{H_2}SO$) - 3H à 2,40 ppm (S, $C\underline{H_3}$ Ar) - 3H à 2,34 ppm (S, $C\underline{H_3}Ar$) - 6H à 1,45 ppm (2S, $(C\underline{H_3})_2C$).

**RMN n° 133 - (b):**

1H à 12,5 ppm (S.e., Ar N$\underline{H}$CO) - 1H à 8,78 ppm (D, J = 9 Hz, CON$\underline{H}$) - 3H à 8,30 ppm (S.e.,
- CH
|
$CH_3$
$N^{\oplus}\underline{H_3}$) - 1H à 8,20 ppm (S, $\underline{H}$ thiazole en 3) - 1H à 7,00 ppm (S, $\underline{H}$ thiazole) - 1H à 6,00 ppm (D de D, $J_1$ = 9 Hz, $J_2$ = 4 Hz, $\underline{H_7}$) - 1H à 5,42 ppm (2D, J = 13 Hz, $C\underline{H_2}OCO$) - 1H à 5,00 ppm (D, J = 4 Hz, $\underline{H_6}$) - 1H à 4,90 ppm (D, J = 13 Hz, $C\underline{H_2}OCO$) - 1H à 4,15 ppm (M,
C$\underline{H}$
|
$CH_3$
$N^{\oplus}H_3$) - 1H à 4,00 ppm (D, J = 17 Hz, $C\underline{H_2}SO$) - 1H à 3,76 ppm (D, J = 17 Hz, $C\underline{H_2}SO$) - 6H à 1,44 ppm (2S, $(C\underline{H_3})_2C$) - 3H à 1,40 ppm (D, J = 7 Hz, $C\underline{H_3}CH$).

**RMN n° 134 - (b):**

1H à 8,85 ppm (D, J = 9 Hz, CON$\underline{H}$) - 1H à 8,50 ppm (S, $\underline{H}$ thiazole en 3) - 3H à 7,90 ppm (S.e., $CH_2N^{\ominus}\underline{H_3}$) - 1H à 7,00 ppm (S, $\underline{H}$ thiazole) - 1H à 6,00 ppm (D de D, $J_1$ = 9 Hz, $J_2$ = 4 Hz, $\underline{H_7}$) - 1H à 5,42 ppm (D, J = 13 Hz, $C\underline{H_2}OCO$) - 1H à 4,99 ppm (D, J = 4 Hz, $\underline{H_6}$) - 1H à 4,84 ppm (D, J = 13 Hz, $C\underline{H_2}OCO$) - 1H à 4,00 ppm (D, J = 17 Hz, $C\underline{H_2}SO$) - 1H à 3,75 ppm (D, J = 17 Hz, $C\underline{H_2}SO$) - 4H à 3,20 ppm (M, $C\underline{H_2}C\underline{H_2}N^{\ominus}H_3$) - 6H à 1,45 ppm (2S, $(C\underline{H_3})_2C$).

**RMN n° 135 - (b):**

1H à 8,80 ppm (D, J = 9 Hz, CON$\underline{H}$) - 1H à 8,57 ppm (S, $\underline{H}$ thiazole en 3) - 3H à 8 50 ppm (S.e., $CH_2N^{\ominus}\underline{H_3}$) - 1H à 7,00 ppm (S, $\underline{H}$ thiazole) - 1H à 6,00 ppm (D de D, $J_1$ = 9 Hz, $J_2$ = 4 Hz, $\underline{H_7}$) - 1H à 5,40 ppm (D, J = 13 Hz, $C\underline{H_2}OCO$) - 1H à 5,00 ppm (D, J = 4 Hz, $\underline{H_6}$) - 1H à 4,90 ppm (D, J = 13 Hz, $C\underline{H_2}OCO$) - 2H à 4,45 ppm (M, $C\underline{H_2}N^{\ominus}H_3$) - 1H à 4,00 ppm (D, J = 17 Hz, $C\underline{H_2}SO$) - 1H à 3 72 ppm (D, J = 17 Hz, $C\underline{H_2}SO$) - 6H à 1,45 ppm (2S, $(C\underline{H_3})_2C$).

**RMN n° 136 - (b):**

1H à 8,90 ppm (D, J = 9 Hz, CON$\underline{H}$) - 1H à 8,60 ppm (S, $\underline{H}$ thiazole en 3) - 1H à 8,50 ppm (S.e., $CH_2N^{\ominus}\underline{H_3}$) - 1H à 5,98 ppm (S, $\underline{H}$ thiazole) - 1H à 6,00 ppm (D de D, $J_1$ = 9 Hz, $J_2$ = 4 Hz, $\underline{H_7}$) - 1H à 5,42 ppm (D, J = 13 Hz, $C\underline{H_2}OCO$) - 1H à 5,00 ppm (D, J = 4 Hz, $\underline{H_6}$) - 1H à 4,84 ppm (D, J = 13 Hz, $C\underline{H_2}OCO$) - 2H à 4,45 ppm (M, $C\underline{H_2}N^{\oplus}H_3$) - 1H à 4,00 ppm (D, J = 17 Hz, $C\underline{H_2}SO$) -1H à 3,72 ppm (D, J = 17 Hz, $C\underline{H_2}SO$) - 4H à 2,40 ppm

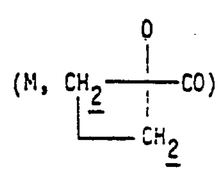

- 2H à 1,86 ppm

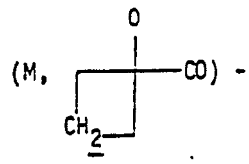

**RMN n° 137 - (b):**

1H à 8,90 ppm (T, J = 8 Hz,

||

NHCH$_2$) - 1H à 8,75 ppm (D, J = 9 Hz, CONH) - 3H à 8,00 ppm (S.e., CH$_2$N$^{\oplus}$H$_3$) - 2H à 7,88 ppm (D, J = 8 Hz, HAr 2', 6') - 2H à 7,40 ppm (D, J = 8 Hz, HAr 3', 5') - 1H à 6,96 ppm (S, H thiazole) - 1H à 6,00 ppm (D de D, $J_1$ = 9 Hz, $J_2$ = 4 Hz, $H_7$) - 1H à 5,44 ppm (D, J = 13 Hz, CH$_2$OCO) - 1H à 4,98 ppm (D, J = 4 Hz, $H_6$) - 1H à 4,80 ppm (D, J = 13 Hz, CH$_2$OCO) - 2H à 4,40 ppm (D, J = 8 Hz, Ar CH$_2$NH) - 1H à 4,06 ppm et 1H à 3,70 ppm (D, J = 17 Hz, CH$_2$S → 0) - 2H à 3,57 ppm (M, OC CH$_2$N$^{\oplus}$H$_3$) - 2H à 1,47 ppm (2S, (CH$_3$)$_2$C).

En opérant comme dans l'exemple 2 ou l'exemple 3, on obtient les composés selon l'invention, sous forme de trifluoracétates décrits dans le tableau II ci-après.

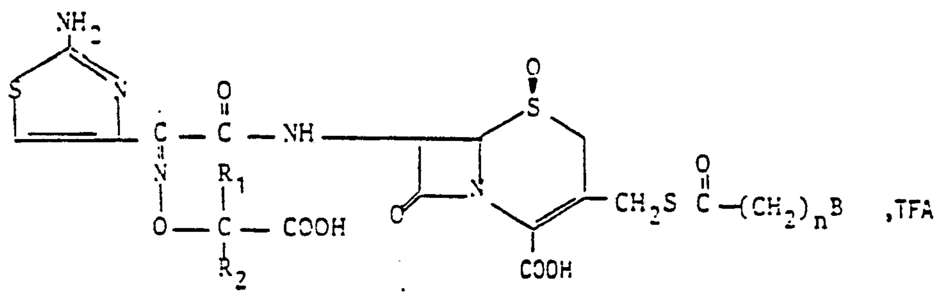

Ces composés sont identifiés par un numéro de référence et on donne pour chacun d'eux les valeurs de $R_1$, $R_2$, n et B et le spectre de RMN.

On donne également l'éluant de chromatographie qui sert pour isoler (V): dernier produit intermédiaire avant déblocage des fonctions acides et amines de la molécule. Cet intermédiaire V est caractérisé par son spectre infra-rouge, les longueurs d'ondes indiquées en cm$^{-1}$ correspondent dans l'ordre aux fréquences de vibrations d'élongation du carbonyl en 8 du bêta lactame, des esters tertiobutyliques et du thioester en 3, de l'amide en et du carbamate protecteur de l'amine. Lorsque 2 longueurs d'onde seulement sont indiquées la seconde correspond à une bande large qui recouvre les fréquences de vibration d'élongation à la fois des esters, de l'amide et du carbamate protecteur de l'amine et du thioester.

Il arrive pour certains produits que la fréquence de vibration du thioester soit à la même longueur d'onde que celle des esters tertiobutyliques. Cela est indiqué dans le tableau par + COS en face de la fréquence de vibration correspondante.

**Tableau II**

| n°SR | n | 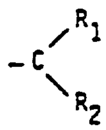 | B | Eluant de chromatographie de l'intermédiaire V vol/vol | IR $\nu$ CO $cm^{-1}$ intermédiaire V | n° RMN |
|---|---|---|---|---|---|---|
| 41 970 | 0 | 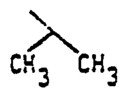 | $-(CH_2)_3\ NH_2$ | $CH_2Cl_2$ 90<br>Ac O Et 10 | 1800<br>1720<br>1690 | 1 |
| 41 971 | " | " | $-(CH_2)_4\ NH_2$ | $CH_2Cl_2$ 90<br>Ac O Et 10 | 1800<br>1715<br>1690 | 2 |
| 41 972 | " | " | $-(CH_2)_5\ NH_2$ | $CH_2Cl_2$ 92<br>Ac O Et 8 | 1800<br>1720<br>1690 | 3 |
| 41 973 | " | " | 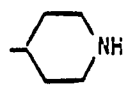 | $CH_2Cl_2$ 90<br>Ac O Et 10 | 1800<br>1725<br>1690 | 4 |
| 42 074 | " | " | 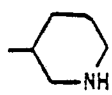 (R) | $CH_2Cl_2$ 90<br>Ac O Et 10 | 1800<br>1725<br>1690 | 5 |
| 42 076 | " | " | $-(CH_2)_3\ NH\ CH_3$ | $CH_2Cl_2$ 90<br>Ac O Et 10 | 1800<br>1725<br>1690 | 6 |
| 42 077 | " | " | $-(CH_2)_7\ NH_2$ | $CH_2Cl_2$ 95<br>Ac O Et 5 | 1800<br>1720<br>1690 | 7 |

| n°SR | n | 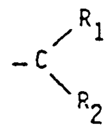 | B | Eluant de chromatographie de l'intermédiaire V. vol/vol | IR ∂CO $cm^{-1}$ intermédiaire V | n° RMN |
|---|---|---|---|---|---|---|
| 42 118 | 0 | 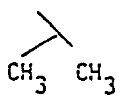 | 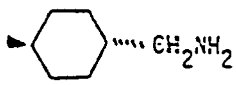 | $CH_2Cl_2$ 95<br>Ac O Et 5 | 1803<br>1720<br>1690 | 8 |
| 42 119 | " | " | 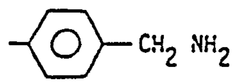 | $CH_2Cl_2$ 92,5<br>Ac O Et 7,5 | 1805<br>1720 | 9 |
| 42 187 | " | " | 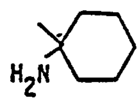 | $CH_2Cl_2$ 95<br>Ac O Et 5 | 1805<br>1725 | 10 |
| 42 199 | " | $- CH_2 -$ | 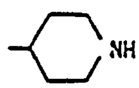 | $CH_2Cl_2$ 90<br>Ac O Et 10 | 1800<br>1725<br>1690 | 11 |
| 42 218 | " | 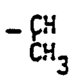 | " | $CH_2Cl_2$ 92,5<br>Ac O Et 7,5 | 1805<br>1725<br>1690 | 12 |
| 42 219 | " | 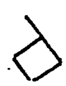 | 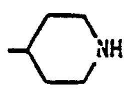 | $CH_2Cl_2$ 92,5<br>Ac O Et 7,5 | 1805<br>1725<br>1690 | 13 |
| 42 220 | " | $- CH_2$ | 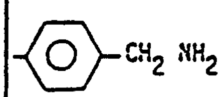 | $CH_2Cl_2$ 95<br>Ac O Et 5 | 1800<br>1720 | 14 |
| 42 221 | " | 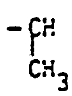 | " | $CH_2Cl_2$ 95<br>Ac O Et 5 | 1800<br>1720 | 15 |
| 42 222 | " | 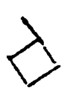 | " | $CH_2Cl_2$ 95<br>Ac O Et 5 | 1805<br>1720 | 16 |

| n° SR | n | 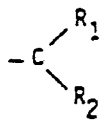 | B | Eluant de chromatographie de l'intermédiaire V vol/vol | IR $\nu$ CO $cm^{-1}$ intermédiaire V | n° RMN |
|---|---|---|---|---|---|---|
| 42 531 | 0 | 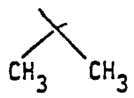 | 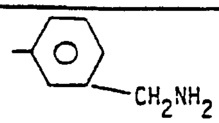 | $CH_2$ $Cl_2$ 100<br>Me OH 0,5 | 1800<br>1720 | 17 |
| 42 532 | " | 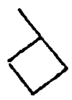 | 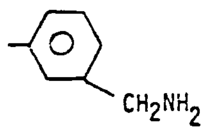 | $CH_2$ $Cl_2$ 100<br>Me OH 0,5 | 1800<br>1720 | 18 |
| 42 533 | " | 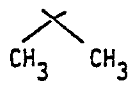 | 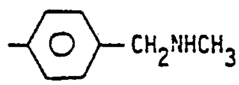 | $CH_2$ $Cl_2$ 100<br>Me OH 0,5 | 1800<br>1720<br>1690 | 19 |
| 42 534 | " | 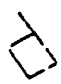 | 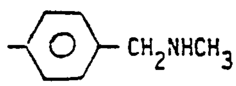 | $CH_2$ $Cl_2$ 100<br>Me OH 0,5 | 1800<br>1720<br>1690 | 20 |
| 42 535 | " | 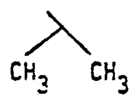 | 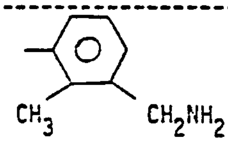 | $CH_2$ $Cl_2$ 95<br>Ac O Et 5 | 1800<br>1720 | 21 |
| 42 536 | " | 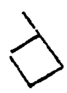 | 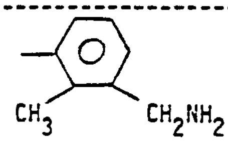 | $CH_2$ $Cl_2$ 95<br>Ac O Et 5 | 1800<br>1720 | 22 |
| 42 659 | " | 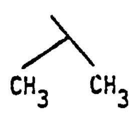 | 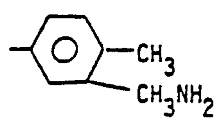 | $CH_2$ $Cl_2$ 100<br>Me OH 0,4 | 1802<br>1720 | 23 |
| 42 660 | " | 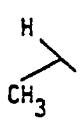 | 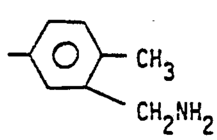 | $CH_2$ $Cl_2$ 100<br>Me OH 0,5 | 1802<br>1720 | 24 |
| 42 661 | " | 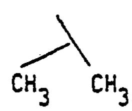 | 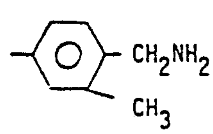 | $CH_2$ $Cl_2$ 95<br>Ac O Et 5 | 1802<br>1720 | 25 |

| n° SR | n | 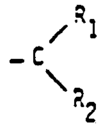 | B | Eluant de chromatographie de l'intermédiaire V vol/vol | IR ∂ CO $cm^{-1}$ intermédiaire V | n° RMN |
|---|---|---|---|---|---|---|
| 42 662 | 0 | 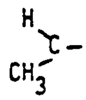 | 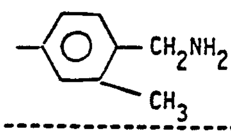 | $CH_2Cl_2$ 95<br>Ac O Et 5 | 1802<br>1720 | 26 |
| 42 663 | " | 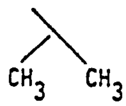 | 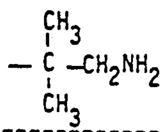 | $CH_2Cl_2$ 100<br>Me OH 0,4 | 1802<br>1720<br>1680 | 27 |
| 42 664 | " | " | 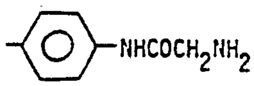 | $CH_2Cl_2$ 95<br>Ac O Et 5 | 1802<br>1720 | 28 |
| 42 665 | " | 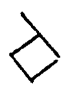 | 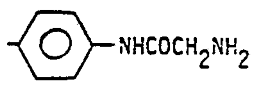 | $CH_2Cl_2$ 95<br>Ac O Et 5 | 1802<br>1720 | 29 |
| 42 672 | 1 | 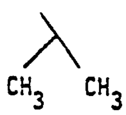 | 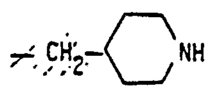 | $CH_2Cl_2$ 90<br>Ac O Et 10 | 1802<br>1720<br>1680 $CH_2Cl_2$ | 30 |
| 42 673 | 0 | " | 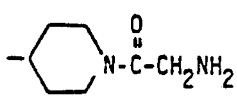 | $CH_2Cl_2$ 100<br>Me OH 1 | 1802<br>1715<br>1690 $CH_2Cl_2$ | 31 |
| 42 674 | " | 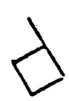 | 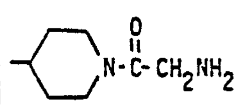 | $CH_2Cl_2$ 100<br>Me OH 1 | 1802<br>1715<br>1690 $CH_2Cl_2$ | 32 |
| 42 685 | " | 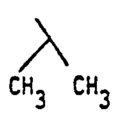 | 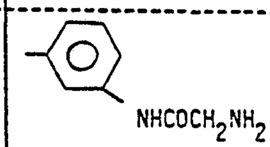 | $CH_2Cl_2$ 95<br>Ac O Et 5 | 1805<br>1725<br>1695 | 33 |
| 42 686 | " | 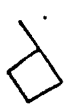 | 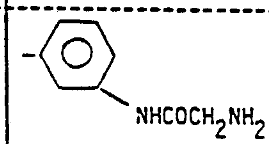 | $CH_2Cl_2$ 95<br>Ac O Et 5 | 1805<br>1725<br>1695 | 34 |
| 42 847 | " | 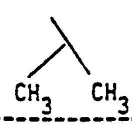 | 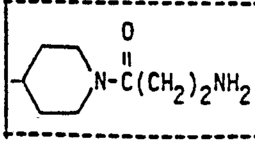 | $CH_2Cl_2$ 100<br>Me OH 1 | 1800<br>1710 $CH_2Cl_2$ | 35 |

| n° SR | n | 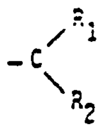 | B | Eluant de chromatographie de l'intermédiaire V vol/vol | IR $\nu$ CO $cm^{-1}$ intermédiaire V | n° RMN |
|---|---|---|---|---|---|---|
| 42 850 | 0 | 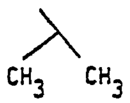 | 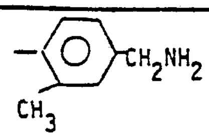 | $CH_2Cl_2$ 100<br>Me OH 0,7 | 1805<br>1720 | 36 |
| 42 851 | " | 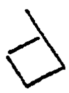 | 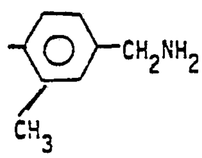 | $CH_2Cl_2$ 100<br>Me OH 0,7 | 1805<br>1720 | 37 |
| 42 853 | " | 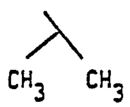 | 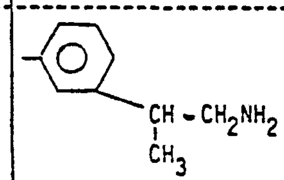 | $CH_2Cl_2$ 95<br>Ac O Et 5 | 1802<br>1720 | 38 |
| 42 854 | " | 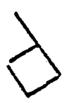 | 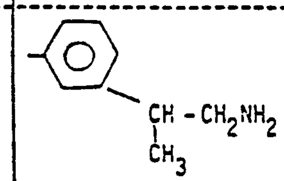 | $CH_2Cl_2$ 100<br>Me OH 0,4 | 1802<br>1720 | 39 |
| 42 856 | " | 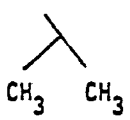 | 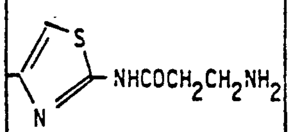 | $CH_2Cl_2$ 85<br>Ac O Et 15 | 1803<br>1720<br>1685 | 40 |
| 42 858 | " | " | 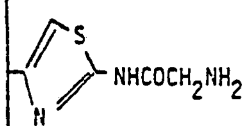 | $CH_2Cl_2$ 100<br>Me OH 1 | 1802<br>1725<br>1690 | 41 |
| 42 859 | " | " | 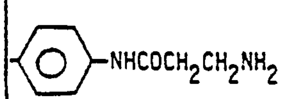 | $CH_2Cl_2$ 70<br>Ac O Et 30 | 1802<br>1720<br>1690 | 42 |
| 42 860 | " | 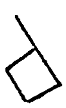 | 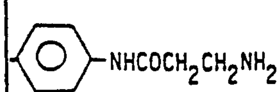 | $CH_2Cl_2$ 70<br>Ac O Et 30 | 1802<br>1720 | 43 |

| n° SR | n | 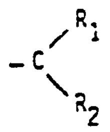 | B | Eluant de chromatographie de l'intermédiaire V vol/vol | IR $\nu$ CO $cm^{-1}$ intermédiaire V | n° RMN |
|---|---|---|---|---|---|---|
| 42 863 | 0 | 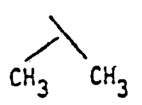 | 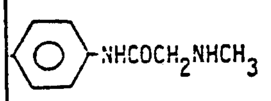 | $CH_2Cl_2$ 90<br>Ac O Et 10 | 1800<br>1720<br>1690 | 44 |
| 42 868 | " | " | 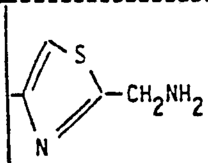 | $CH_2Cl_2$ 100<br>Me OH 0,7 | 1802<br>1720 | 45 |
| 42 902 | " | " | 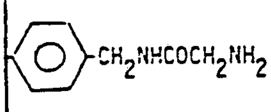 | $CH_2Cl_2$ 100<br>Me OH 1 | 1802<br>1720<br>1670 | 46 |

**Spectres de RMN:**

Les spectres sont enregistrés à 60 MHz, signalés par (a) ou à 250 MHz, signalés par (b); lorsqu'il existe deux diastéréoisomères dans la molécule, les signaux dédoublés sont indiqués par *.

**RMN n° 1 - (a):**

8H entre 6 et 9 ppm (signal large, $C\underline{H}_2$, TFA, $CO_2\underline{H}$) - 1H à 8,40 ppm (D, J = 9 Hz, $CON\underline{H}$) - 1H à 6,86 ppm (S, $\underline{H}$ thiazole) - 1H à 6,00 ppm (D de D, $J_1$ = 9 Hz, $J_2$ = 4 Hz, $\underline{H_7}$) - 1H à 4,97 ppm (D, J = 4 Hz, $\underline{H_6}$) - 1H à 4,20 ppm (AB, $J_{AB}$ = 13 Hz, $C\underline{H}_2SCO$) - 3H à 3,70 ppm (M, $CH_2SCO$ et $CH_2SO$) - 4H à 2,75 ppm (M, $C\underline{H}_2NH_2$ et $C\underline{H}_2COS$) - 2H à 1,77 ppm (M, $CH_2C\underline{H}_2CH_2$) - 6H à 1,45 ppm (S, $(C\underline{H}_3)_2C$).

**RMN n° 2 - (a):**

8H entre 6,5 et 9 ppm (signal large, $CO_2\underline{H}$, TFA, $N\underline{H}_2$) - 1H à 8,40 ppm (D, J = 9 Hz, $CON\underline{H}$) - 1H à 6,88 ppm (S, $\underline{H}$ thiazole) - 1H à 6,0 ppm (D de D, $J_1$ = 9 Hz, $J_2$ = 4 Hz, $\underline{H_7}$) - 1H à 5,0 ppm (D, J = 4 Hz, $\underline{H_6}$) - 1H à 4,20 ppm (A de AB, J = 13 Hz, $C\underline{H}_2SCO$) - 3H à 3,80 ppm (M, $C\underline{H}_2SO$ et $C\underline{H}_2SCO$) - 4H à 2,65 ppm (M, $C\underline{H}_2NH_2$ et $C\underline{H}_2COS$) - 10H à 1,45 ppm (S.e., $(C\underline{H}_3)_2C$ et $CH_2$ $(C\underline{H}_2)_2CH_2$).

**RMN n° 3 - (a):**

8H entre 6,5 et 8,7 ppm (signal large, ($N\underline{H}_2$, $CO_2\underline{H}$, TFA) - 1H à 8,40 ppm (D, J = 9 Hz, $CON\underline{H}$) - 1H à 6,87 ppm (S, $\underline{H}$ thiazole) - 1H à 6,0 ppm (D de D, $J_1$ = 9 Hz, $J_2$ = 4 Hz, $\underline{H_7}$) - 1H à 5,0 ppm (D, $J_1$ = 4 Hz, $\underline{H_6}$) - 1H à 4,20 ppm (A de AB, J = 13 Hz, $C\underline{H}_2SO$) - 3H à 3,70 ppm (M, $C\underline{H}_2SCO$ et $C\underline{H}_2SO$) - 4H à 2,80 ppm (M, $C\underline{H}_2NH_2$ et $C\underline{H}_2CO$) 12H à 1,45 ppm (S.e., $(C\underline{H}_3)_2C$ et $CH_2$ $(C\underline{H}_2)_3CH_2$).

**RMN n° 4 (a):**

7H entre 6,5 et 9,5 ppm (signal large, $N\underline{H}_2$, $N\underline{H}$, $CO_2\underline{H}$, TFA) - 1H à 8,40 ppm (D, J = 9 Hz, $CON\underline{H}$) - 1H à 6,90 ppm (S, $\underline{H}$ thiazole) - 1H à 6,0 ppm (D de D, $J_1$ = 9 Hz, $J_2$ = 4 Hz, $\underline{H}_7$) - 1H à 5,0 ppm (D, J = 4 Hz, $\underline{H}_6$) - 1H à 4,20 ppm (A de AB, $J_{AB}$ = 13 Hz, $C\underline{H}_2SCO$) - 3H à 3,70 ppm (M, $C\underline{H}_2SCO$ et $C\underline{H}_2SO$) - 5H à 3,0 ppm (M, $C\underline{H}_2N$ et $C\underline{H}COS$) - 4H à 1,90 ppm (M, $C\underline{H}_2CH_2N$) - 6H à 1,45 ppm (S, $(C\underline{H}_3)_2C$).

**RMN n° 5 - (b):**

5H entre 7 et 9 ppm (signal large, $N\underline{H}_2$, TFA, $N\underline{H}$) - 1H à 8,34 ppm (2D, J = 9 Hz, $CON\underline{H}$)* - 1H à 6,8 ppm (S, $\underline{H}$ thiazole) - 1H à 5,97 ppm (D de D, $J_1$ = 9 Hz, $J_2$ = 4 Hz, $\underline{H}_7$) - 1H à 4,95 ppm (D, J = 4 Hz, $\underline{H}_6$) - 1H à 4,16 ppm (2D, J = 13 Hz, $C\underline{H}_2SCO$)*-1H à 3,76 ppm (D, J = 13 Hz, $C\underline{H}_2SCO$) - 2H à 3,66 ppm (S, $C\underline{H}_2SO$) - 1H à 3,4 ppm, 1H à 3,16 ppm et 2H à 2,95 ppm (M, $C\underline{H}_2N$) - 1H à 2,80 ppm (M, $C\underline{H}COS$) - 4H entre 1,5 et 2,1 ppm (M, $C\underline{H}_2C\underline{H}_2CH_2N$) - 6H à 1,44 ppm (S, $(C\underline{H}_3)_2C$).

**RMN n° 6 - (a):**

3H à 8,60 ppm (S.e., $CON\underline{H}$, $N^{\oplus}H_2$) - 3H à 7,80 ppm (S.e., $N^{\oplus}\underline{H}_3$) - 1H à 6,85 ppm (S, $\underline{H}$ thiazole) - 1H à 6,00 ppm (D de D, $J_1$ = 9 Hz, $J_2$ = 4 Hz, $\underline{H}_7$) - 1H à 5,00 ppm (D, J = 4 Hz, $\underline{H}_6$) - 1H à 4,15 ppm (A de AB, $J_{AB}$ = 13 Hz, $C\underline{H}_2SCO$) - 1H à 3,80 ppm (B de AB, $J_{AB}$ = 13 Hz, $C\underline{H}_2SCO$) - 2H à 3,70 ppm (S.e., $C\underline{H}_2SO$) - 7H à 2,50 ppm (M, $C\underline{H}_3NH$, $C\underline{H}_2NH$, $C\underline{H}_2$
CS) - 2H à 1,80 ppm (M, $CH_2C\underline{H}_2CH_2NH$) - 6H à 1,45 ppm (2S, $(C\underline{H}_3)_2C$).
|

**RMN n° 7 - (a):**

1H à 8,35 ppm (D, J = 9 Hz, $CON\underline{H}$) - 8H entre 6,5 et 10 ppm ($CO_2\underline{H}$, $N\underline{H}_2$, TFA) - 1H à 6,82 ppm (S, $\underline{H}$ thiazole) - 1H à 6,00 ppm (D de D, $J_1$ = 9 Hz, $J_2$ = 4 Hz, $\underline{H}_7$) - 1H à 5,00 ppm (D, J = 4 Hz, $\underline{H}_6$) - 1H à 4,15 ppm (A de AB, $J_{AB}$ = 13 Hz, $C\underline{H}_2SCO$)-3H à 3,66 ppm (M, $C\underline{H}_2SO$ et B de AB, $C\underline{H}_2SCO$) - 4H à 2,65 ppm (M, $C\underline{H}_2CO$ et $C\underline{H}_2NH_2$) - 6H à 1,42 ppm (S, $(C\underline{H}_3)_2C$) - 10H à 1,25 ppm (S.e., $(C\underline{H}_2)_5CH_2NH_2$).

**RMN n° 8 - (b):**

1H à 8,34 ppm (D, J = 9 Hz, $CON\underline{H}$) - 3H à 7,80 ppm (S.e., $N^{\oplus}\underline{H}_3$) - 3H à 7,40 ppm (S.e., $N^{\oplus}\underline{H}_3$) - 1H à 6,78 ppm (S, $\underline{H}$ thiazole) - 1H à 5,94 ppm (D de D, $J_1$ = 9 Hz, $J_2$ = 4 Hz, $\underline{H}_7$) - 1H à 4,94 ppm (D, J = 4 Hz, $\underline{H}_6$) - 1H à 4,14 ppm (D, J = 13 Hz, $C\underline{H}_2$ SCO) - 1H à 3,69 ppm (D, J = 13 Hz, $C\underline{H}_2SO$) - 2H à 3,63 ppm (S, $C\underline{H}_2SO$) - 2H à 2,60 ppm (M, $C\underline{H}_2NH_2$) -1H à 2,45 ppm (M, $C\underline{H}COS$) - 4H à 1,84 ppm (M, $C\underline{H}_2CHCOS$) - 6H à 1,44 ppm (2S, $(C\underline{H}_3)_2C$) - 2H à 1,40 ppm et 2H à 1,0 ppm (M, $C\underline{H}_2CHCH_2NH_2$).

**RMN n° 9 - (b):**

1H à 8,36 ppm (D, J = 9 Hz, $N\underline{H}CO$) - 3H à 8,30 ppm (S.e., $N^{\oplus}\underline{H}_3$) - 2H à 7,94 ppm (D, J = 8 Hz, $\underline{H}$ ortho CO) - 2H à 7,55 ppm (D, J = 8 Hz, $\underline{H}$ méta CO) - 3H à 7,40 ppm (S.e., $N^{\oplus}\underline{H}_3$) - 1H à 6,76 ppm (S, $\underline{H}$ thiazole) - 1H à 5,95 ppm (D de D, $J_1$ = 9 Hz, $J_2$ = 4 Hz, $\underline{H}_7$) - 1H à 4,95 ppm (D, J = 4 Hz, $\underline{H}_6$) - 1H à 4,40 ppm (D, J = 13 Hz, $C\underline{H}_2SCO$) - 2H à 4,10 ppm (M, $C\underline{H}_2NH_2$) - 1H à 3,90 ppm (D, J = 13 Hz, $C\underline{H}_2SCO$) - 2H à 3,74 ppm (S, $C\underline{H}_2SO$) - 6H à 1,42 ppm (2S, $(C\underline{H}_3)_2C$).

**RMN n° 10 - (a):**

1H à 8,30 ppm (D, J = 9 Hz, $CON\underline{H}$) - 8H entre 6,5 et 9 ppm (S.e., $N^{\oplus}\underline{H}_3$, $CO_2\underline{H}$) - 1H à 6,80 ppm (S, $\underline{H}$ thiazole) - 1H à 5,95 ppm (M, $\underline{H}_7$) - 1H à 4,90 ppm (D, J = 4 Hz, $\underline{H}_6$) - 1H à 4,25 ppm (A de AB, $J_{AB}$ = 13 Hz, $C\underline{H}_2SCO$) - 1H à 3,90 ppm (B de AB, $J_{AB}$ = 13 Hz, $C\underline{H}_2SCO$) - 2H à 3,65 ppm (S.e., $C\underline{H}_2SO$) - 10H entre 1 et 2,3 ppm (M,

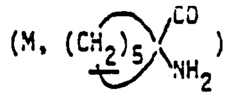

- 6H à 1,43 ppm (S, $(CH_3)_2C$).

**RMN n° 11 -(b):**

1H à 8,79 ppm (D, J = 9 Hz, CONH) - 1H à 8,60 ppm et 1H à 8,40 ppm (S.e., $N^{\oplus}H_2$) - 3H à 7,30 ppm (S.e., $N^{\oplus}H_3$) - 1H à 6,84 ppm (S, H thiazole) - 1H à 5,84 ppm (D de D, $J_1$ = 9 Hz, $J_2$ = 4 Hz, $H_7$) - 1H à 4,90 ppm (D, J = 4 Hz, $H_6$) - 2H à 4,55 ppm (S, $CH_2ON$) - 1H à 4,15 ppm (D, J = 13 Hz, $CH_2SCO$) - 1H à 3,74 ppm (D, J = 13 Hz, $CH_2SCO$) - 2H à 3,54 ppm (S, $CH_2SO$) - 2H à 3,20 ppm et 3H à 2,90 ppm (M, $CH_2NH$ et CHCOS) - 2H à 1,95 ppm et 2H à 1,65 ppm (M, $CH_2CH_2NH$).

**RMN n° 12 - (b):**

2H à 8,60 ppm (M, CONH, $N^{\oplus}H_2$) - 1H à 8,40 ppm (S.e., $N^{\oplus}H_2$) - 3H à 7,30 ppm (S.e., $N^{\oplus}H_3$) - 1H à 6,81 ppm (2S, H thiazole)* - 1H à 5,92 ppm (D de D, $J_1$ = 9 Hz, $J_2$ = 4 Hz, $H_7$) - 1H à 4,92 ppm (2D superposés, $H_6$)* - 1H à 4,60 ppm (Q, J = 7 Hz, CHON) - 1H à 4,16 ppm (D, J = 13 Hz, $CH_2SCO$) = 1H à 3,75 ppm (D, J = 13 Hz, $CH_2SCO$) 2H à 3,65 ppm (S, $CH_2SO$) - 2H à 3,25 ppm et 3H à 2,95 ppm (M, CHCOS et

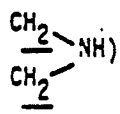

- 2H à 1,95 ppm et 2H à 1 70 ppm (M,

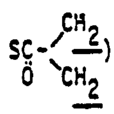

- 3H à 1,45 ppm (D, J = 7 Hz, $CH_3CH$).

**RMN n° 13 - (b):**

2H à 8,70 ppm (M, CONH et $N^{\oplus}H_2$) - 1H à 8,40 ppm (M, $N^{\oplus}H_2$) - 3H à 7,40 ppm (S.e., $N^{\oplus}H_3$) - 1H à 6,79 ppm (S, H thiazole) - 1H à 5,90 ppm (D de D, $J_1$ = 9 Hz, $J_2$ = 4 Hz, $H_7$) - 1H à 4,94 ppm (D, J = 4 Hz, $H_6$) - 1H à 4,16 ppm (D, J = 13 Hz, $CH_2SCO$) - 1H à 3,74 ppm (D, J = 13 Hz, $CH_2SCO$) - 2H à 3,70 ppm (S, $CH_2SO$) - 2H à 3,25 ppm (M, $CH_2NH$) - 3H à 2,90 ppm (M, $CH_2NH$ et CHCOS) - 4H à 2,40 ppm (M,

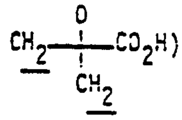

- 6H à 1,80 ppm (M,

(M, 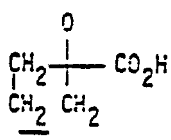 et 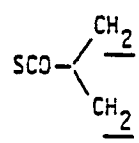 ).

**RMN n° 14 - (b):**

1H à 8,65 ppm (D, J = 9 Hz, CONH) - 3H à 8,20 ppm (S.e., $NH_3$) - 2H à 7,92 ppm (D, J = 8 Hz, H ortho CO) - 2H à 7,56 ppm (D, J = 8 Hz, H méta CO) - 3H à 7,30 ppm S.e., $N^{\oplus}H_3$) - 1H à 6,82 ppm (S, H thiazole) - 1H à 5,88 ppm (D, $J_1$ = 9 Hz, $J_2$ = 4 Hz, $H_7$) - 1H à 4,92 ppm (D, J = 4 Hz, $H_6$) - 2H à 4,55 ppm (S, $CH_2ON$) - 1H à 4,37 ppm (D, J = 13 Hz, $CH_2SCO$) - 2H à 4,13 ppm (M, $CH_2NH_2$) - 1H à 4,42 ppm (D, J = 13 Hz, $CH_2SCO$) - 2H à 3,74 ppm (S, $CH_2SO$).

**RMN n° 15 - (b):**

1H à 8,60 ppm (2D, J = 9 Hz, CONH)* - 3H à 8,20 ppm (S.e., $N^{\oplus}H_3$) - 2H à 7,92 ppm (D, J = 8 Hz, H ortho CO)- 2H à 7,56 ppm (D, J = 8 Hz, H méta CO) - 3H à 7,30 ppm (S.e., $N^{\oplus}H_3$) - 1H à 6,82 ppm (2S, H thiazole)* - 1H à 5,92 ppm (D de D, $J_1$ = 9 Hz, $J_2$ = 4 Hz, $H_7$) - 1H à 4,95 ppm (2D, superposés, $H_6$)* - 1H à 4,60 ppm (Q, J = 7 Hz, CHON) - 1H à 4,60 ppm (D, J = 13 Hz, $CH_2SCO$) - 2H à 4,13 ppm (M, $CH_2NH_2$) - 1H à 3,90 ppm (D, J = 13 Hz, $CH_2SCO$) - 2H à 3,75 ppm (S, $CH_2SO$) - 3H à 1,45 ppm (D, J = 7 Hz, $CH_3CH$).

**RMN n° 16 - (b):**

1H à 8,61 ppm (D, J = 9 Hz, CONH) - 5H à 8,40 ppm (S.e., $N^{\oplus}H_3$, $CO_2H$) - 2H à 7,95 ppm (D, J = 8 Hz, H ortho CO) - 2H à 7,61 ppm (D, j + 8 Hz, H méta CO) - 3H à 7,30 ppm (S.e., $N^{\oplus}H_3$) - 1H à 6,76 ppm (S, H thiazole) - 1H à 5,92 ppm (D de D, $J_1$ = 9 Hz, $J_2$ = 4 Hz, $H_7$) - 1H à 4,93 ppm (D, J = 4 Hz, $H_6$) - 1H à 4,42 ppm (D, J = 13 Hz, $CH_2SCO$) - 2H à 4,10 ppm (S.e., $CH_2NH_2$) - 1H à 3,92 ppm (D, J = 13 Hz, $CH_2SCO$) - 2H à 3,74 ppm (S, $CH_2SO$) - 4H à 2,35 ppm (M,

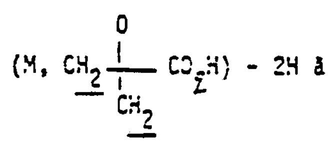

1,85 ppm (M,

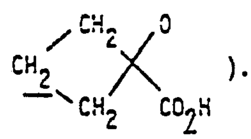

**RMN n° 17 - (b).**

1H à 8,62 ppm (D, J = 9 Hz, CONH) - 3H à 8,20 ppm (S.e., $CH_2N^{\oplus}H_3$) - 1H à 8,00 ppm (S.e., HAr 2') - 1H à 7,90 ppm (D, J = 8 Hz, HAr 6') - 1H à 7,72 ppm (D, J = 8 Hz, HAr 4') - 1H à 7,55 ppm (T, J = 8 Hz, H Ar 5') - 1H à 6,93 ppm (S, H thiazole) - 1H à 5,95 ppm (D de D, $J_1$ = 9 Hz, $J_2$ = 4 Hz, $H_7$) - 1H à 4,93 ppm (D, J = 4 Hz, $H_6$) - 1H à 4,43 ppm (D, J = 13 Hz, $CH_2SCO$) - 2H à 4,10 ppm (Q, J = 7 Hz, $CH_2N^{\oplus}H_3$) - 1H à 3,94 ppm (D, J = 13 Hz, $CH_2SCO$) - 2H à 3,75 ppm (S.e., $CH_2SO$) - 6H à 1,45 ppm (2S, $(CH_3)_2C$).

**RMN n° 18 - (b):**

1H à 8,80 ppm (D, J = 9 Hz, CONH) - 3H à 8,20 ppm (S.e., $CH_2N^{\oplus}H_3$) - 1H à 8,00 ppm (S.e., HAr 2') - 1H à 7,90 ppm (D, J = 8 Hz, HAr 6') - 1H à 7,72 ppm (D, J = 8 Hz, HAr 4') - 1H à 7,55 ppm (T, J = 8 Hz, HAr 5') - 1H à 6,92 ppm (S, H thiazole) - 1H à 5,94 ppm (D de D, $J_1$ = 9 Hz, $J_2$ = 4 Hz, $H_7$) - 1H à 4,96 ppm (D, J = 4 Hz, $H_6$) - 1H à 4,44 ppm (D, J = 13 Hz, $CH_2SCO$) - 2H à 4,10 ppm (Q, J = 7 Hz, $CH_2N^{\oplus}H_3$) - 1H à 3,94 ppm (D, J = 13 Hz, $CH_2SCO$) - 2H à 3,77 ppm (S.e., $CH_2SO$) - 4H à 2,40 ppm (M,

(M, 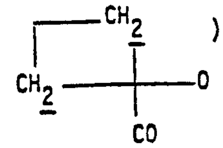 )

2H à 1,90 ppm (M,

(M, 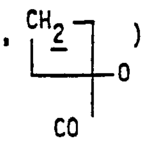 ) -

**RMN n° 19 - (b):**

2H à 8,80 ppm (S.e., $N^{\oplus}\underline{H_2}CH_3$) - 1H à 8,60 ppm (D, J = 9 Hz, $CON\underline{H}$) - 2H à 7,95 ppm (D, J = 8 Hz, $\underline{H}$Ar 2', 6') - 2H à 7,60 ppm (D, J = 8 Hz, Ar 3', 5') - 1H à 6,94 ppm (S, $\underline{H}$ thiazole) - 1H à 5,95 ppm (D de D, $J_1$ = 9 Hz, $J_2$ = 4 Hz, $\underline{H_7}$) - 1H à 4,95 ppm (D, J = 4 Hz, $\underline{H_6}$) - 1H à 4,42 ppm (D, J = 13 Hz, $C\underline{H_2}SCO$) - 2H à 4,16 ppm (T, J = 7 Hz, $C\underline{H_2}N^{\oplus}H_2CH_3$) - 1H à 3,92 ppm (D, J = 13 Hz, $C\underline{H_2}SCO$) - 2H à 3,75 ppm (S.e., $C\underline{H_2}SO$) - 3H à 2,55 ppm (T, J = 7 Hz, $CH_2N^{\oplus}H_2C\underline{H_3}$) - 6H à 1,45 ppm (2S, $(CH_3)_2C$).

**RMN n° 20 - (b):**

1H à 8,84 ppm (D, J = 9 Hz, $CON\underline{H}$) - 2H à 8,80 ppm (S.e., $N^{\oplus}\underline{H_2}$ - $CH_3$) - 2H à 7,95 ppm (D, J = 8 Hz, $\underline{H}$Ar, 2', 6') - 2H à 7,60 ppm (D, J = 8 Hz, $\underline{H}$Ar 3', 5') = 1H à 6,95 ppm (S, $\underline{H}$ thiazole) - 1H à 5,94 ppm (D de D, $J_1$ = 9 Hz, $J_2$ = 4 Hz, $\underline{H_7}$) - 1H à 4,95 ppm (D, J = 4 Hz, $\underline{H_6}$) - 1H à 4,45 ppm (D, J = 13 Hz, $CH_2SCO$) - 2H à 4,16 ppm (T, J = 7 Hz, $C\underline{H_2}N^{\oplus}H_2CH_3$) 1H à 3,92 ppm (D, J = 13 Hz, $C\underline{H_2}SCO$) - 2H à 3,75 ppm (S.e., $C\underline{H_2}SO$) 3H à 2,55 ppm (T, J = 7 Hz, $CH_2N^{\oplus}H_2$ - $C\underline{H_3}$) - 4H à 2,40 ppm (M,

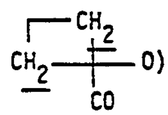 ) -

2H à 1,90 ppm (M,

(M, 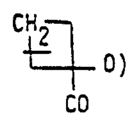 )

**RMN n° 21 - (b):**

1H à 8,66 ppm (D, J = 9 Hz, $CON\underline{H}$) - 3H à 8,10 ppm (S.e., $CH_2N^{\oplus}\underline{H_3}$) - 2H à 7,55 ppm (M, $\underline{H}$Ar 4', 6') - 1H à 7,37 ppm (T, J = 8 Hz, $\underline{H}$Ar 5') - 1H à 6,95 ppm (S, $\underline{H}$ thiazole) - 1H à 5,96 ppm (D de D, $J_1$ = 9 Hz, $J_2$ = 4 Hz, $\underline{H_7}$) - 1H à 4,96 ppm (D, J = 4 Hz, $\underline{H_6}$) - 1H à 4,39 ppm (D, J = 13 Hz, $C\underline{H_2}SCO$). - 2H à 4,07 ppm (M, Ar $C\underline{H_2}N^{\oplus}H_3$) - 1H à 3,90 ppm (D, J = 13 Hz, $C\underline{H_2}$ SCO) - 2H à 3,78 ppm (S, $C\underline{H_2}SO$) - 3H à 2,26 ppm (S, $C\underline{H_3}$Ar) - 6H à 1,45 ppm (2S, $(C\underline{H_3})_2C$)

**RMN n° 22 - (b):**

1H à 8,89 ppm (D, J = 9 Hz, CONH) - 3H à 8,10 ppm (S.e., $CH_2N^{\oplus}H_3$) - 2H à 7,55 ppm (M, HAr 4', 6') - 1H à 7,37 ppm (T, J = 8 Hz, HAr 5') - 1H à 6,95 ppm (S, H thiazole) - 1H à 5,95 ppm (D de D, $J_1$ = 9 Hz, $J_2$ = 4 Hz, $H_7$) - 1H à 5,0 ppm (D, J = 4 Hz, $H_6$) - 1H à 4,40 ppm (D, J = 13 Hz, $CH_2SCO$) - 2H à 4,05 ppm (M, Ar $CH_2N^{\oplus}H_3$) - 1H à 3,90 ppm (D, J = 13 Hz, $CH_2$ SCO) - 2H à 3,79 ppm (S, $CH_2SO$) - 4H à 2,40 ppm (M,

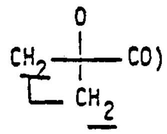

- 3H à 2,25 ppm (S, $CH_3Ar$) - 2H à 1,90 ppm (M,

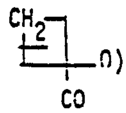

**RMN n° 23 - (b):**

1H à 8,64 ppm (D, J = 9 Hz, CONH) - 3H à 8,14 ppm (S.e., $CH_2N^{\oplus}H_3$) - 1H à 7,95 ppm (S, HAr 2') - 1H à 7,60 ppm (D, J = 8 Hz, HAr 6') - 1H à 7,39 ppm (D, J = 8 Hz, HAr 5') - 1H à 6,95 ppm (S, H thiazole) - 1H à 5,95 ppm (D de D, $J_1$ = 9 Hz, $J_2$ = 4 Hz, $H_7$) - 1H à 4,96 ppm (D, J = 4 Hz, $H_6$) - 1H à 4,37 ppm (D, J = 13 Hz, $CH_2SCO$) - 2H à 4,08 ppm (M, $CH_2N^{\oplus}H_3$) - 1H à 3,94 ppm (D, J = 13 Hz, $CH_2SCO$) - 2H à 3,74 ppm (S.e., $CH_2SO$) - 3H à 2,37 ppm (S, Ar $CH_3$) - 6H à 1,45 ppm (2S, $(CH_3)_2C$).

**RMN n° 24 - (b):**

1H à 8,80 ppm (2D, J = 9 Hz, CONH) - 3H à 8,20 ppm (S.e., $CH_2N^{\oplus}H_3$) - 1H à 7,94 ppm (S, HAr 2') - 1H à 7,80 ppm (D, J = 8 Hz, HAr 6') - 1H à 7,39 ppm (D, J = 8 Hz, HAr 5') - 1H à 6,95 ppm (2S, H thiazole) - 1H à 5,94 ppm (D de D, $J_1$ = 9 Hz, $J_2$ = 4 Hz, $H_7$) - 1H à 4,95 ppm (2D, J = 4 Hz, $H_6$) - 1H à 4,70 ppm (M, $CH_3CHON$) - 1H à 4,37 ppm (2D, J = 13 Hz, $CH_2SCO$) - 2H à 4,08 ppm (M, $CH_2N^{\oplus}H_3$) - 1H à 3,94 ppm (D, J = 13 Hz, $CH_2SCO$) - 2H à 3,73 ppm (S.e., $CH_2SO$) - 3H à 2,36 ppm (S, Ar $CH_3$) - 3H à 1,42 ppm (D, J = 7 Hz, $CH_3CH$ ON).

**RMN n° 25 - (b):**

1H à 8,66 ppm (D, J = 9 Hz, CONH) - 3H à 8,17 ppm (S.e., $CH_2N^{\oplus}H_3$) - 2H à 7,80 ppm (M, HAr 2', 6') - 1H à 7,50 ppm (D, J = 8 Hz, HAr 5') - 1H à 6,95 ppm (S, H thiazole) - 1H à 5,95 ppm (D de D, $J_1$ = 9 Hz, $J_2$ = 4 Hz, $H_7$) - 1H à 4,95 ppm (D, J = 4 Hz, $H_6$) - 1H à 4,40 ppm (D, J = 13 Hz, $CH_2SCO$) - 2H à 4,06 ppm (M, $CH_2N^{\oplus}H_3$) - 1H à 3,90 ppm (D, J = 13 Hz, $CH_2$ SCO) - 2H à 3,74 ppm (S, $CH_2SO$) - 3H à 2,46 ppm (S, Ar $CH_3$) - 6H à 1,46 ppm (2S, $(CH_3)_2C$).

**RMN n° 26 - (b):**

1H à 8,80 ppm (2D, J = 9 Hz, CONH) - 3H à 8,20 ppm (S.e., $CH_2N^{\oplus}H_3$) - 2H à 7,80 ppm (M, HAr 2', 6') - 1H à 7,46 ppm (D, J = 8 Hz, HAr 5') - 1H à 6,95 ppm (2S, H thiazole) - 1H à 5,94 ppm (D de D, $J_1$ = 9 Hz, $J_2$ = 4 Hz, $H_7$) - 1H à 4,95 ppm (2D, J = 4 Hz, $H_6$)- 1H à 4,66 ppm (M, $CH_3CH$- ON) - 1H à 4,40 ppm (2D, J = à 13 Hz, $CH_2SCO$) - 2H à 4,06 ppm (M, $CH_2N^{\oplus}H_3$) - 1H à 3,90 ppm (D, J = 13 Hz, $CH_2SCO$) - 2H à 3,76 ppm (S.e., $CH_2SO$) - 3H à 2,40 ppm (S, Ar $CH_3$) - 3H à 1,44 ppm (D, J = 7 Hz, $CH_3CH$ ON).

**RMN n° 27 (b):**

1H à 8,66 ppm (D, J = 9 Hz, CONH) - 3H à 7,80 ppm (S.e., $CH_2N^{\oplus}H_3$) - 1H à 6,95 ppm (S, H thiazole) - 1H à 5,98 ppm (D de D, $J_1$ = 9 Hz, $J_2$ = 4 Hz, $H_7$) - 1H à 4,95 ppm (D, J = 4 Hz, $H_6$) - 1H à 4,18 ppm (D, J = 13 Hz, $CH_2SCO$) - 1H à 3,78 ppm (D, J = 13 Hz, $CH_2SO$) - 2H à 3,69 ppm (S.e., $CH_2SO$) - 2H à 2,95 ppm (M, $CH_2N^{\oplus}H_3$) - 6H à 1,45 ppm (2S, $(CH_3)_2$-C-O) - 6H à 1,22 ppm (S, $(CH_3)_2C$ COS).

**RMN n° 28 - (b):**

1H à 10,80 ppm (S, Ar NHCO) - 1H à 8,66 (D, J = 9 Hz, CONH) 3H à 8,05 ppm (S.e., $CH_2N^{\oplus}H_3$) - 2H à 7,89 ppm (D, J = 8 Hz, HAr 2', 6') - 2H à 7,74 ppm (D, J = 8 Hz, HAr 3', 5') - 1H à 6,96 ppm (S, H thiazole) - 1H à 5,95 ppm (D de D, $J_1$ = 9 Hz, $J_2$ = 4 Hz, $H_7$) - 1H à 4,95 ppm (D, J = 4 Hz, $H_6$) - 1H à 4,42 ppm (D, J = 13 Hz, $CH_2SCO$) - 1H à 3,88 ppm (D, J = 13 Hz, $CH_2SCO$) - 4H à 3,80 ppm (M, $CH_2SO$ et $CH_2N^{\oplus}H_3$) - 6H à 1,45 ppm (2S, $(CH_3)_2C$).

**RMN n° 29 - (b):**

1H à 10,83 ppm (S, Ar NHCO) - 1H à 8,89 ppm (D, J = 9 Hz, CONH) - 3H à 8,10 ppm (S.e., $CH_2N^{\oplus}H_3$) - 2H à 7,90 ppm (D, J = 8 Hz, HAr 2', 6') - 2H à 7,71 ppm (D, J = 8 Hz, HAr 3', 5') - 1H à 6,95 ppm (S, H thiazole) - 1H à 5,94 ppm (D de D, $J_1$ = 9 Hz, $J_2$ = 4 Hz, $H_7$) - 1H à 4,95 ppm (D, J = 4 Hz, $H_6$) - 1H à 4,45 ppm (D, J = 13 Hz, $CH_2SCO$) - 1H à 3,87 ppm (D, J = 13 Hz, $CH_2SCO$) - 4H à 3,80 ppm (M, $CH_2SO$ et $CH_2N^{\oplus}H_3$) - 4H à 2,40 ppm (M,

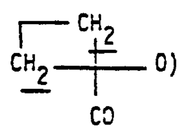

- 2H à 1,90 ppm (M,

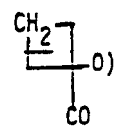

**RMN n° 30 (b):**

1H à 8,50 ppm (S.e., $N^{\oplus}H_2$ pipéridine) - 1H à 8,20 ppm (S.e., $N^{\oplus}H_2$ pipéridine) - 1 H à 8,36 ppm (D, J = 9 Hz, CONH) - 1H à 6,76 ppm (S, H thiazole) - 1 H à 5,95 ppm (C de D, $J_1$ = 9 Hz, $J_2$ = 4 Hz, $H_7$) - 1H à 4,92 ppm (D, J = 4 Hz, $H_6$) - 1H à 4,16 ppm (D, J = 13 Hz, $CH_2SCO$) - 1H à 3,72 ppm (D, J = 13 Hz, $CH_2SCO$) - 2H à 3,14 ppm (S, $CH_2SO$) - 2H à 3,17 ppm et 2H à 2,80 ppm (M, $CH_2$ en α $N^{\oplus}H_2$) - 2H à 2,55 ppm (D, J = 7 Hz, $CH_2$ CO S) - 1H à 2,00 ppm (M, $CHCH_2COS$) - 2H à 1,75 ppm et 2H à 1,30 ppm (M, $CH_2$ en β $N^{\oplus}H_2$) - 6H à 1,45 ppm (2S, $(CH_3)_2C$).

**RMN n° 31 - (b):**

1H à 8,34 ppm (D, J = 9 Hz, CONH) - 3H à 7,92 ppm (S.e., $CH_2N^{\oplus}H_3$) - 3H à 7,30 ppm (S.e., $N^{\oplus}H_3$ thiazole) - 1H à 6,76 ppm (S, H thiazole) - 1H à 5,95 ppm (D de D, $J_1$ = 9 Hz, $J_2$ = 4 Hz, $H_7$) - 1H à 4,92 ppm (D, J = 4 Hz, $H_6$) - 1H à 4,26 ppm (DE, J = 12 Hz, $H_2$ Eq pipéridine) - 1H à 4,15 ppm (D, J = 13 Hz, $CH_2SCO$) - 2H à 3,84 ppm (M, $CH_2N^{\oplus}H_3$) - 1H à 3,72 ppm (D, J = 13 Hz, $CH_2SCO$) - 3H à 3,60 ppm (M, $CH_2SO$ + $H_6$ Eq pipéridine) - 1H à 3,05 ppm (TE J = 12 Hz, $H_2$Ax pipéridine) - 1H à 2,87 ppm (TE, CHCOS) - 1H à 2,74 ppm (TE, J = 12 Hz, $H_6$Ax pipéridine) - 2H à 1,86 ppm (M, H en 3 et 5 pipéridine) - 2H à 1,45 ppm (M, H en 3 et 5 pipéridine) - 6H à 1,45 ppm (2S, $(CH_3)_2C$).

**RMN n° 32 - (b):**

1H à 8,66 ppm (D, J = 9 Hz, CON$\underline{H}$) - 3H à 7,90 ppm (S.e., $CH_2N^{\oplus}\underline{H}_3$) - 3H à 7,30 ppm (S.e., - $N^{\oplus}\underline{H}_3$ thiazole) - 1H à 6,78 ppm (S, $\underline{H}$ thiazole) - 1H à 5,90 ppm (D de D, $J_1$ = 9 Hz, $J_2$ = 4 Hz, $\underline{H}_7$) - 1H à 4,94 ppm (D, J = 4 Hz, $\underline{H}_6$) - 1H à 4,26 ppm (De., J = 12 Hz, $\underline{H}_2$ Eq piperidine) - 1H à 4,16 (D, J = 13 Hz, $C\underline{H}_2SCO$) - 2H à 3,82 ppm (M, $C\underline{H}_2$ Gly) - 1H à 3,74 ppm (D, J = 13 Hz, $C\underline{H}_2SO$) - 3H à 3,63 ppm (S.e., $C\underline{H}_2SO$ et $\underline{H}_6$ Eq pipéridine) - 1H à 3,05 ppm (Te., J = 13 Hz, $\underline{H}_2$Ax pipéridine) - 1H à 2,87 ppm (Te., J = 12 Hz,
S - C C$\underline{H}$) - 1H à 2,75 ppm
||

(Te., J = 12 Hz, $\underline{H}_6$Ax pipéridine) - 4H à 2,44 ppm (M

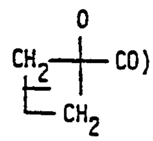

4H à 1,90 ppm (M,

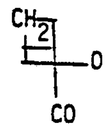

et $\underline{H}_3$ Eq et $\underline{H}_5$ Eq pipéridine) - 2H à 1,50 ppm (M, $H_3$ Ax pipéridine).

**RMN n° 33 - (b):**

1H à 10,70 ppm (S, Ar N$\underline{H}$CO) - 1H à 8,70 ppm (D, J = 9 Hz, CON$\underline{H}$) - 1H à 8,30 ppm (S, $\underline{H}$Ar 2') - 3H à 8,10 ppm (S.e., $CH_2N^{\oplus}\underline{H}_3$) - 1H à 7,75 ppm (D, J = 8 Hz, $\underline{H}$Ar 6') - 1H à 7,60 ppm (D, J = 8 Hz, $\underline{H}$Ar 4') - 1H à 7,50 ppm (T, J = 8 Hz, $\underline{H}$Ar 5') - 1H à 6,95 ppm (S, $\underline{H}$ thiazole) - 1H à 5,95 ppm (D de D, $J_1$ = 9 Hz, $J_2$ = 4 Hz, $\underline{H}_7$) - 1H à 4,95 ppm (D, J = 4 Hz, $\underline{H}_6$) - 1H à 4,45 ppm (D, J = 13 Hz, $C\underline{H}_2SCO$) - 1H à 3,90 ppm (D, J = 13 Hz, $C\underline{H}_2SCO$). - 4H à 3,75 ppm (M, $C\underline{H}_2SO$ et $C\underline{H}_2$ Gly) - 6H à 1,45 ppm (2S, $(C\underline{H}_3)_2C$)

**RMN n° 34 - (b):**

1H à 10,65 ppm (S.e., Ar N$\underline{H}$CO) - 1H à 8,80 ppm (D, J = 9 Hz, N$\underline{H}$CO)-1H à 8,25 ppm (S.e., $\underline{H}$Ar 2') - 3H à 8,05 ppm (S.e., $CH_2N^{\oplus}\underline{H}_3$)-1H à 7,75 ppm (D, J = 8 Hz, $\underline{H}$Ar 6') - 1H à 7,58 ppm (D, J = 8 Hz, $\underline{H}$Ar 4') - 1H à 7,50 ppm (T, J = 8 Hz, $\underline{H}$Ar 5') - 1H à 6,95 ppm (S, $\underline{H}$ thiazole) - 1 H à 5,95 ppm (D de D, $J_1$ = 9 Hz, $J_2$ = 4 Hz, $\underline{H}_7$) - 1 H à 4,95 ppm (D, J = 4 Hz, $\underline{H}_6$) - 1H à 4,45 ppm (D, J = 13 Hz, $C\underline{H}_2SCO$) - 1H à 3,90 ppm (D, J = 13 Hz, $C\underline{H}_2$ SCO) - 4H à 3,75 ppm (M, $C\underline{H}_2SO$ et $C\underline{H}_2$ Gly) 4H à 2,40 ppm (M,

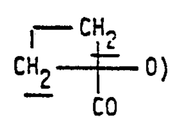

- 2H à 1,90 ppm (M,

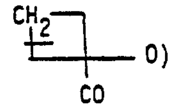

**RMN n° 35 - (b):**

1H à 8,40 ppm (D, J = 9 Hz, CONH) - 6H à 7,60 ppm (S.e., $N^{\oplus}H_3$) - 1H à 6,80 ppm (S, H thiazole) - 1H à 5,95 ppm (D de D, $J_1$ = 9 Hz, $J_2$ = 4 Hz, $H_7$) - 1H à 4,95 ppm (D, J = 4 Hz, $H_6$) - 1H à 4,30 ppm (M, $H_2$e pipéridine) - 1H à 4,16 ppm (D, J = 13 Hz, $CH_2SCO$) - 1H à 3,75 ppm (D, J = 13 Hz, $CH_2SCO$) - 2H à 3,60 ppm (S, $CH_2SO$) - 1H à 3,55 ppm (M, $H_6$e pipéridine) - 4H à 2,90 ppm (M, $CH_2N^{\oplus}H_3$ et $H_2$a et $H_6$a pipéridine) - 3H à 2,60 ppm (M, $CH_2$ $CH_2N^{\oplus}H_3$ et $H_4$ pipéridine) - 2H à 1,80 ppm et 2H à 1,50 ppm (2M, $H_3$ et $H_5$ pipéridine) - 6H à 1,45 ppm (2S, $(CH_3)_2C$).

**RMN n° 36 - (b):**

1H à 8,70 ppm (D, J = 9 Hz, CONH) - 3H à 8,20 ppm (S.e., $CH_2N^{\oplus}H_3$) - 1H à 7,75 ppm (D, J = 8 Hz, HAr 6') - 2H à 7,43 ppm (M, HAr 3', 5') - 1H à 6,98 ppm (S.e., H thiazole) - 1H à 6,00 ppm (D de D, $J_1$= 9 Hz, $J_2$ = 4 Hz, $H_7$) - 1H à 4,97 ppm (D, J = 4 Hz, $H_6$) - 1H à 4,34 ppm (D, J = 13 Hz, $CH_2SCO$) - 2H à 4,00 ppm (M, $CH_2N^{\oplus}H_3$) - 1H à 3,90 ppm (D, J = 13 Hz, $CH_2SCO$) 2H à 3,77 ppm (S.e., $CH_2SO$) - 1H à 2,34 ppm (S, $CH_3Ar$) - 6H à 1,46 ppm (2S, $(CH_3)_2C$).

**RMN n° 37 - (b)**

1H à 8,80 ppm (D, J = 9 Hz, CONH) - 3H à 8,15 ppm (S.e., $CH_2N^{\oplus}H_3$) - 1H à 7,72 ppm (D, J = 8 Hz, HAr 6') - 2H à 7,40 ppm (M, HAr 3', 5') - 1H à 6,95 ppm (S, H thiazole) - 1H à 5,95 ppm (D de D, $J_1$ = 9 Hz, $J_2$ = 4 Hz, $H_7$) - 1H à 4,93 ppm (D, J = 4 Hz, $H_6$) - 1H à 4,34 ppm (D, J = 13 Hz, $CH_2SCO$) - 2H à 4,00 ppm (M, $CH_2N^{\oplus}H_3$) - 1H à 3,90 ppm (D, J = 13 Hz, $CH_2SCO$) - 2H à 3,78 ppm (S.e., $CH_2SO$) - 2H à 2,34 ppm (S, $CH_3Ar$) - 4H à 2,30 ppm (M,

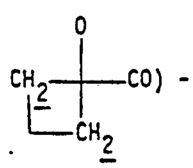

2H à 1,80 ppm (M,

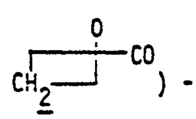

**RMN n° 38 - (b):**

1H à 8,60 ppm (D, J = 9 Hz, CONH) - 7H à 7,70 ppm (M, HAr et $CH_2N^{\oplus}H_3$) - 1H à 6,98 ppm (S, H thiazole) - 1H à 5,98 ppm (D de D, $J_1$ = 9 Hz, $J_2$ = 4 Hz, $H_7$) - 1H à 4,96 ppm (D, J = 4 Hz, $H_6$) - 1H à 4,38 ppm (D, J = 13 Hz, $CH_2SCO$) - 1H à 3,94 ppm (D, J = 13 Hz, $CH_2SCO$) - 2H à 3,75 ppm (S.e., $CH_2SO$) - 3H à 3,00 ppm (M, $CHCH_2N^{\oplus}H_3$) - 6H à 1,47 ppm (2S, $(CH_3)_2C$) - 3H à 1,19 ppm (D, J = 7 Hz, $CH_3CH$).

**RMN n° 39 - (b):**

1H à 8,80 ppm (D, J = 9 Hz, CONH) - 7H à 7,80 ppm (M, HAr et $N^{\oplus}H_3CH_2$) - 1H à 6,95 ppm (S, H thiazole) - 1H à 5,95 ppm (D de D, $J_1$ = 9 Hz, $J_2$ = 4 Hz, $H_7$) - 1H à 4,97 ppm (D, J = 4 Hz, $H_6$) - 1H à 4,40 ppm (D, J = 13 Hz, $CH_2SCO$) - 1H à 3,93 ppm (D, J = 13 Hz, $CH_2SCO$) - 2H à 3,75 ppm (S.e., $CH_2SO$) - 3H à 3,00 ppm (M, $CHCH_2N^{\oplus}H_3$) - 4H à 2,40 ppm (M,

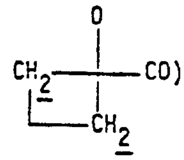

- 2H à 1,85 ppm (M,

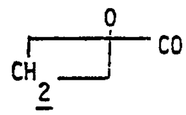

3H à 1,25 ppm (D, J = 7 Hz, $CH_3CH$).

**RMN n° 40 - (b):**

1H à 12,6 ppm (S.e., Ar NHCO) - 1H à 8,75 ppm (D, J = 9 Hz, CONH) - 1H à 8,00 ppm (S, H thiazole en 3) - 3H à 7,75 ppm (S.e., $CH_2N^{\oplus}H_3$) - 1H à 7,00 ppm (S, H thiazole) - 1H à 5,98 ppm (D de D, $J_1$ = 9 Hz, $J_2$ = 4 Hz, $H_7$) - 1H à 4,95 ppm (D, J = 4 Hz, $H_6$) - 1H à 4,40 ppm (D, J = 13 Hz, $CH_2SCO$) - 1H à 3,78 ppm (D, J = 13 Hz, $CH_2SCO$) - 2H à 3,75 ppm (S.e., $CH_2SO$) - 2H à 3,50 ppm (M, $CH_2N^{\oplus}H_3$) - 2H à 2,80 ppm (M, $CH_2CH_2N^{\oplus}H_3$) - 6H à 1,45 ppm (2S, $(CH_3)_2C$).

**RMN n° 41 - (b):**

1H à 12,8 ppm (S.e., $NHCOCH_2$) - 1H à 8,80 ppm (D, J = 9 Hz, CONH) 3H à 8,25 ppm (S.e., $CH_2N^{\oplus}H_3$) - 1H à 8,16 ppm (S, H thiazole en 3) - 1H à 7,00 ppm (S, H thiazole) - 1H à 5,96 ppm (D de D, $J_1$ = 9 Hz, $J_2$ = 4 Hz, $H_7$) - 1H à 4,97 ppm (D, J = 4 Hz, $H_6$) - 1H à 4,40 ppm (D, J = 13 Hz, $CH_2SCO$) 3H à 3,90 ppm (M, $CH_2N^{\oplus}H_3$ et $CH_2SCO$) - 2H à 3,75 ppm (S.e., $CH_2SO$) - 6H à 1,44 ppm (2S, $(CH_3)_2C$).

**RMN n° 42 - (b):**

1H à 10,6 ppm (S, Ar NHCO) - 1H à 8,70 ppm (D, J = 9 Hz, CONH) - 2H à 7,90 ppm (M, HAr 2', 6') - 5H à 7,60 ppm (M, $CH_2N^{\oplus}H_3$ et HAr 3', 5') - 1H à 7,00 ppm (S, H thiazole) - 1H à 6,00 ppm (D de D, $J_1$ = 9 Hz, $J_2$ = 4 Hz, $H_7$) - 1H à 4,95 ppm (D, J = 4 Hz, $H_6$) - 1H à 4,42 ppm (D, J = 13 Hz, $CH_2SCO$) 1H à 3,90 ppm (D, J = 13 Hz, $CH_2SCO$) - 2H à 3,75 ppm (S.e., $CH_2SO$) - 2H à 3,05 ppm (M, $CH_2N^{\oplus}H_3$) - 2H à 2,72 ppm (M, $CH_2CH_2N^{\oplus}H_3$) - 6H à 1,45 ppm (2S, $(CH_3)_2C$).

**RMN n° 43 - (b):**

1H à 10,5 ppm (S, Ar NHCO) - 1H à 8,85 ppm (D, J = 9 Hz, CONH) - 2H à 7,90 ppm et 2H à 7,75 ppm (M, HAr) - 3H à 7,70 ppm (S.e., $CH_2N^{\oplus}H_3$) - 1H à 6,95 ppm (S, H thiazole) - 1H à 5,45 ppm (D de D, $J_1$ = 9 Hz, $J_2$ = 4 Hz, $H_7$) - 1H à 4,97 ppm (D, J = 4 Hz, $H_6$) - 1H à 4,40 ppm (D, J = 13 Hz, $CH_2SCO$) 1H à 3,86 ppm (D, J = 13 Hz, $CH_2SCO$) - 2H à 3,75 ppm (S.e., $CH_2SO$) - 2H à 3,02 ppm (N, $CH_2N^{\oplus}H_3$) - 2H à 2,72 ppm (M, $CH_2CH_2N^{\oplus}H_3$) - 4H à 2,40 ppm (M,

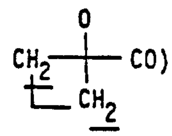

- 2H à 1,85 ppm (M,

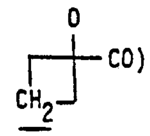

**RMN n° 44 - (b):**

1H à 10,9 ppm (S, Ar NHCO) - 2H à 8,80 ppm (S.e., $CH_2N^{\oplus}H_2CH_3$) - 1H à 8,70 ppm (D, J = 9 Hz, CONH) - 2H à 7,90 ppm (D, J = 8 Hz, HAr 2', 6') - 2H à 7,70 ppm (D, J = 8 Hz, HAr 3', 5') - 1H à 6,98 ppm (S, H thiazole) - 1H à 6,00 ppm (D de D, $J_1$ = 9 Hz, $J_2$ = 4 Hz, $H_7$) - 1H à 4,97 ppm (D, J = 4 Hz, $H_6$) - 1H à 4,42 ppm (D, J = 13 Hz, $CH_2SCO$) - 3H à 3,90 ppm (M, $CH_2N^{\oplus}H_2CH_3$ et $CH_2SCO$) - 2H à 3,75 ppm (S.e., $CH_2SO$) - 3H à 2,55 ppm (M, $CH_3N^{\oplus}H_2$ - $CH_2$)6H à 1,46 ppm (2S, $(CH_3)_2C$).

**RMN n° 45 - (b):**

1H à 8,75 ppm (D, J = 9 Hz, CONH) - 1H à 8,55 ppm (D, H thiazole en 3) - 3H à 8,50 ppm (S.e., $CH_2N^{\ominus}H_2$) - 1H à 7,00 ppm (S, H thiazole) - 1H à 5,95 ppm (D de D, $J_1$ = 9 Hz, $J_2$ = 4 Hz, $H_7$) - 1H à 4,96 ppm (D, J = 4 Hz, $H_6$) - 2H à 4,46 ppm (M, $CH_2N^{\ominus}H_3$) - 1H à 4,30 ppm (D, J = 13 Hz, $CH_2SCO$) - 1H à 3,90 ppm (D, J = 13 Hz, $CH_2SCO$) - 2H à 3,73 ppm (S.e., $CH_2SO$) - 6H à 1,44 ppm (2S, $(CH_3)_2C$).

**RMN n° 46 - (b):**

1H à 8,87 ppm (T, J = 8 Hz, $NHCH_2$ Ar) - 1H à 8,64 ppm (D, J = 9 Hz, NHCO) - 3H à 8,00 ppm (S.e., $H_3N^{\ominus}$ $CH_2$) - 2H à 7,84 ppm (D, J = 8 Hz, H Ar 2', 6') - 2H à 7,42 ppm (D, J = 8 Hz, HAr 3', 5') - 1H à 6,94 ppm (S, H thiazole) - 1H à 5,95 ppm (D de D, $J_1$ = 9 Hz, $J_2$ = 4 Hz, $H_7$) - 1H à 4,95 ppm (D de D, J = 4 Hz, $H_6$) - 3H à 4,40 ppm (M, Ar $CH_2NH$ et $CH_2SCO$) - 1H à 3,88 ppm (D, J = 13 Hz, $CH_2SCO$) - 2H à 3,74 ppm (S.e., $CH_2S \rightarrow O$) - 2H à 3,58 ppm (M, OC $CH_2N^{\ominus}H_3$) - 6H à 1,47 ppm (2S, $(CH_3)_2C$).

Les produits de l'invention ont été étudiés en ce qui concerne leurs propriétés pharmacologiques et, plus spécialement, l'action bactériostatique. Celle-ci a été déterminée in vitro par la méthode des dilutions, l'étude a porté sur des souches à Gram négatif.

Les résultats, exprimés en concentrations minimales inhibitrices (CMI - µ/ml), sont rassemblés dans le tableau suivant (Tableau III).

**Tableau III**

CMI (µg/ml) sur différentes souches

| Produits N° SR | Souches | | | | | | |
|---|---|---|---|---|---|---|---|
| | Escherichia coli SOL RL 90 | Citrobacter 49 | Proteus vulgaris RL 99 bis | Serratia RL 72 | Klebsiella RO 30 | Enterobacter RO 154 | Pseudomonas RL 112 |
| 41 730 | 0,5 | 0,25 | ⩽ 0,12 | 1 | 0,25 | 0,25 | 4 |
| 41 733 | 2 | 2 | 0,5 | 8 | 1 | 1 | 8 |
| 41 806 | 1 | 1 | 0,5 | 4 | 0,5 | 8 | 8 |

| | | | | | | | |
|---|---|---|---|---|---|---|---|
| 41 854 | 0,25 | 0,5 | ≤ 0,12 | 0,5 | 0,25 | 2 | 4 |
| 41 855 | 0,25 | 1 | ≤ 0,12 | 0,5 | 0,25 | 2 | 8 |
| 41 856 | 0,5 | 2 | ≤0,12 | 2 | 0,25 | 4 | 16 |
| 41 858 | 2 | 1 | 0,5 | 4 | 0,5 | 8 | 8 |
| 41 859 | 1 | 2 | 0,25 | 4 | 0,5 | 8 | 16 |
| 41 860 | 2 | 1 | 0,5 | 4 | 0,5 | 8 | 16 |
| 41 862 | 0,25 | 0,25 | ≤ 0,12 | 0,5 | 0,25 | 1 | 4 |
| 41 885 | 1 | 4 | 0,5 | 4 | 0,5 | 8 | 8 |
| 41 887 | 2 | 4 | 0,25 | 4 | 0,5 | 8 | 8 |
| 41 888 | 2 | 2 | 1 | 8 | 1 | 8 | 8 |
| 41 889 | 1 | 1 | 0,5 | 4 | 1 | 8 | 8 |
| 41 891 | 0,5 | 0,5 | 0,5 | 4 | 0,5 | 8 | 8 |
| 41 967 | 0,25 | 0,5 | ≤ 0,12 | 0,5 | 0,25 | 2 | 4 |
| 41 975 | 0,25 | 0,5 | 0,5 | 4 | 0,5 | 8 | 8 |
| 41 976 | 1 | 0,5 | 0,25 | 8 | 0,5 | 8 | 8 |
| 42 022 | 0,5 | 0,25 | ≤ 0,12 | 0,5 | 0,25 | 2 | 4 |
| 42 024 | 0,5 | 0,5 | 0,25 | 0,5 | 0,25 | 2 | 4 |
| 42 025 | 0,5 | 0,5 | 0,25 | 1 | 0,5 | 4 | 4 |
| 42 026 | 0,5 | 0,25 | ≤0,12 | 1 | 0,25 | 4 | 8 |
| 42 027 | 0,25 | 0,25 | ≤ 0,12 | 0,5 | 0,25 | 2 | 4 |
| 42 028 | 0,25 | 0,5 | ≤ 0,12 | 0,5 | 0,25 | 2 | 4 |
| 42 029 | 0,25 | 0,25 | ≤ 0,12 | 0,5 | 0,25 | 2 | 8 |
| 42 042 | ≤ 0,12 | ≤ 0,12 | ≤ 0,12 | 2 | ≤ 0,12 | 1 | 4 |
| 42 073 | 1 | 0,5 | 0,5 | 2 | 0,5 | 4 | 4 |
| 42 074 | 1 | 0,5 | 0,5 | 4 | 0,5 | 8 | 8 |
| 42 117 | 0,25 | 0,25 | ≤ 0,12 | 0,5 | ≤ 0,12 | 1 | 4 |
| 42 118 | 1 | 0,5 | 0,25 | 4 | 0,5 | 16 | 8 |
| 42 119 | 0,5 | 0,25 | ≤ 0,12 | 4 | 0,5 | 8 | 8 |
| 42 208 | 0,5 | 0,5 | ≤ 0,12 | 1 | 0,5 | 0,25 | 4 |
| 42 209 | 0,25 | ≤ 0,12 | ≤ 0,12 | 0,5 | 0,25 | ≤ 0,12 | 4 |
| 42 210 | 1 | 0,5 | 0,25 | 1 | 0,5 | 0,25 | 4 |
| 42 211 | ≤ 0,12 | ≤ 0,12 | ≤ 0,12 | ≤ 0,12 | ≤ 0,12 | ≤ 0,12 | 64 |
| 42 212 | ≤ 0,12 | ≤ 0,12 | ≤ 0,12 | 0,25 | ≤ 0,12 | ≤ 0,12 | 4 |
| 32 213 | 0,25 | ≤ 0,12 | ≤ 0,12 | 0,5 | 0,25 | ≤ 0,12 | 16 |
| 42 214 | ≤ 0,12 | ≤ 0,12 | ≤ 0,12 | 0,5 | ≤ 0,12 | ≤ 0,12 | 16 |
| 42 215 | ≤ 0,12 | ≤ 0,12 | ≤ 0,12 | 0,25 | ≤ 0,12 | ≤ 0,12 | 8 |
| 42 216 | ≤ 0,12 | ≤ 0,12 | ≤ 0,12 | 0,5 | ≤ 0,12 | ≤ 0,12 | 8 |
| 42 217 | ≤ 0,12 | ≤ 0,12 | ≤ 0,12 | 0,25 | ≤ 0,12 | ≤ 0,12 | 16 |
| 42 218 | 0,5 | 0,25 | 0,5 | 2 | 0,5 | 0,5 | 64 |
| 42 219 | 0,5 | ≤ 0,12 | ≤ 0,12 | 1 | 0,5 | 0,5 | 4 |
| 42 221 | 0,25 | ≤ 0,12 | ≤ 0,12 | 2 | ≤ 0,12 | 0,25 | 64 |
| 42 222 | 0,5 | ≤ 0,12 | ≤ 0,12 | 1 | 0,5 | 0,5 | 8 |
| 42 320 | ≤ 0,125 | ≤ 0,125 | ≤ 0,125 | 0,5 | ≤ 0,125 | ≤ 0,125 | 4 |
| 42 321 | 0,5 | 0,5 | ≤ 0,125 | 1 | 0,5 | 0,25 | 8 |
| 42 371 | 0,5 | 0,25 | ≤ 0,125 | 0,5 | 0,25 | 0,25 | 8 |
| 42 372 | 0,25 | ≤ 0,125 | ≤ 0,125 | 0,25 | 0,25 | ≤ 0,125 | 4 |
| 42 374 | 0,5 | 0,25 | ≤ 0,125 | 1 | 0,25 | 0,25 | 4 |
| 42 379 | 0,25 | ≤ 0,125 | ≤ 0,125 | 0,5 | ≤ 0,125 | ≤ 0,125 | 8 |
| 42 380 | ≤ 0,125 | ≤ 0,125 | ≤ 0,125 | 1 | ≤ 0,125 | ≤ 0,125 | 4 |
| 42 395 | ≤ 0,125 | ≤ 0,125 | ≤ 0,125 | 0,5 | ≤ 0,125 | ≤ 0,125 | 4 |
| 42 396 | ≤ 0,125 | ≤ 0,125 | ≤ 0,125 | 1 | ≤ 0,125 | ≤ 0,125 | 8 |
| 42 397 | ≤ 0,125 | ≤ 0,125 | ≤ 0,125 | 1 | ≤ 0,125 | ≤ 0,125 | 8 |
| 42 456 | 0,25 | ≤ 0,125 | ≤ 0,125 | 0,5 | ≤ 0,125 | ≤ 0,125 | 4 |
| 42 457 | 0,5 | 0,25 | ≤ 0,125 | 0,5 | 0,25 | 0,25 | 8 |
| 42 461 | 0,25 | ≤ 0,125 | ≤ 0,125 | 2 | ≤ 0,125 | ≤ 0,125 | 8 |
| 42 462 | 0,25 | ≤ 0,125 | ≤ 0,125 | 0,5 | ≤ 0,125 | ≤ 0,125 | 4 |
| 42 463 | ≤ 0,125 | ≤ 0,125 | ≤ 0,125 | 0,5 | ≤ 0,125 | ≤ 0,125 | 4 |
| 42 464 | ≤ 0,125 | ≤ 0,125 | ≤ 0,125 | 0,5 | ≤ 0,125 | ≤ 0,125 | 4 |
| 42 465 | ≤ 0,125 | ≤ 0,125 | ≤ 0,125 | 2 | 0,5 | 0,5 | 8 |
| 42 466 | 0,5 | 0,25 | ≤ 0,125 | 2 | 0,5 | 0,5 | 4 |
| 42 467 | 0,25 | ≤ 0,125 | ≤ 0,125 | 2 | 0,25 | 0,5 | 4 |
| 42 471 | 0,5 | 0,25 | ≤ 0,125 | 0,5 | 0,25 | 0,25 | 8 |
| 42 472 | 0,25 | ≤ 0,125 | ≤ 0,125 | 0,25 | ≤ 0,125 | ≤ 0,125 | 4 |
| 42 473 | 0,25 | ≤ 0,125 | ≤ 0,125 | 0,25 | ≤ 0,125 | ≤ 0,125 | 4 |
| 42 474 | 0,25 | ≤ 0,125 | ≤ 0,125 | 0,25 | 0,25 | ≤ 0,125 | 4 |

| | | | | | | | |
|---|---|---|---|---|---|---|---|
| 42 531 | 0,5 | 0,25 | ≤ 0,125 | 0,5 | 0,25 | 0,5 | 8 |
| 42 532 | 0,5 | 0,25 | ≤ 0,125 | 1 | 0,25 | 0,5 | 8 |
| 42 533 | 0,5 | 0,25 | ≤ 0,125 | 1 | 0,25 | 0,5 | 8 |
| 42 534 | 0,5 | 0,25 | ≤ 0,125 | 1 | 0,25 | 0,5 | 8 |
| 42 535 | 0,5 | 0,25 | ≤ 0,125 | 1 | 0,25 | 0,5 | 8 |
| 42 536 | 0,5 | 0,25 | ≤ 0,125 | 0,5 | 0,25 | 0,5 | 8 |
| 42 537 | 0,25 | 0,25 | ≤ 0,125 | 0,5 | 0,25 | 0,25 | 8 |
| 42 538 | 0,5 | 0,25 | ≤ 0,125 | 0,5 | 0,25 | 0,5 | 8 |
| 42 540 | 0,25 | 0,125 | ≤ 0,125 | 0,25 | ≤ 0,125 | ≤ 0,125 | 4 |
| 42 541 | 0,5 | 0,25 | ≤ 0,125 | 0,5 | ≤ 0,125 | 0,25 | 4 |
| 42 542 | 0,5 | 0,5 | ≤ 0,125 | 0,5 | ≤ 0,25 | 0,5 | 8 |
| 42 546 | 0,25 | 0,25 | ≤ 0,125 | 1 | 0,25 | 0,25 | 4 |
| 42 547 | 0,5 | 0,25 | ≤ 0,125 | 0,5 | 0,25 | 0,5 | 4 |
| 42 548 | 0,25 | ≤ 0,125 | ≤ 0,125 | 0,5 | 0,125 | 0,25 | 2 |
| 42 549 | 0,25 | ≤ 0,125 | ≤ 0,125 | 0,5 | 0,25 | 0,25 | 4 |
| 42 581 | 0,125 | | | | 0,125 | 2 | 4 |
| 42 582 | 0,25 | | | | 0,25 | 2 | 8 |
| 42 583 | 1 | | | | 0,5 | 4 | 8 |
| 42 584 | 0,25 | | | | 0,25 | 2 | 4 |
| 42 585 | 0,25 | | | | 0,25 | 8 | 4 |
| 42 586 | 0,25 | | | | 0,25 | 16 | 4 |
| 42 587 | 0,25 | | | | 0,25 | 8 | 4 |
| 42 657 | 0,25 | | | | 0,25 | 4 | 8 |
| 42 658 | 0,125 | | | | 0,125 | 8 | 4 |
| 42 661 | 0,5 | | | | 0,25 | 1 | 8 |
| 42 663 | 4 | | | | 1 | 4 | 8 |
| 42 664 | 1 | | | 4 | 0,5 | 4 | 8 |
| 42 665 | 0,5 | | | | 0,5 | 2 | 8 |
| 42 674 | 1 | | | | 0,5 | 2 | 8 |
| 42 675 | 0,125 | | | | 0,125 | 1 | 4 |
| 42 676 | 0,25 | | | | 0,25 | 2 | 8 |
| 42 685 | 2 | | | | 1 | 2 | 8 |
| 42 686 | 2 | | | | 1 | 2 | 8 |
| 42 687 | 2 | | | | 1 | 4 | 8 |
| 42 688 | 0,125 | | | | 0,25 | 4 | 4 |
| 42 689 | 0,125 | | | | 0,25 | 4 | 4 |
| 42 690 | 0,5 | | | | 0,5 | 8 | 4 |
| 42 781 | 0,25 | | | | 0,125 | 2 | 4 |
| 42 782 | 0,25 | | | | 0,125 | 2 | 4 |
| 42 783 | 0,25 | | | | 0,125 | 2 | 8 |
| 42 811 | 0,125 | | | | 0,125 | 2 | 4 |
| 42 814 | 0,5 | | | | 0,25 | 8 | 8 |
| 42 815 | 0,125 | | | | 0,25 | 8 | 8 |
| 42 817 | 0,125 | | | | 0,25 | 4 | 4 |
| 42 818 | 0,125 | | | | 0,125 | 4 | 4 |
| 42 848 | 0,125 | | | | 0,125 | 2 | 4 |
| 42 849 | 0,125 | | | | 0,125 | 2 | 4 |
| 42 850 | 0,5 | | | | 0,25 | 2 | 8 |
| 42 851 | 0,5 | | | | 0,25 | 1 | 8 |
| 42 852 | 0,25 | | | | 0,125 | 1 | 8 |
| 42 857 | 0,5 | | | | 1 | 8 | 8 |

Les résultats présentés dans le tableau III montrent la bonne activité sur les bactéries Gram négatif des produits, selon l'invention.

Pour évaluer la stabilité de ces produits envers les bêta-lactamases, on détermine leur CMI sur des souches isogéniques productrices et non productrices de bêta-lactamases.

Les résultats sont exprimés en µg/ml dans le tableau IV.

**Tableau IV**

CMI (µg/ml) SUR DES SOUCHES PRODUCTRICES DE BETA-LACTAMASES
ET NON PRODUCTRICES (indiquées par *)

| Produits | SOUCHES | | | | | |
|---|---|---|---|---|---|---|
| | Escherichia coli | | Serratia liquefaciens | | Proteus vulgaris | |
| N° SR | 255 | 255/L.7 | SL/326 A | SL 1326 S | GN 76/C. 1 | GN 76/C. 1/3 |
| | | * | | * | | * |
| 41 730 | 0,25 | ≤0,12 | 0,5 | ≤0,12 | 0,25 | 0,25 |
| 41 854 | ≤0,12 | ≤0,12 | 0,25 | ≤0,12 | 0,25 | 0,25 |
| 41 855 | ≤0,12 | ≤0,12 | 0,25 | ≤0,12 | ≤0,12 | ≤0,12 |
| 41 862 | ≤0,12 | 0,12 | ≤0,12 | ≤0,12 | ≤0,12 | ≤0,12 |
| 41 967 | ≤0,12 | ≤0,12 | 0,25 | ≤0,12 | ≤0,12 | ≤0,12 |
| 41 973 | 0,25 | 0,25 | 0,25 | ≤0,12 | 0,5 | 0,5 |
| 41 975 | 0,5 | 0,5 | 0,25 | ≤0,12 | 0,5 | 0,5 |
| 42 022 | 0,25 | 0,25 | ≤0,12 | ≤0,12 | ≤0,12 | ≤0,12 |
| 42 024 | 0,25 | ≤0,12 | 0,25 | 0,06 | 0,25 | 0,25 |
| 42 027 | 0,12 | 0,06 | 0,25 | ≤0,06 | 0,12 | 0,12 |
| 42 028 | 0,12 | 0,12 | 0,12 | ≤0,06 | 0,25 | 0,25 |
| 42 042 | ≤0,12 | ≤0,12 | 0,25 | ≤0,12 | ≤0,12 | ≤0,12 |
| 42 073 | 0,5 | 0,25 | 0,25 | ≤0,12 | 0,5 | 0,5 |
| 42 074 | 0,5 | 0,25 | 0,25 | 0,12 | 0,5 | 0,5 |
| 42 117 | 0,125 | 0,06 | 0,12 | ≤0,06 | ≤0,06 | ≤0,06 |
| 42 118 | 0,5 | 0,25 | 0,25 | 0,12 | 0,5 | 0,5 |
| 42 119 | 0,25 | 0,12 | 0,25 | ≤0,06 | 0,12 | 0,12 |
| 42 320 | 0,0625 | 0,0625 | 0,125 | ≤0,01312 | ≤0,0312 | 0,125 |
| 42 395 | 0,0625 | 0,0625 | 0,125 | ≤0,0312 | ≤0,0312 | ≤0,0312 |
| 42 456 | 0,0625 | ≤0,0312 | 0,25 | 0,0625 | ≤0,0312 | 0,25 |
| 42 457 | 0,125 | ≤0,0312 | 0,0625 | 0,0625 | ≤0,0312 | 0,0625 |
| 42 466 | 0,125 | 0,125 | 1 | ≤0,0312 | ≤0,0312 | 0,0625 |
| 42 467 | 0,0625 | ≤0,0312 | 0,125 | 0,125 | ≤0,0312 | 0,0625 |
| 42 474 | 0,0625 | 0,0625 | 0,125 | ≤0,0312 | 0,0625 | 0,0625 |
| 42 531 | 0,125 | 0,0625 | 0,125 | 0,125 | 0,0625 | 0,0625 |
| 42 533 | 0,125 | 0,0625 | 0,125 | ≤0,0312 | 0,0625 | 0,125 |
| 42 535 | 0,125 | ≤0,0312 | 0,0625 | 0,125 | 0,0625 | 0,125 |
| 42 546 | 0,0625 | ≤0,0312 | 0,25 | ≤0,0312 | ≤0,0312 | 0,125 |
| 42 547 | 0,125 | ≤0,0312 | 2 | 0,125 | 0,0625 | 0,25 |
| 42 548 | 0,625 | ≤0,0312 | 0,125 | ≤0,0312 | 0,0625 | 0,0625 |
| 42 549 | 0,125 | 0,0625 | 0,125 | ≤0,0312 | ≤0,0312 | 0,0625 |
| 42 664 | 0,25 | 0,0625 | 0,25 | 0,0625 | 0,125 | 0,25 |
| 42 673 | 0,25 | 0,25 | 0,25 | 0,0625 | 0,25 | 0,25 |

D'après les résultats du tableau IV, les produits, selon l'invention, ont une activité égale ou comparable sur les souches productrices et non productrices de béta-lactamases, ce qui montre la bonne stabilité envers les béta-lactamases.

L'efficacité thérapeutique des produits est déterminée sur le modèle septicémique de la souris. La septicémie est provoquée par inoculation intrapéritonéale de 0,5 ml d'une dilution appropriée d'une suspension de la souche Escherichia coli SOL 90. Les produits sont administrés en solution dans un tampon phosphate pH 7,0 sous volume de 0,2 ml par voie sous-cutanée à des lots de 10 souris, 1 à 5 heures après inoculation du microorganisme. Au bout de 4 jours d'observation au cours desquels la mortalité est notée, les doses efficaces 50 % (DE 50) sont calculées par la méthode de Muench et Reed.

Les résultats obtenus figurent dans le tableau V.

**Tableau V**

$DE_{50}$ (mg/kg) Dans le modèle septicémique chez la souris

| Produits | Souches | |
|---|---|---|
| N° SR | Escherichia coli SOL 90 | Klebsiella RO 30 |
| 41 730 | 1,4 | 0,79 |
| 41 854 | 0,23 | 0,19 |
| 41 855 | 0,16 | 0,17 |
| 41 862 | 0,13 | 0,10 |
| 41 973 | 0,22 | 0,4 |
| 42 042 | 0,05 | ND |
| 42 073 | 0,33 | ND |
| 42 117 | 0,11 | ND |
| 42 119 | 0,16 | ND |

ND = non déterminé

D'après les résultats du tableau V, les produits, selon l'invention, montrent une bonne activité thérapeutique IN VIVO.

Par ailleurs, d'après les essais effectués jusqu'à ce jour sur les animaux, la toxicité des produits selon l'invention a paru suffisamment faible pour permettre leur utilisation en thérapeutique.

Les produits de l'invention peuvent donc être employés comme antibiotiques en médecine humaine ou vétérinaire. Ils présentent un large spectre sur les Gram négatif et peuvent être utilisés dans toutes les infections bactériennes à germes sensibles.

Les produits peuvent être administrés par voie générale (parentérale, orale ou par voie topique).

Les compositions pharmaceutiques sont réalisées à partir des composés (I) sous une forme soluble obtenue par salification d'une des fonctions acides de la molécule ou d'une des fonctions amine de la chaîne B.

Les compositions pharmaceutiques peuvent être solides ou liquides et se présenter, par exemple, sous forme de préparations injectables, de comprimés, gélules, granulés, pommades, crèmes, gels ou suppositoires. La posologie peut varier dans de larges proportions, en particulier suivant le type et la gravité de l'infection à traiter et suivant le mode d'administration. Le plus souvent, chez l'adulte, par voie injectable, elle est comprise entre 0,250 g et 4 g par jour.

3A tire d'exemple de composition pharmaceutique, on peut préparer des solutions injectables de sel de sodium du SR 41 973:

A une solution de 3 g de dichlorhydrate du SR 41 973 dans 25 ml d'eau, on ajoute goutte à goutte une solution saturée de bicarbonate de sodium. Lorsque le pH est 3, la solution est filtrée. Le pH est alors ajusté à 3,6 par addition de quelques gouttes de solution saturée de bicarbonate de sodium. La solution est refroidie à + 4°C et le SR 41 973 commence à cristalliser. On ajoute lentement et goutte à goutte 75 ml d'acétone. Après 1/2 heure d'agitation à + 4°C, les cristaux sont filtrés et lavés deux fois par 20 ml d'un mélange (1 - 1) d'eau et d'acétone et enfin lavés 2 fois par 20 ml d'acétone. Le produit est séché au dessicateur sur P 205. On obtient 1,9 g du composé SR 41 973.

1,25 g de SR 41 973 ainsi obtenu est mis en suspension dans 15 ml d'eau à + 4°C. Une solution de 0,168 g de bicarbonate de sodium dans 3 ml d'eau est ajoutée goutte à goutte. La solution limpide ainsi obtenue est congelée et lyophylisée, après filtration stérile, pour obtenir du sel de sodium du 41 973 prêt à l'injection.

**Revendications** pour les états contractants: BE, CH, DE, FR, GB, IT, LI, LU, NL, SE.

1. Dérivés de la famille des céphalosporines de formule:

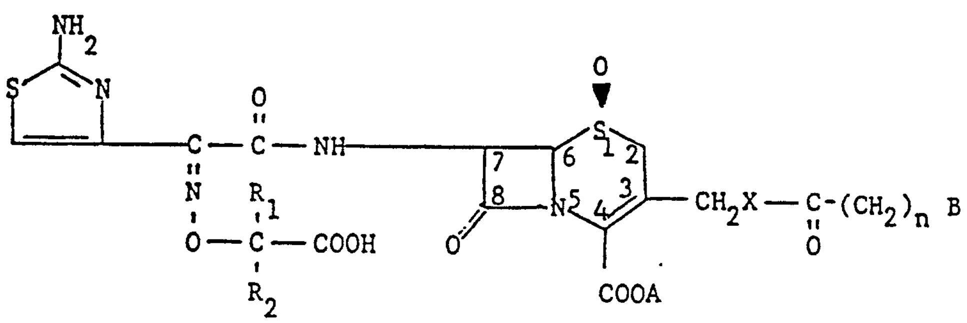 (I)

dans laquelle:

. le groupe COOA en position 4 est un radical acide, ou un sel alcalin ou alcalino-terreux, ou un sel d'aminoacide ou d'amine, par exemple la triéthylamine ou les éthanolamines, ou un radical ester facilement hydrolysable ou métaboliquement labile et pharmaceutiquement acceptable;

. X désigne un atome d'oxygène ou un atome de soufre;
. n est 0 ou 1;
. $R_1$ et $R_2$ désignent chacun indépendamment l'hydrogène ou un groupe méthyle, ou
. $R_1$ et $R_2$ pris ensemble avec l'atome de carbone auquel ils sont liés forment un noyau cyclobutyle ou cyclopentyle;
. B est le reste d'une amine primaire ou secondaire choisi parmi les groupements suivants:

- Z-NH-R, où Z est un groupe alkylène à chaîne droite ou ramidifiée ayant de 2 à 7 atomes de carbone, éventuellement interrompue par un atome de soufre et éventuellement substituée par un groupe hydroxyle, thiol, méthylthio, amino, acétamido, carbamoyle, phényle, hydroxyphényle ou imidazolyle,

Z peut aussi être un groupe cyclopentylidène ou cyclohexylidène,
R représente un atome d'hydrogène ou un groupe alkyle ayant de 1 à 3 atomes de carbone;
- Z'-Alk-NH-R où Z' représente un groupe 1,2-phénylène ou 1,3-phénylène ou 1,4-phénylène éventuellement substitué par un atome d'halogène ou par 1 ou 2 groupes méthoxy ou par 1, 2 ou 3 groupes méthyle ou bien Z' représente un groupe 1,2-cyclohexylène, 1,3-cyclohexylène ou 1,4-cyclohexylène,
Alk représente un groupe alkylène droit ou ramifié ayant de 1 à 3 atomes de carbone,
R est tel que défini ci-dessus,
- Z'-N-CO-Y-NH-R", où Z' est tel que défini ci-dessus,

|
R'

Y désigne un groupe alkylène $(CH_2)_m$ dans lequel m = 0, 1, 2, 3 ou 4, un groupe alkylène ramifié ayant 2 ou 3 atomes de carbone, ou encore, Y avec NH-R" constitue un cycle

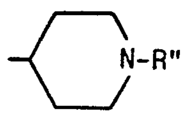

R' et R", identiques ou différents, ont la même signification que celle donnée pour R ci-dessus,
- Z"-NH-R, où Z" est un groupe 1,3-cyclohexylène ou 1,4-cyclohexylène et R est tel que défini précédemment,

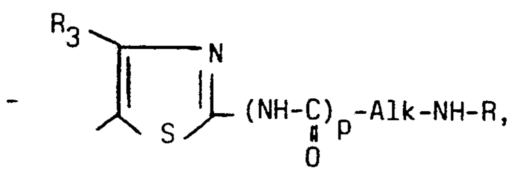

où $R_3$ représente un atome d'hydrogène ou un groupe méthyle,
p = 0 ou 1 et Alk et R sont tels que définis précédemment,
- un groupe 2-pipéridyl, 3-pipéridyl ou 4-pipéridyl éventuellement substitué sur l'atome d'azote par un groupe $-CO-Alk-NH_2$

ou 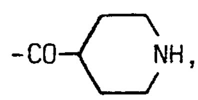

où Alk est tel que défini précédemment, et les sels desdits composés avec des acides pharmaceutiquement acceptables.

2. Dérivés selon la revendication 1, caractérisés en ce qu'ils sont sous l'une des formes syn et anti.

3. Trifluoroacétate de l'acide de formule:

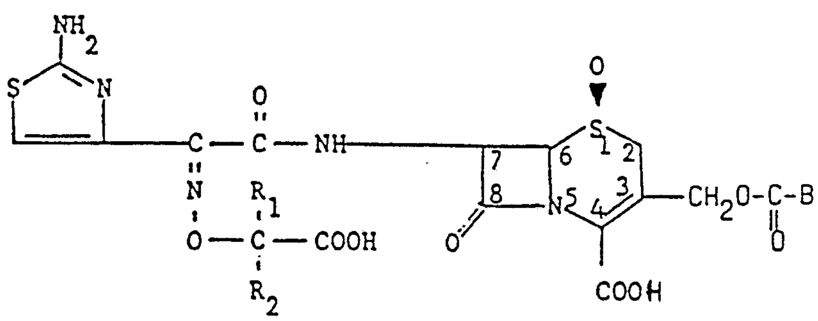

(I)

dans laquelle:
- lorsque $R_1$ et $R_2$ représentent chacun un groupe méthyle, B est le reste de l'amine de formule:

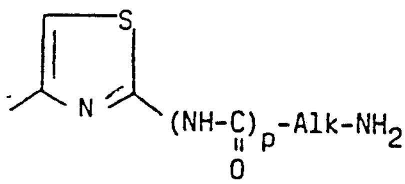

dans laquelle le groupe

$$-(NH-\underset{\underset{O}{\|}}{C})_p-Alk-NH_2$$

est choisi parmi les groupes

$$-NH-\underset{\underset{O}{\|}}{C}-CH_2-NH_2\ ;\ -NH-\underset{\underset{O}{\|}}{C}-CH_2-CH_2-NH_2\ ;-NH-\underset{\underset{O}{\|}}{C}-\underset{\underset{CH_3}{|}}{CH}-NH_2\ ;\ -CH_2-CH_2-NH_2\ :$$

$$-CH_2NH_2\ ;$$

ou B est le reste d'une amine de formule:

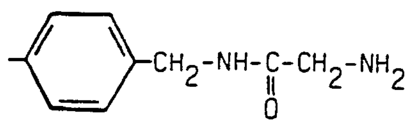

ou

- lorsque $R_1$ et $R_2$ représentent avec l'atome de carbone auquel ils sont liés un groupe cyclobutyle, B est le reste d'une amine de formule:

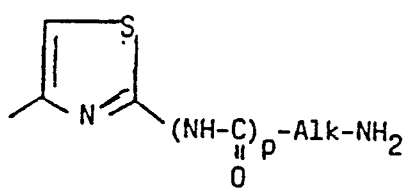

dans laquelle le groupe

$$(NH-\underset{\underset{O}{\|}}{C})_p-Alk-NH_2$$

est choisi parmi les groupes

$$-NH-\underset{\underset{O}{\|}}{C}-CH_2-NH_2\ \text{et}\ CH_2-NH_2\ ;$$

isomère syn.

4. Trifluoroacétate de l'acide de formule:

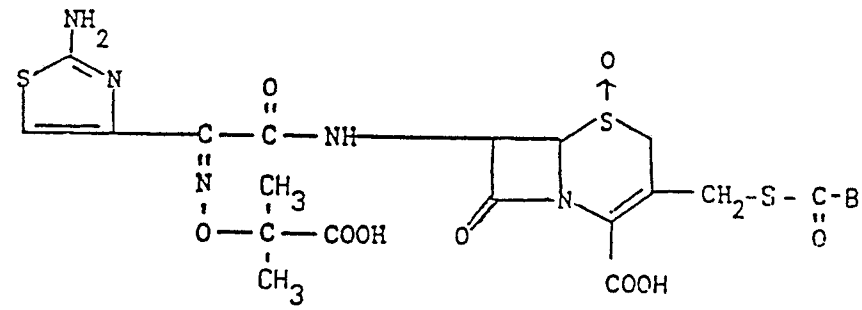

dans laquelle B est le reste d'une amine de formule:

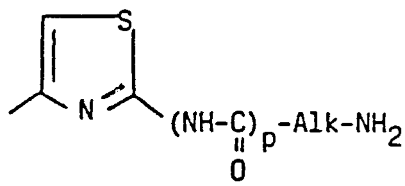

dans laquelle le groupe

$(NH-\underset{\underset{O}{\|}}{C})_p-Alk-NH_2$

est choisi parmi les groupes

$-NH-\underset{\underset{O}{\|}}{C}-CH_2-NH_2$ ; $-NH-\underset{\underset{O}{\|}}{C}-CH_2-CH_2-NH_2$ ; $-CH_2-NH_2$ ;

ou B est le reste d'une amine de formule:

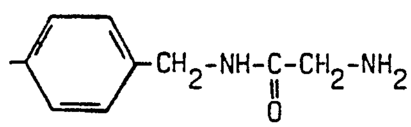 ;

isomère syn.

5. Procédé pour l'obtention des dérivés de la famille des céphalosporines selon l'une des revendications 1 ou 2, caractérisé en ce qu'il consiste:

1) à acyler l'amino-7 bromométhyl-3 céphème-3 carboxylate de tertiobutyle-4 S-oxyde-1 de formule II:

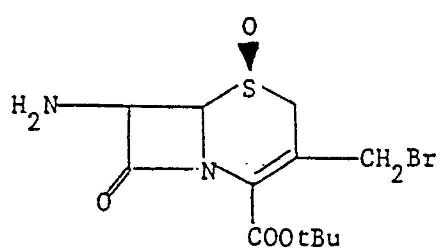

(II)

par l'acide de formule III:

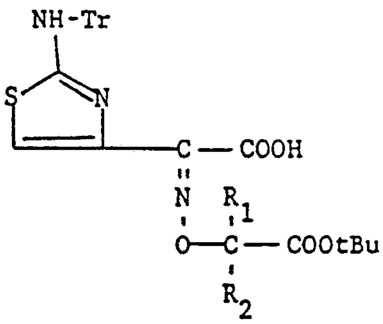

(III)

dans laquelle Tr est le groupe trityle, tBu le groupe tertiobutyle, $R_1$ et $R_2$ sont tels que définis à la revendication 1, pour obtenir le composé de formule IV

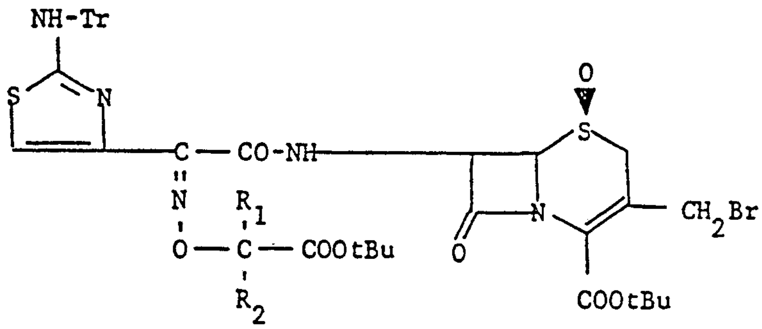

(IV)

2) à ajouter audit composé de formule IV un acide $B-(CH_2)_n-COOH$ ou un thioacide $B-(CH_2)_nCOSH$, dans lesquels n est 0 ou 1 et B est le reste d'une amine primaire ou secondaire, tel que défini à la revendication 1, la fonction amine dudit acide ayant été préalablement protégée pour former le composé de formule V

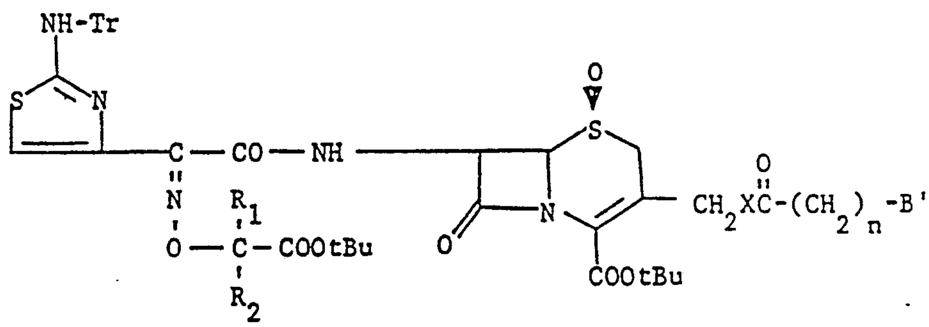

(V)

dans laquelle Tr, $R_1$, $R_2$, n sont tels que définis ci-dessus et B' représente le groupe B, dans lequel la fonction amine est protégée,

3) à éliminer les groupes protecteurs sur les fonctions amines et carboxy pour obtenir le composé de formule I dans laquelle A est l'hydrogène,

4) éventuellement à transformer ledit composé obtenu en un composé de formule I dans laquelle A est autre que H, par action sur ledit composé d'une base minérale ou organique ou par estérification.

5) à transformer éventuellement ledit composé de formule I en un de ses sels par réaction avec un acide pharmaceutiquement acceptable,

6. Procédé selon la revendication 5, caractérisé en ce que l'addition de l'acide $B-(CH_2)_n-COOH$ est réalisée en utilisant le sel de sodium ou de potassium dudit acide, ladite addition étant réalisée dans un solvant apolaire aprotique.

7. Procédé selon la revendication 5, caractérisé en ce que l'addition du thioacide $B'-(CH_2)_n-COSH$ est réalisée en utilisant le sel de sodium ou de potassium dudit acide, ladite addition étant réalisée dans un solvant apolaire.

8. Procédé pour l'obtention des composés selon la revendication 3, caractérisé en ce qu'il consiste à ajouter, dans le diméthylformamide, au composé de formule:

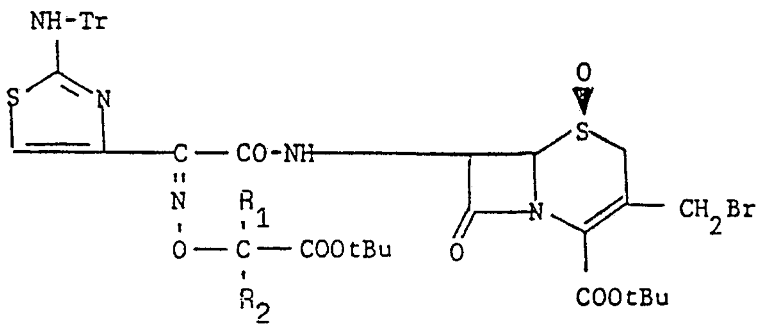

(IV)

isomère syn,

dans laquelle Tr est le groupe trityle, tBu le groupe tertiobutyle, $R_1$ et $R_2$ sont tels que définis à la revendication 3, le sel de potassium de l'acide de formule B'-COOH, dans laquelle B' est le reste d'une amine (B) tel que défini à la revendication 3 et dont la fonction amine a été protégée par le groupe tertiobutoxycarbonyle; et à dissoudre le composé obtenu dans de l'acide trifluoroacétique.

9. Procédé pour l'obtention des composés selon la revendication 4, caractérisé en ce qu'il consiste à ajouter au composé de formule:

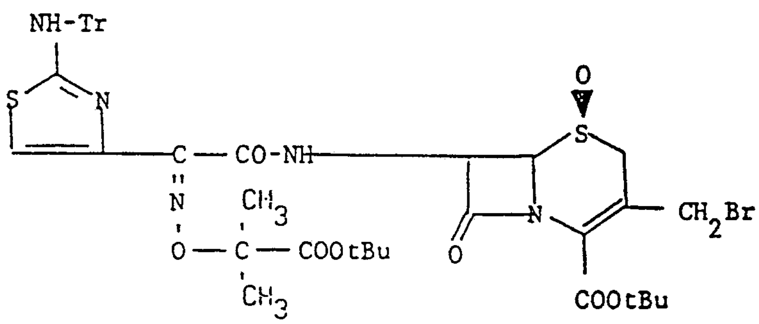

(IV)

isomère syn, dans laquelle Tr est le groupe trityle et tBu le groupe tertiobutyle,

- dans le tétrahydrofurane, le sel de potassium du thioacide de formule B'-COSH, ou
- dans l'acétone anhydre et en présence d'hydrogénocarbonate de potassium et d'iodure de sodium, le thioacide de formule B'-COSH, B' étant le reste d'une amine (B), tel que défini à la revendication 4 et dont la fonction amine a été protégée par le groupe tertiobutoxycarbonyle;

et à dissoudre le composé obtenu dans de l'acide trifluoroacétique.

10. Composition pharmaceutique, caractérisée en ce qu'elle contient, à titre d'ingrédient actif, un dérivé selon l'une quelconque des revendications 1 à 4 en combinaison avec un, véhicule pharmaceutiquement acceptable.

11. Composition pharmaceutique, à action bactériostatique, caractérisée en ce qu'elle contient, à titre d'ingrédient actif, un dérivé selon l'une quelconque des revendications 1 à 4 en combinaison avec un véhicule pharmaceutiquement acceptable.

12. Composition pharmaceutique selon la revendication 11, caractérisée en ce qu'elle est conditionnée sous forme d'ampoules injectables et contient 0,25 g à 4 g du dérivé selon l'une quelconque des revendications 1 à 4.

**Revendications** pour l'état contractant: AT

1. Procédé pour l'obtention de dérivés de la famille des céphalosporines de formule:

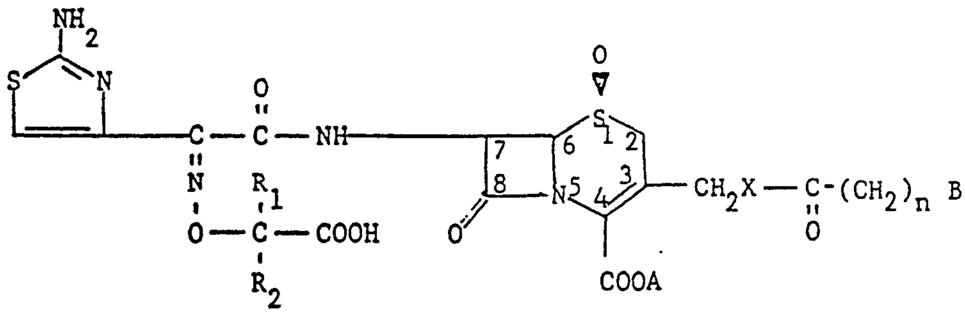 (I)

dans laquelle:

. le groupe COOA en position 4 est un radical acide, ou un sel alcalin ou alcalino-terreux, ou un sel d'aminoacide ou d'amine, par exemple la triéthylamine ou les éthanolamines, ou un radical ester facilement hydrolysable ou métaboliquement labile et pharmaceutiquement acceptable;

. X désigne un atome d'oxygène ou un atome de soufre;

. n est 0 ou 1;

. $R_1$ et $R_2$ désignent chacun indépendamment l'hydrogène ou un groupe méthyle, ou

. $R_1$ et $R_2$ pris ensemble avec l'atome de carbone auquel ils sont liés forment un noyau cyclobutyle ou cyclopentyle;

. B est le reste d'une amine primaire ou secondaire choisi parmi les groupements suivants:

- Z-NH-R, où Z est un groupe alkylène à chaîne droite ou ramifiée ayant de 2 à 7 atomes de carbone, éventuellement interrompue par un atome de soufre et éventuellement substituée par un groupe hydroxyle, thiol, méthylthio, amino, acétamido, carbamoyle, phényle, hydroxyphényle ou imidazolyle,

Z représente un atome d'hydrogène ou un groupe alkyle ayant de 1 à 3 atomes de carbone;

- Z'-Alk-NH-R où Z' représente un groupe 1,2-phénylène ou 1,3-phénylène ou 1,4-phénylène éventuellement substitué par un atome d'halogène ou par 1 ou 2 groupes méthoxy ou par 1, 2 ou 3 groupes méthyle ou bien Z' représente un groupe 1,2-cyclohexylène, 1,3-cyclohexylène ou 1,4-cyclohexylène,

Alk représente un groupe alkylène droit ou ramifié ayant de 1 à 3 atomes de carbone,

R est tel que défini ci-dessus,

- Z'-N-CO-Y-NH-R", où Z' est tel que défini ci-dessus,
 |
 R'

Y désigne un groupe alkylène $(CH_2)_m$ dans lequel m = 0, 1, 2, 3 ou 4, un groupe alkylène ramifié ayant 2 ou 3 atomes de carbone, ou encore, Y avec NH-R" constitue un cycle

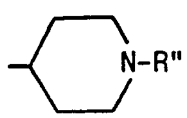

R' et R", identiques ou différents, ont la même signification que celle donnée pour R ci-dessus,

- Z"-NH-R, où Z" est un groupe 1,3-cyclohexylène ou 1,4-cyclohexylène et R est tel que défini précédemment,

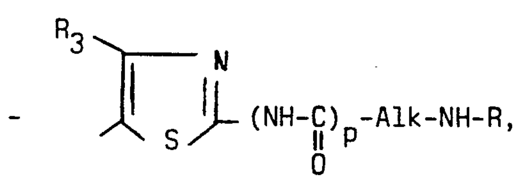

où $R_3$ représente un atome d'hydrogène ou un groupe méthyle,

p = 0 ou 1 et Alk et R sont tels que définis précédemment,

- un groupe 2-pipéridyl, 3-pipéridyl ou 4-pipéridyl éventuellement substitué sur l'atome d'azote par un groupe $-CO-Alk-NH_2$

ou 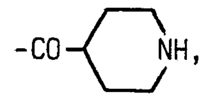

où Alk est tel que défini précédemment, et les sels desdits composés avec des acides pharmaceutiquement acceptables, caractérisé en ce qu'il consiste:

1) à acyler l'amino-7 bromométhyl-3 céphème-3 carboxylate de tertiobutyle-4 S-oxyde-1 de formule II

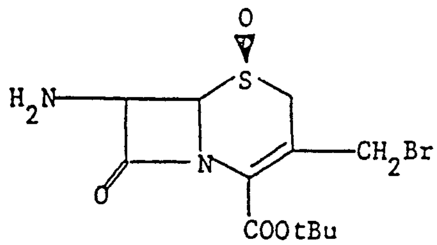

(II)

par l'acide de formule III:

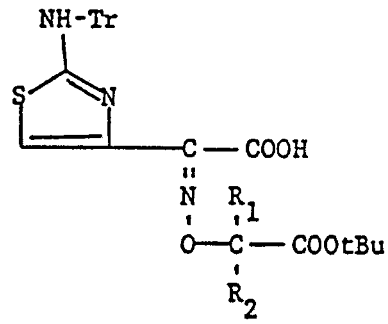

(III)

dans laquelle Tr est le groupe trityle, tBu le groupe tertiobutyle, $R_1$ et $R_2$ sont tels que définis ci-dessus pour obtenir le composé de formule IV

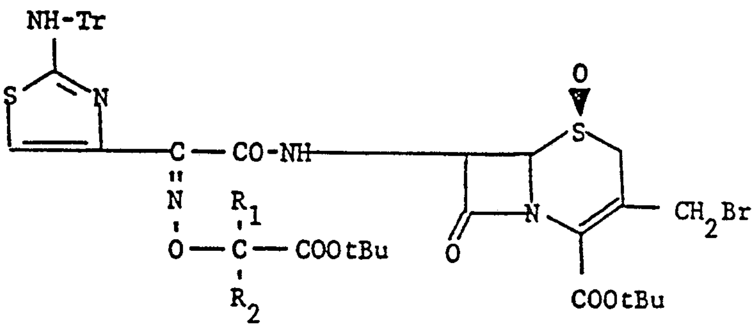

(IV)

2) à ajouter audit composé de formule IV un acide $B-(CH_2)_n-COOH$ ou un thioacide $B-(CH_2)_nCOSH$, dans lesquels n est 0 ou 1 et B est le reste d'une amine primaire ou secondaire, tel que défini ci-dessus, la fonction amine dudit acide ayant été préalablement protégée pour former le composé de formule V

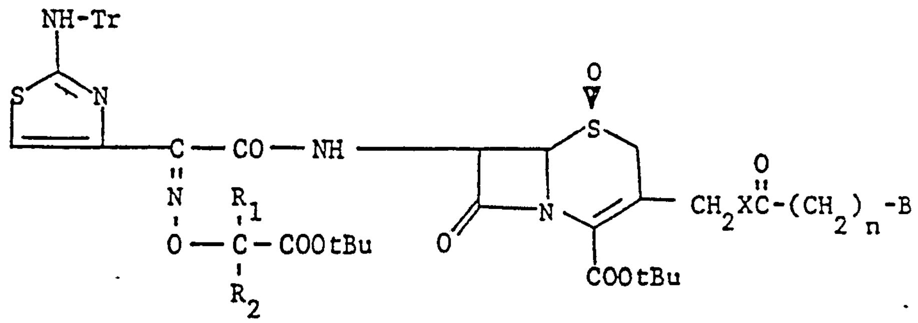

(V)

dans laquelle Tr, $R_1$, $R_2$, n sont tels que définis précédemment, et B' représente le groupe B dans lequel la fonction amine est protégée,

3) à éliminer les groupes protecteurs sur les fonctions amines et carboxy pour obtenir le composé de formule I, dans laquelle A est l'hydrogène,

4) éventuellement à transformer ledit composé obtenu en un composé de formule I, dans laquelle A est autre que H par action sur ledit composé d'une base minérale ou organique ou par estérification,

5) à transformer éventuellement ledit composé de formule I en un de ses sels par réaction avec un acide pharmaceutiquement acceptable.

2. Procédé selon la revendication 1, caractérisé en ce que l'acide de formule (III) est utilisé sous forme syn ou anti.

3. Procédé selon l'une des revendications 1 ou 2, caractérisé en ce que l'addition de l'acide $B-(CH_2)_n-COOH$ est réalisée en utilisant le sel de sodium ou de potassium dudit acide, ladite addition étant réalisée dans un solvant apolaire aprotique.

4. Procédé selon l'une des revendications 1 ou 2, caractérisé en ce que l'addition du thioacide $B'-(CH_2)_n-COSH$ est réalisée en utilisant le sel de sodium ou de potassium dudit acide, ladite addition étant réalisée dans un solvant apolaire.

5. Procédé pour l'obtention du trifluoroacétate de l'acide de formule:

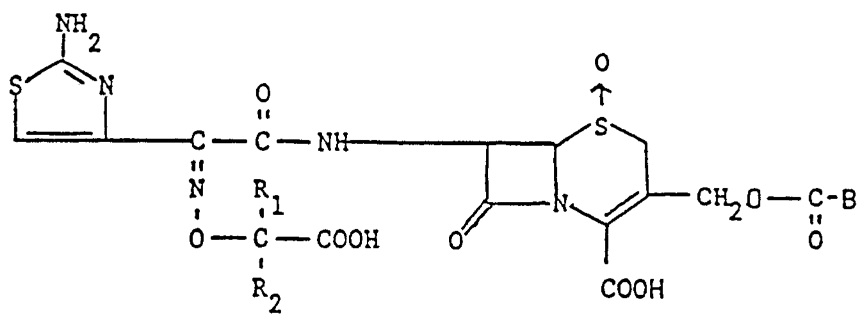

dans laquelle:

- lorsque $R_1$ et $R_2$ représentent chacun un groupe méthyle,
B est le reste d'une amine de formule:

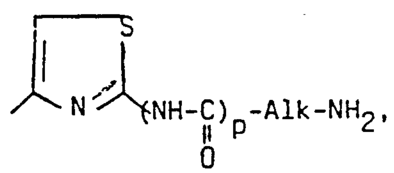

dans laquelle le groupe

$$-(NH-\underset{\underset{O}{\|}}{C})_p-Alk-NH_2$$

est choisi parmi les groupes

$$-NH-\underset{\underset{O}{\|}}{C}-CH_2-NH_2, \quad -NH-\underset{\underset{O}{\|}}{C}-CH_2-CH_2-NH_2, \quad -NH-\underset{\underset{O}{\|}}{C}-\underset{\underset{CH_3}{|}}{CH}-NH_2,$$

$$-CH_2-CH_2-NH_2, \ -CH_2-NH_2 \ ;$$

ou B est le reste d'une amine de formule:

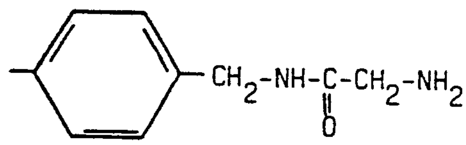

ou

- lorsque $R_1$ et $R_2$ représentent avec l'atome de carbone auquel ils sont liés, un groupe cyclobutyle, B est le reste d'une amine de formule:

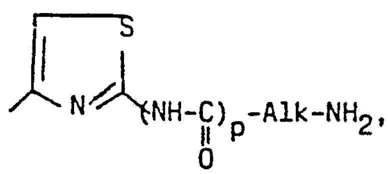

dans laquelle le groupe

$$-(NH-\underset{\underset{O}{\|}}{C})_p-Alk-NH_2$$

est choisi parmi les groupes

$$-NH-\underset{\underset{O}{\|}}{C}-CH_2-NH_2 \ et -CH_2-NH_2 \ ;$$

isomère syn,

caractérisé en ce qu'il consiste à ajouter, dans le diméthylformamide, au composé de formule:

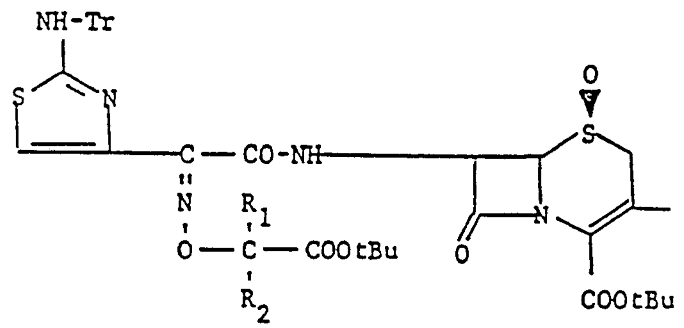

(IV)

, isomère syn,

dans laquelle Tr est le groupe trityle, tBu le groupe tertiobutyle, $R_1$ et $R_2$ sont tels que définis ci-dessus, le sel de potassium de l'acide de formule B'-COOH dans laquelle B' est le reste d'une amine (B) tel que défini ci-dessus et dont la fonction amine a été protégée par le groupe tertiobutoxycarbonyle; et à dissoudre le composé obtenu dans de l'acide trifluoroacétique.

6. Procédé pour l'obtention du trifluoroacétate de l'acide de formule:

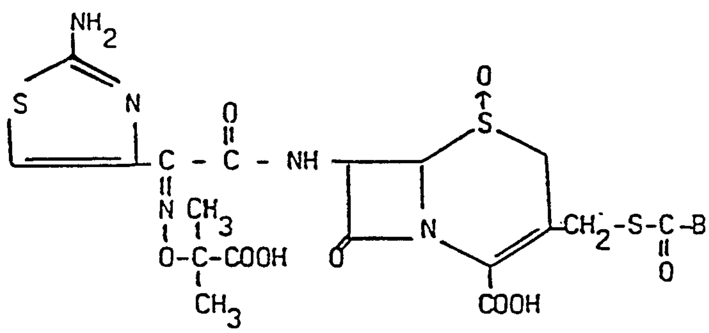

dans laquelle B est le reste d'une amine de formule:

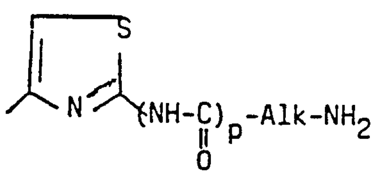

dans laquelle le groupe

$$(NH-\underset{\underset{O}{\|}}{C})_p-Alk-NH_2$$

est choisi parmi les groupes

$$-NH-\underset{\underset{O}{\|}}{C}-CH_2-NH_2\ ;\ -NH-\underset{\underset{O}{\|}}{C}-CH_2-CH_2-NH_2\ ;\ -CH_2-NH_2\ ;$$

ou B est le reste d'une amine de formule:

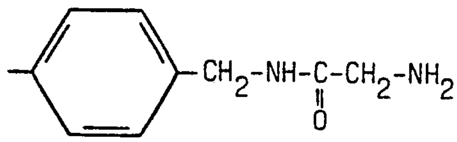

isomère syn,

caractérisé en ce qu'il consiste à ajouter au composé de formule:

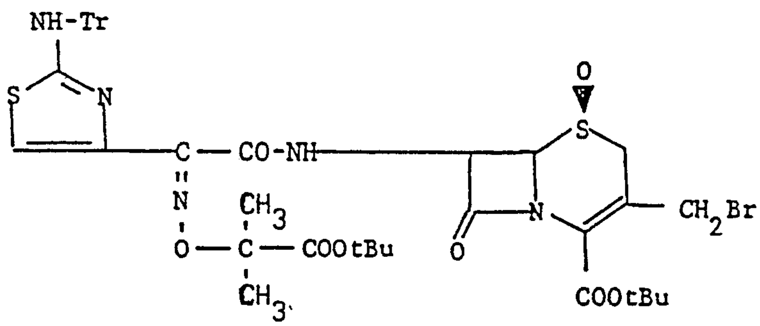

(IV)

isomère syn, dans laquelle Tr est le groupe trityle et tBu le groupe tertiobutyle;
- dans le tétrahydrofurane, le sel de potassium du thioacide de formule B'-COSH, ou
- dans l'acétone anhydre et en présence d'hydrogénocarbonate de potassium et d'iodure de sodium, le thioacide de formule B'-COSH,

B' étant le reste d'une amine (B), tel que défini ci-dessus et dont la fonction amine a été protégée par le groupe tertiobutoxycarbonyle;

et à dissoudre le composé obtenu dans de l'acide trifluoroacétique.

7. Utilisation des dérivés de la famille des céphalosporines obtenus selon l'une quelconque des revendications 1 à 6, en combinaison avec un véhicule pharmaceutiquement acceptable pour la préparation de compositions pharmaceutiques.

8. Utilisation des dérivés de la famille des céphalosporines obtenus selon l'une quelconque des revendications 1 à 6, en combinaison avec un véhicule pharmaceutiquement acceptable pour la préparation de compositions pharmaceutiques, à action bactériostatique.

9. Utilisation selon la revendication 8, pour la préparation de compositions pharmaceutiques, conditionnées sous forme d'ampoules injectables, contenant de 0,25 à 4 g dudit dérivé.

**Patentansprüche** für die Vertragsstaaten BE, CH, DE, FR, GB, IT, LI, LU, NL, SE:

1. Derivate der Familie der Cephalosporine der Formel

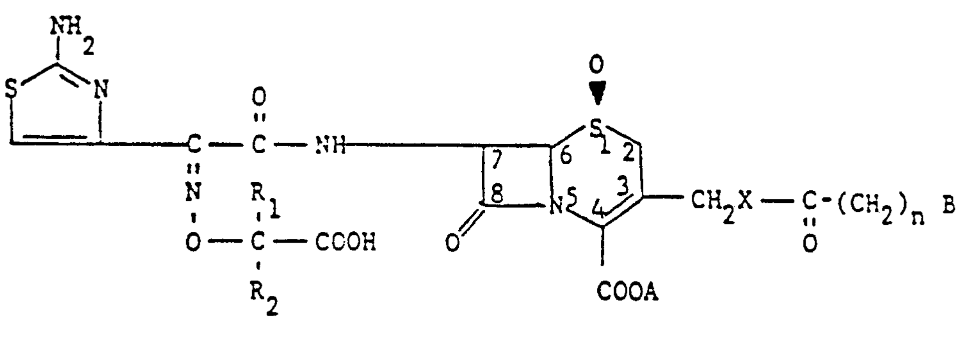

(I)

worin

. die Gruppe COOA in Position 4 ein Säurerest oder ein Alkali- oder Erdalkalisalz oder ein Salz einer Aminosäure oder eines Amins, beispielsweise Triethylamin oder Ethanolamine, oder ein leicht hydroylsierbarer oder metabolisch labiler und pharmazeutisch akzeptabler Esterrest ist;

. X ein Sauerstoffatom oder ein Schwefelatom bezeichnet;

. n für 0 oder 1 steht;

. $R_1$ und $R_2$ jeweils unabhängig Wasserstoff oder eine Methylgruppe darstellen, oder

. $R_1$ und $R_2$, zusammen mit dem Kohlenstoffatom, an das sie gebunden sind, einen Cyclobutyl- oder Cyclopentylkern bilden;

. B der Rest eines primären oder sekundären Amins ist, ausgewählt aus den folgenden Gruppen:

- Z-NH-R, worin Z eine Alkylengruppe mit gerader oder verzweigter Kette mit 2 bis 7 Kohlenstoffatomen, gegebenenfalls unterbrochen von einem Schwefelatom und gegebenenfalls substituiert durch eine Hydroxyl-, Thiol-, Methylthio-, Amino-, Acetamido-, Carbamoyl-, Phenyl-, Hydroxyphenyloder Imidazolylgruppe, ist,

Z auch eine Cyclopentyliden- oder Cyclohexylidengruppe sein kann,

R ein Wasserstoffatom oder eine Alkylgruppe mit 1 bis 3 Kohlenstoffatomen darstellt;

- Z'-Alk-NH-R, worin Z' eine 1,2-Phenylen- oder 1,3 -Phenylen- oder 1,4-Phenylengruppe, gegebenenfalls substituiert durch ein Halogenatom oder durch 1 oder 2 Methoxygruppen oder durch 1, 2 oder 3 Methylgruppen, darstellt, oder Z' auch eine 1,2-Cyclohexylen-, 1,3-Cyclohexylen- oder 1,4 -Cyclohexylengruppe bedeutet,

Alk für eine gerade oder verzweigte Alkylengruppe mit 1 bis 3 Kohlenstoffatomen steht,

R wie oben definiert ist;

- Z'-N-CO-Y-NH-R", worin Z' wie oben definiert ist,
   |
   R'

Y eine Alkylengruppe $(CH_2)_m$, worin m = 0, 1, 2, 3 oder 4, eine verzweigte Alkylengruppe mit 2 oder 3 Kohlenstoffatomen,

bezeichnet, oder aber Y mit NH-R" einen Ring

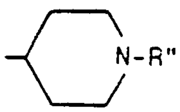

bildet, R' und R", gleich oder verschieden, die wie oben für R angegebene Bedeutung haben,

- Z"-NH-R-, worin Z" eine 1,3-Cyclohexylen- oder 1,4-Cyclohexylengruppe darstellt und R wie zuvor definiert ist,

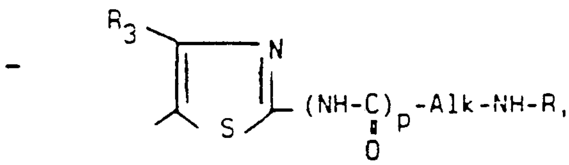

worin $R_3$ ein Wasserstoffatom oder eine Methylgruppe darstellt,

p = 0 oder 1 und Alk und R wie zuvor definiert sind,

- einer 2-Piperidyl-, 3-Piperidyl- oder 4-Piperidylgruppe, die gegebenenfalls am Stickstoffatom durch eine Gruppe

-CO-Alk-$NH_2$ oder -CO-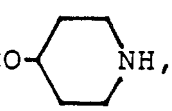

worin Alk wie zuvor definiert ist, substituiert ist;

und die Salze dieser Verbindungen mit pharmazeutisch akzeptablen Säuren.

2. Derivate nach Anspruch 1, dadurch gekennzeichnet, daß sie in einer der Formen syn- und anti- vorliegen.

3. Trifluoracetat der Säure der Formel

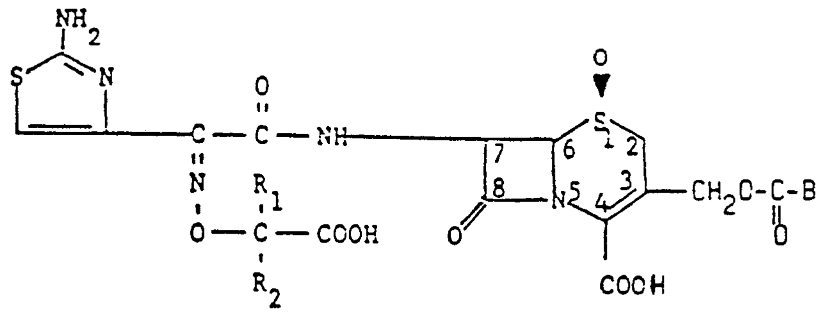

(I)

worin

- wenn $R_1$ und $R_2$ jeweils eine Methylgruppe darstellen, B der Rest des Amins der Formel

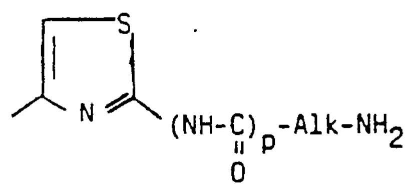

ist, worin die Gruppe

$$-(NH-\underset{\underset{O}{\|}}{C})_p-Alk-NH_2$$

ausgewählt ist aus den Gruppen

$-NH-\underset{\underset{O}{\|}}{C}-CH_2-NH_2$ ; $-NH-\underset{\underset{O}{\|}}{C}-CH_2-CH_2-NH_2$ ; $-NH-\underset{\underset{O}{\|}}{C}-\underset{\underset{CH_3}{|}}{CH}-NH_2$ ; $-CH_2-CH_2-NH_2$ ;

$-CH_2NH_2$ ;

oder B der Rest eines Amins der Formel

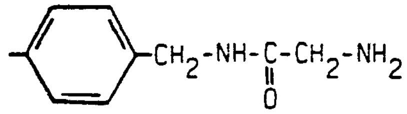

ist,

oder

- wenn $R_1$ und $R_2$ mit dem Kohlenstoffatom, an das sie gebunden sind, eine Cyclobutylgruppe darstellen, B der Rest eines Amins der Formel

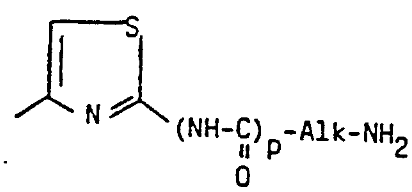

ist worin die Gruppe

$$(NH-\underset{\underset{O}{\|}}{C})_p-Alk-NH_2$$

ausgewählt ist aus den Gruppen

$$-NH-\underset{\underset{O}{\|}}{C}-CH_2-NH_2 \text{ und } CH_2-NH_2;$$

syn-Isomeres.

4. Trifluoracetat der Säure der Formel

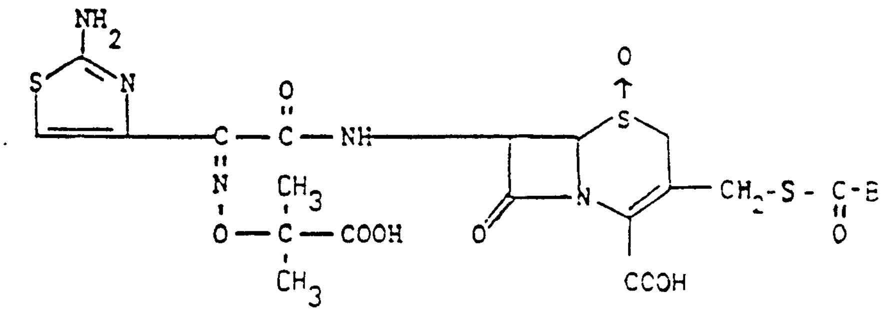

worin B der Rest eines Amins der Formel

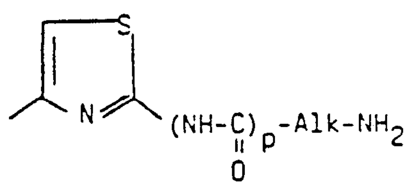

ist, worin die Gruppe

$$(NH-\underset{\underset{O}{\|}}{C})_p-Alk-NH_2$$

ausgewählt ist aus den Gruppen

$$-NH-\underset{\underset{O}{\|}}{C}-CH_2-NH_2 \;;\; -NH-\underset{\underset{O}{\|}}{C}-CH_2-CH_2-NH_2 \;;\; -CH_2-NH_2 \;;$$

oder B der Rest eines Amins der Formel

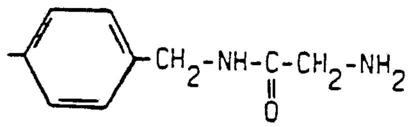

ist;
syn-Isomeres.

5. Verfahren zur Herstellung der Derivate der Familie der Cephalosporine nach einem der Ansprüche 1 oder 2, dadurch gekennzeichnet, daß es darin besteht, daß

1) 7-Amino-3-brommethyl-3-cephem-4-tert. Butylcarboxylat-1-S-oxid der Formel II

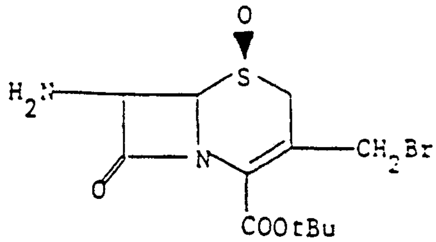

(II)

mit der Säure der Formel III

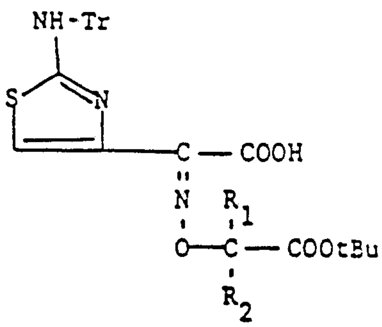

(III)

worin Tr die Gruppe Trityl ist, tBu die Gruppe tert. Butyl ist, $R_1$ und $R_2$ wie in Anspruch 1 definiert sind, acyliert wird, um die Verbindung der Formel IV

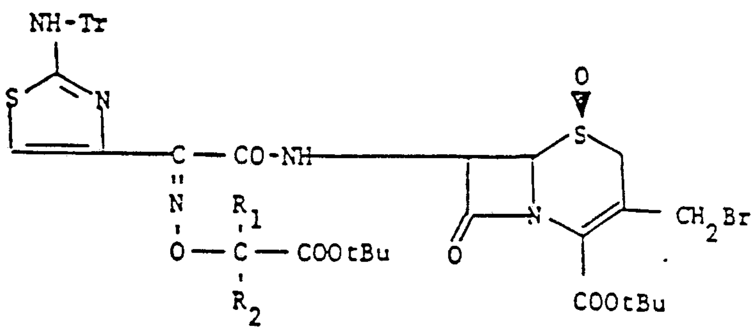

(IV)

zu erhalten,

2) zur Verbindung der Formel IV eine Säure B-$(CH_2)_n$-COOH oder eine Thiosäure B-$(CH_2)_n$COSH gegeben wird, worin n für 0 oder 1 steht und B der Rest eines primären oder sekundären Amins, wie in Anspruch 1 definiert, ist, wobei die Aminfunktion des Säureamins zuvor geschützt wurde, zur Bildung der Verbindung der Formel V

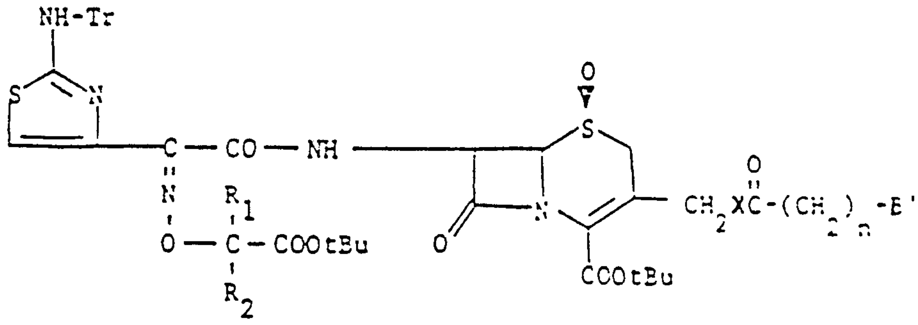

(V)

worin Tr, $R_1$, $R_2$, n wie oben definiert sind und B' die Gruppe B darstellt, in der die Aminfunktion geschützt ist,

3) die Schutzgruppen an den Amin- und Carboxyfunktionen entfernt werden, um die Verbindung der Formel I zu erhalten, in der A Wasserstoff ist,

4) gegebenenfalls die erhaltene Verbindung in eine Verbindung der Formel I übergeführt, wird, worin A anders als H ist, u. zw. durch Einwirken einer mineralischen oder organischen Base auf diese Verbindung oder durch Veresterung,

5) gegebenenfalls die Verbindung der Formel I durch Reaktion mit einer pharmazeutisch akzeptablen Säure in eines ihrer Salze übergeführt wird.

6. Verfahren nach Anspruch 5, dadurch gekennzeichnet, daß die Zugabe der Säure B-$(CH_2)_n$-COOH unter Verwendung des Natrium- oder Kaliumsalzes dieser Säure erfolgt, wobei die Zugabe in einem aprotischen apolaren Lösungsmittel durchgeführt wird.

7. Verfahren nach Anspruch 5, dadurch gekennzeichnet, daß die Zugabe der Thiosäure B'-$(CH_2)_n$-COSH unter Verwendung des Natrium- oder Kaliumsalzes dieser Säure erfolgt, wobei die Zugabe in einem apolaren Lösungsmittel durchgeführt wird.

8. Verfahren zur Herstellung der Verbindungen nach Anspruch 3, dadurch gekennzeichnet, daß es darin besteht, daß in Dimethylformamid zur Verbindung der Formel

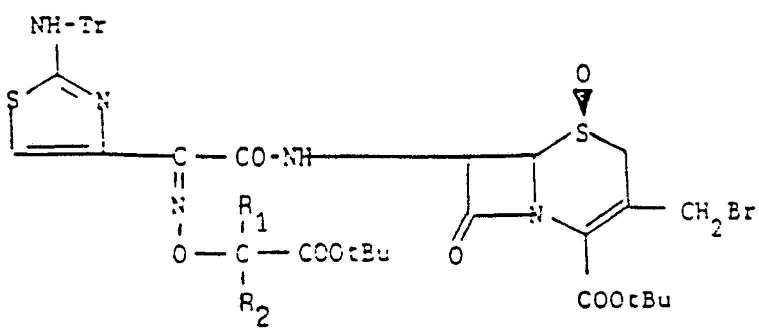

(IV)

syn-Isomeres,

worin Tr die Gruppe Trityl, tBu die Gruppe tert. Butyl ist, $R_1$ und $R_2$ wie in Anspruch 3 definiert sind, das Kaliumsalz der Säure der Formel B'-COOH, worin B' einen Aminrest (B) wie in Anspruch 3 definiert und dessen Aminfunktion durch die Gruppe tert. Butoxycarbonyl geschützt ist, darstellt, zugegeben wird und die erhaltene Verbindung in Trifluoressigsäure gelöst wird.

9. Verfahren zur Herstellung der Verbindungen nach Anspruch 4, dadurch gekennzeichnet, daß es darin besteht, daß zur Verbindung der Formel

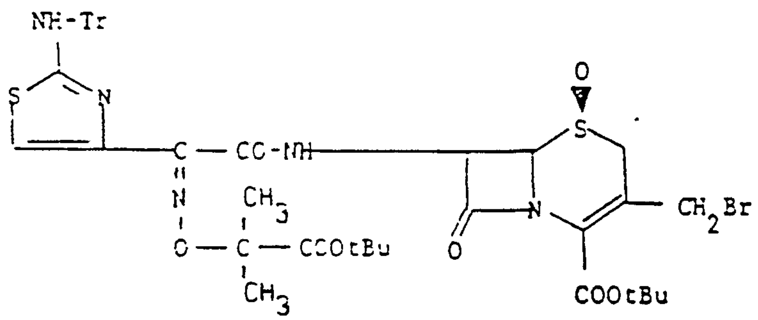

(IV)

syn-Isomeres, worin Tr die Gruppe Trityl und tBu die Gruppe tert. Butyl ist,
- in Tetrahydrofuran das Kaliumsalz der Thiosäure der Formel B'-COSH
- oder in wasserfreiem Aceton und in Gegenwart von Kaliumhydrogencarbonat und Natriumjodid die Thiosäure der Formel B'-COSH,

worin B' den Rest eines Amins (B), wie in Anspruch 4 definiert und dessen Aminfunktion durch die Gruppe tert. Butoxycarbonyl geschützt ist, darstellt, zugegeben wird und die erhaltene Verbindung in Trifluoressigsäure gelöst wird.

10. Pharmazeutische Zusammensetzung, dadurch gekennzeichnet, daß sie als aktives Ingrediens ein Derivat nach einem der Ansprüche 1 bis 4 in Kombination mit einem pharmazeutisch akzeptablen Vehikel enthält.

11. Pharmazeutische Zusammensetzung mit bakteriostatischer Wirkung, dadurch gekennzeichnet, daß sie als aktives Ingrediens ein Derivat nach einem der Ansprüche 1 bis 4 in Kombination mit einem pharmazeutisch akzeptablen Vehikel enthält.

12. Pharmazeutische Zusammensetzung nach Anspruch 11,
dadurch gekennzeichnet, daß sie in Form von Injektionsampullen konditioniert ist und 0,25 g bis 4 g des Derivats nach einem der Ansprüche 1 bis 4 enthält.

**Patentansprüche** für den Vertragsstaat: AT

1. Verfahren zur Herstellung von Derivaten der Familie der Cephalosporine der Formel

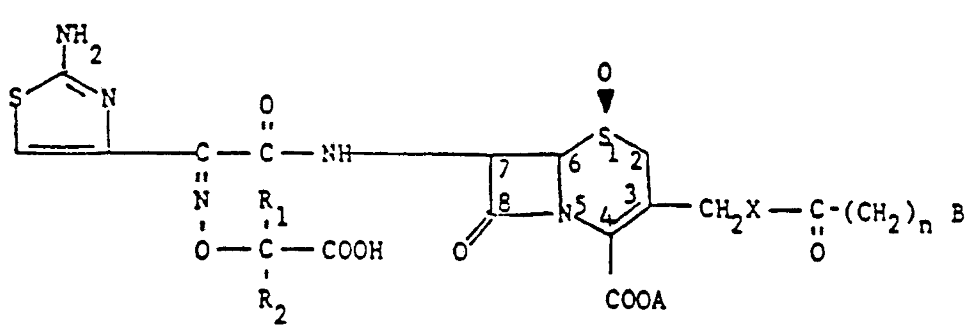

(I)

worin
die Gruppe COOA in Position 4 ein Säurerest oder ein Alkali- oder Erdalkalisalz oder ein Salz einer

Aminosäure oder eines amins, beispielsweise Triethylamin oder Ethanolamine, oder ein leicht hydroylsierbarer oder metabolisch labiler und pharmazeutisch akzeptabler Esterrest ist;

. X ein Sauerstoffatom oder ein Schwefelatom bezeichnet;

. n für 0 oder 1 steht;

. $R_1$ und $R_2$ jeweils unabhängig Wasserstoff oder eine Methylgruppe darstellen, oder

. $R_1$ und $R_2$, zusammen mit dem Kohlenstoffatom, an das sie gebunden sind, einen Cyclobutyl- oder Cyclopentylkern bilden;

. B der Rest eines primären oder sekundären amins ist, ausgewählt aus den folgenden Gruppen:

- Z-NH-R, worin Z eine Alkylengruppe mit gerader oder verzweigter Kette mit 2 bis 7 Kohlenstoffatomen, gegebenenfalls unterbrochen von einem Schwefelatom und gegebenenfalls substituiert durch eine Hydroxyl-, Thiol-, Methylthio-, Amino-, Acetamido-, Carbamoyl-, Phenyl-, Hydroxyphenyloder Imidazolylgruppe, ist,

Z auch eine Cyclopentyliden- oder Cyclohexylidengruppe sein kann,

R ein Wasserstoffatom oder eine Alkylgruppe mit 1 bis 3 Kohlenstoffatomen darstellt;

- Z'-Alk-NH-R, worin Z' eine 1,2-Phenylen- oder 1,3-Phenylen- oder 1,4-Phenylengruppe, gegebenenfalls substituiert durch ein Halogenatom oder durch 1 oder 2 Methoxygruppen oder durch 1, 2 oder 3 Methylgruppen, darstellt, oder Z' auch eine 1,2-Cyclohexylen-, 1,3-Cyclohexylen- oder 1,4-Cyclohexylengruppe bedeutet,

Alk für eine gerade oder verzweigte Alkylengruppe mit 1 bis 3 Kohlenstoffatomen steht,

R wie oben definiert ist;

- Z'-N-CO-Y-NH-R", worin Z' wie oben definiert ist,
    |
    R'

Y eine Alkylengruppe $(CH_2)_m$, worin m = 0, 1, 2, 3 oder 4, eine verzweigte Alkylengruppe mit 2 oder 3 Kohlenstoffatomen,

bezeichnet, oder aber Y mit NH-R" einen Ring

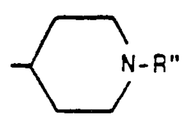

bildet, R' und R", gleich oder verschieden, die wie oben für R angegebene Bedeutung haben,

- Z"-NH-R-, worin Z" eine 1,3-Cyclohexylen- oder 1,4-Cyclohexylengruppe darstellt und R wie zuvor definiert ist,

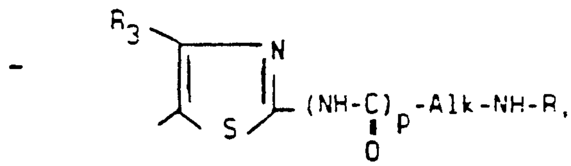

worin $R_3$ ein Wasserstoffatom oder eine Methylgruppe darstellt,

p = 0 oder 1 und Alk und R wie zuvor definiert sind,

- einer 2-Piperidyl-, 3-Piperidyl- oder 4-Piperidylgruppe, die gegebenenfalls am Stickstoffatom durch eine Gruppe

-CO-Alk-$NH_2$ oder -CO- 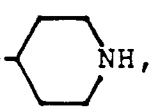

worin Alk wie zuvor definiert ist, substituiert ist;

und der Salze dieser Verbindungen mit pharmazeutisch akzeptablen Säuren,

dadurch gekennzeichnet, daß es darin besteht, daß

1) 7-Amino-3-brommethyl-3-cephem-4-tert. Butylcarboxylat-1-S-oxid der Formel II

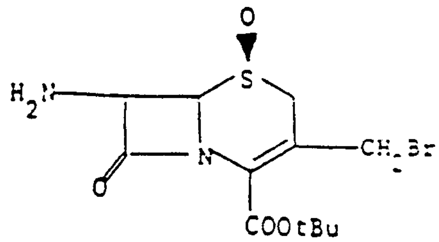

(II)

mit der Säure der Formel III

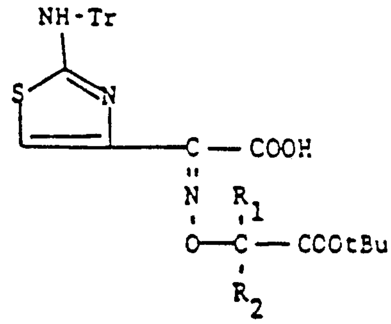

(III)

worin Tr die Gruppe Trityl ist, tBu die Gruppe tert. Butyl ist, $R_1$ und $R_2$ wie oben definiert sind, acyliert wird, um die Verbindung der Formel IV

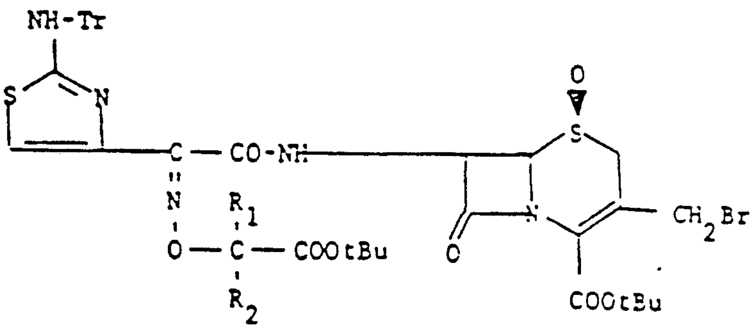

(IV)

zu erhalten,

2) zur Verbindung der Formel IV eine Säure B-$(CH_2)_n$-COOH oder eine Thiosäure B-$(CH_2)_n$COSH gegeben wird, worin n für 0 oder 1 steht und B den Rest eines primären oder sekundären Amins, wie oben definiert, darstellt, wobei die Aminfunktion des Säureamins zuvor geschützt wurde, zur Bildung der Verbindung der Formel V

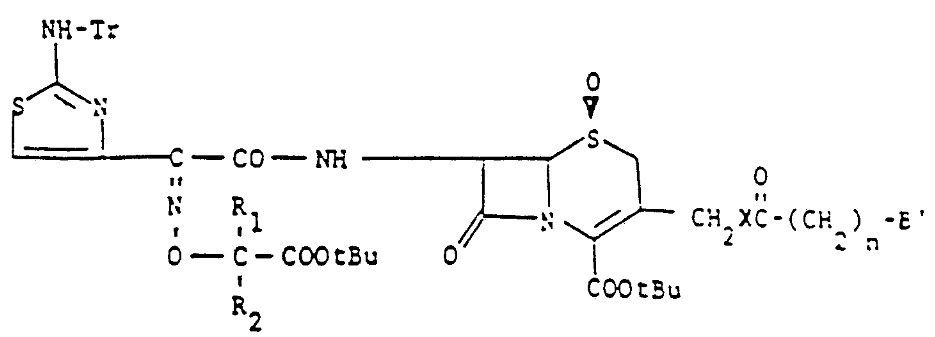

(V)

worin Tr, $R_1$, $R_2$, n wiezuvor definiert sind und B' die Gruppe B darstellt, in der die Aminfunktion geschützt ist,

3) die Schutzgruppen an den Amin- und Carboxyfunktionen entfernt werden, um die Verbindung der Formel I zu erhalten, in der A Wasserstoff ist,

4) gegebenenfalls die erhaltene Verbindung in eine Verbindung der Formel I übergeführt wird, worin A anders als H ist, u. zw. durch Einwirken einer mineralischen oder organischen Base auf diese Verbindung oder

durch Veresterung,

5) gegebenenfalls die Verbindung der Formel I durch Reaktion mit einer pharmazeutisch akzeptablen Säure in eines ihrer Salze übergeführt wird.

2. Verfahren nach Anspruch 1, dadurch gekennzeichnet, daß die Säure der Formel (III) in der syn- oder anti-Form verwendet wird.

3. Verfahren nach einem der Ansprüche 1 oder 2, dadurch gekennzeichnet, daß die Zugabe der Säure B-$(CH_2)_n$-COOH unter Verwendung des Natrium- oder Kaliumsalzes dieser Säure erfolgt, wobei die Zugabe in einem aprotischen apolaren Lösungsmittel durchgeführt wird.

4. Verfahren nach einem der Ansprüche 1 oder 2, dadurch gekennzeichnet, daß die Zugabe der Thiosäure B'-$(CH_2)_n$-COSH unter Verwendung des Natrium- oder Kaliumsalzes dieser Säure erfolgt, wobei die Zugabe in einem apolaren Lösungsmittel durchgeführt wird.

5. Verfahren zur Herstellung des Trifluoracetats der Säure der Formel

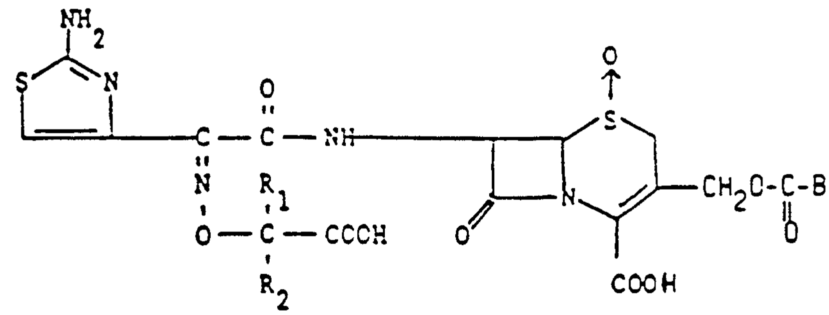

worin

- wenn $R_1$ und $R_2$ jeweils eine Methylgruppe darstellen, B der Rest des Amins der Formel

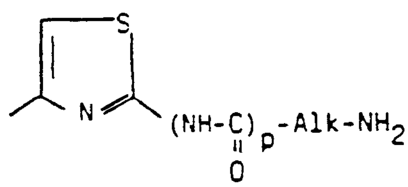

ist, worin die Gruppe

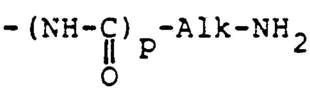

ausgewählt ist aus den Gruppen

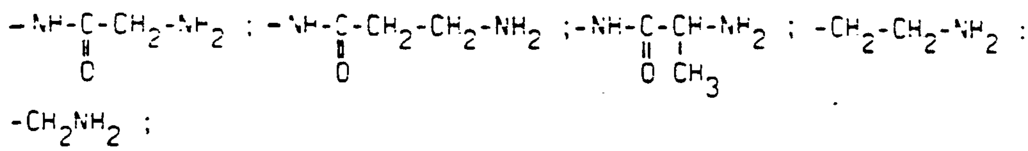

$-CH_2NH_2$ ;

oder B der Rest eines Amins der Formel

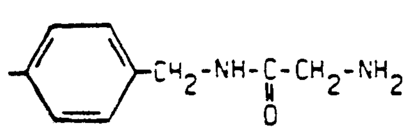

ist,

oder

- wenn $R_1$ und $R_2$ mit dem Kohlenstoffatom, an das sie gebunden sind, eine Cyclobutylgruppe darstellen, B der Rest eines Amins der Formel

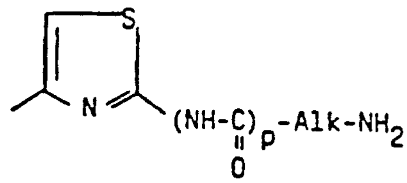

ist, worin die Gruppe

$$-(NH-\underset{\underset{O}{\|}}{C})_p-Alk-NH_2$$

ausgewählt ist aus den Gruppen

$$-NH-\underset{\underset{O}{\|}}{C}-CH_2-NH_2 \text{ und } CH_2-NH_2;$$

syn-Isomeres,

dadurch gekennzeichnet, daß es darin besteht, daß in Dimethylformamid zur Verbindung der Formel

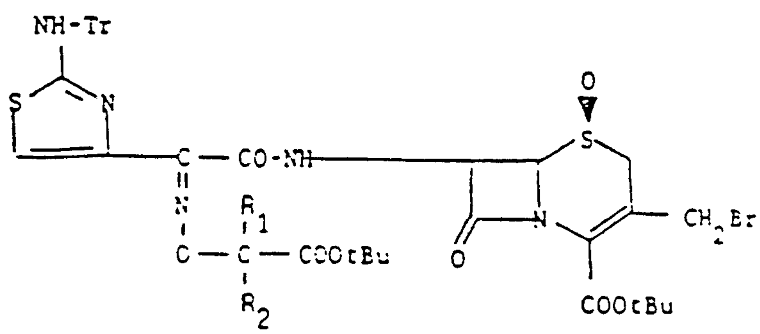

(IV)

syn-Isomeres,

worin Tr die Gruppe Trityl, tBu die Gruppe tert. Butyl ist, $R_1$ und $R_2$ wie oben definiert sind, das Kaliumsalz der Säure der Formel B'-COOH, worin B' ein Aminrest (B) ist, wie oben definiert und dessen Aminfunktion durch die Gruppe tert. Butoxycarbonyl geschützt ist, zugegeben wird und die erhaltene Verbindung in Trifluoressigsäure gelöst wird.

6. Verfahren zur Herstellung des Trifluoracetats der Säure der Formel

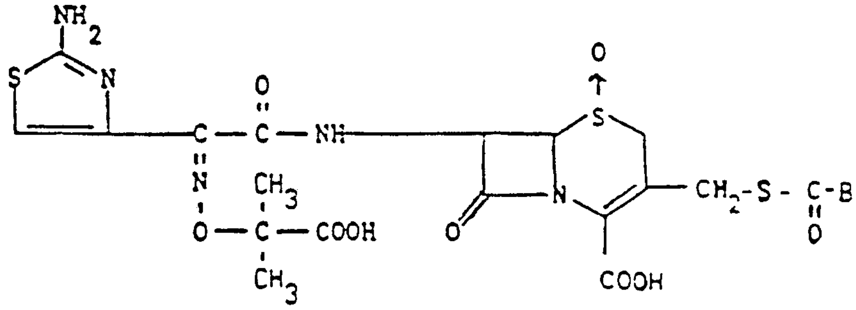

worin B der Rest eines Amins der Formel

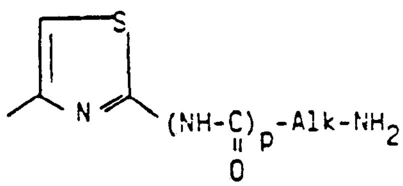

ist, worin die Gruppe

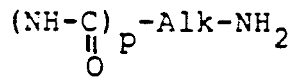

ausgewählt ist aus den Gruppen

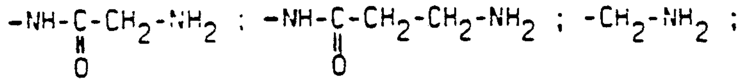

oder B der Rest eines Amins der Formel

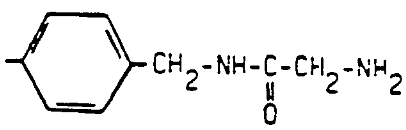

ist;
syn-Isomeres,
dadurch gekennzeichnet, daß es darin besteht, daß zur Verbindung der Formel

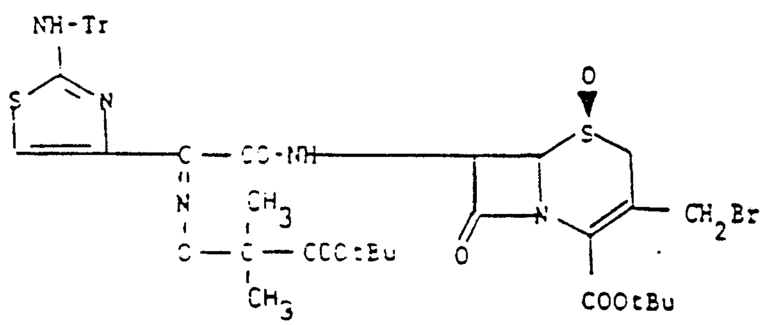

(IV)

syn-Isomeres, worin Tr die Gruppe Trityl und tBu die Gruppe tert. Butyl ist,
- in Tetrahydrofuran das Kaliumsalz der Thiosäure der Formel B'-COSH
- oder in wasserfreiem Aceton und in Gegenwart von Kaliumhydrogencarbonat und Natriumjodid die Thiosäure der Formel B'-COSH,

worin B' der Rest eines Amins (B) ist, wie oben definiert und dessen Aminfunktion durch die Gruppe tert. Butoxycarbonyl geschützt ist, zugegeben wird und die erhaltene Verbindung in Trifluoressigsäure gelöst wird.

7. Verwendung der nach einem der Ansprüche 1 bis 6 erhaltenen Derivate der Familie der Cephalosporine in Kombination mit einem pharmazeutisch akzeptablen Vehikel zur Herstellung von pharmazeutischen Zusammensetzungen.

8. Verwendung der nach einem der Ansprüche 1 bis 6 erhaltenen Derivate der Familie der Cephalosporine in Kombination mit einem pharmazeutisch akzeptablen Vehikel zur Herstellung von pharmazeutischen Zusammensetzungen mit bakteriostatischer Wirkung.

9. Verwendung nach Anspruch 8 zur Herstellung von pharmazeutischen Zusammensetzungen, die in Form von Injektionsampullen konditioniert sind und 0,25 bis 4 g des Derivats enthalten.

**Claims** for the contracting states: BE, CH, DE, FR, GB, IT, LI, LU, NL, SE.

1. Derivatives of the family of cephalosporins of the formula:

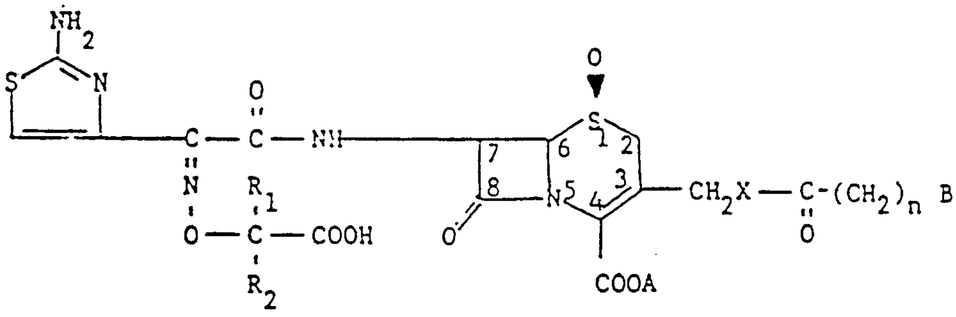

(I)

in which:

the COOA group at the 4 position is an acidradical, or an alkali or alkaline-earth salt or an amino acid or amine salt, for example triethylamine or ethanolamines, or an ester radical easily hydrolyzable or metabolically labile and pharmaceutically acceptable;

. X denotes an oxygen atom or a sulfur atom;

. n is zero or 1;

. $R_1$ and $R_2$ each denote independently hydrogen or a methyl group or,

. $R_1$ and $R_2$ taken together with the carbon atom to which they are linked form a cyclobutyl or cyclopentyl nucleus;

. B is the residue of a primary or secondary amine selected among the following groups:

- Z-NH-R where Z is a straight or branched chain alkylene group having from 2 to 7 carbon atoms, optionally interrupted by a sulfur atom and optionally substituted by a hydroxyl, thiol, methylthio, amino, acetamido, carbamoyl, phenyl, hydroxyphenyl or imidazoyl group;

Z can also be a cyclopentylidene or cyclohexylidene group;

R represents a hydrogen atom or an alkyl group having from 1 to 3 carbon atoms;

- Z'-Alk-NH-R where Z' represents a 1,2-phenylene or 1,3-phenylene or 1,4-phenylene group optionally substituted by a halogen atom or by 1 or 2 methoxy groups or by 1, 2 or 3 methyl group or Z' represents a 1,2 -cyclohexylene, 1,3-cyclohexylene or 1,4-cyclohexylene group;

Alk represents a straight or branched alkylene group having from 1 to 3 carbon atoms;

R is as defined above;

- Z'-N(R')-CO-Y-NH-R" where Z' is as defined above,

Y denotes an alkylene $(CH_2)_m$ group in which m = 0, 1, 2, 3 or 4, a branched alkylene group having 2 or 3 carbon atoms

or again Y with NH-R" constitutes a ring

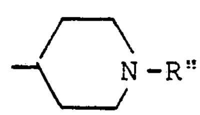

R' and R", identical or different, have the same meaning as that given for R above,

- Z"-NH-R where Z" is a 1,3-cyclohexylene or 1,4-cyclohexylene group and R is as previously defined,

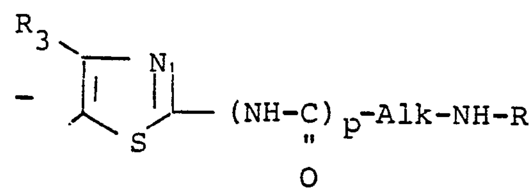

where $R_3$ represents a hydrogen atom or a methyl group,

p = 0 or 1 and Alk and R are as previously defined.

- a 2-piperidyl, 3-piperidyl or 4-piperidyl group optionally substituted on the nitrogen atom by a $-CO-Alk-NH_2$ or a

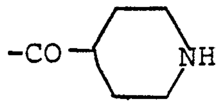

group where Alk is as previously defined, and the salts of said compounds with pharmaceutically acceptable acids.

2. Derivatives according to claim 1, characterized in that they are in one of the syn and anti forms.

3. Trifluoroacetate of acid of formula:

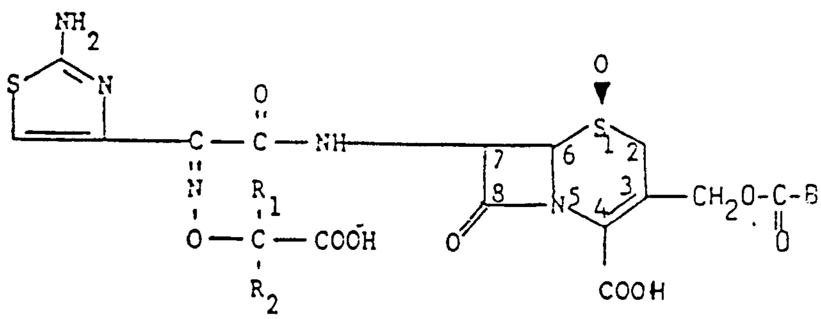

(I)

in which:

- when $R_1$ et $R_2$ each represent a methyl group, B is the residue of amine of formula:

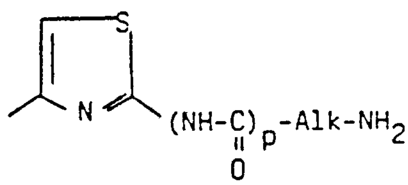

in which:

$$(NH-\underset{\underset{O}{\|}}{C})_p-Alk-NH_2$$

group is selected among the

$$-NH-\underset{\underset{O}{\|}}{C}-CH_2-NH_2\ ;\ -NH-\underset{\underset{O}{\|}}{C}-CH_2-CH_2-NH_2\ ;\ -NH-\underset{\underset{O}{\|}}{C}-\underset{\underset{CH_3}{|}}{CH}-NH_2;\ -CH_2-CH_2-NH_2;\ -CH_2NH_2$$

groups;

or B is the residue of an amine of formula:

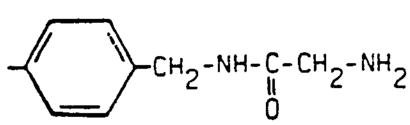

or

- when $R_1$ and $R_2$ represent with the carbon atom to which they are linked a cyclobutyl group, B is the

residue of an amine of formula:

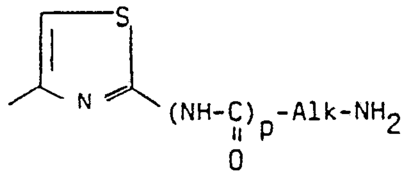

in which the

$$(NH-\underset{\underset{O}{\parallel}}{C})_p-Alk-NH_2$$

group is selected among the

$$-NH-\underset{\underset{O}{\parallel}}{C}-CH_2-NH_2 \text{ and } CH_2-NH_2 \text{ groups ;}$$

groups;
syn isomer.

4. Trifluoroacetate of acid of formula:

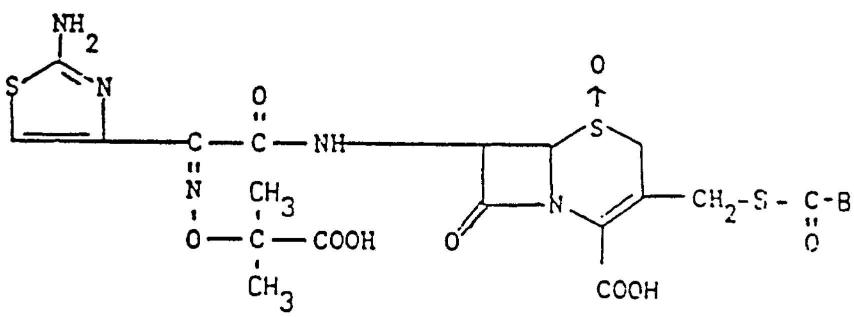

in which B is the residue of an amine of formula:

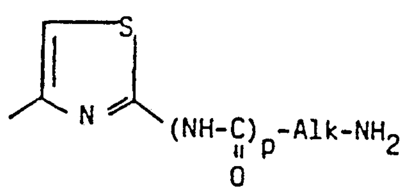

in which the

$$(NH-\underset{\underset{O}{\parallel}}{C})_p-Alk-NH_2$$

group is selected among the

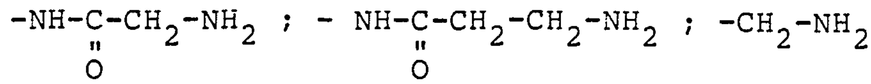

groups or B is the residue of an amine of formula:

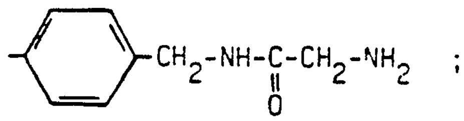

syn isomer.

5. Process for the production of derivatives of the family of cephalosporins according to one of claims 1 or 2, characterized in that it consists in:

1) acylating 4-tertiobutyl 1-S-oxide 7-amino 3-bromomethyl 3-cepheme carboxylate of formula II:

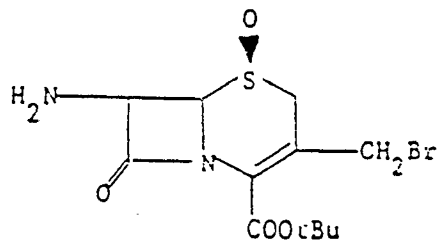

(II)

by the acid of formula III:

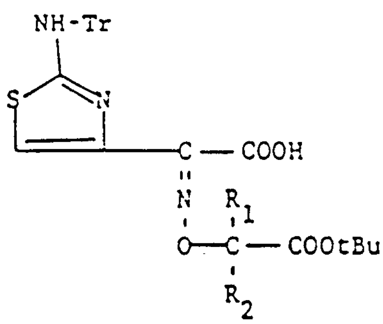

(III)

in which Tr is the trityl group, tBU the tertiobutyl group, $R_1$ and $R_2$ are as defined in clam 1, to obtain the compound of formula IV:

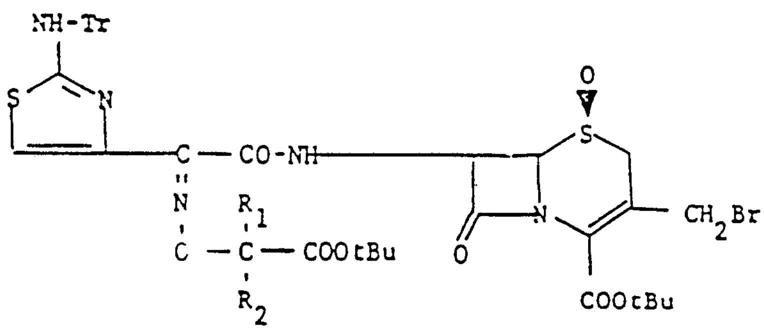

(IV)

2) adding to said compound of formula IV an acid B-$(CH_2)_n$-COOH or a thioacid B-$(CH_2)_n$COSH, in which n is zero or 1 and B is the residue of a primary or secondary amine as defined in claim 1, the amine function of said acid having been previously protected, to form the compound of formula V:

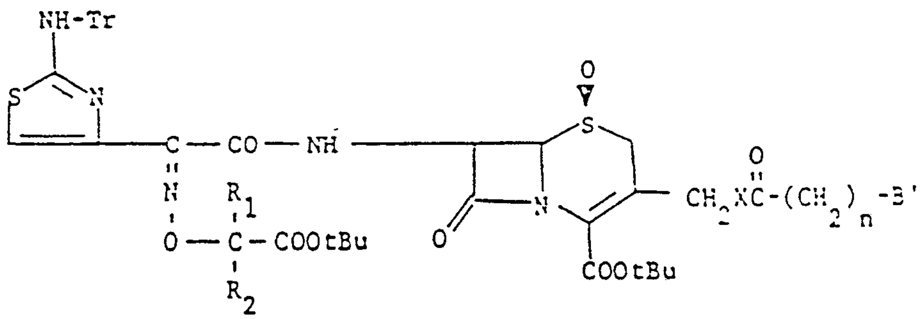

(V)

in which Tr, $R_1$, $R_2$, n are as defined above and B' represents the group B in which the amine function is protected,

3) removing the protective groups on the amine and carboxy functions to obtain the compound of formula I in which A is hydrogen,

4) optionally converting said compound obtained into a compound of formula I in which A is other than H by acting on said compound with an inorganic or organic base of by esterification,

5) optionally converting said compound of formula I into one of its salts by reaction with a pharmaceutically acceptable acid.

6. Process according to claim 5, characterized in that the addition of the acid B-$(CH_2)_n$ -COOH is effected by using the sodium or potassium salt of said acid, said addition being carried out in an aprotic apolar solvent.

7. Process according to claim 5, characterized in that the addition of the thioacid B'-$(CH_2)_n$-COSH is effected by using the sodium or potassium salt of said acid, said addition being carried out in an apolar solvent.

8. Process for the production of the compounds according to claim 3, characterized in that it consists in adding, in the dimethylformamide, to the compound of formula:

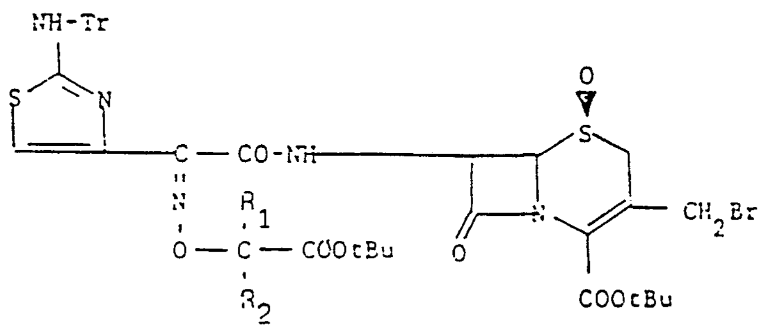

(IV)

syn isomer,

in which Tr is the trityl group, tBu the tertiobutyl group, $R_1$ and $R_2$ are as defined in claim 3, the potassium salt of the acid of formula B'-COOH, in which B' is the residue of an amine (B) as defined in claim 3 and of which the amine function has been protected by the tertiobutoxycarbonyl group; and dissolving the resulting compound in the trifluoroacetic acid.

9. Process for the production of the compounds according to claim 4, characterized in that it consists in adding to the compound of formula:

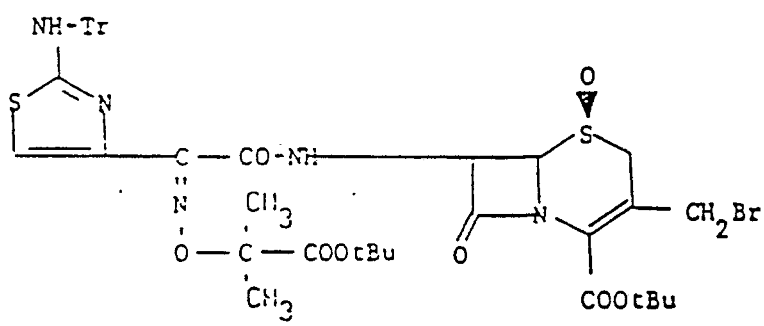

(IV)

syn isomer, in which Tr is the trityl group and tBu the tertiobutyl group,

- in the tetrahydrofuran, the potassium salt of the thioacid of formula B'-COSH, or

- in the anhydrous acetone and in the presence of potassium hydrogenocarbonate and sodium iodide, the thioacid of formula B'-COSH,

B' being the residue of an amine (B), as defined in claim 4 and of which the amine function has been protected by the tertiobutoxycarbonyl group; and dissolving the resulting compound in the trifluoroacetic acid.

10. Pharmaceutical composition, characterized in that it contains, as active ingredient, a derivative according to any one of claims 1 to 4 in combination with a pharmaceutically acceptable vehicle.

11. Pharmaceutical composition with bacteriostatic action, characterized in that it contains, as active ingredient, a derivative according to any one of claims 1 to 4, in combination with a pharmaceutically acceptable vehicle.

12. Pharmaceutical composition according to claim 11, characterized in that it is packaged in the form of injectable ampoules and contains 0.25 to 4 g of the derivative according to any one of claims 1 to 4.

**Claims** for the contracting state: AT

1. Process for the preparation of derivatives of the family of cephalosporins of the formula:

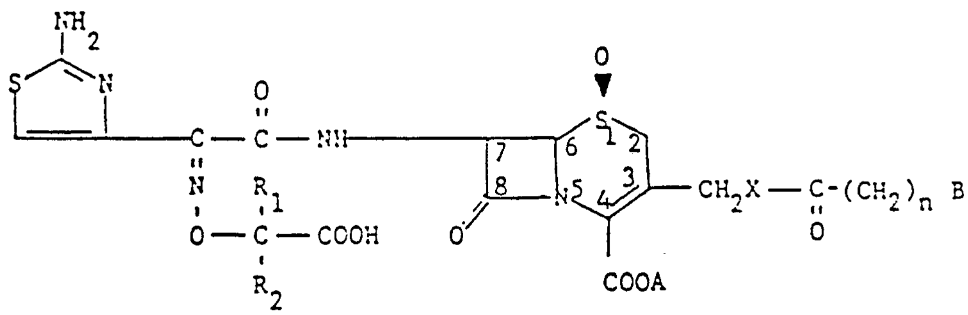 (I)

in which:

The COOA group at the 4 position is an acid radical, or an alkali or alkaline-earth salt or an amino acid or amine salt, for example triethylamine or ethanolamines, or an ester radical easily hydrolyzable or metabolically labile and pharmaceutically acceptable;

. X denotes an oxygen atom or a sulfur atom;

. n is zero or 1;

. $R_1$ and $R_2$ each denote independently hydrogen or a methyl group or,

. $R_1$ and $R_2$ taken together with the carbon atom to which they are linked form a cyclobutyl or cyclopentyl nucleus;

. B is the residue of a primary or secondary amine selected among the following groups:

- Z-NH-R where Z is a straight or branched chain alkylene group having from 2 to 7 carbon atoms, optionally interrupted by a sulfur atom and optionally substituted by a hydroxyl, thiol, methylthio, amino, acetamido, carbamoyl, phenyl, hydroxyphenyl or imidazoyl group,

Z can also be a cyclopentylidene or cyclohexylidene group,

R represents a hydrogen atom or an alkyl group having from 1 to 3 carbon atoms;

- Z'-Alk-NH-R where Z' represents a 1,2-phenylene or 1,3-phenylene or 1,4-phenylene group optionally substituted by a halogen atom or by 1 or 2 methoxy groups or by 1, 2 or 3 methyl group or Z' represents a 1,2-cyclohexylene, 1,3-cyclohexylene or 1,4-cyclohexylene group,

Alk represents a straight or branched alkylene group having from 1 to 3 carbon atoms;

R is as defined above,

- Z'-N-CO-Y-NH-R" where Z' is as defined above,

     |
     R'

Y denotes an alkylene $(CH_2)_m$ group in which m = 0, 1, 2, 3 or 4, a branched alkylene group having 2 or 3 carbon atoms

or again Y with NH-R" constitutes a ring

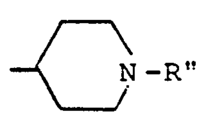

R' and R", identical or different, have the same meaning as that given for R above;

- Z"-NH-R where Z" is a 1,3-cyclohexylene or 1,4-cyclohexylene group and R is as previously defined.

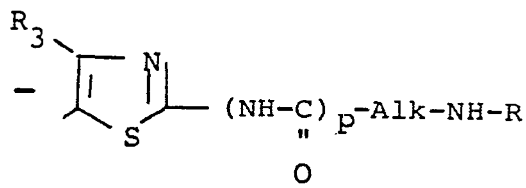

where $R_3$ represents a hydrogen atom or a methyl group,

p = 0 or 1 and Alk and R are as previously defined;

- a 2-piperidyl, 3-piperidyl or 4-piperidyl group optionally substituted on the nitrogen atom by a -CO-Alk-$NH_2$ or a

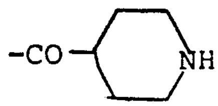

group where Alk is as previously defined, and the salts of said compounds with pharmaceutically acceptable acids, characterized in that it consists:

1) in acylating 4-tertiobutyl 1-S-oxide 7-amino 3-bromomethyl 3-cepheme carboxylate of formula II:

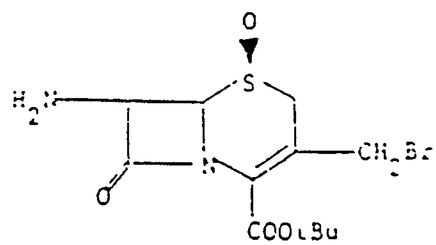

(II)

by the acid of formula III:

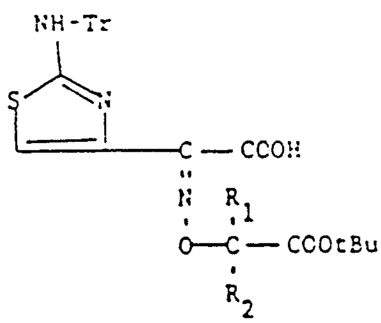

(III)

in which Tr is the trityl group, tBu the tertiobutyl group, $R_1$ and $R_2$ are as defined herein above, to obtain the compound of formula IV:

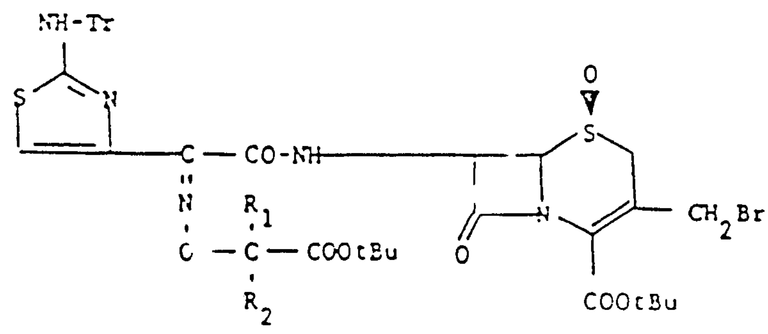

(IV)

2) in adding to said compound of formula IV an acid B-$(CH_2)_n$-COOH or a thioacid B-$(CH_2)_n$COSH, in which n is zero or 1 and B is the residue of a primary or secondary amine as defined herein above, the amine function of said acid having been, previously protected, to form the compound of formula V:

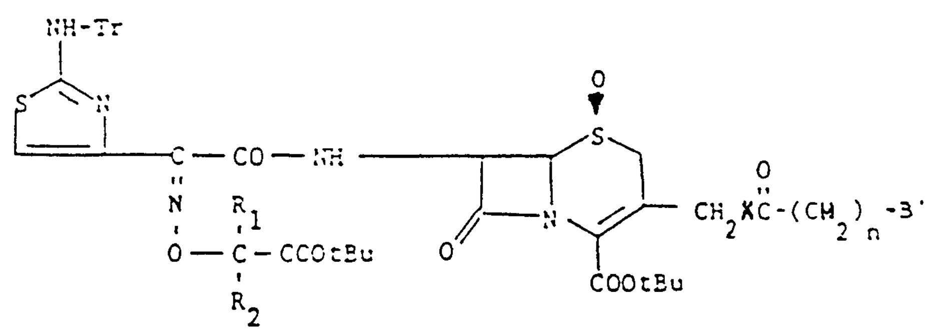

(V)

in which Tr, $R_1$, $R_2$, n are as defined above and B' represents the group B in which the amine function is protected,

3) in removing the protective groups on the amine and carboxy functions to obtain the compound of formula I in which A is hydrogen,

4) in optionally converting said compound obtained into a compound of formula I in which A is other than H by acting on said compound with an inorganic or organic base of by esterification,

5) in optionally converting said compound of formula I into one of its salts by reaction with a pharmaceutically acceptable acid.

2. Process according to claim 1, characterized in that the acid of formula III is used in the syn or anti form.

3. Process according to one of claims 1 or 2, characterized in that the addition of the acid B-$(CH_2)_n$-COOH is effected by using the sodium or potassium salt of said acid, said addition being carried out in an aprotic apolar solvent.

4. Process according to one of claims 1 or 2, characterized in that the addition of the thioacid B'-$(CH_2)_n$-COSH is effected by using the sodium or potassium salt of said acid, said addition being carried out in an apolar solvent.

5. Process for the production of trifluoroacetate of acid of formula:

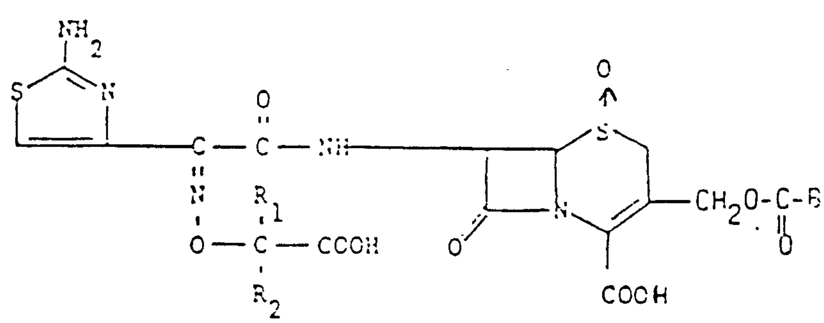

in which:

- when $R_1$ et $R_2$ each represent a methyl group, B is the residue of amine of formula:

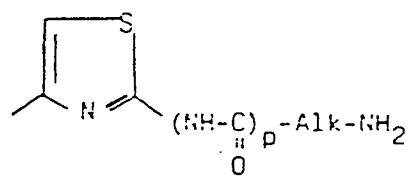

in which:

$$(NH-\underset{\underset{O}{\|}}{C})_p-Alk-NH_2$$

group is selected among the

$$-NH-\underset{\underset{O}{\|}}{C}-CH_2-NH_2\ ;\ -NH-\underset{\underset{O}{\|}}{C}-CH_2-CH_2-NH_2\ ;\ -NH-\underset{\underset{O}{\|}}{C}-\underset{\underset{CH_3}{|}}{CH}-NH_2;\ -CH_2-$$

$$CH_2-NH_2;\ -CH_2NH_2$$

groups;

or B is the residue of an amine of formula:

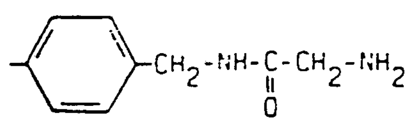

or

- when $R_1$ and $R_2$ represent with the carbon atom to which they are linked a cyclobutyl group, B is the residue of an amine of formula:

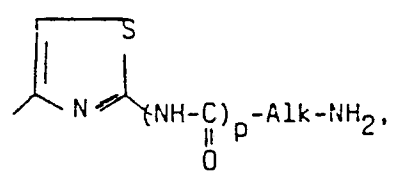

in which the

$$(NH-\underset{\underset{O}{\|}}{C})_p-Alk-NH_2$$

group is selected among the

$$-NH-\underset{\underset{O}{\|}}{C}-CH_2-NH_2 \text{ and } CH_2-NH \text{ groups ;}$$

and $CH_2$-NH groups;

syn isomer, characterized in that it consists in adding, in the dimethylformamide, to the compound of formula:

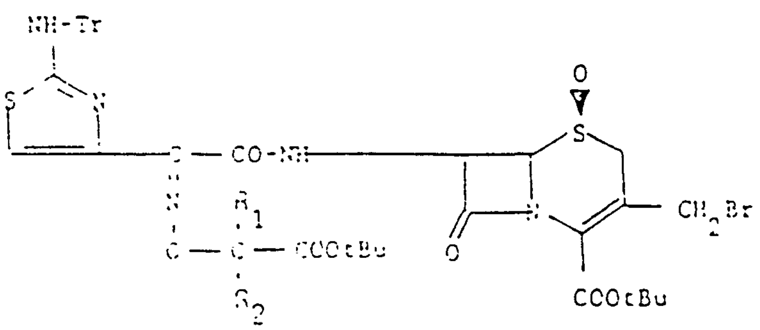

(IV)

syn isomer,

in which Tr is the trityl group, tBu the tertiobutyl group, $R_1$ and $R_2$ are as defined above, the potassium salt of the acid of formula B'-COOH, in which B' is the residue of an amine (B) as defined above, and of which the amine function has been protected by the tertiobutoxycarbonyl group; and in dissolving the resulting compound in the trifluoroacetic acid.

6. Process for the production of trifluoroacetate of acid of formula:

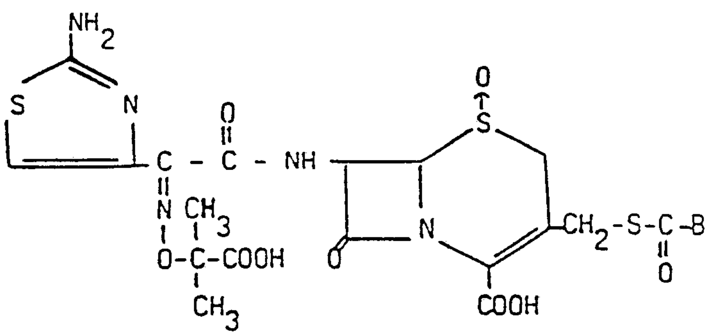

in which B is the residue of an amine of formula:

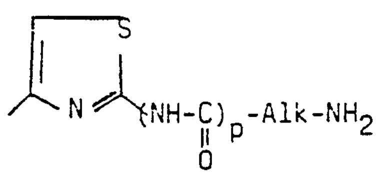

in which the

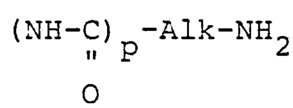

group is selected among

the 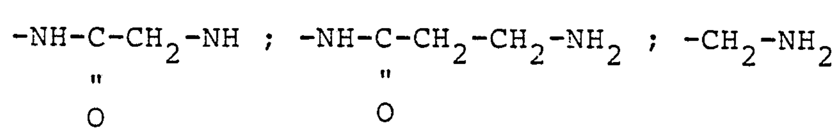

groups or B is the residue of an amine of formula:

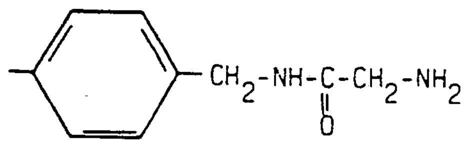

syn isomer,

characterized in that it consists in adding to the compound of formula:

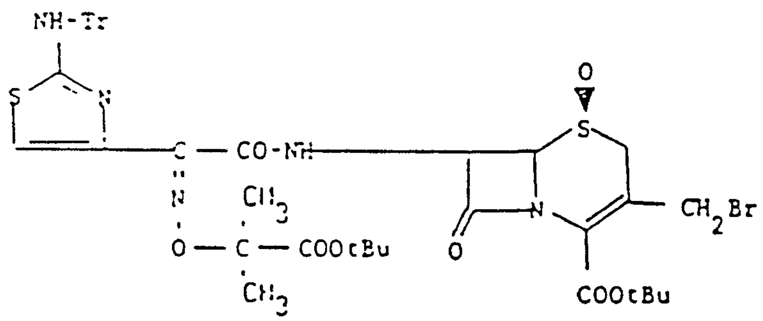

(IV)

syn isomer, in which Tr is the trityl group, tBu the tertiobutyl group,

- in the tetrahydrofuran, the potassium salt of the thioacid of formula B'-COSH, or
- in the anhydrous acetone and in the presence of potassium hydrogenocarbonate and sodium iodide, the thioacid of formula B'-COSH,

B' being the residue of an amine (B), as defined above and of which the amine function has been protected by the tertiobutoxycarbonyl group; and in dissolving the resulting compound in the trifluoroacetic acid.

7. Use of the derivatives of the family of cephalosporins obtained according to any one of claims 1 to 6, in combination with a pharmaceutically acceptable vehicle for the preparation of pharmaceutical compositions.

8. Use of the derivatives of the family of cephalosporins obtained according to any one of claims 1 to 6, in combination with a pharmaceutically acceptable vehicle for the preparation of pharmaceutical compositions with bacteriostatic action.

9. Use according to claim 8, for the preparation of pharmaceutical compositions, packaged in the form of injectable ampoules containing 0.25 to 4 g of the said derivative.